# EUROPEAN PATENT APPLICATION

(11) **EP 1 270 044 A2**
(43) Date of publication of application: **02.01.2003**
(21) Application number: 02254207.0
(22) Date of filing: 17.06.2002
(51) Int. Cl.: A61P 17/02, A61K 38/17, G01N 33/50, G01N 33/68

(54) **Wound healing biomarkers**

(30) Priority: 18.06.2001 GB 0114869; 13.07.2001 US 305346 P
(71) Applicant: Pfizer Limited, Sandwich, Kent CT13 9NJ (GB); PFIZER INC., New York, N.Y. 10017 (US)
(72) Inventor: Burslem, Martyn Frank, Sandwich, Kent CT13 9NJ (GB); Cooper, Lisa, Sandwich, Kent CT13 9NJ (GB); Johnson, Claire Michelle, Sandwich, Kent CT13 9NJ (GB); Martin, Paul, Sandwich, Kent CT13 9NJ (GB)
(74) Representative: Hayles, James Richard

(57) **Abstract**

This invention provides biomarkers such as genes and the corresponding mRNA transcripts or protein products that are identified as being involved in wound healing processes. Also provided are methods for identification of compounds useful for the treatment of wounds, wound healing disorders or inflammation and compounds identified by such methods. Methods are provided for monitoring the progress of wound healing and for identification of individuals with wound healing disorders.

## Description

This invention relates to the fields of molecular biology and wound healing. More particularly this invention relates to methods and probes for investigating and evaluating the presence of RNA species that are differentially expressed in wound and normal tissue. The invention also relates to use of specific genes and their translation products to monitor wound healing and/or to detect disorders or diseases characterised by impaired or excessive wound healing. The invention also relates to methods for the identification of compounds useful for the treatment of wounds, inflammation and wound healing disorders, compounds identified by such screening methods, the use of such compounds in the manufacture of medicaments or in methods of medical treatment.

In recent years much has been learned about the cell biology of wound repair, but the current understanding of the regulation and coordination of the various processes that underlie wound repair is still poor. Effective and wide-ranging therapeutic intervention will largely depend on the identification of the full repertoire of signals controlling each step in the tissue repair process and a detailed understanding of the mechanisms involved in effective tissue repair.

Most skin lesions heal rapidly and efficiently within a week or two. However, the result of this process is not perfect; often there remains a connective tissue scar where the collagen matrix has been poorly reconstituted, epidermal appendages that have been lost at the site of damage do not regenerate. In some conditions, wounds become chronic, i.e. do not heal at all.

Most wounds will cause leakage of blood from damaged blood vessels, the formation of a clot serves as a temporary shield and provides a provisional matrix over and through which cells can migrate during the repair process. The clot also serves as a reservoir of cytokines and growth factors, released as the activated platelets degranulate, which provide chemotactic signals for circulating inflammatory cells to migrate to the wound site, initiate the tissue movements of reepithelialisation and connective tissue contraction and stimulate the angiogenic response.

The inflammatory component of the wound healing response was long considered necessary, because it would help eliminate the damaged tissue and any contaminants that may have entered the wound environment and would allow new skin to form.

A genetically modified mouse model is available that lacks macrophages and B-cells (McKercher, S.R. et al (1996) EMBO J. 15, 5647-5658). The mouse model was generated by a knockout mutation in the gene for transcription factor PU.1, a member of the Ets family of transcription factors, the expression of which appears to be strictly limited to cells of the haematopoietic lineage (Lloberas, J. et al (1999) Immunol. Today 20, 184-189).

When the present inventors investigated the wound healing ability of the PU.1 knockout (KO) mouse, it was found that PU.1 mice healed wounds perfectly, with little or no scarring. This provided strong evidence that, contrary to established belief, the inflammatory response is not required for wound healing and indeed may be a factor in scarring and/or fibrosis.

The PU. 1 knockout mouse was used to investigate genes involved in wound healing, and, when results from the knockout mouse are compared with the wildtype mouse, to dissect out the inflammatory components in wound healing. Analysis was carried out to identify genes that are upregulated or downregulated during a wound healing response, occurring with or without the inflammatory response usually occurring in healing wounds. One method for this type of analysis is microarray technology. With DNA microarray technology, it becomes possible to monitor large-scale gene expression over time. Prefabricated arrays of large numbers of especially designed oligonucleotide probes, e.g. as manufactured by Affymetrix (CA), enable simultaneous hybridisation-based analysis of the expression of thousands of genes. Using this method, four stages during repair of a neonatal skin wound in wild type and PU1 knockout mice were investigated: 30 minutes, 3 hours, 12 hours and 24 hours. This allowed the identification of the immediate early wound response genes, the early and late effector genes and genes required to complete reepithelialisation. It also allowed identification of genes involved in the inflammatory response.

The present invention is based on the analysis of genes, the expression of which is upregulated or downregulated during a wound healing response, occurring with or without the inflammatory response usually occurring in healing wounds.

The present invention provides novel targets (genes, or the corresponding RNA or protein products), for therapeutic intervention in diseases or disorders involving impaired wound healing, as well as such targets for therapeutic intervention in diseases or disorders characterised by an excessive wound healing response. The invention also provides the use of antagonists or inhibitors of these novel targets for the treatment of wounds or of disorders characterised by excessive wound healing; the invention also provides the use of agonists or activators of these targets for the treatment of wounds or disorders
characterised by impaired wound healing. It will be appreciated that such antagonists or inhibitors can be compounds that reduce the activity of the target, antibodies specific for the target, as well as antisense oligonucleotides or ribozymes or transcription inhibitors, that have the effect of reducing the amount of the target produced. It will also be appreciated that agonists or activators of these targets can comprise compounds that increase the activity of the target itself, as well as compounds that increase the expression of the target. Increased activity of the target could also be achieved by delivering the target itself, either as protein or its coding region in an appropriate vector as gene therapy.

The present invention also provides markers which are useful in monitoring, for example, the state of healing of a wound; these markers can be used more generally for monitoring diseases or disorders characterised by impaired or by excessive wound healing. In addition, the invention provides markers for wound inflammation. These markers are useful in the clinical assessment of progress of healing, and can be used to aid in the selection of the appropriate therapeutic intervention.

### Definitions

Coding sequence or coding region refers to a nucleic acid molecule having sequence information necessary to produce a gene product when the sequence is expressed.

Antisense oligonucleotide refers to a small nucleic acid molecule, typically between about 10 to 50 nucleotides long, which may be unmodified RNA or DNA or modified RNA or DNA; it is designed to hybridise to the respective transcript, and thereby reduce its translation into protein, e.g. by blocking translation or by facilitating rapid degradation of the respective transcript.

Ribozymes are RNA molecules capable of catalysing RNA cleavage in a sequence specific manner.

Antibody as used herein includes polyclonal and monoclonal antibodies, single chain, chimeric and humanised antibodies, as well as antibody fragments, whether produced by recombinant or proteolytic means. The term is also meant to include the products of any antibody-derived expression libraries, e.g. single-chain Fv or Fab fragment expression libraries.

Marker/biomarker refers to any molecule derived from the gene, e.g., a transcript of the gene, a sense (coding) or antisense (non-coding) probe sequence derived from the gene, or a full length or partial length translation product of the gene or an antibody thereto, which can be used to monitor a condition, disorder, disease, or the status in the progression of a process, e.g. a healing process or the progression in a disease. Biomarkers may be labelled to assist detection, the choice of label will be directed by the nature of the biomarker, suitable labels include radionuclides, enzymes, fluorescent, chemiluminescent, or chromogenic agents as well as substrates, cofactors, inhibitors, magnetic particles.

The gene sequences identified by accession numbers in Table 1 and provided in the sequence listing are mouse (Mus muscularis) sequences. Mammalian homologues of these mouse genes, particularly human or rat homologues are included in the scope of this invention and, given the mouse sequence information, the corresponding gene in human, rat or other mammals can be readily identified and isolated using techniques known in the art.

### Examples

The invention will now be illustrated by a number of examples. While they are typical of the methods that might be used, other procedures known to those skilled in the art may also be used. All Tables referred to are found at the end of this section. References are made to the following figures:

### Figure 1: Profiles of clusters 1 - 20

### Figure 2: Profiles of clusters using specific interesting genes as active profiles

### Example 1: Wounding

Ten horizontal and eight vertical incisional wounds were made to a marked area of 0.5 cm x 1 cm on a day 2-neonatal mouse back skin. The pups were left with their mothers for the wounds to heal. After 30 minutes, 3 hours, 12 hours and 24 hours, the skin was removed from the back and immediately immersed in liquid nitrogen. A control of uninjured skin was taken at 12 hours.

The mice used for this experiment were either wildtype or PU.1 knockout (KO) mice, the latter are unable to produce inflammatory cells and therefore cannot mount an inflammatory response.

### Example 2: RNA extraction

The tissue was homogenised using a glass teflon homogeniser. The RNA was extracted using RNAzol (Biogenesis), following the manufacturer's protocol. The RNA was cleaned up using RNeasy minicolumns from Qiagen, following the manufacturer's protocol.

### Example 3: Affymetrix gene expression analysis

Double stranded cDNA was generated from 10µg total RNA (produced according to example 2) using Superscript Choice kit (Life Technologies) with a T7-polyT primer. Approximately 1µg cDNA was used to generate biotinylated cRNA by *in vitro* transcription using Bioarray High Yield RNA Transcript Labeling Kit (Enzo). 10µg fragmented cRNA was hybridised in 100mM MES, 1M Na⁺, 20mM EDTA, 0.01% Tween 20, 0.1mg/ml herring sperm DNA, 0.5mg/ml acetylated BSA plus 50pM control oligonucleotide and eukaryotic hybridisation controls to MGU74A arrays (Affymetrix) at 45°C for 16 hours. Arrays were washed using Affymetrix protocols in non-stringent buffer (6X SSPE, 0.01% Tween 20, 0.005% antifoam) at 25°C and stringent wash buffer (100mM MES, 0.1 M Na⁺, 0.01% Tween) at 50°C and stained with streptavidin phycoerythrin (10µg/ml) including an antibody amplification step. Arrays were scanned using a laser confocal scanner to generate fluorescence intensities. The data were analysed using Microarray Analysis Suite version 4.0 (Affymetrix). The data were scaled to a target intensity of 300.

### Example 4: Data analysis

The results were firstly analysed to remove bad probe sets and to remove those sequences that remained absent during the course of the experiment. This reduced the set to just under 5000 genes. The data for this set were then analysed using Spotfire Array Explorer 3.0 software (see e.g. US patent no 6,014,661).

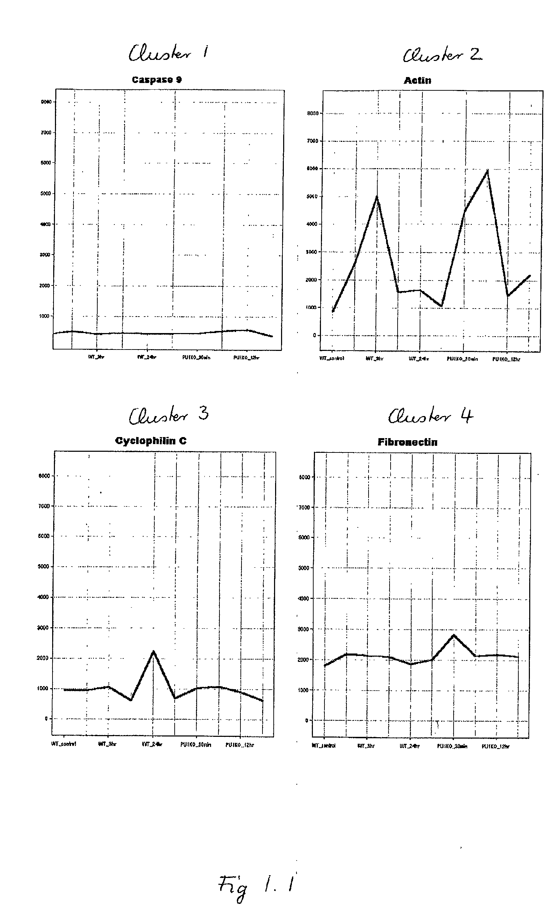

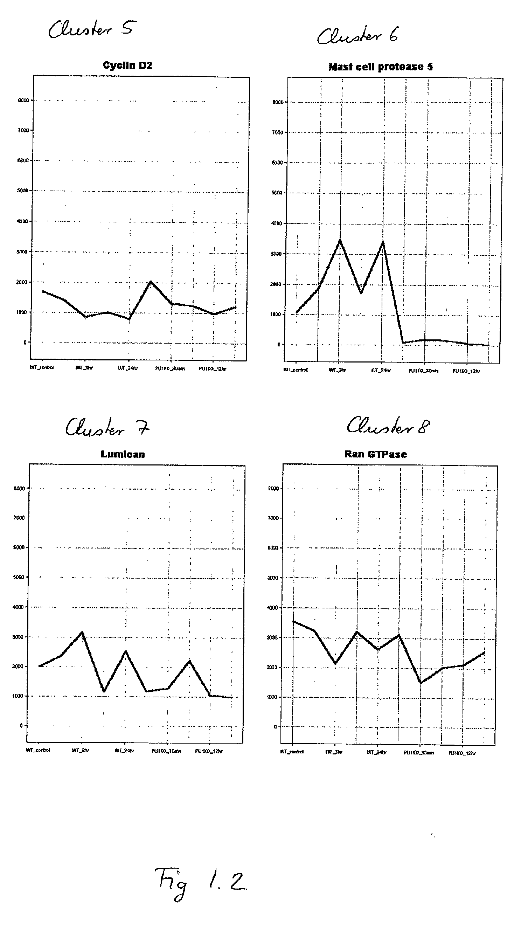

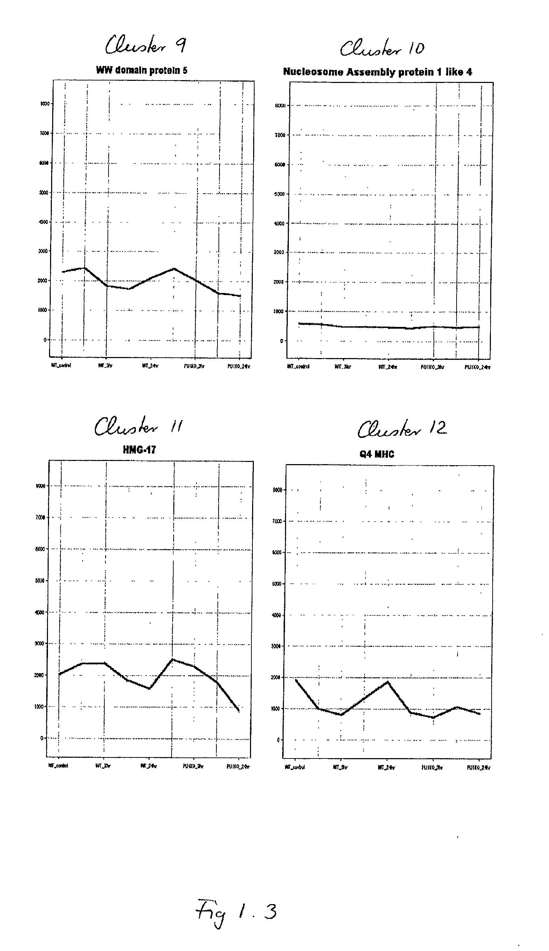

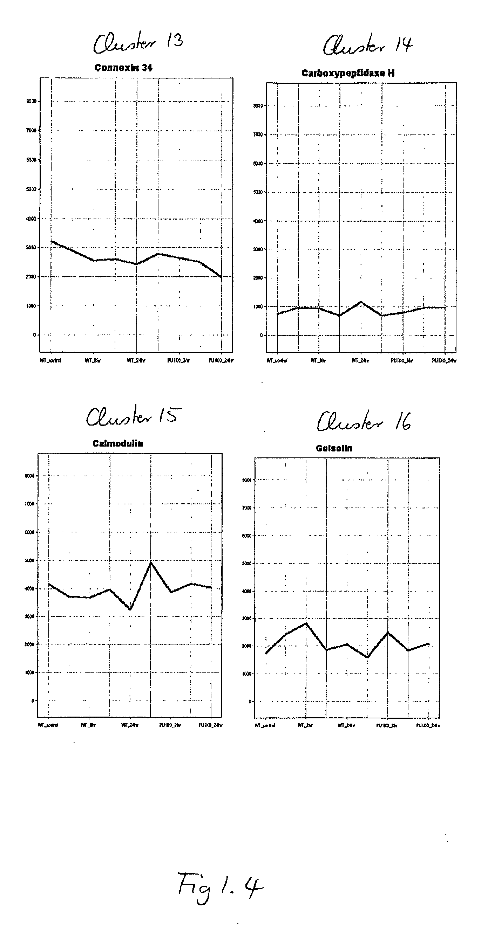

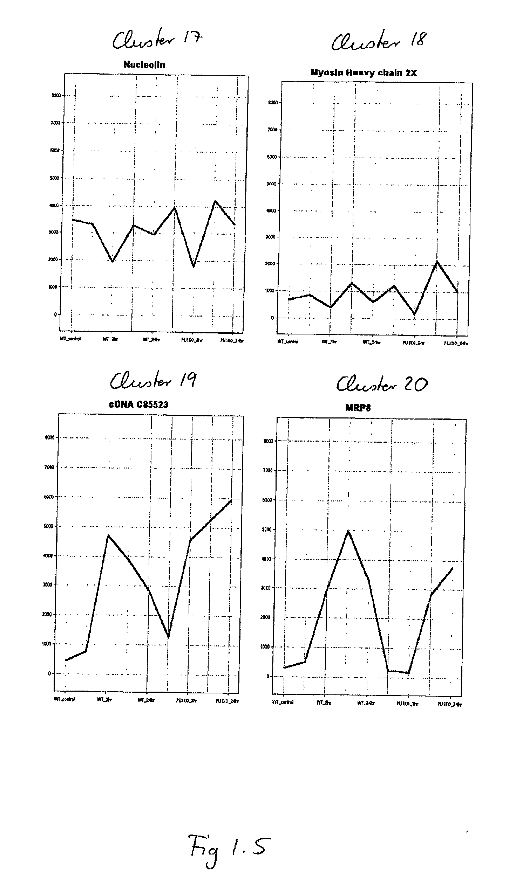

A non-hierarchical clustering method called K-means clustering was used for the analysis. This is an iterative process with each profile assigned to the closest centroid (centroids are the centre point of the profile cluster). New centroids are calculated for the resulting cluster and the profiles are reassigned to the closest centroid. These steps are repeated until a steady state has been reached. In this instance, the method used to generate the centroid was evenly spaced profiles, i.e. profiles in this case are evenly distributed between the maximum and minimum value for each variable in the profile set. The number of clusters was set to 20, assigning the number of clusters expected should be based on an assessment of the distribution of the data. Similarity to the active profile was calculated using Euclidean distances. It is important to ensure that the cluster number chosen does not produce empty clusters or clusters which contain only one profile. The clusters produced by this method resulted in 20 clusters, with a reasonable number of profiles in each (lowest number of profiles: cluster 10 with 141 profiles, highest number: cluster 3 with 337 profiles). The whole set, ordered by cluster, is shown in Table 1 (found at the end of the example section), the summary profile for each cluster is shown in Figure 1. The clusters are presented as the time course of expression for the respective group of genes in the order wild type control (taken at 12 hours), wild type 30 minutes after wounding, wild type 3 hours after wounding, wild type 12 hours after wounding, wild type 24 hours after wounding, followed by the same time points for the PU. 1 KO mice along the X-axis; the Y-axis indicates level of expression (as measured by fluorescence intensity).

These twenty clusters can be further classified into five types:
1. Essential wound healing genes show increased expression during wound healing in both wild type and PU.1 KO mouse wounds. These include the genes found in Cluster 2, Cluster 7, Cluster 11, Cluster 16 and Cluster 20.
2. Genes associated with the inflammatory response, these are found to be upregulated in the wild type mouse, but not in the PU.1 knock out mouse and therefore are not required for wound healing, the relevant genes are found in clusters 5 and 6.
3. Obligatory down-regulated genes. Genes for which expression is downregulated during wound healing in both wild type and PU.1 KO mouse wounds. These include the genes found in Clusters 4, Cluster 13 and Cluster 17.
4. Wound healing genes that are suppressed by inflammation. Genes in this group show increased expression in both genotypes but the level of gene expression is reduced in wildtype in comparison with the PU.1 KO mice. These genes are found in and Cluster 19 and Cluster 3.
5. Genes that are co-expressed with TGF-β and are therefore implicated in scarring and fibrosis.

### 1. Essential wound healing genes

These are found in Cluster 2, Cluster 7, Cluster 11, Cluster 16 and Cluster 20.

### Cluster 2: 310 profiles

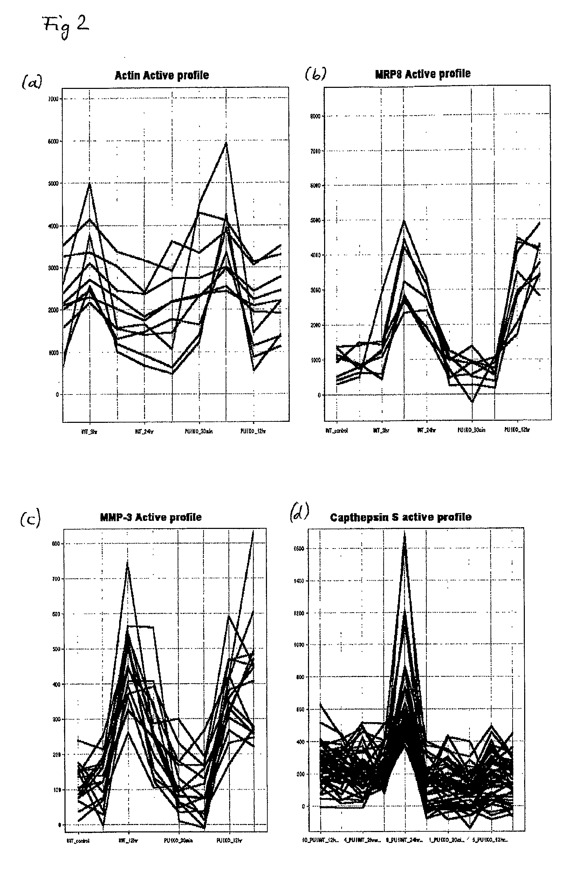

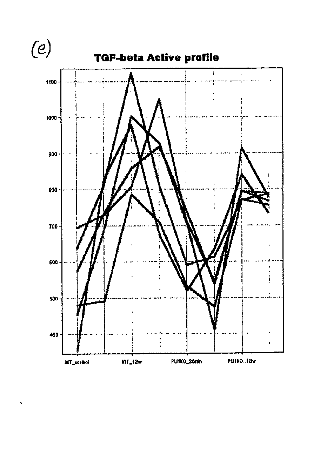

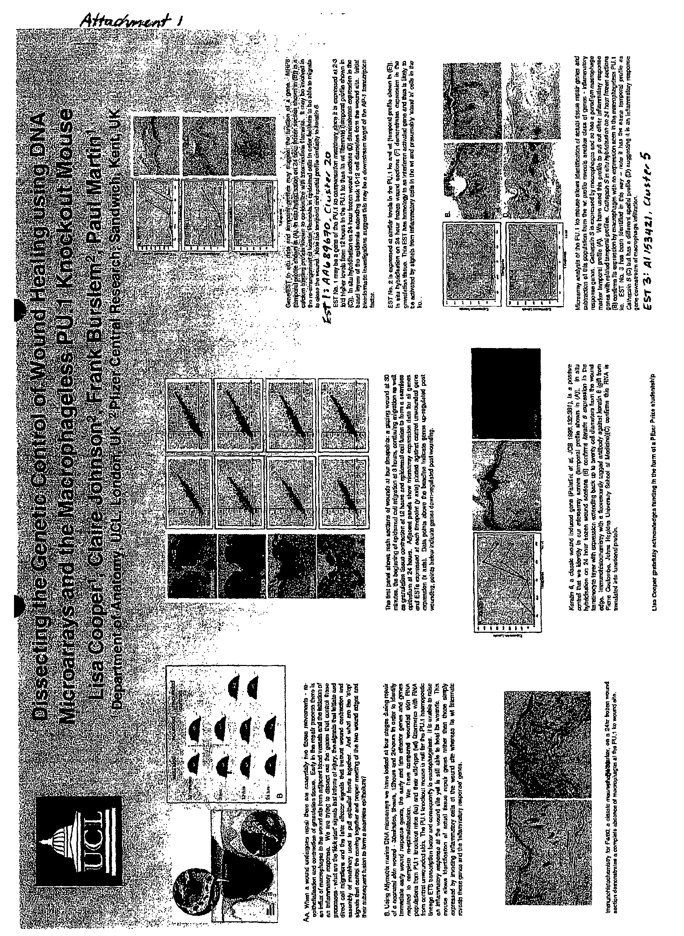

The profile of this cluster identifies genes whose expression peaks 3hrs after wounding in both the wild type and the PU.1 KO mice. Examples of prototypical wound healing genes in cluster 2 are transcription factor JunB (immediate early gene, involved in transcriptional regulation) and actin which is required for cell migration. All genes in this cluster are likely to be essential wound healing genes, and/or marker genes indicative of wound repair. Specifically, the nine genes that show a profile most similar to actin, identified using actin as the active profile, are likely to be important (Fig. 2a, Table 2). One or more of the proteins encoded by nucleotide sequences comprising the sequences of the accession numbers grouped in clusters 2, 7, 11, 16, and 20 as indicated in Table 1 can be used as a marker, or markers indicative of wound repair. An inhibitor or antagonist of a protein encoded by a gene comprising a sequence of one of the accession numbers grouped in clusters 2, 7, 11, 16, and 20 can be used for the treatment of a disease or disorder characterised by an excessive healing response, such as, but not limited to, scarring, fibrosis, restenosis post angioplasty, psoriasis, post-traumatic/surgical adhesions of the peritoneal cavity, joints and ligaments, benign prostatic hyperplasia, glaucoma, and peripheral nerve injury. An antisense oligonucleotide, or ribozyme against, or compounds that downregulate transcription of, any of the genes comprising the sequences of the accession numbers grouped in clusters 2, 7, 11, 16, and 20 can be used for the treatment of diseases and disorders characterised by an excessive healing response, such as, but not limited to, scarring, fibrosis, restenosis post angioplasty, psoriasis, post-traumatic/surgical adhesions of the peritoneal cavity, joints and ligaments, benign prostatic hyperplasia, glaucoma, and peripheral nerve injury.
An increase in activity of a protein encoded by a gene comprising a sequence of one of the accession numbers grouped in clusters 2, 7, 11, 16, and 20 can be achieved by, for example, agonists or stimulators of the protein, gene therapy, application of the protein itself, or substances that upregulate the transcription and/or translation of these genes, means that provide an increase in activity of the protein have application in the treatment of diseases and disorders characterised by impaired healing response such as chronic dermal ulcers, oral mucocystis, emphysema, ulcerative diseases of the gastro-intestinal (GI) tract, cystitis.

### Cluster 7: 218 profiles

The profile of this cluster identifies genes whose expression peaks early after wounding in both the WT and KO mice.
Importantly, the Connective Tissue Growth Factor Gene Family member WISP-1 is in this cluster and therefore identified as an early regulator of wound repair. WISP-1, or a viral or plasmid construct comprising the coding sequence of WISP-1 under control of a promoter active in mammalian cells is useful for the treatment of conditions or disorders involving impaired wound repair.

### Cluster 11: 266 profiles

The profile of this cluster identifies genes whose expression increases during wound repair in both the wildtype and the PU.1 KO mice. PAF acetyl hydrolase is present in this cluster and therefore identified as a marker of wound repair. As this enzyme is required to deactivate the lipid mediator PAF during inflammation, inhibitors of PAF find use as wound healing agents. PAF antagonists are therefore useful as agents for the treatment of diseases and disorders characterised by impaired healing response. Suitable PAF antagonists are disclosed in European Patent EP 0310386, especially modipafant, which is (+)-4-(2-chlorophenyl)-1,4-dihydro-3-ethoxycarbonyl-6-methyl-2-[4-(2-methylimidazo[4,5-c]pyrid-1-yl)phenyl]-5-(N-[2-pyridyl]carbamoyl)pyridine, and is disclosed in European Patent 0310386 [see Example 34(d)].

### Cluster 16: 213 profiles

The profile of this cluster also identifies genes whose expression peaks early after wounding in both the wildtype and the PU.1 KO mice. Interestingly, cyclooxygenase I is present in this cluster and is a marker of wound repair, particularly early wound repair processes. This enzyme is required to produce bioactive eicosanoids, therefore cyclooxygenase I inhibitors are useful for the treatment of diseases and disorders characterised by excessive healing response.

### Cluster 20: 265 profiles

Genes in this cluster are essential wound healing genes, expressed toward the end of the healing process. These genes are involved in cessation of the healing response, and maturation and remodelling of the repaired tissue, transcription and translation products of these genes are markers for advanced wound repair. MRP-8, a cytokine from the S100 family, previously identified as being strongly induced in normal wounds, is a member of this cluster, and can therefore serve as a marker of advanced wound repair. Eight genes were identified using MRP-8 as the active profile (see Figure 2b, Table 3). These eight genes and their transcription or translation products are useful as markers of advanced wound repair. EST AA689670 was identified as a keratinocyte marker of advanced wound repair. The presence of MMP3 in this cluster suggests that inhibitors of MMP-3 will be useful to inhibit wound remodelling. Inhibitors of the sixteen gene products that are identified using MMP3 as the active profile will also find use for inhibition of wound remodelling (Fig. 2c, Table 4).

### 2. Inflammatory genes not required for wound healing: These are genes that are found upregulated in the wildtype but not in the PU.1 KO mice. They are found in clusters 5 and 6:

### Cluster 5: 321 profiles, and Cluster 6: 213 profiles

Genes in these clusters that are not expressed in the PU.1 KO mice represent inflammatory genes not required for wound healing. As excessive inflammation is associated with impaired healing and excessive scarring, these non-essential genes represent targets for the treatment of all types of impaired healing. Antagonists or inhibitors for the products of any of the genes of these clusters are likely to be useful for improving wound healing. Also, genes in these clusters and their transcription and translation products are useful as markers for wound inflammation. Using cathepsin-S as the active profile, 47 genes were found to cluster with cathepsin-S (Fig. 2d, Table 5); these are especially useful as markers for wound inflammation, as well as for screening for inhibitor or antagonist compounds useful for the treatment of impaired healing and excessive scarring. The IMAGE clone number 1429340 is also an example of such an inflammatory marker or potential target gene. Especially inhibitors of ERK2 map kinase will be useful for the treatment of diseases and disorders characterised by impaired healing response.

### 3. Obligatory down-regulated genes.

These include the genes found in Clusters 4 (247 profiles), Cluster 13 (234 profiles) and Cluster 17 (238 profiles).
These clusters represent genes that are down regulated during healing, in both wildtype and PU.1 KO mice. This down regulation may be required for repair to occur. Therefore inhibitors of these genes will be useful to promote wound healing and activators of these genes to prevent overhealing. Apolipoprotein E is found within this cluster and thus compounds that modulate the Apolipoprotein E expression or activity will be useful as agents for the control of wound repair.

### 3. Genes required for wound healing which are suppressed by inflammation:

### Cluster 19: 187 profiles

This cluster represents essential wound healing genes that show decreased expression during the late macrophage dependent inflammatory response. As excessive inflammation is associated with impaired healing and excessive scarring, these genes, their transcription and translation products are targets for the treatment of impaired healing and excessive scarring. Focal adhesion kinase is found in this cluster and thus inhibitors of focal adhesion kinase can be used to treat treat diseases and disorders of over-repair. Also, the endogenous plasminogen activator inhibitor, PAI-1 is found to be down-regulated by inflammation, therefore inhibitors of Plasminogen activators (especially Urokinase-type plasminogen activator) can be used for the treatment of inflammation-impaired wound healing.

### Cluster 3: 337 profiles

This cluster also contains early wound healing genes that are suppressed by resident inflammatory cells such as mast cells and dendritic cells. Genes in this cluster include fibronectin, which is known to be the earliest scaffold for wound repair. Agents which overcome the resident inflammatory cell suppression of fibronectin synthesis will be useful for the treatment of impaired wound healing. More specifically, agonists of the fibroblast growth factor (FGF) receptor and agonists of the ROR alpha 1 nuclear hormone receptor can be used for the treatment of inflammation impaired wound healing.

### 5. Genes involved in scarring.

TGF-β is known to be a major factor in governing scarring and fibrosis, using TGF-β as the active profile, a cluster of six genes was identified (Fig. 2e, Table 6) each of which thus is implicated in scarring and fibrosis.

Various means to decrease the activity and/or amount of the expression products of these genes can be used for the treatment of diseases and disorders characterised by excessive healing response such as scarring, fibrosis, restenosis post angioplasty, psoriasis, post-traumatic/surgical adhesions of the peritoneal cavity, joints and ligaments, benign prostatic hyperplasia, glaucoma, and peripheral nerve injury. Such means include an inhibitor or antagonist of, or antibody to, any one of the proteins encoded by a gene comprising the sequence of one of the accession numbers clustered with TGF-β; or an antisense oligonucleotide, or ribozyme against, or a compound that downregulates transcription and or translation of, any of the genes comprising the sequence of one of the accession numbers clustered with TGF-β.

Further non-limiting examples are found in attachment 1.

### Example 5: Delivery, wounding and culture of E11.5 mouse embryos

Embryonic day 11.5 (E11.5) embryos were obtained by means of timed matings of CD1 mice, with embryos considered to be E0.5 at noon on the day of plug checking. E11.5 embryos were dissected from the uterus in Tyrode's saline and prepared for culture as described by P Martin & D Cockcroft, (1999) Culture of post-implantation mouse embryos, in Molecular embryology methods and protocols (editors Sharpe, P and Mason, I). New York; Humana Press p7 - 22. Standard wounding was performed by amputating the left hindlimb at its base using iridectomy scissors as described by McCluskey and Martin (1995) Developmental Biology, 17: 102-114. Using an electrolytically sharpened tungsten needle, incisional wounds of approximately 500µm in length were also generated on the left forelimb, as well as superficial puncture wounds on the flank. Wounded embryos were transferred to 50ml Falcon tubes containing 4ml of culture medium (3ml of Tyrode's saline plus 1ml of normal rat serum) and roller-cultured for up to 24 hours in an atmosphere of 95% oxygen/5% carbon dioxide at 30rpm and 37°C (Martin and Cockcroft (1999), as above).

### Example 6: Frozen section in situ hybridisation of wound tissue

Sections were hybridised (as described by DG Wilkinson & MA Nieto (1993) Methods Enzymol 225: 361-373) at 55°C overnight with digoxigenin labelled antisense radioprobes specific for the gene of interest. Expression was visualised by BCIP/NBT precipitation (Boehringer Mannheim) and sections were viewed on a Zeiss Axiophot microscope, after mounting in Citifluor (UKC). The labelled antisense radioprobe biomarkers derived from the gene of interest demonstrate expression of the gene of interest.

EST 1 of attachment 1 is identified by accession number AA689670, this sequence corresponds to SEQ ID No. 32; the gene comprising this sequence is found in cluster 20. AA689670 (SEQ ID No. 32) is a keratinocyte marker of advanced wound repair. Biomarkers derived from the gene comprising SEQ ID NO. 32 are useful as markers of advanced wound repair. In situ hybridisation on 24 hour frozen wound sections using an antisense radioprobe biomarker derived from the gene shows that this gene is expressed in the basal layers of the epidermis and expression extends back 10 to 12 cell diameters from the wound site.

EST 3 of attachment 1 is identified by accession number AI153421, this sequence corresponds to SEQ ID No. 19; the gene comprising this sequence is found in cluster 5. AI153421 (SEQ ID No. 19) is a biomarker for wound inflammation. Biomarkers derived from the gene comprising SEQ ID No. 19 are useful as markers of wound inflammation and/or impaired healing. In situ hybridisation on 24 hour frozen wound tissue sections using an antisense radioprobe biomarker derived from the gene shows that this gene is expressed in all tissue layers by cells as part of the inflammatory response downstream of macrophage infiltration.

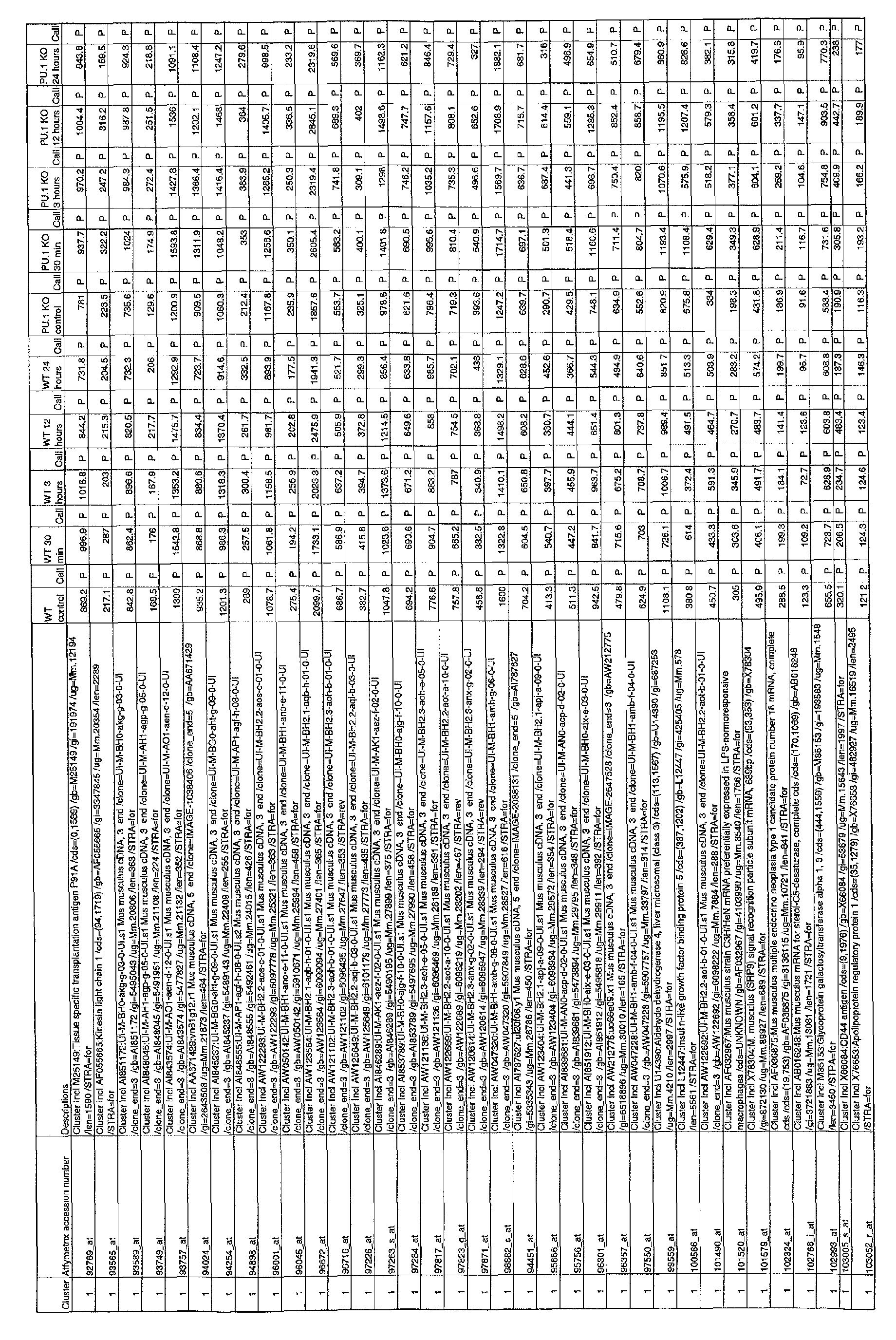

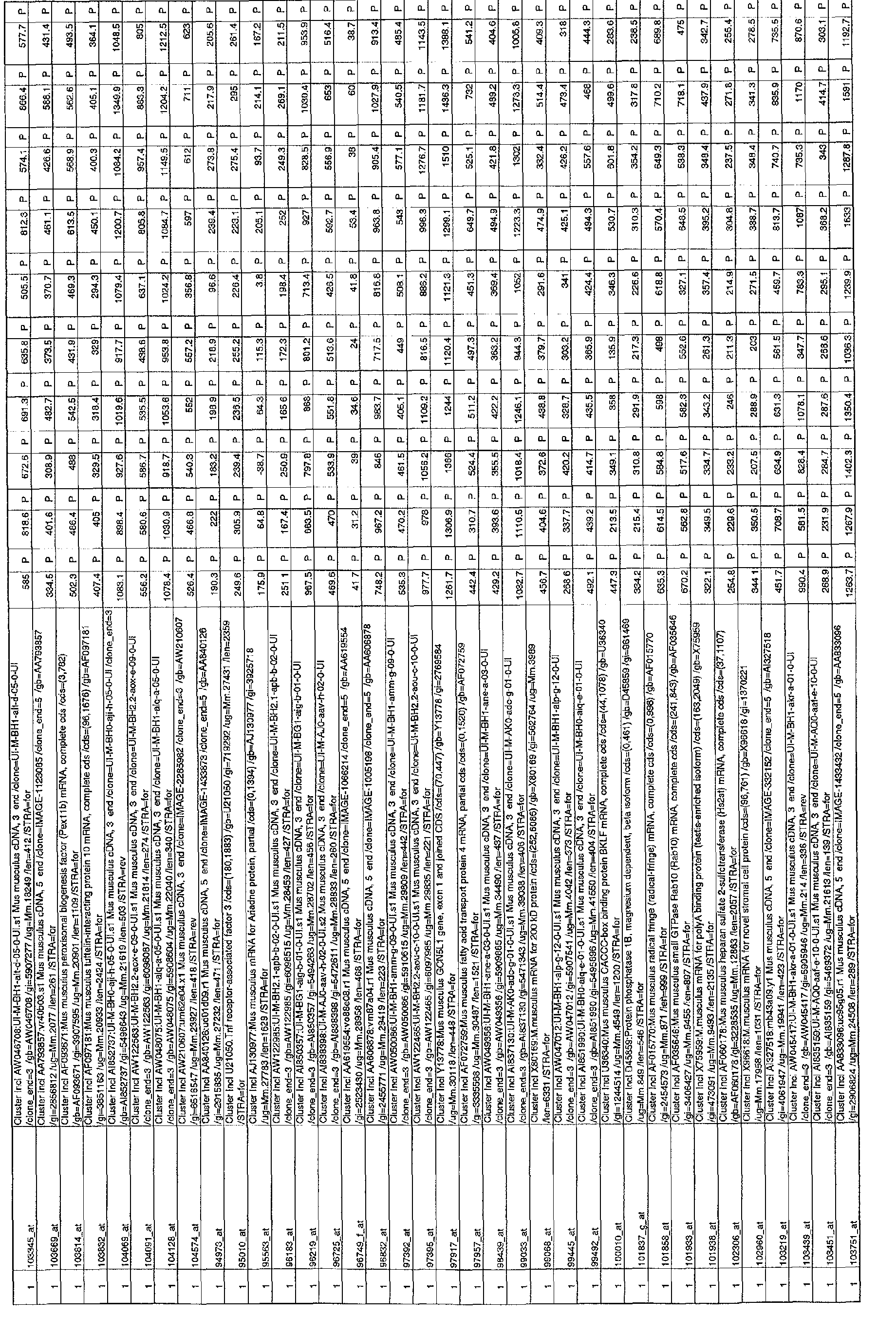

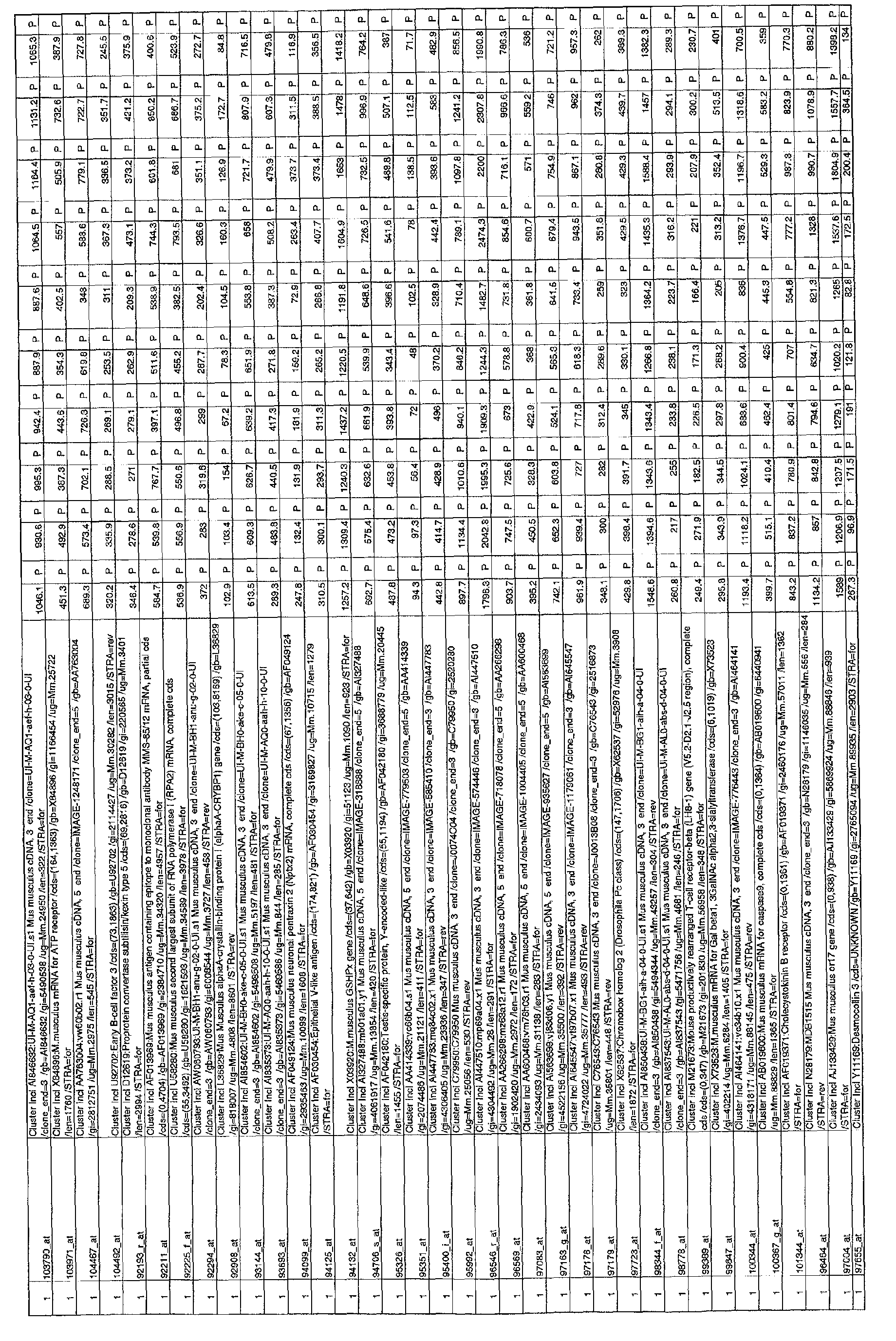

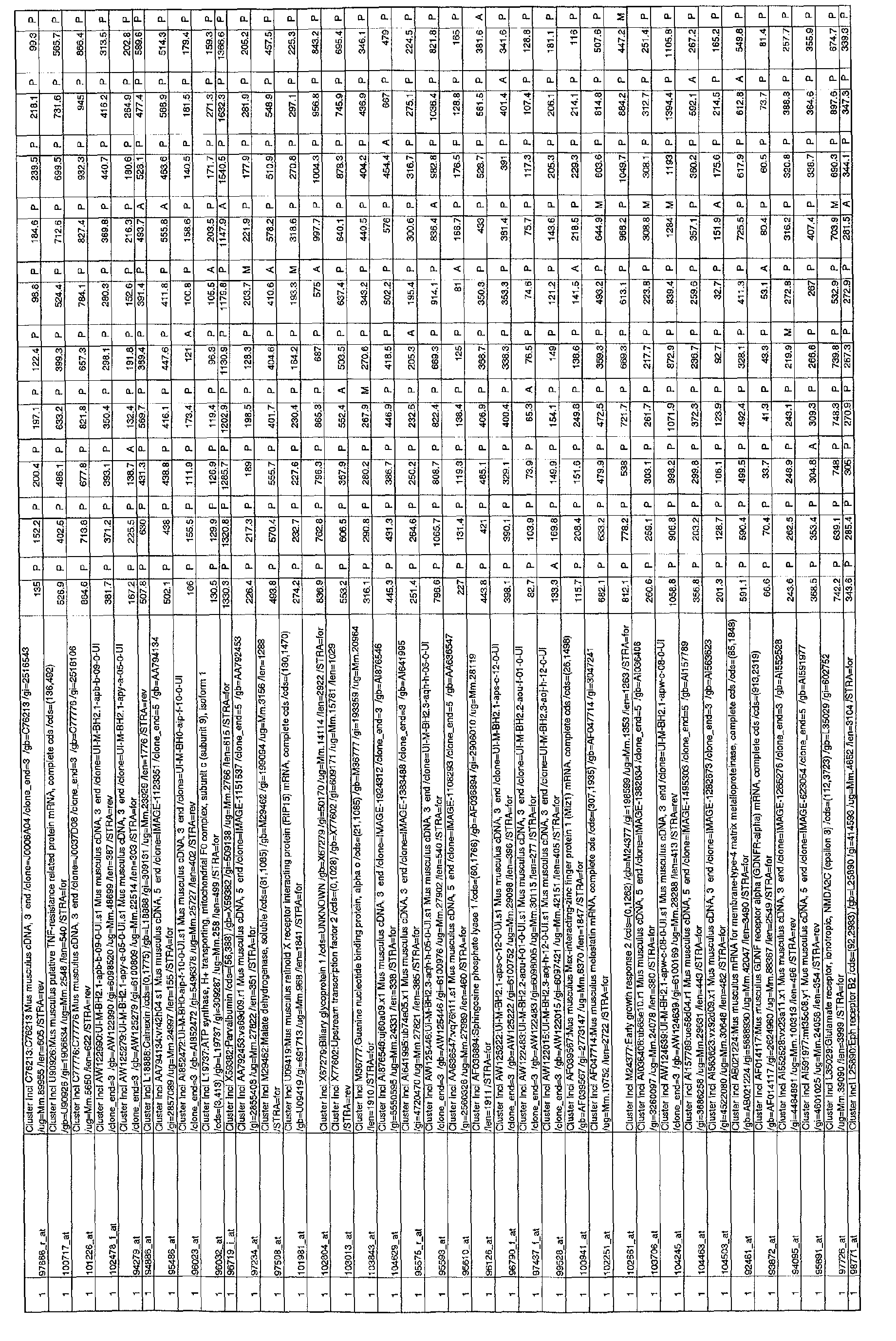

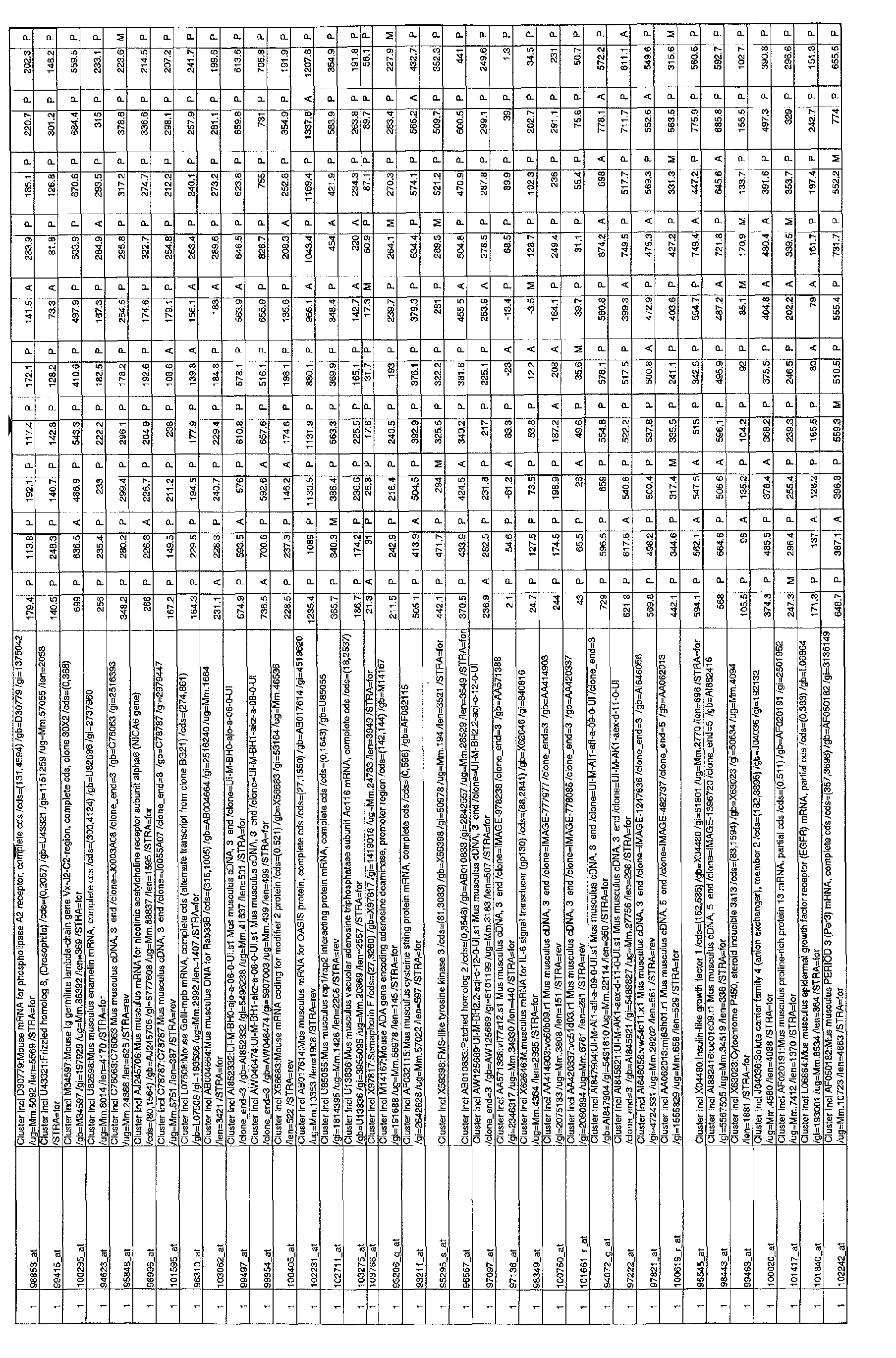

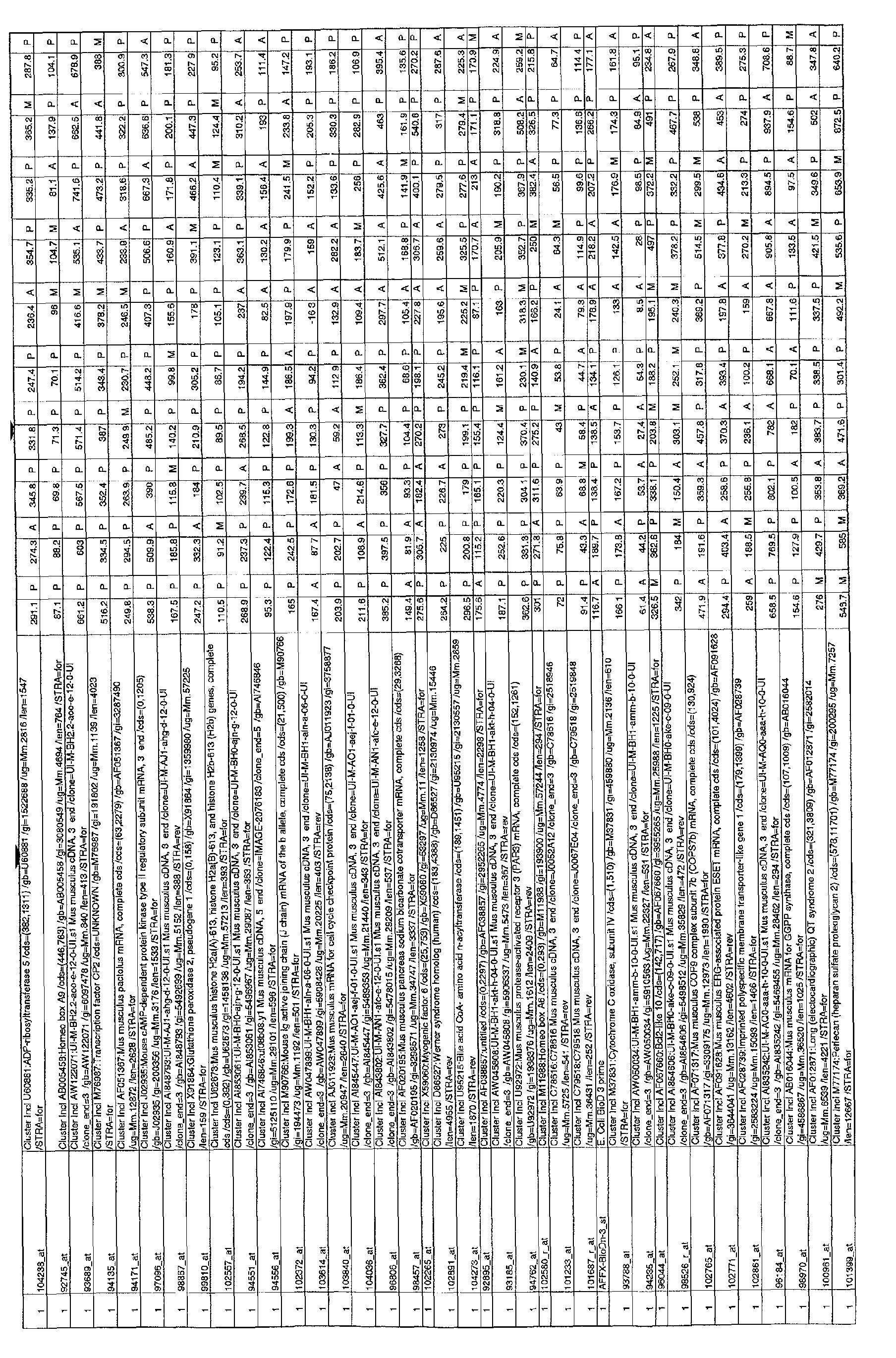

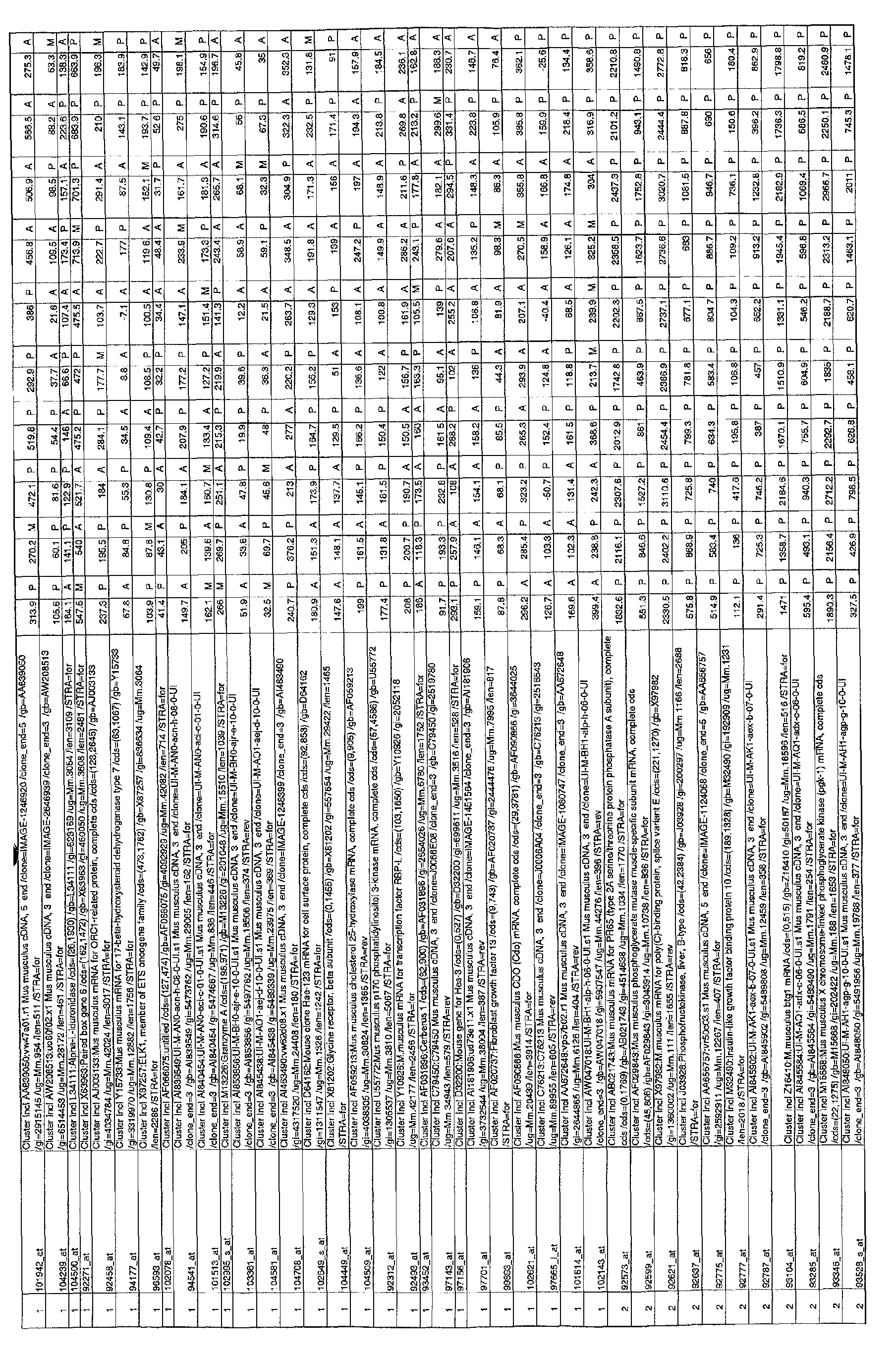

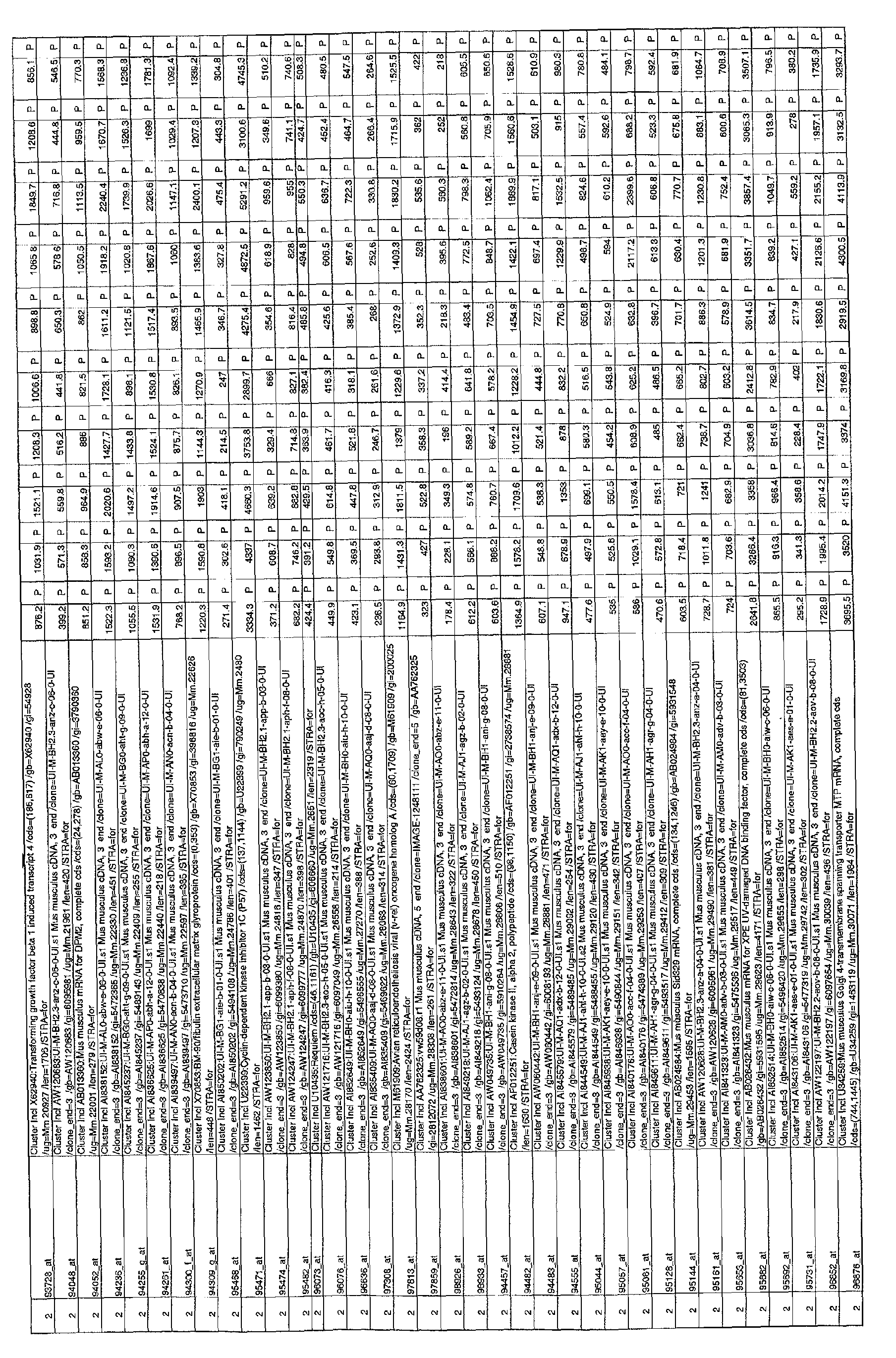

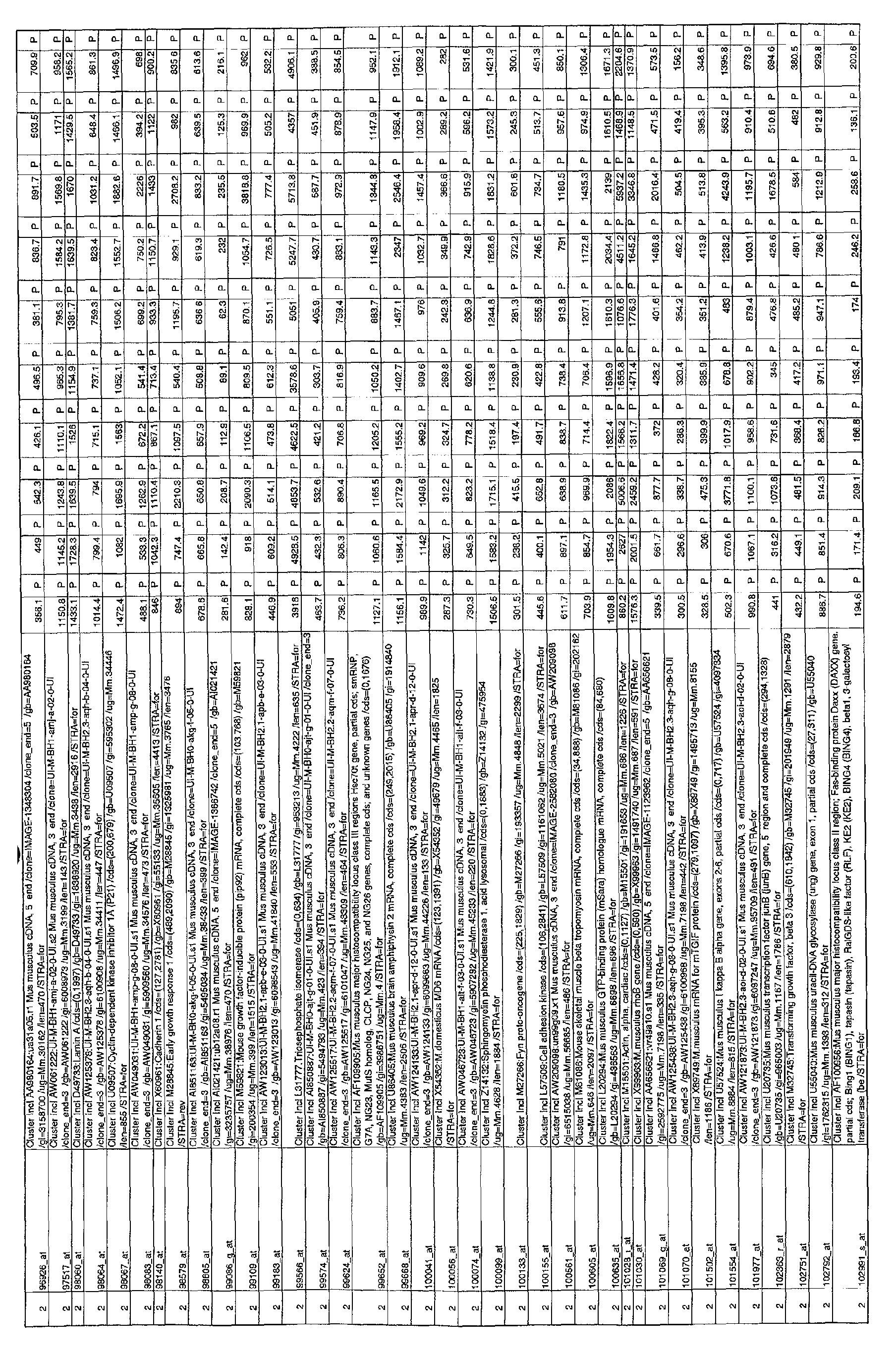

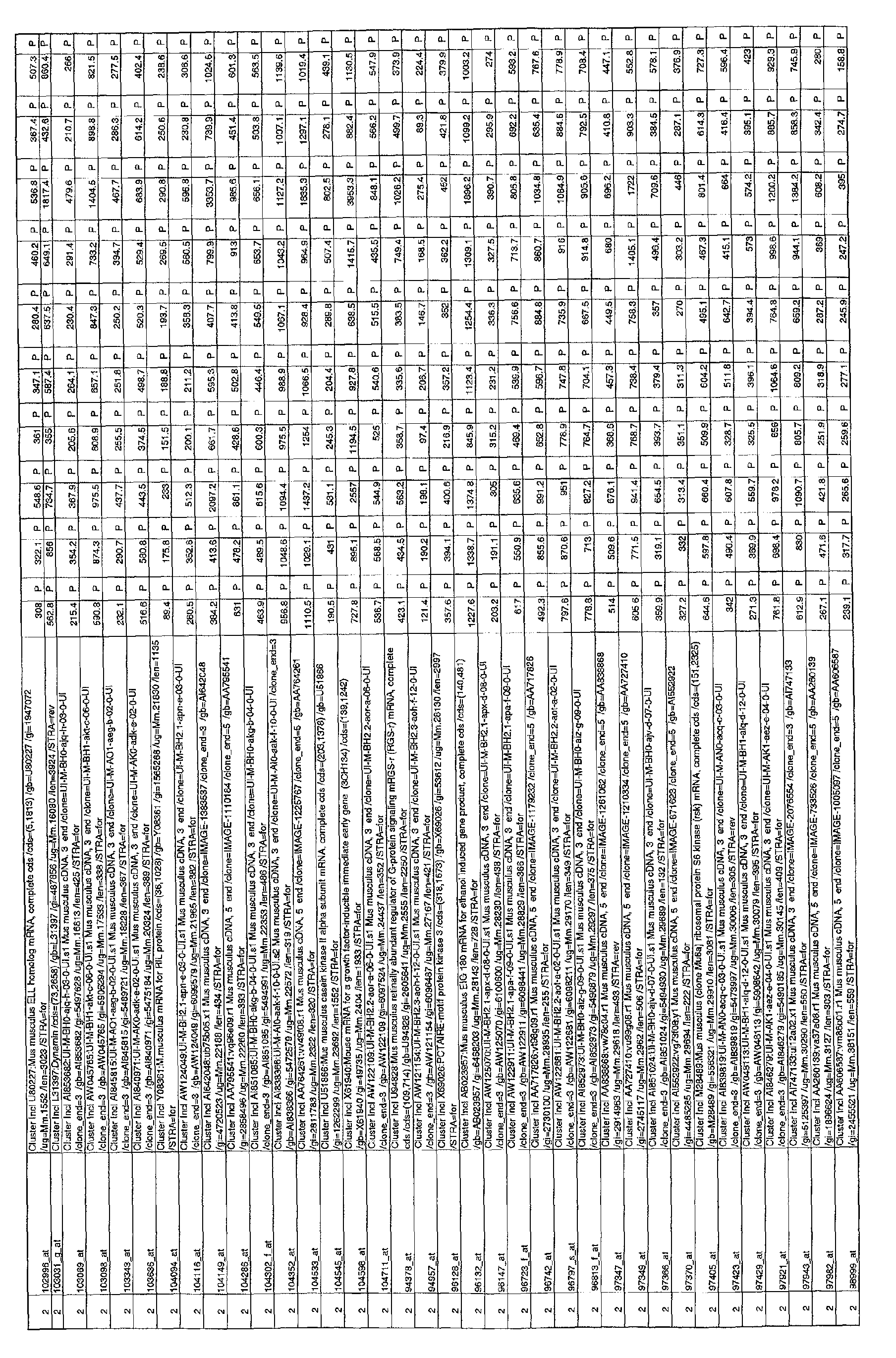

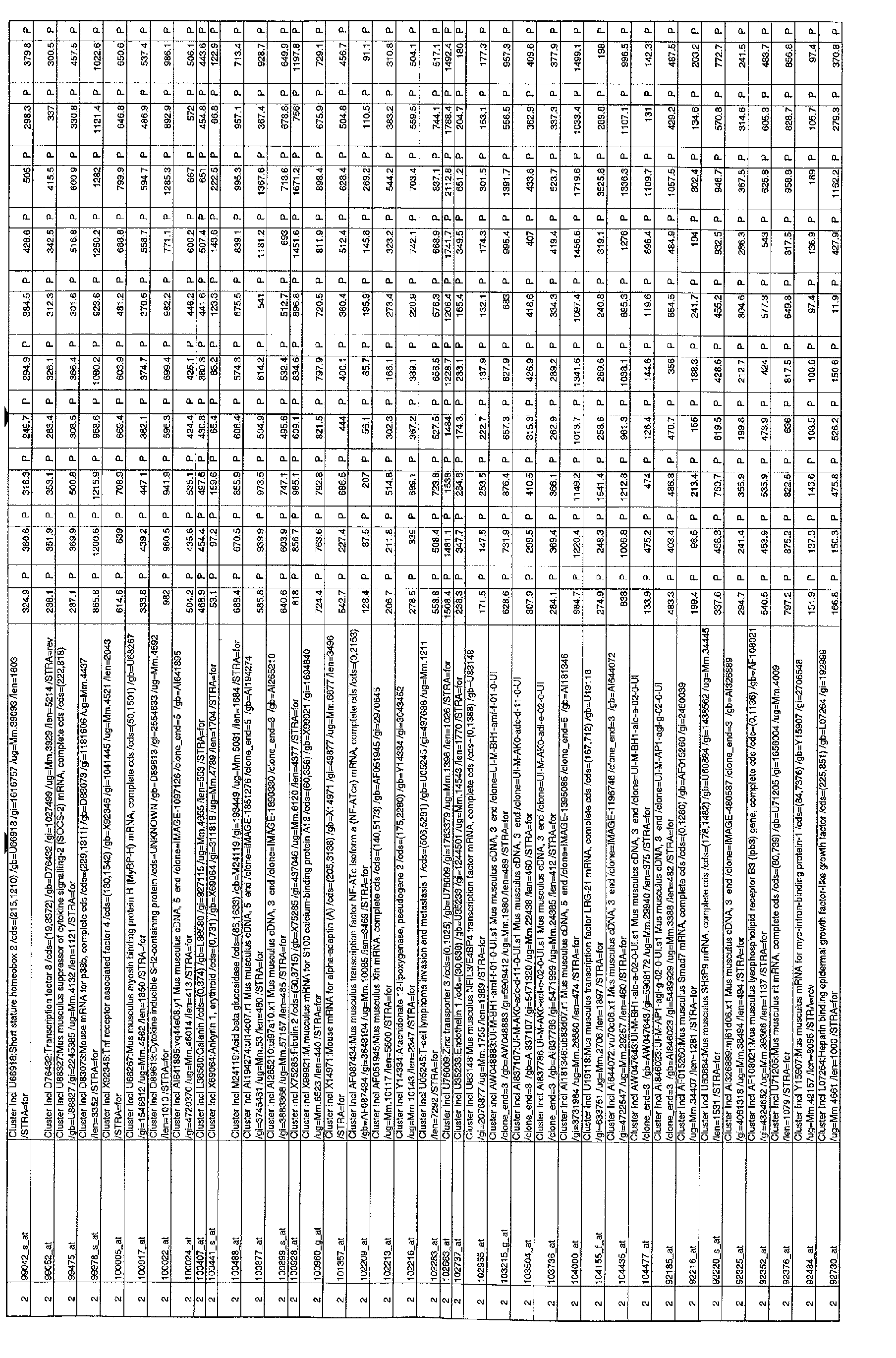

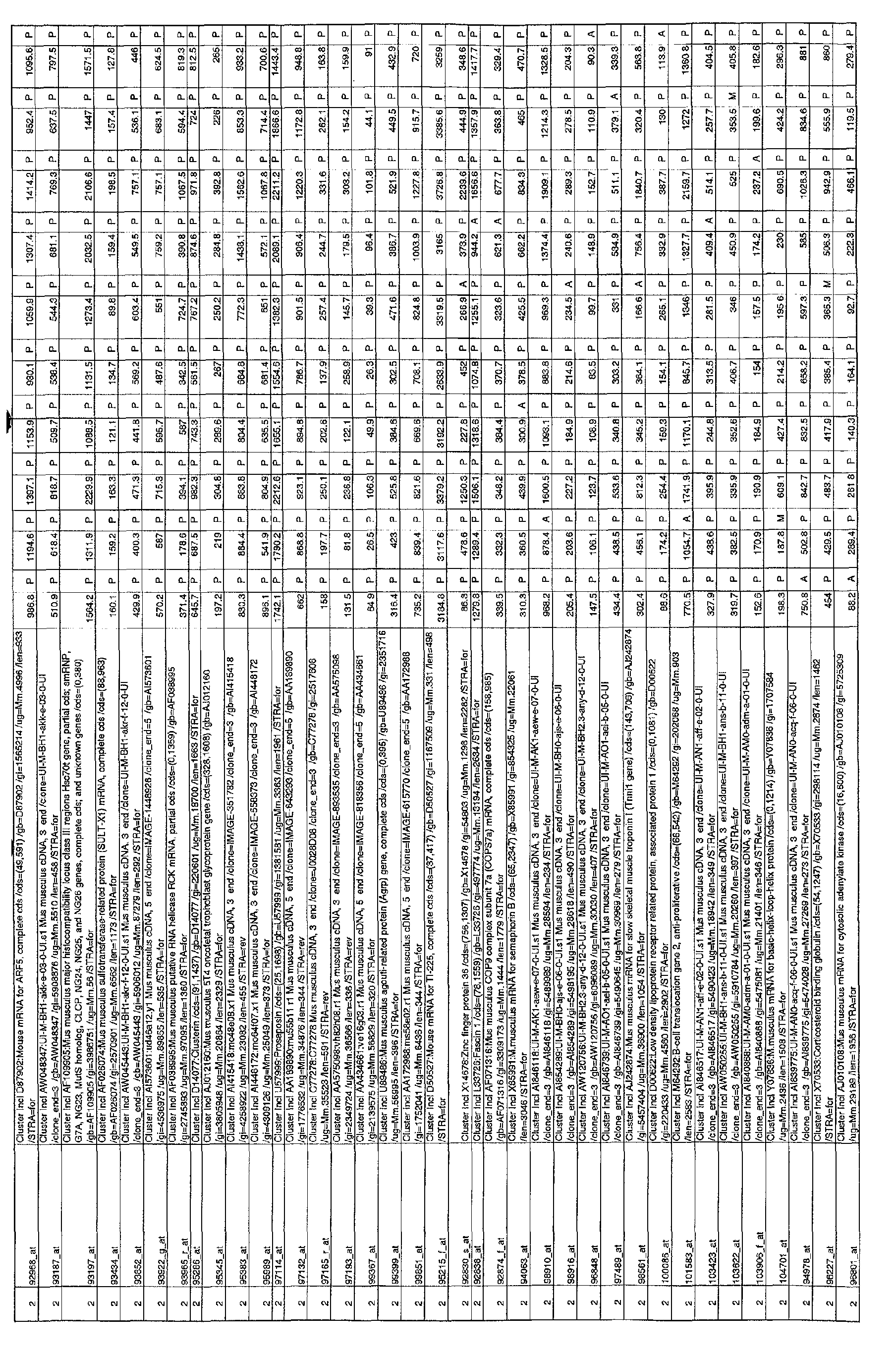

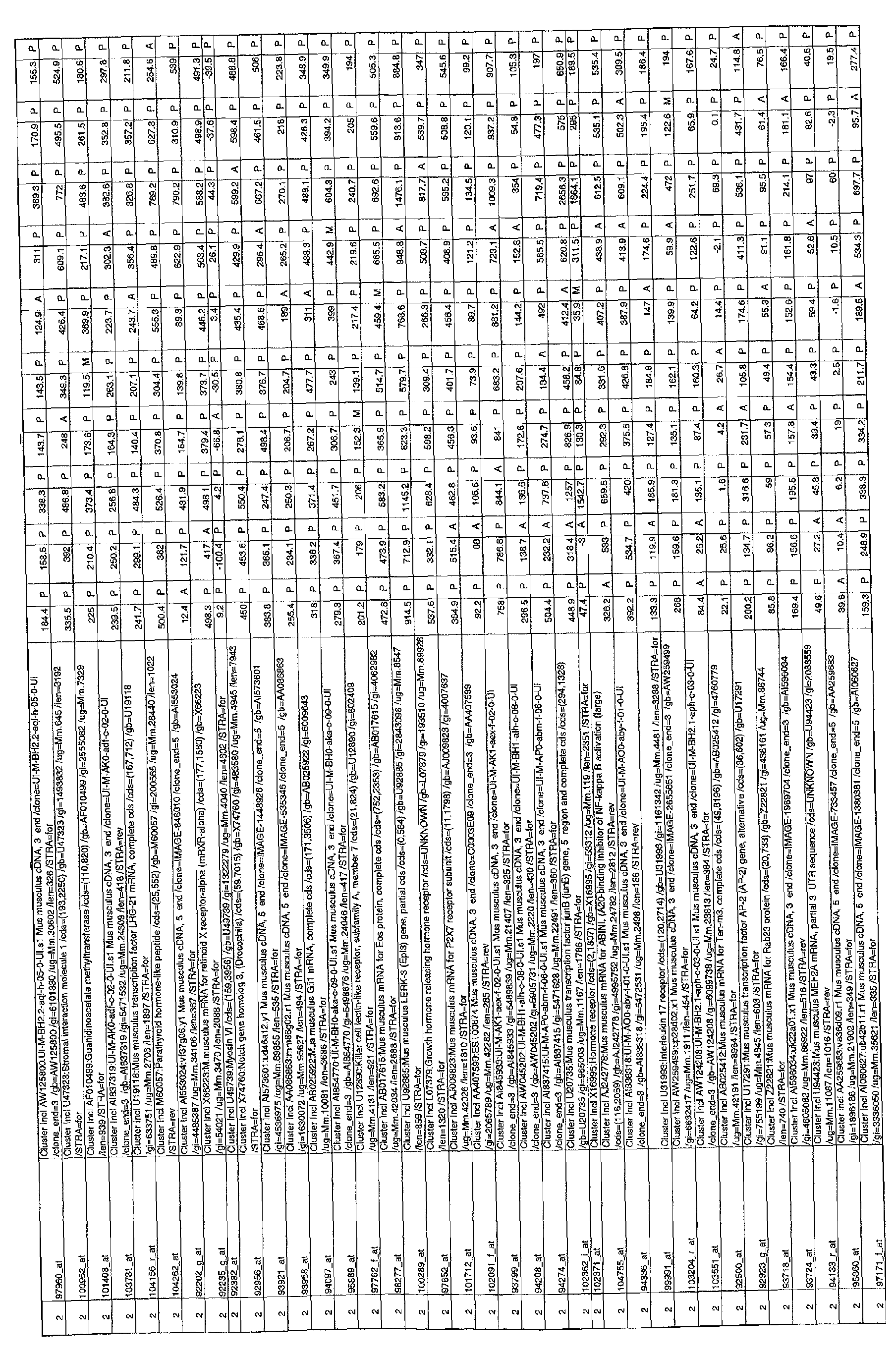

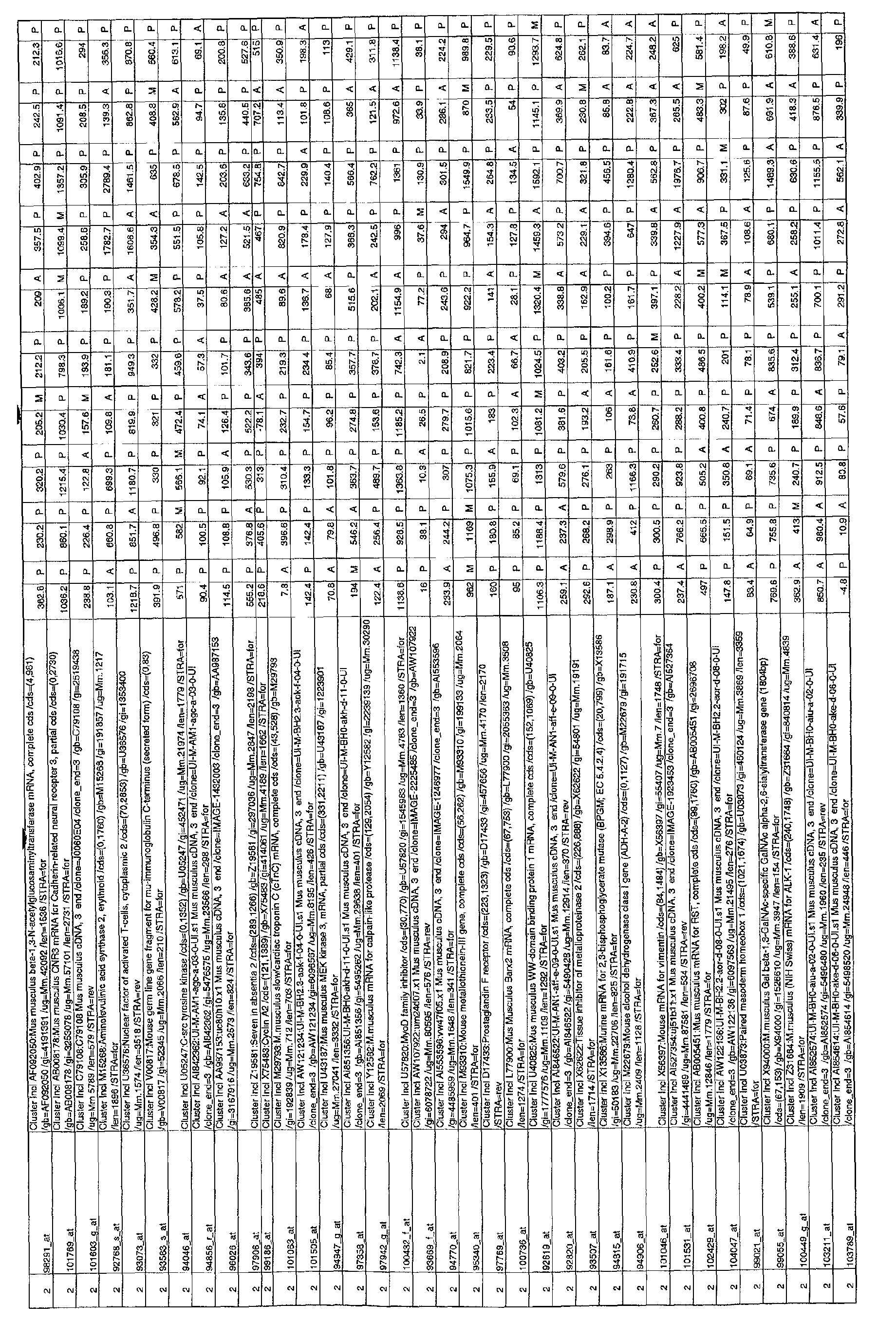

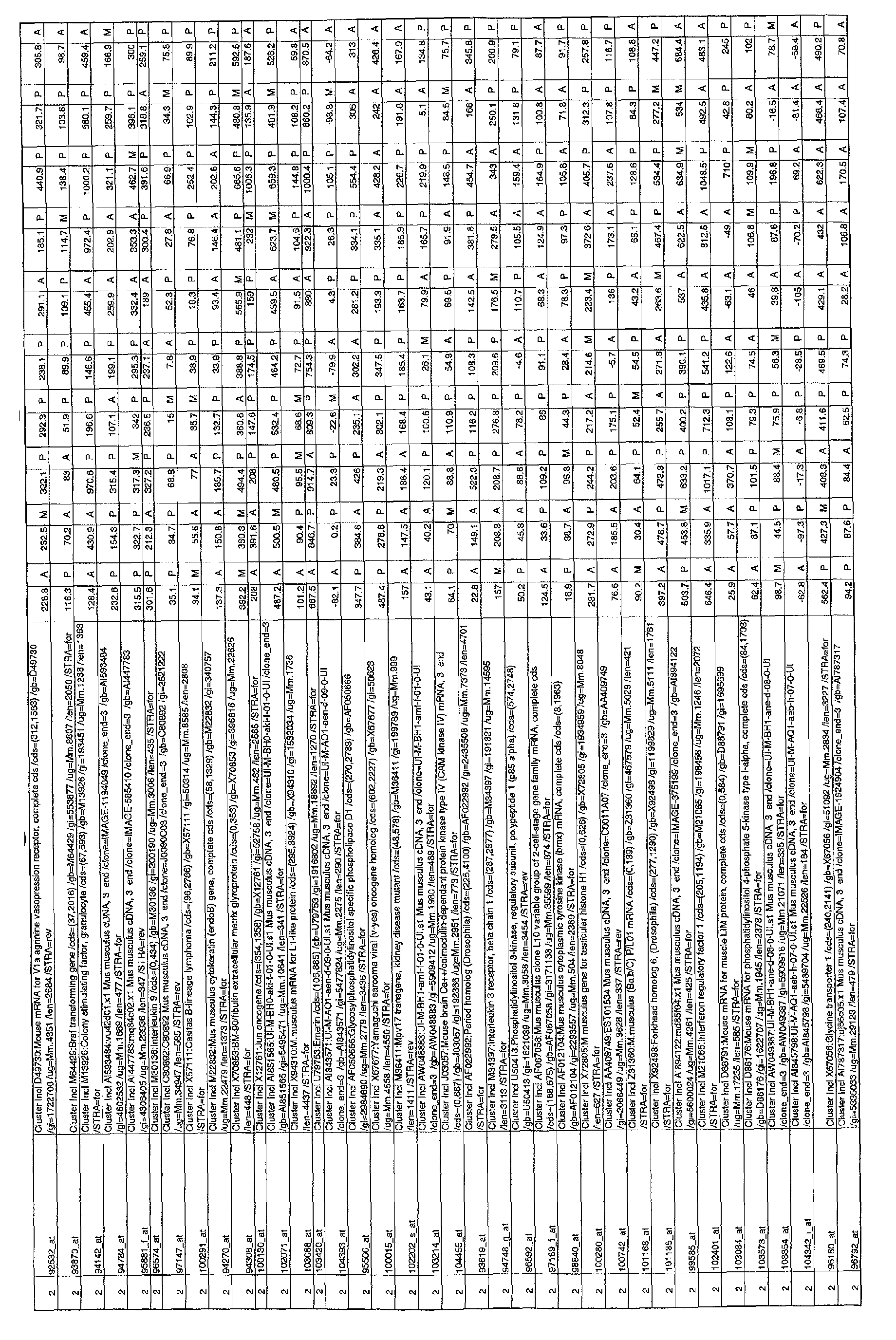

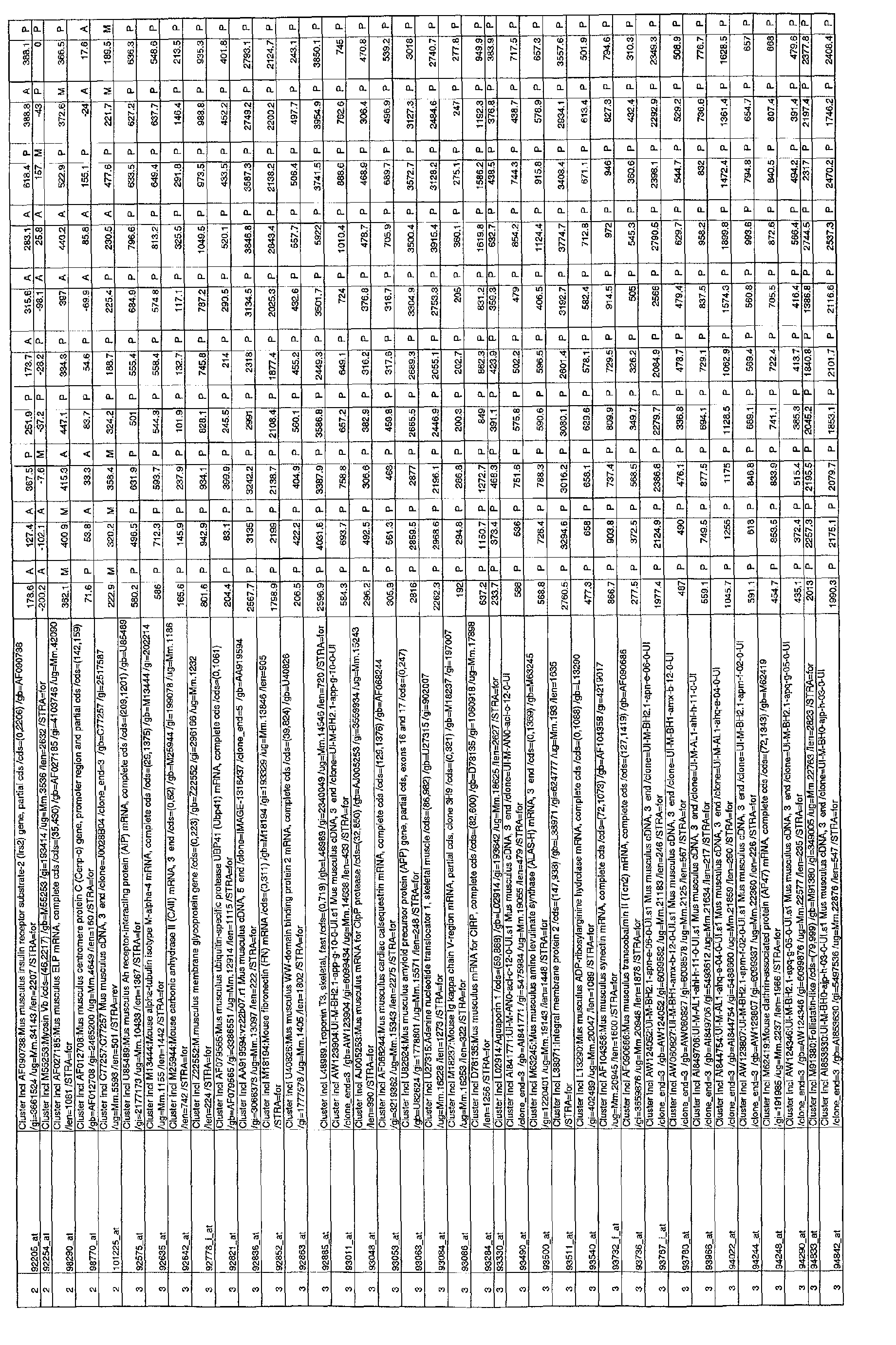

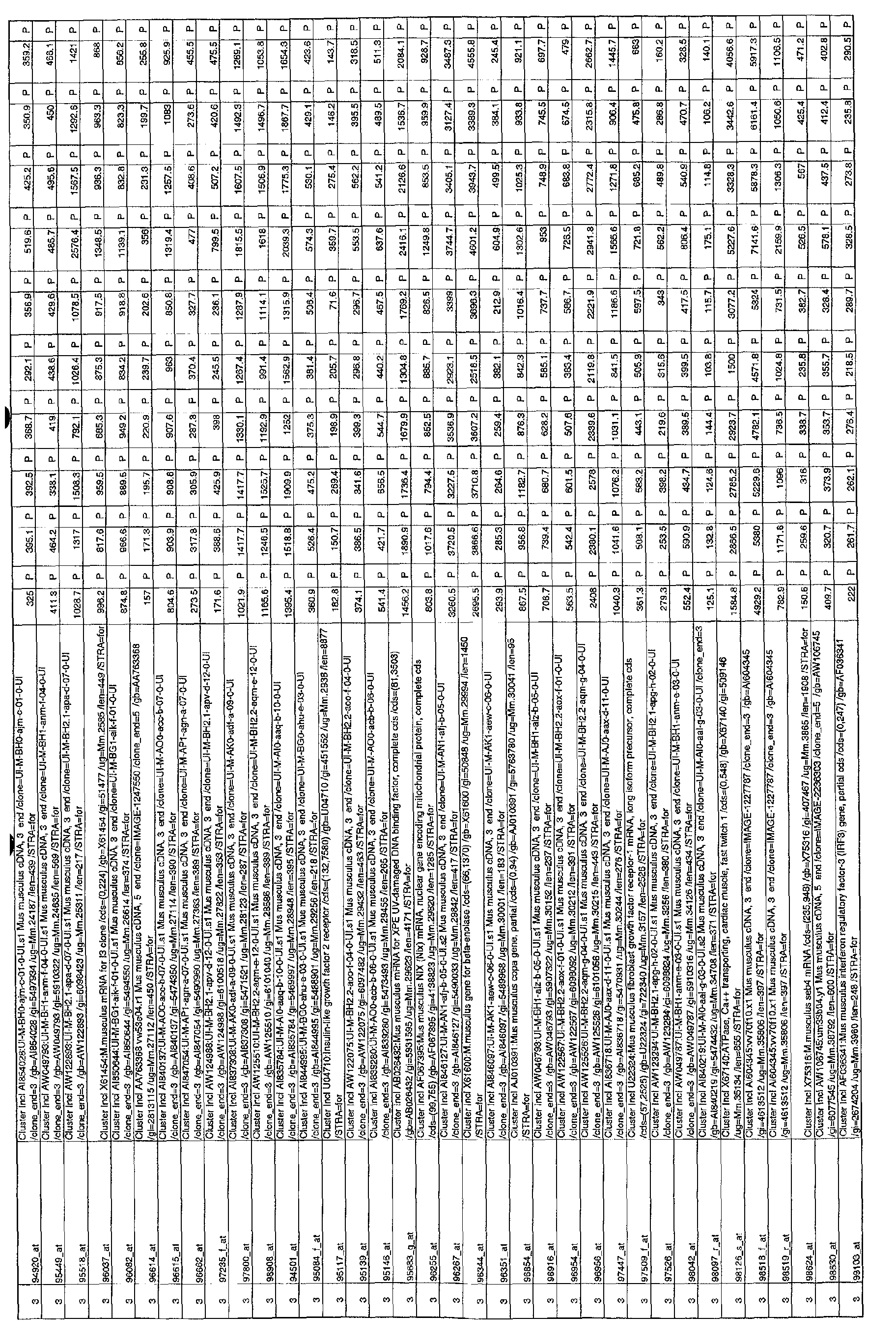

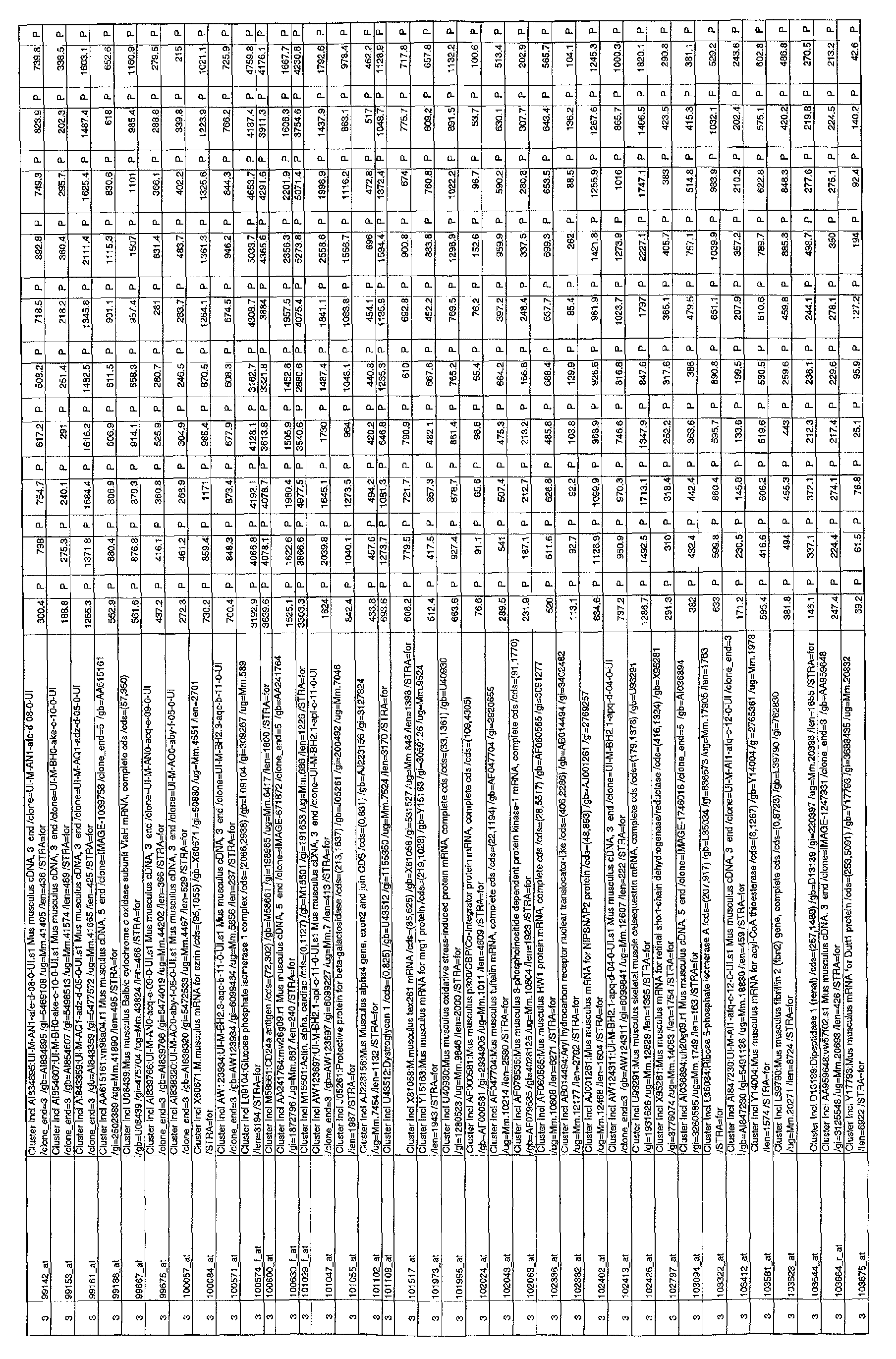

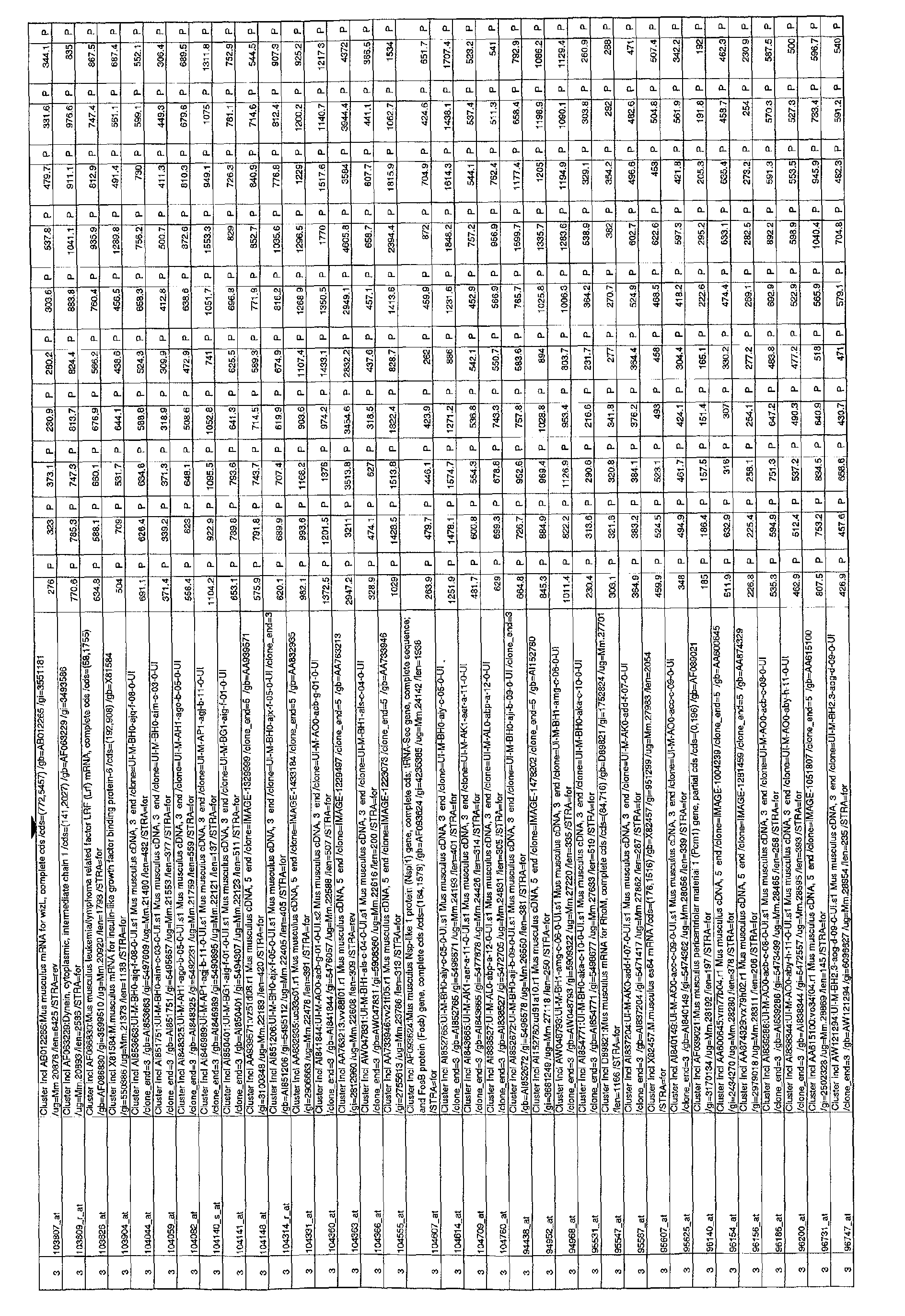

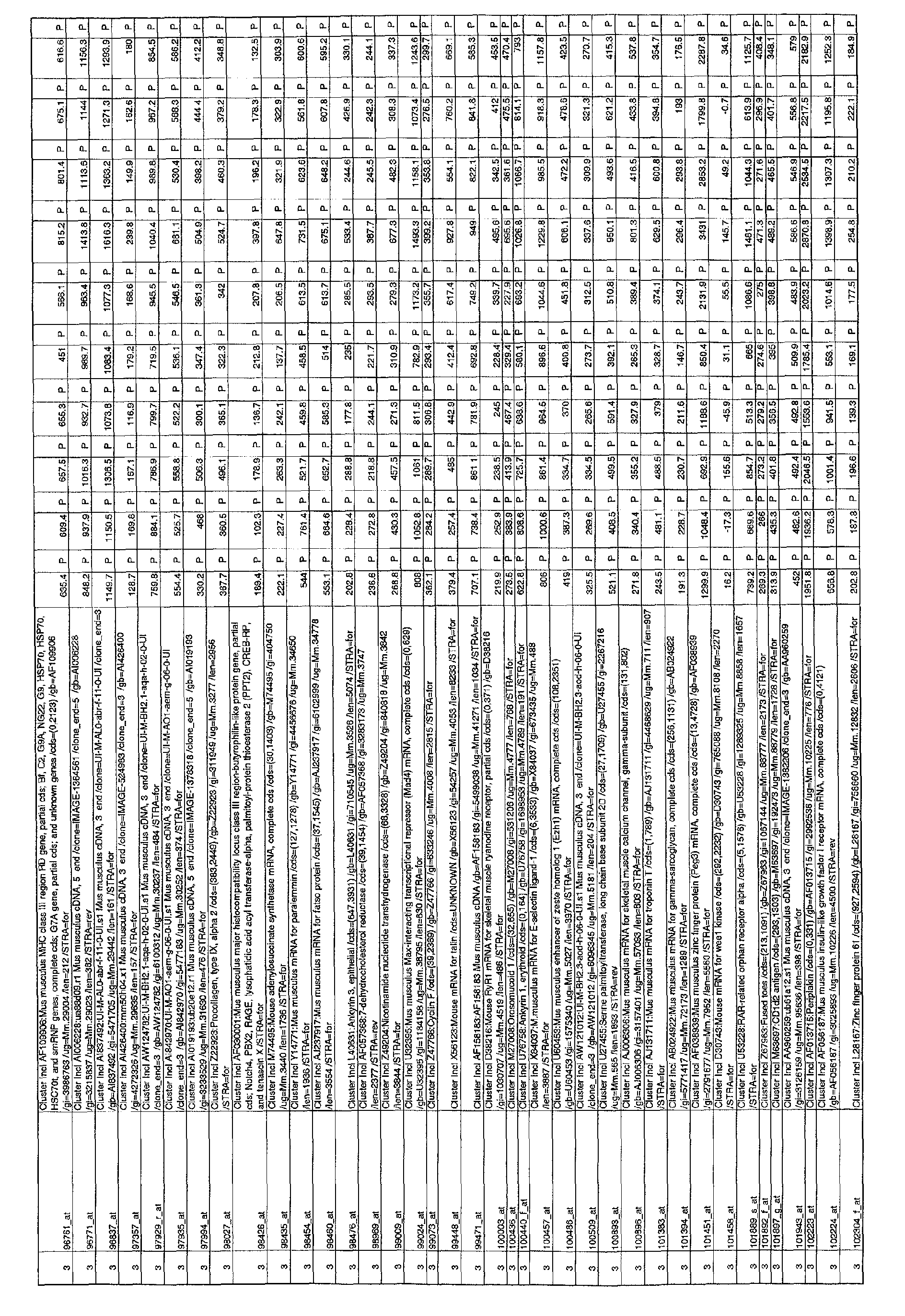

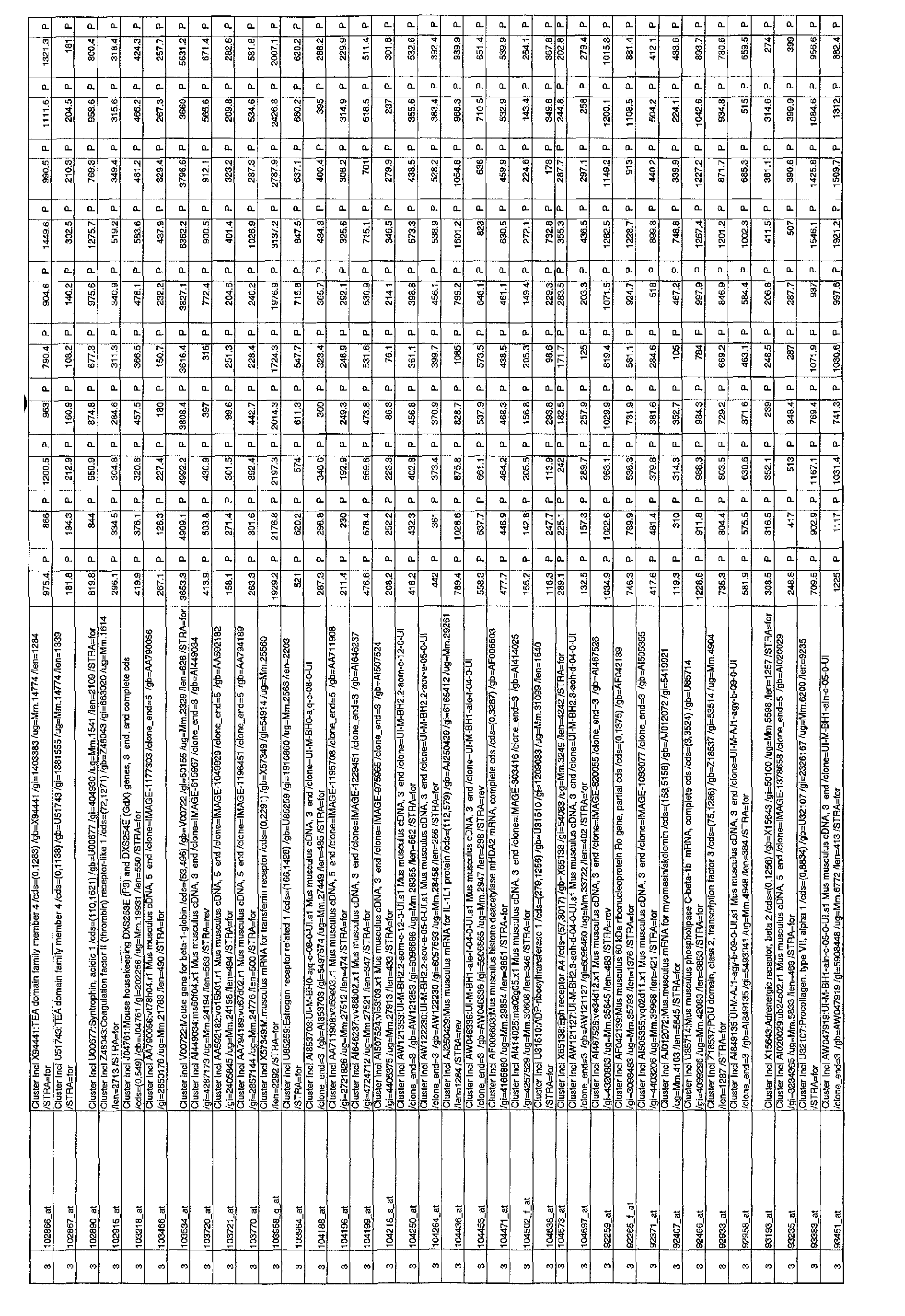

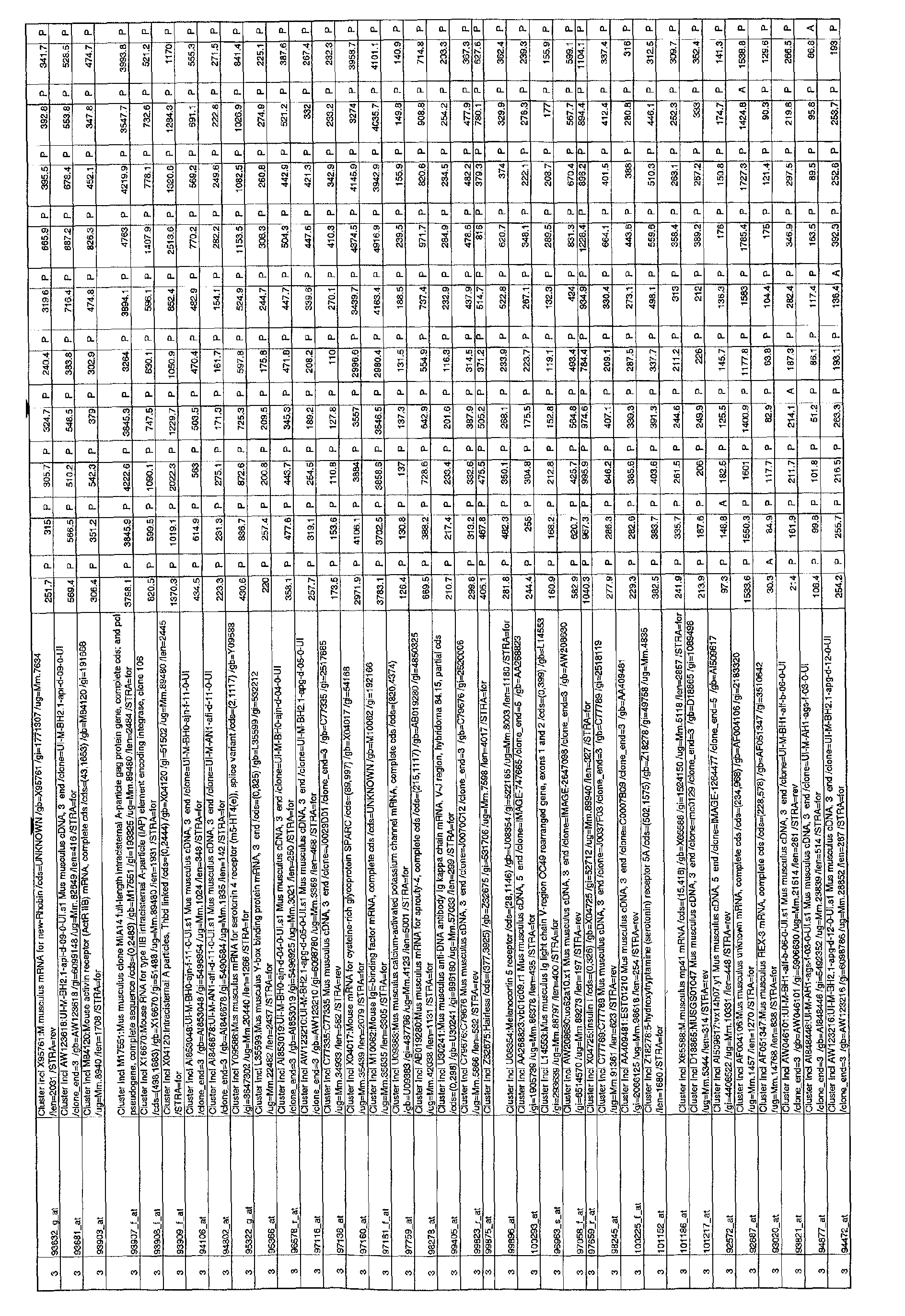

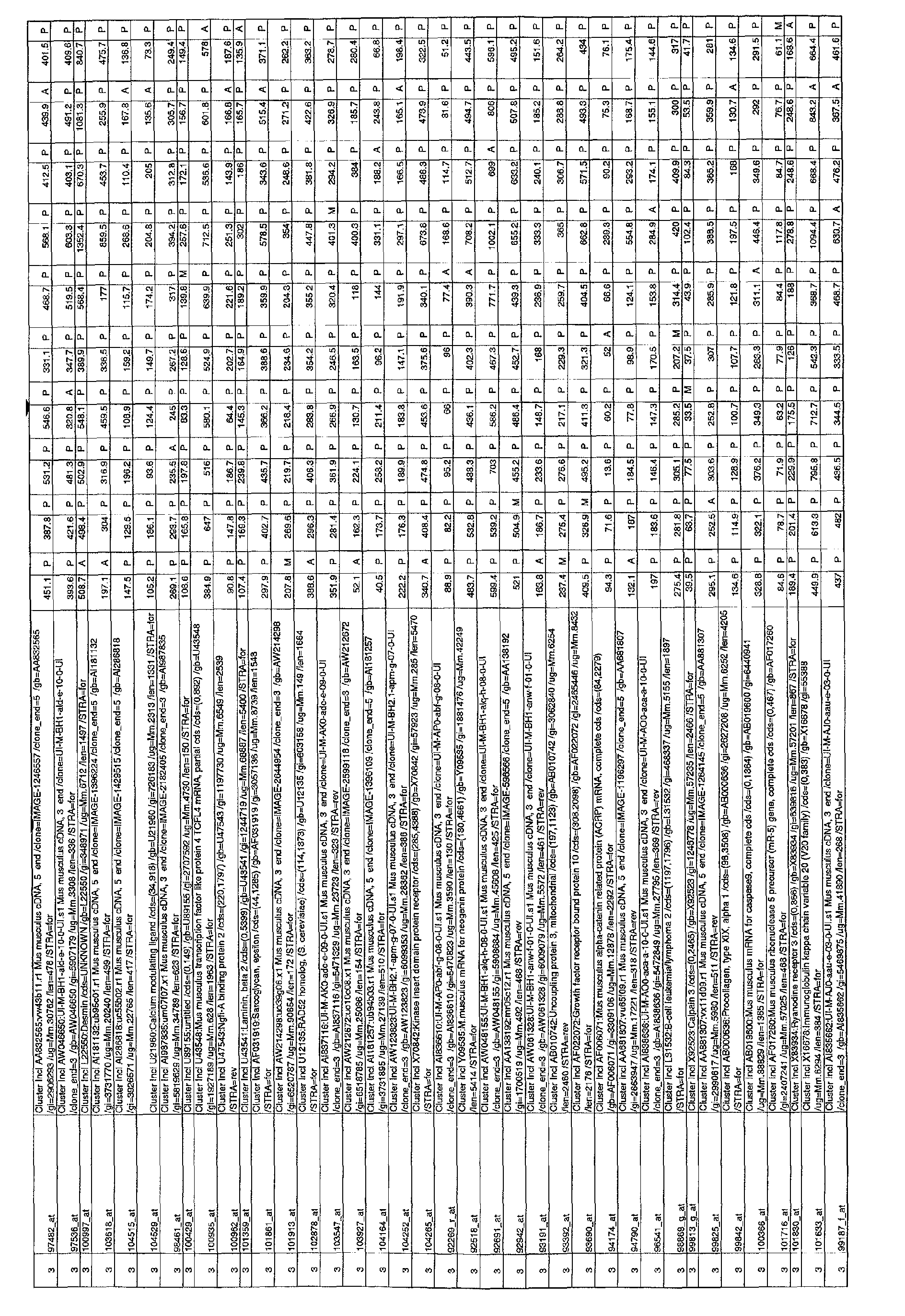

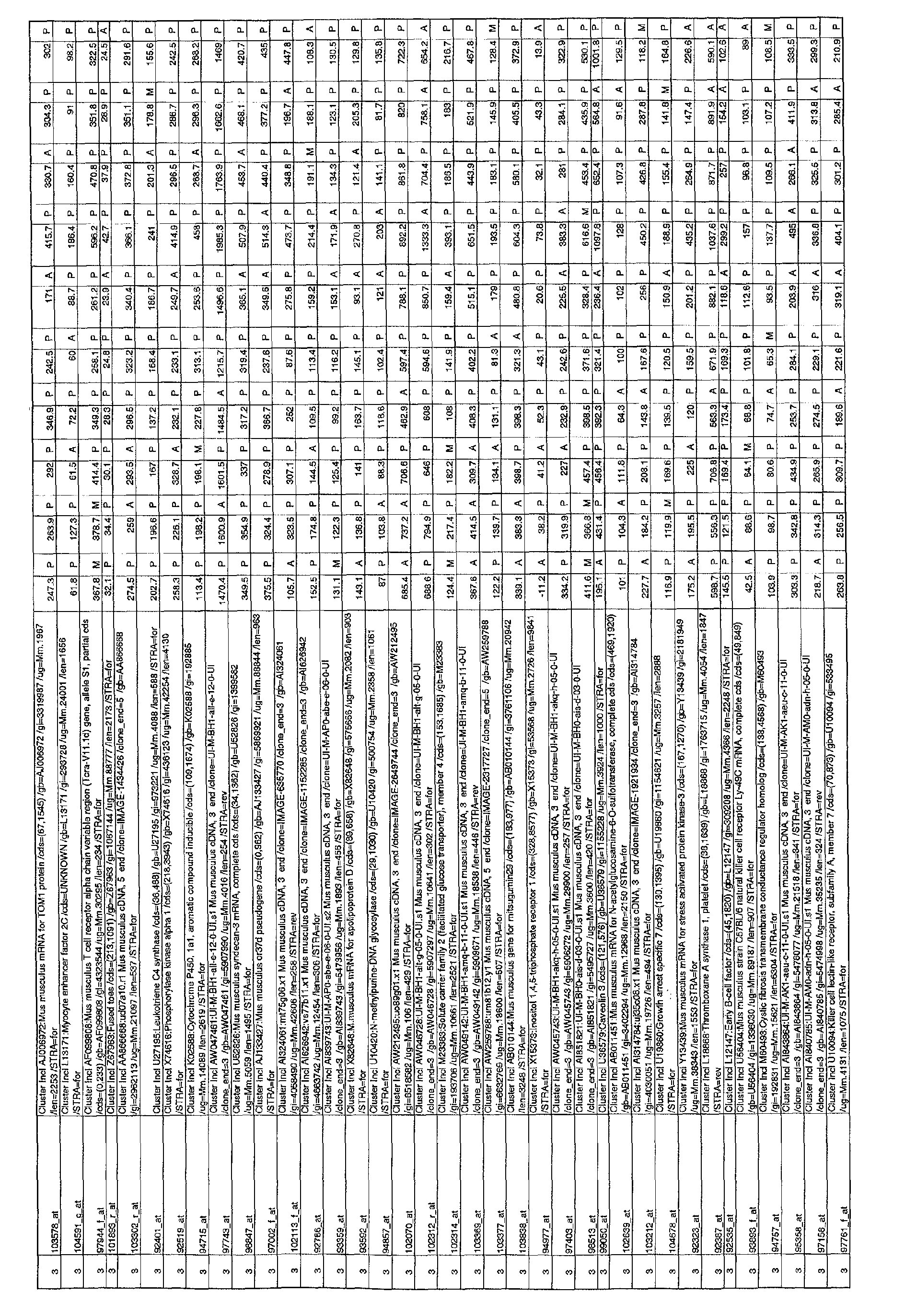

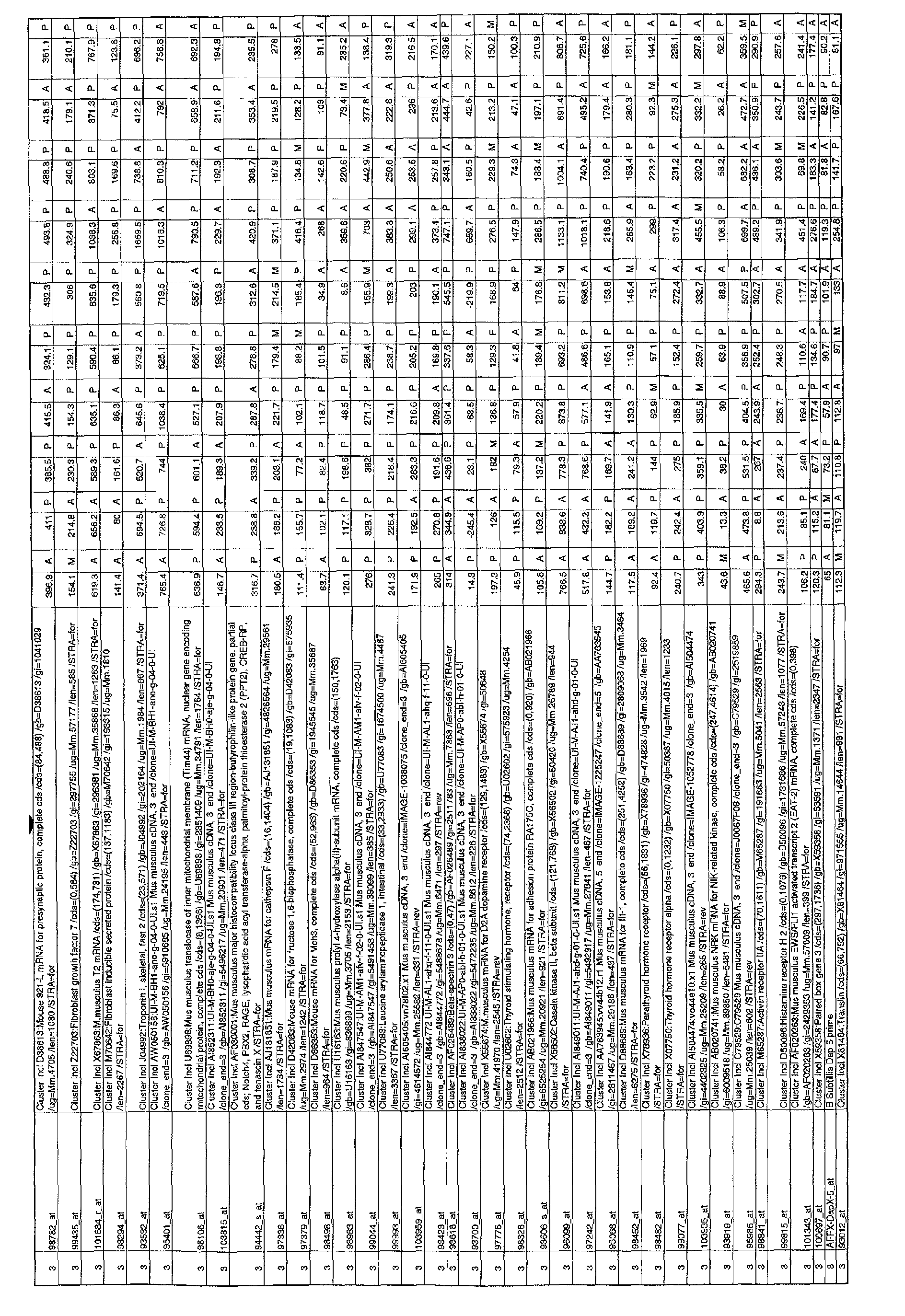

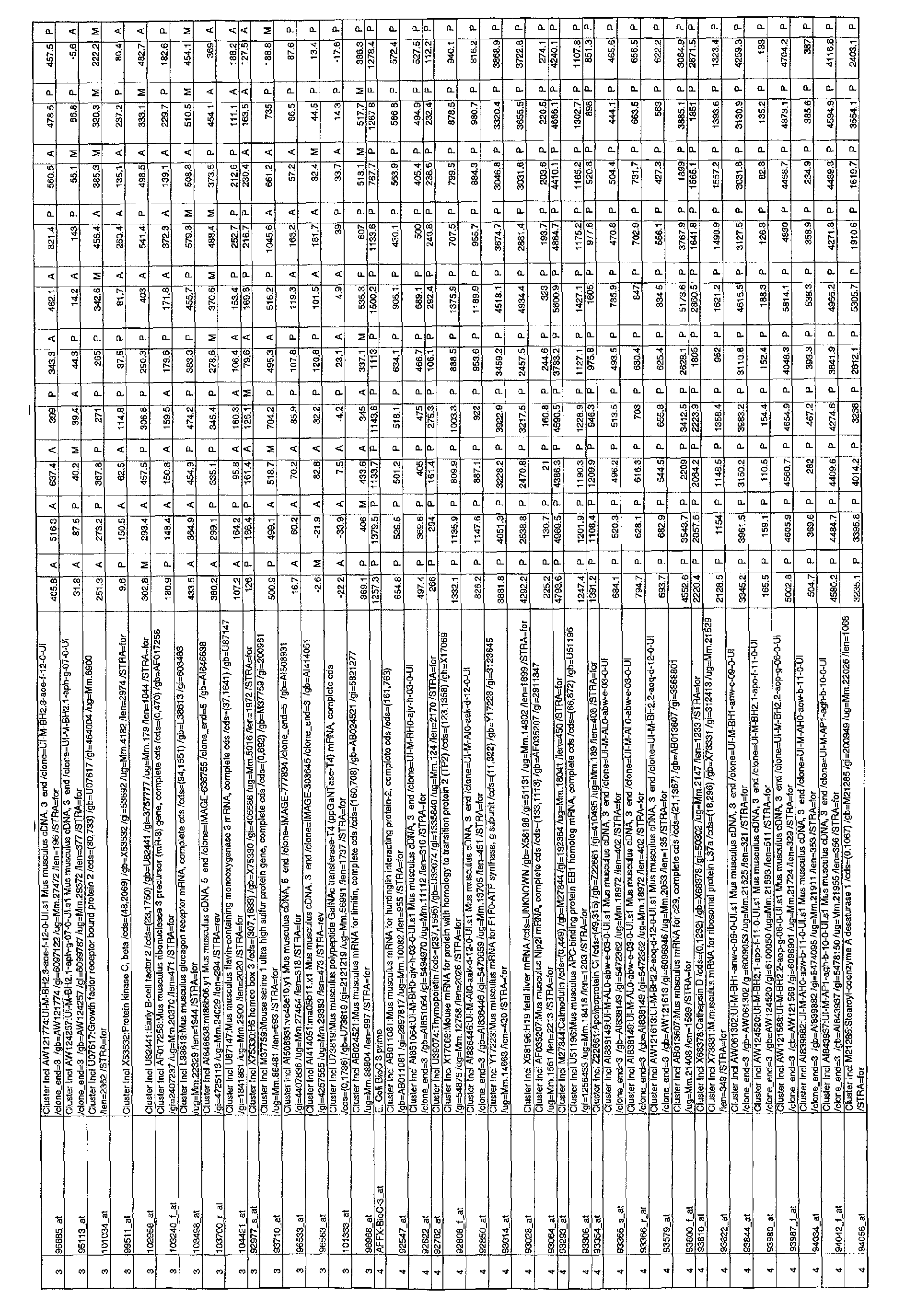

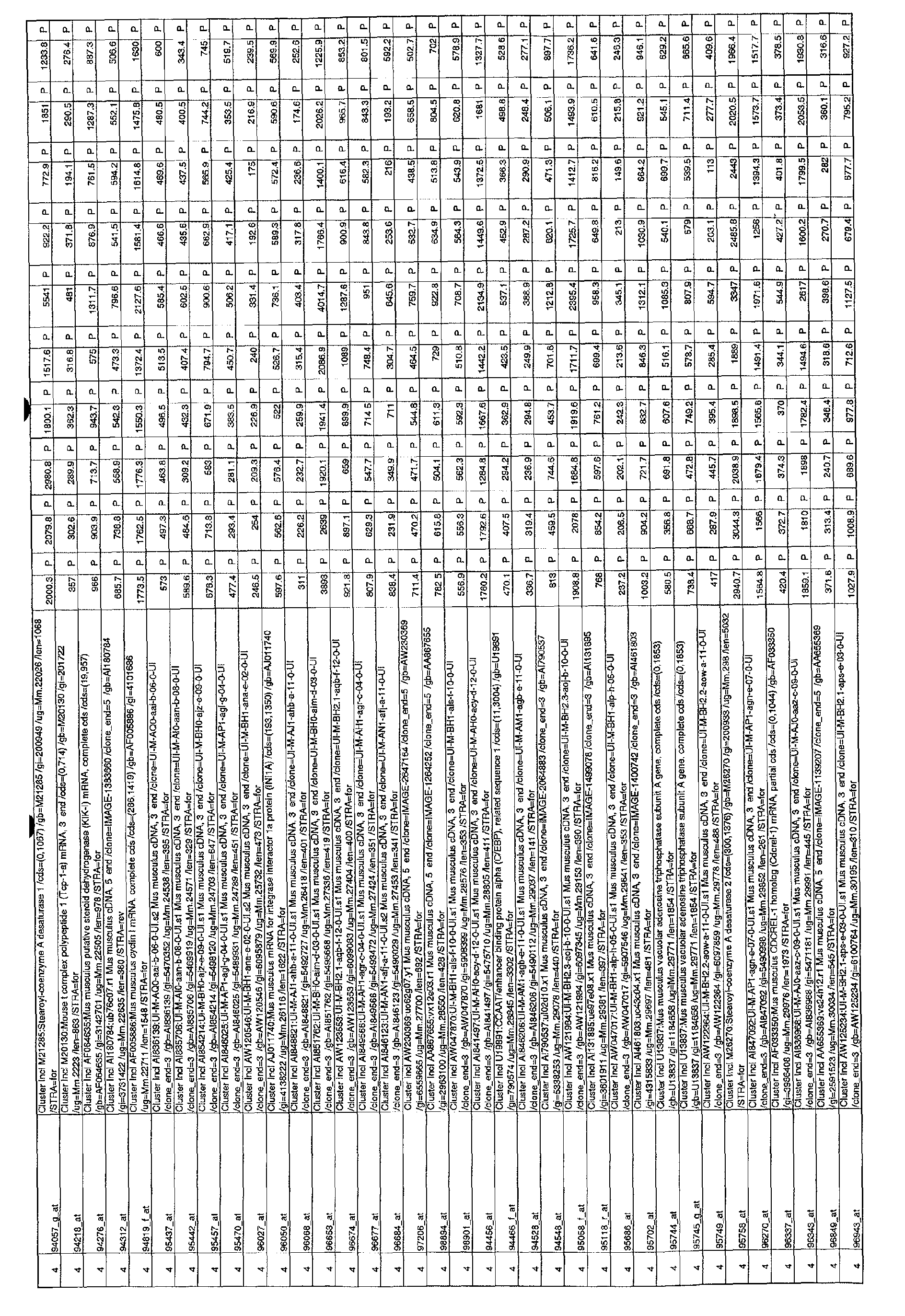

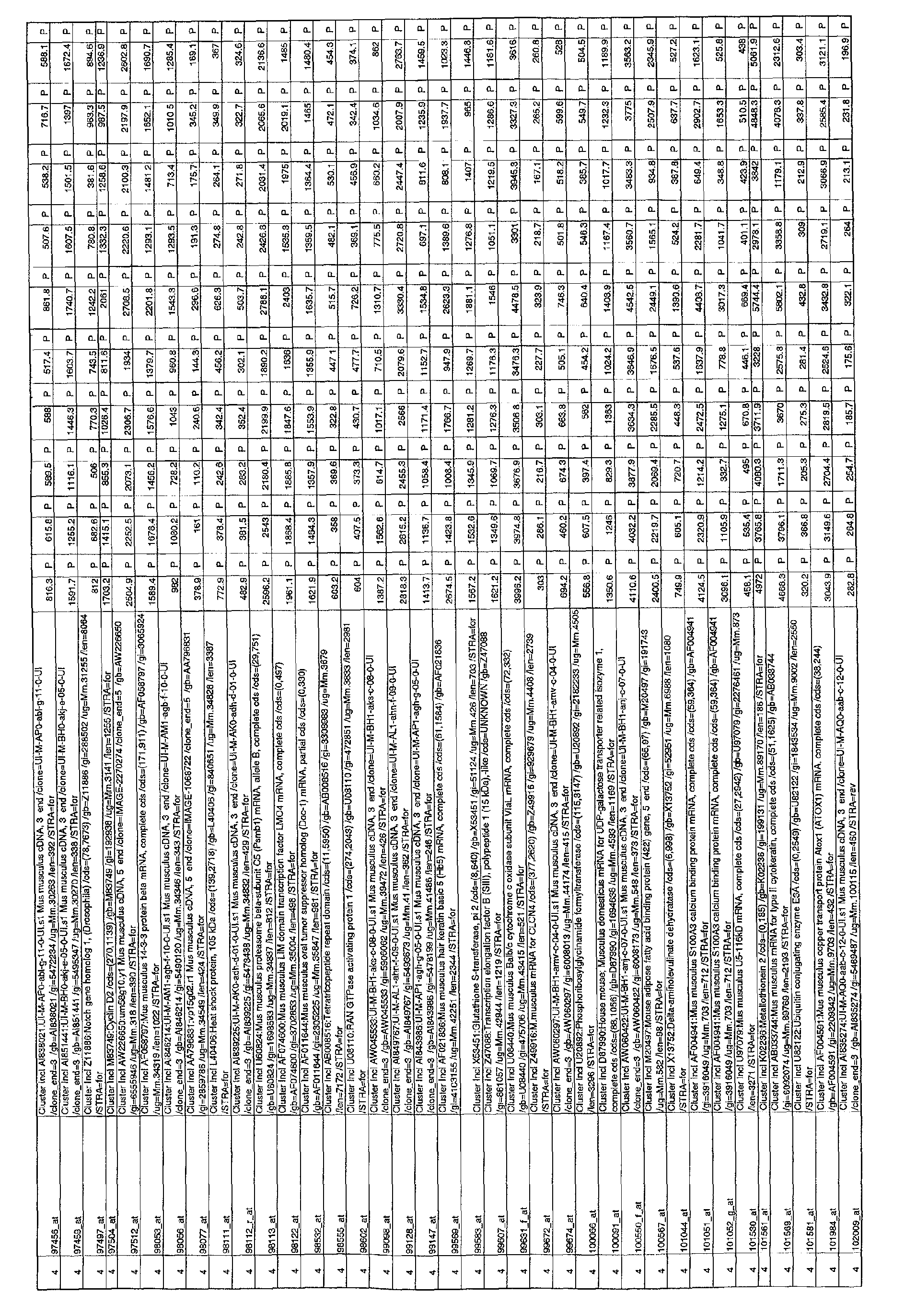

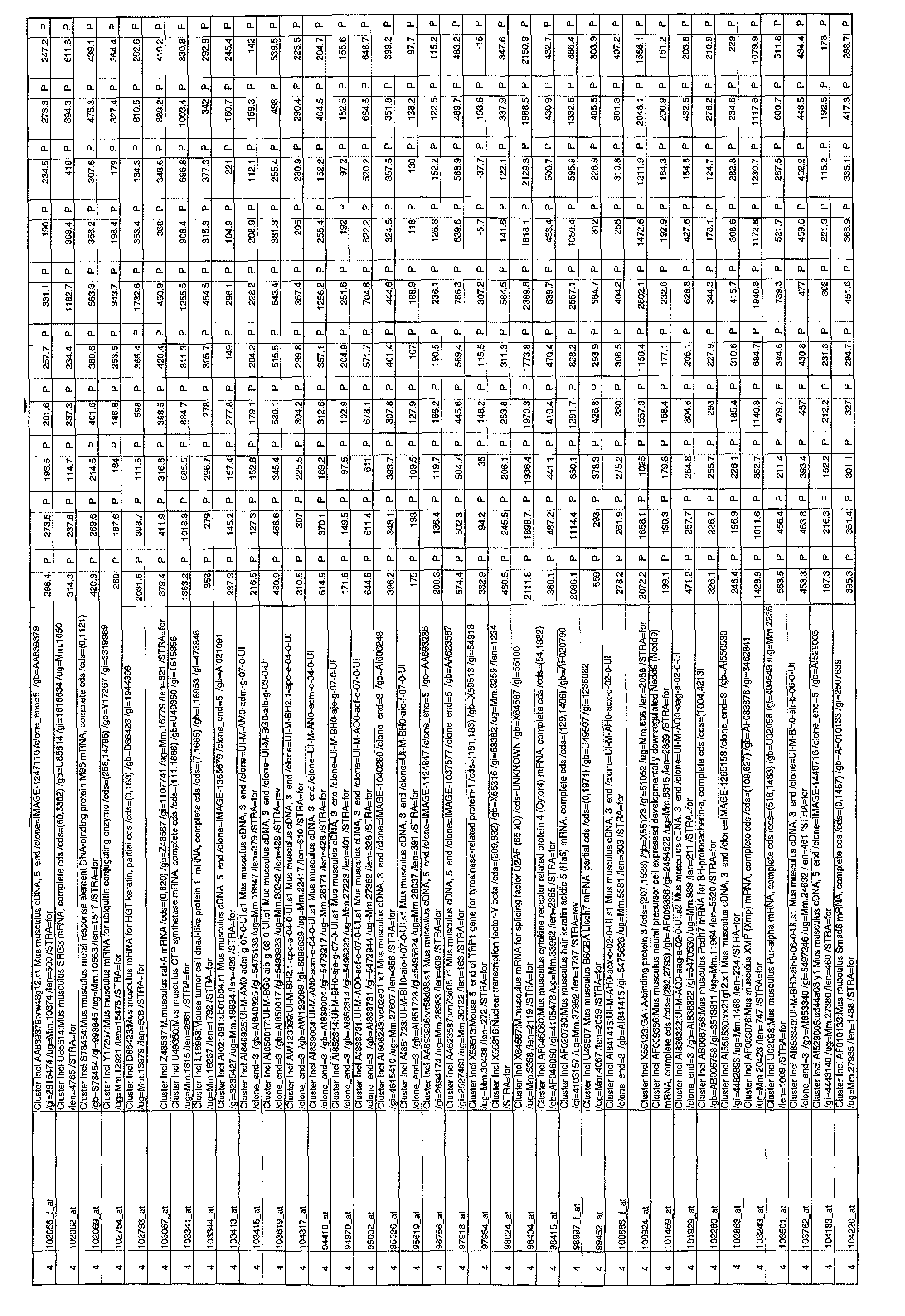

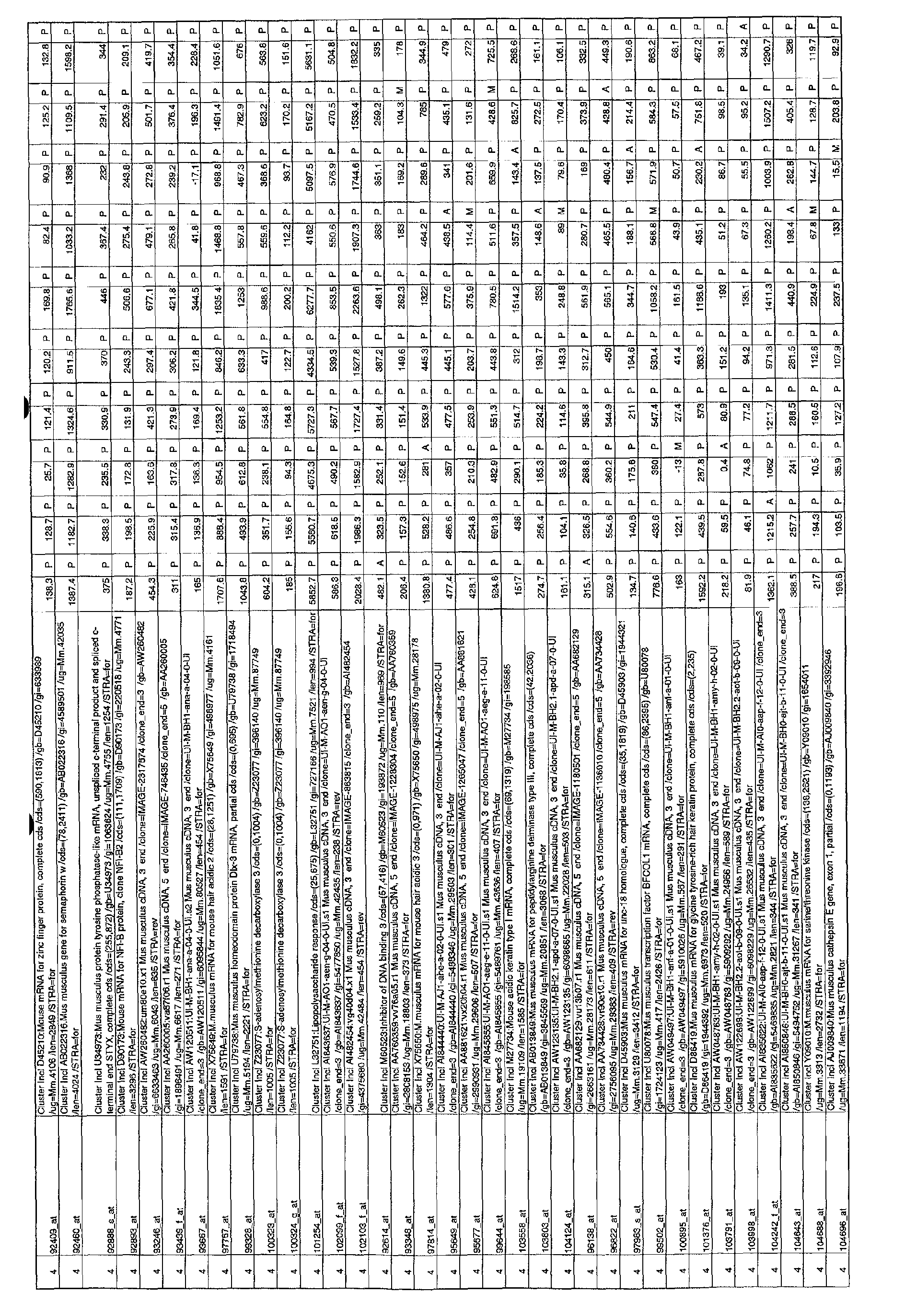

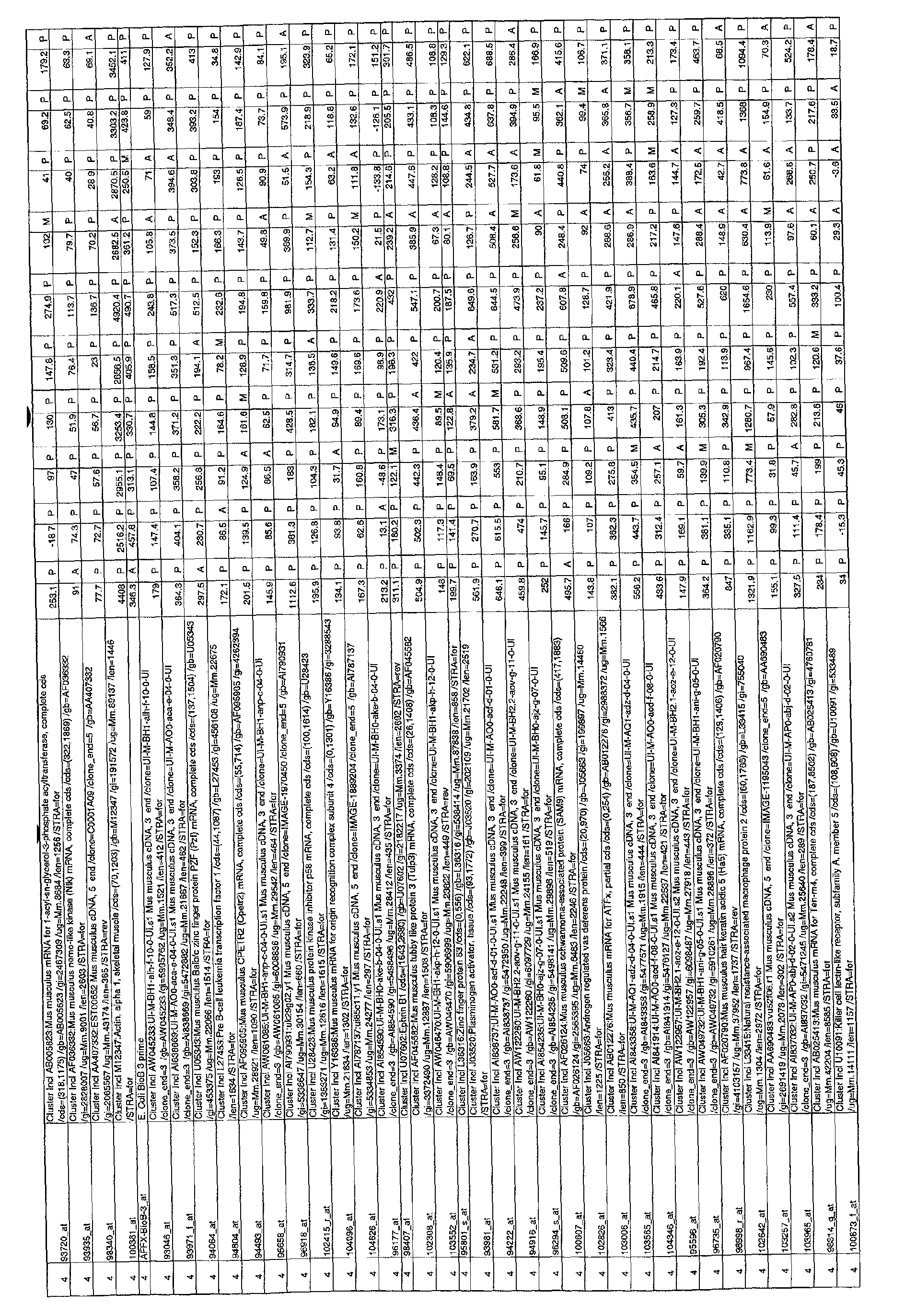

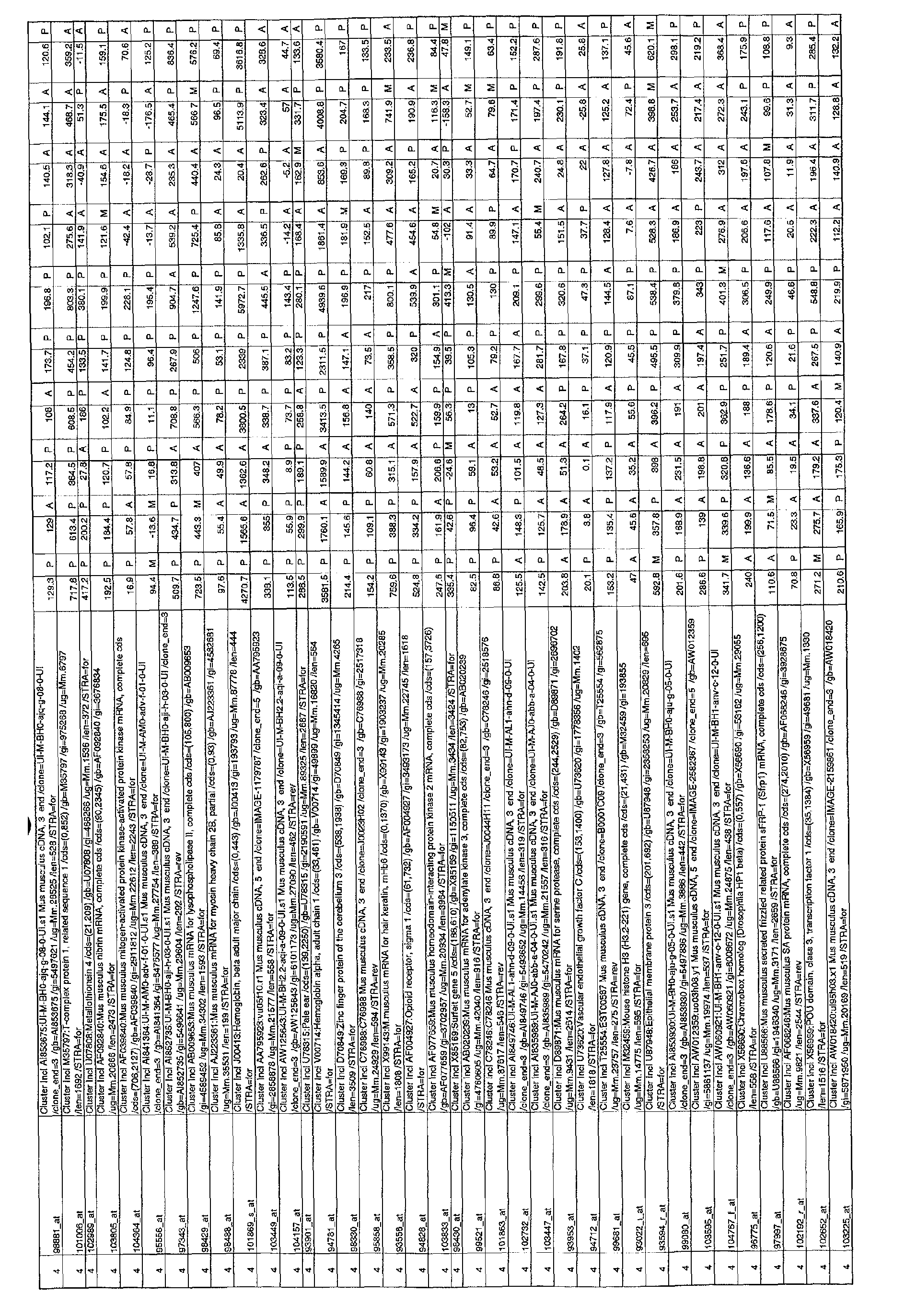

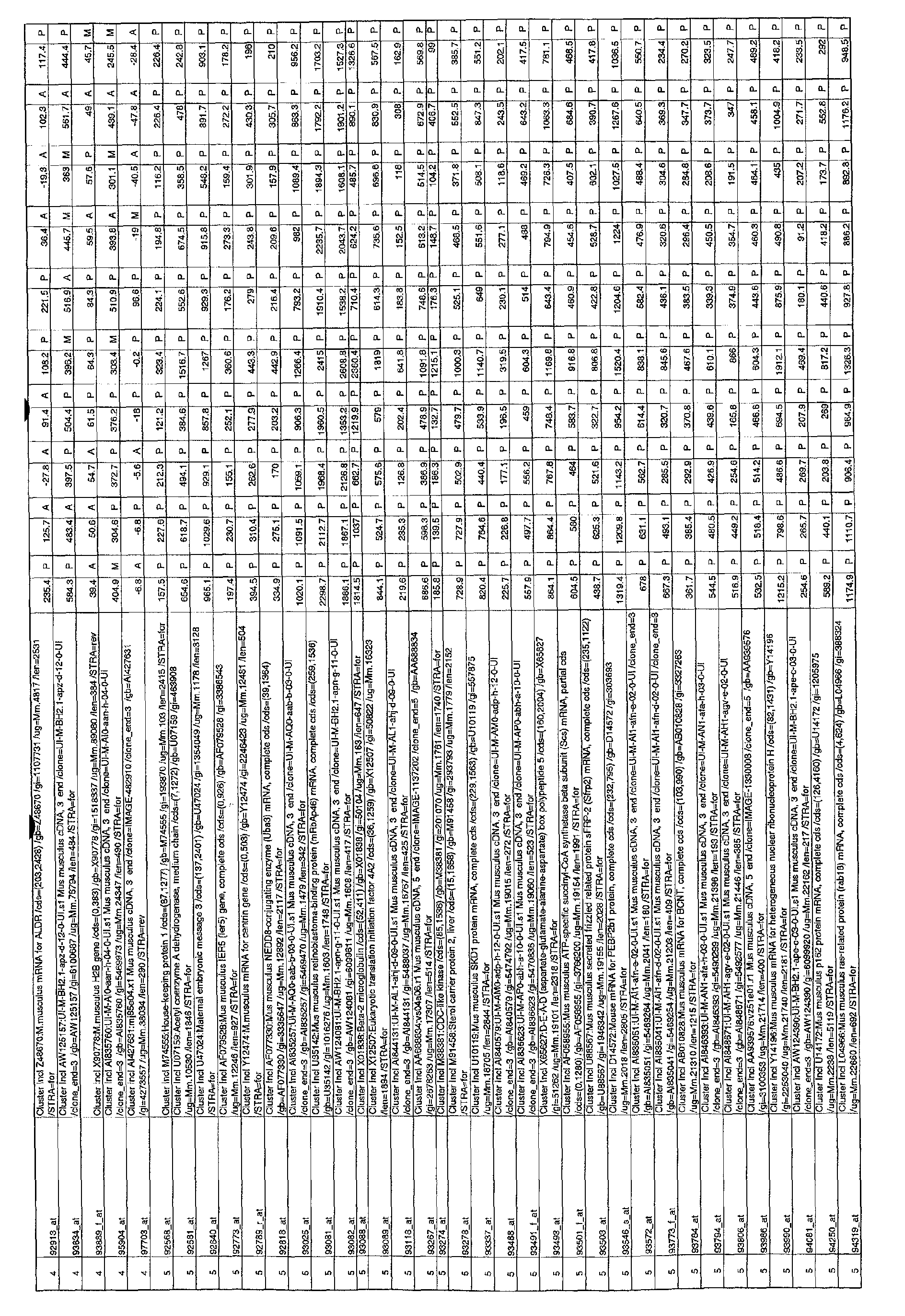

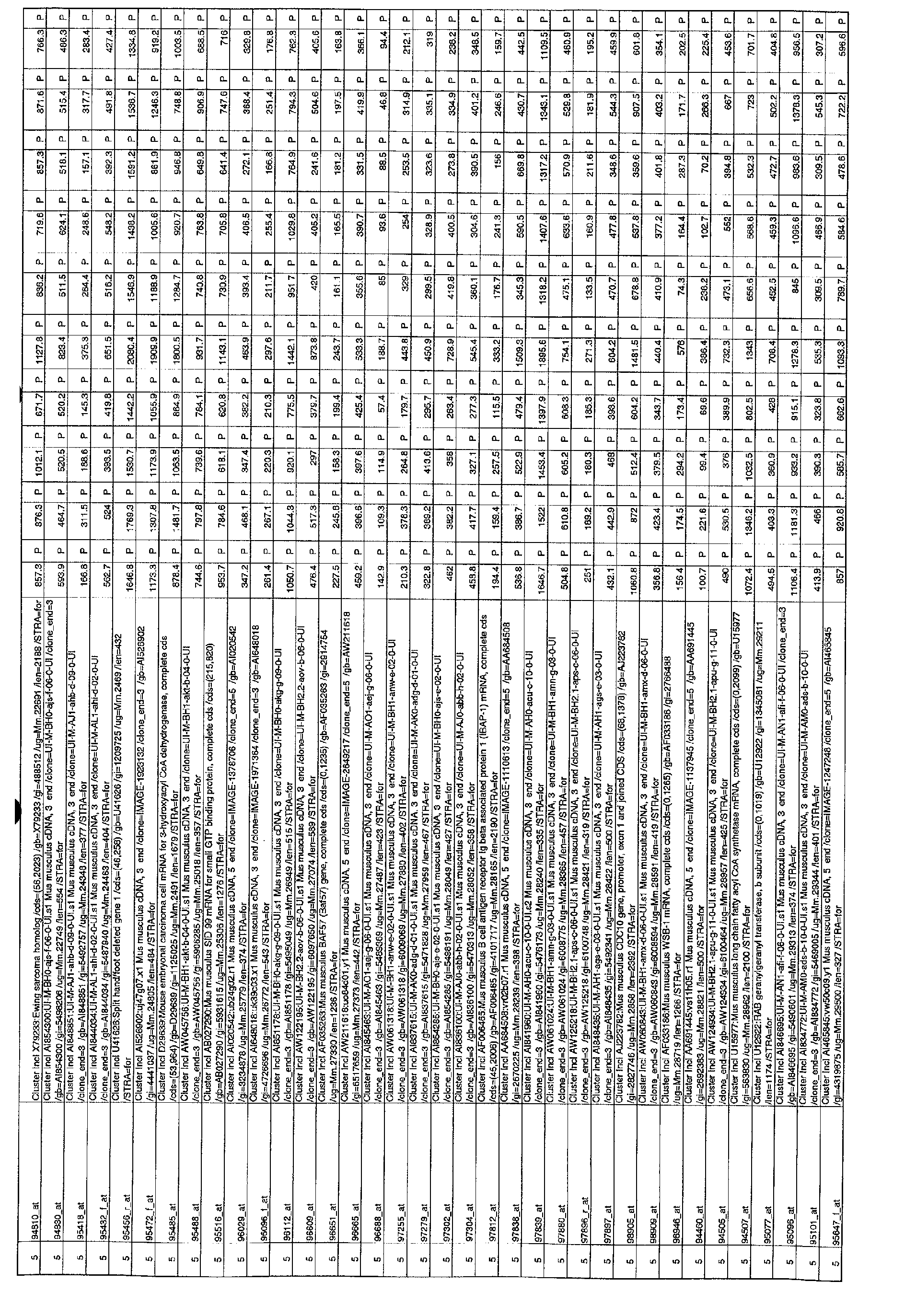

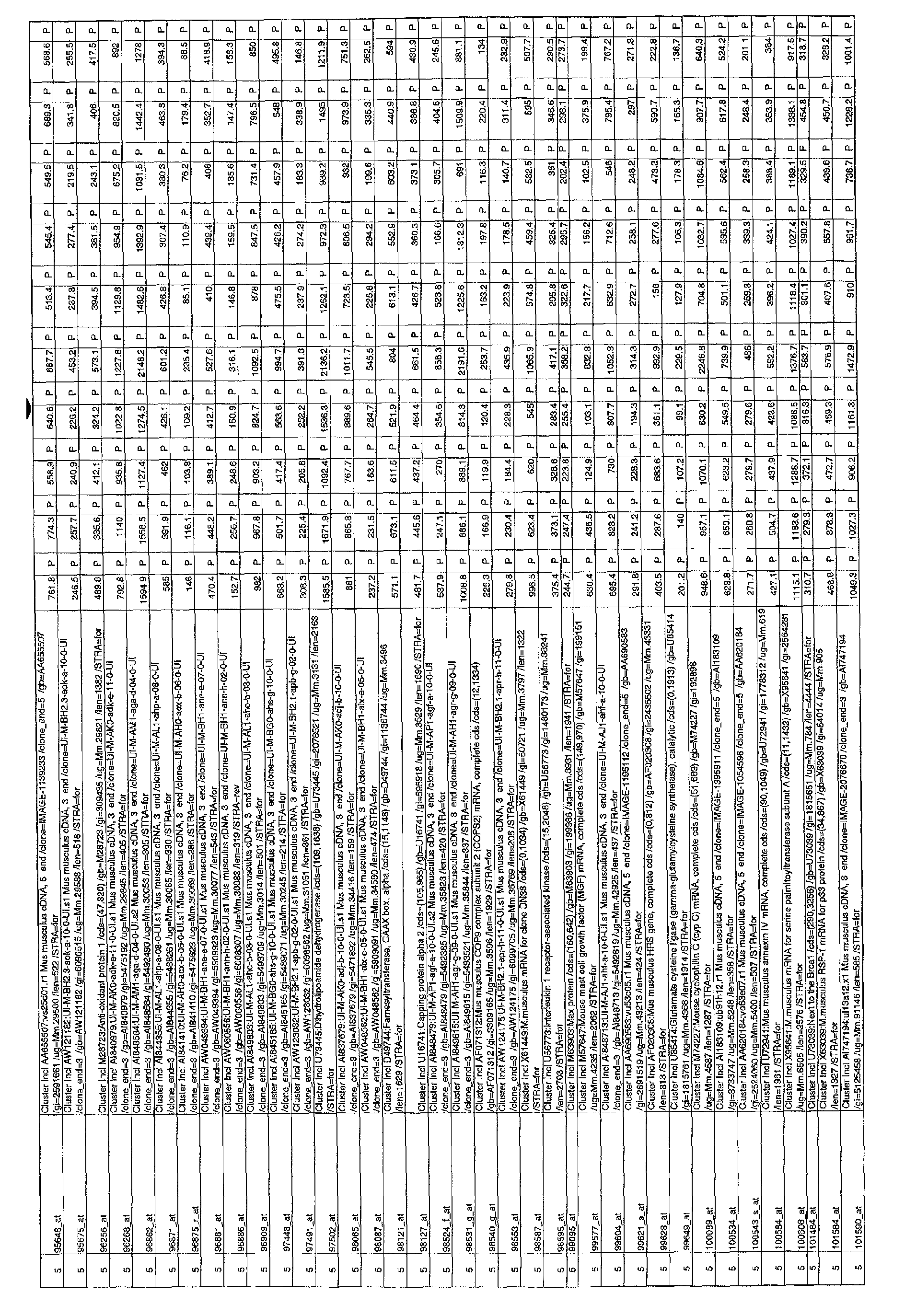

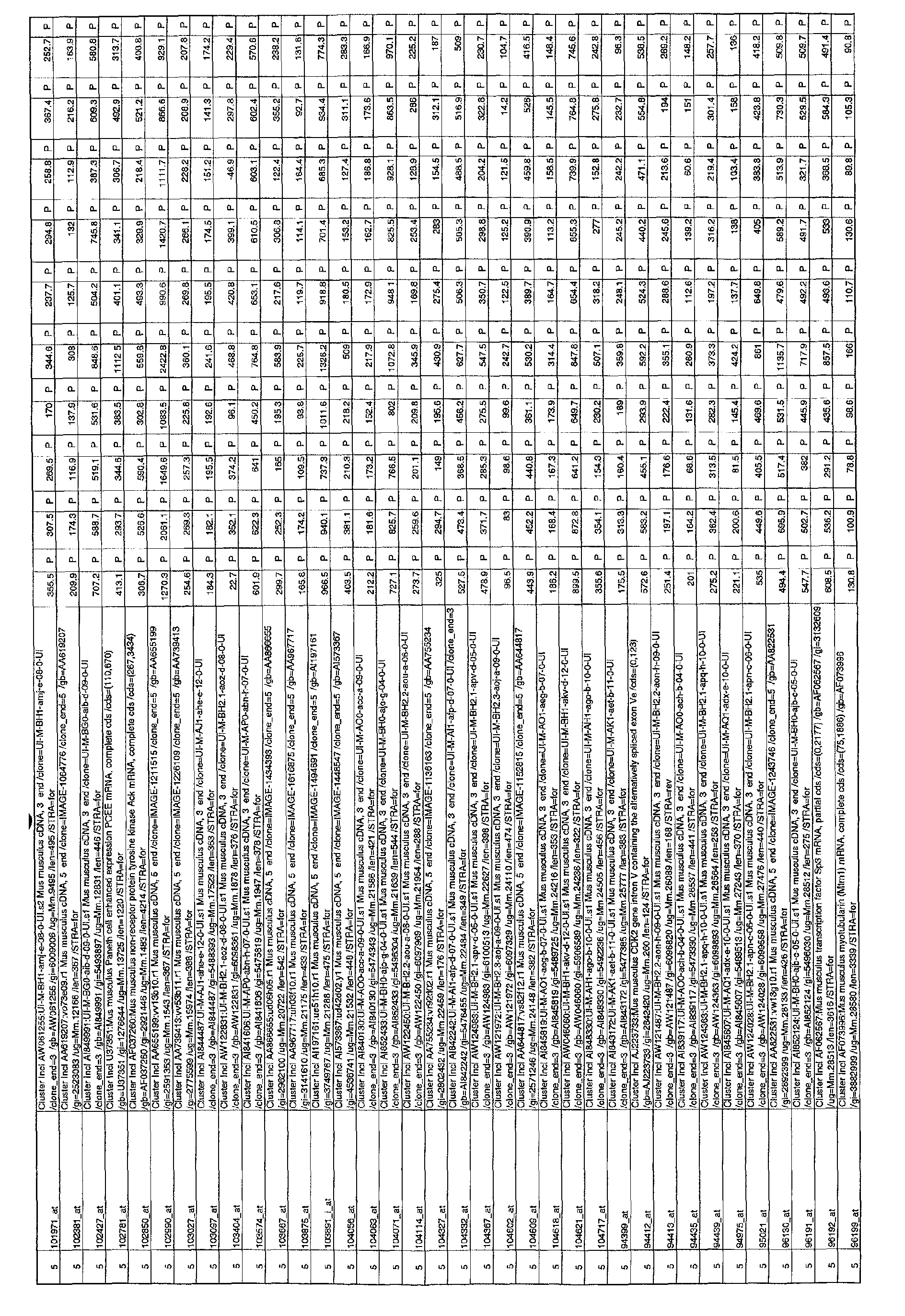

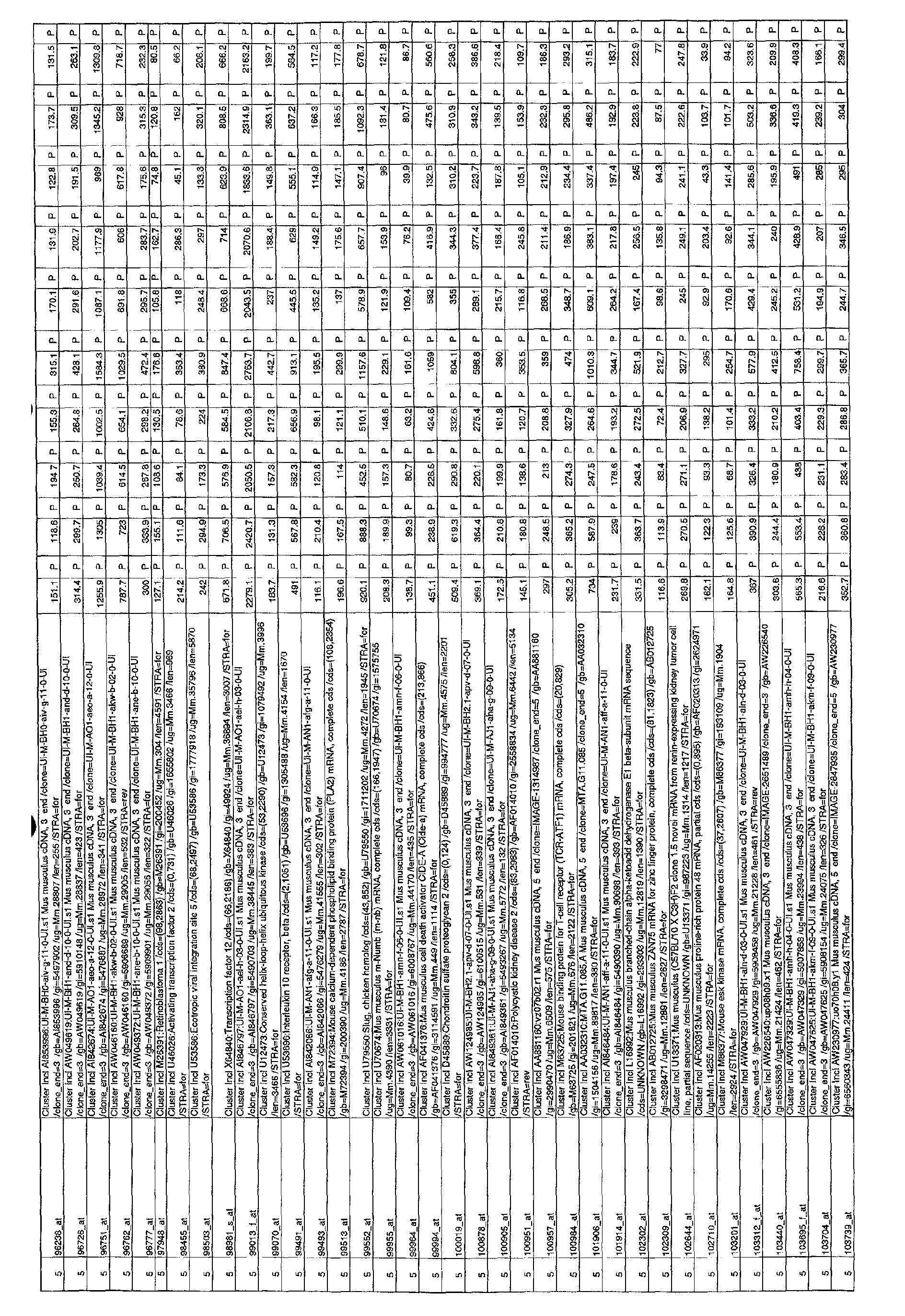

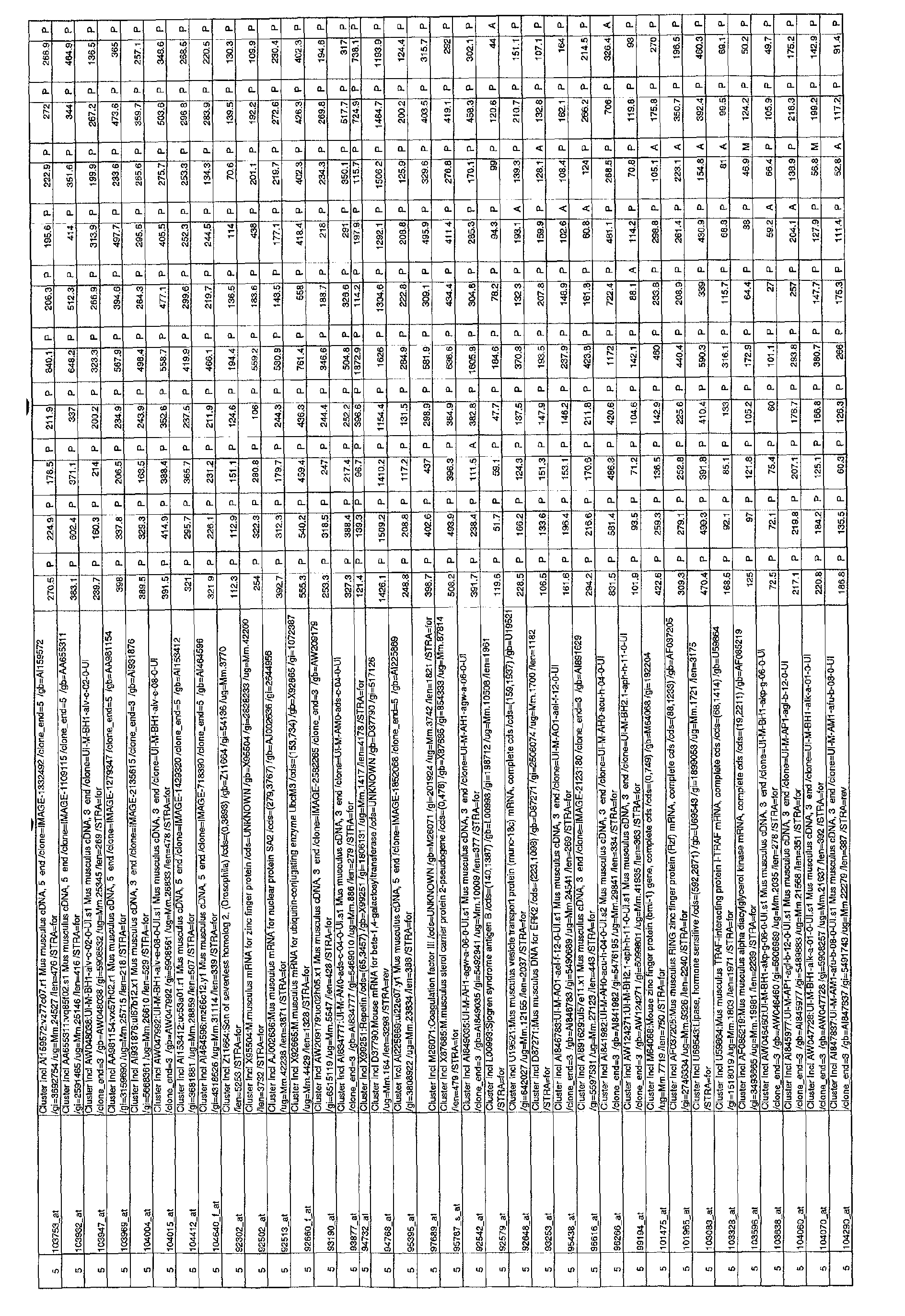

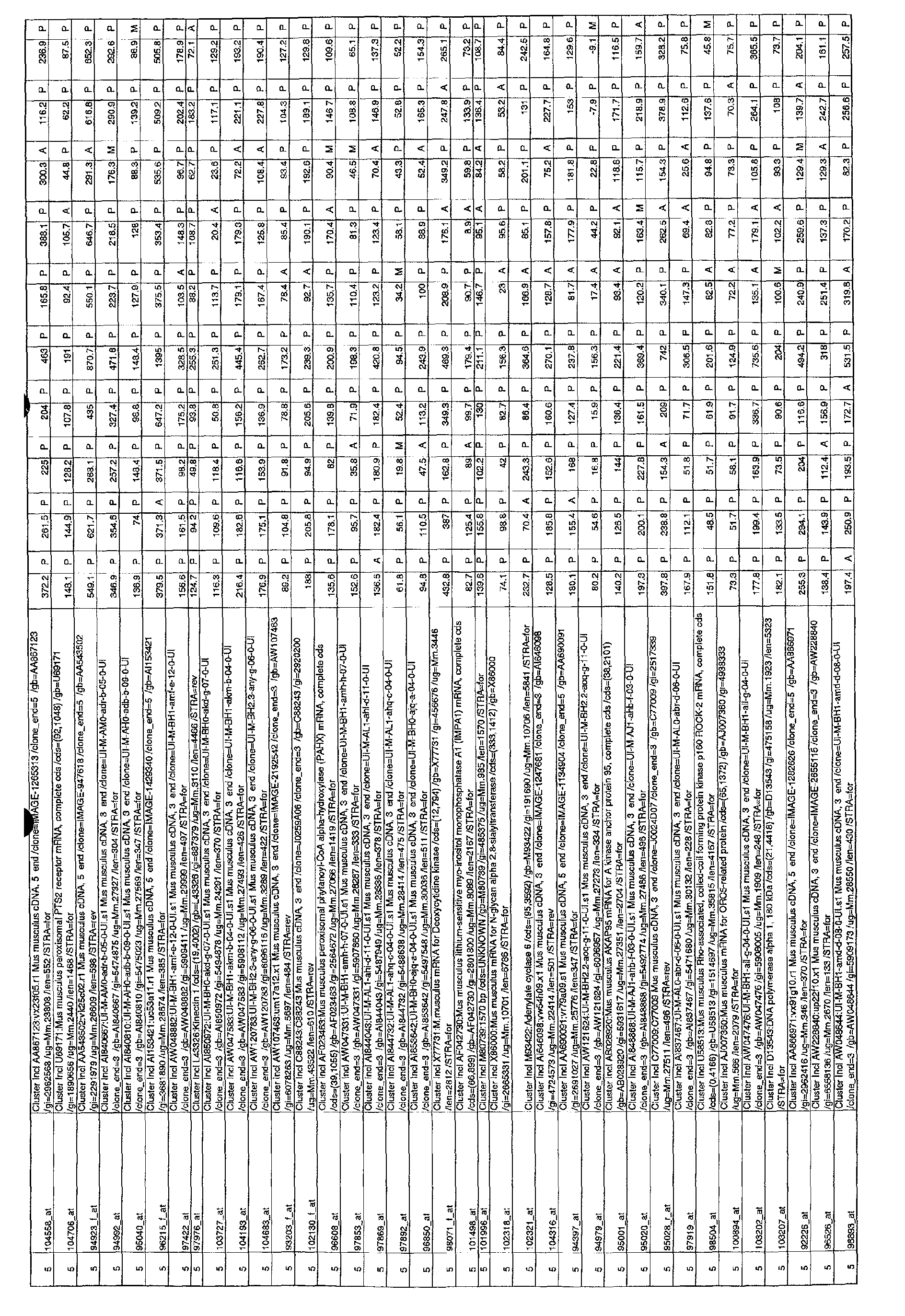

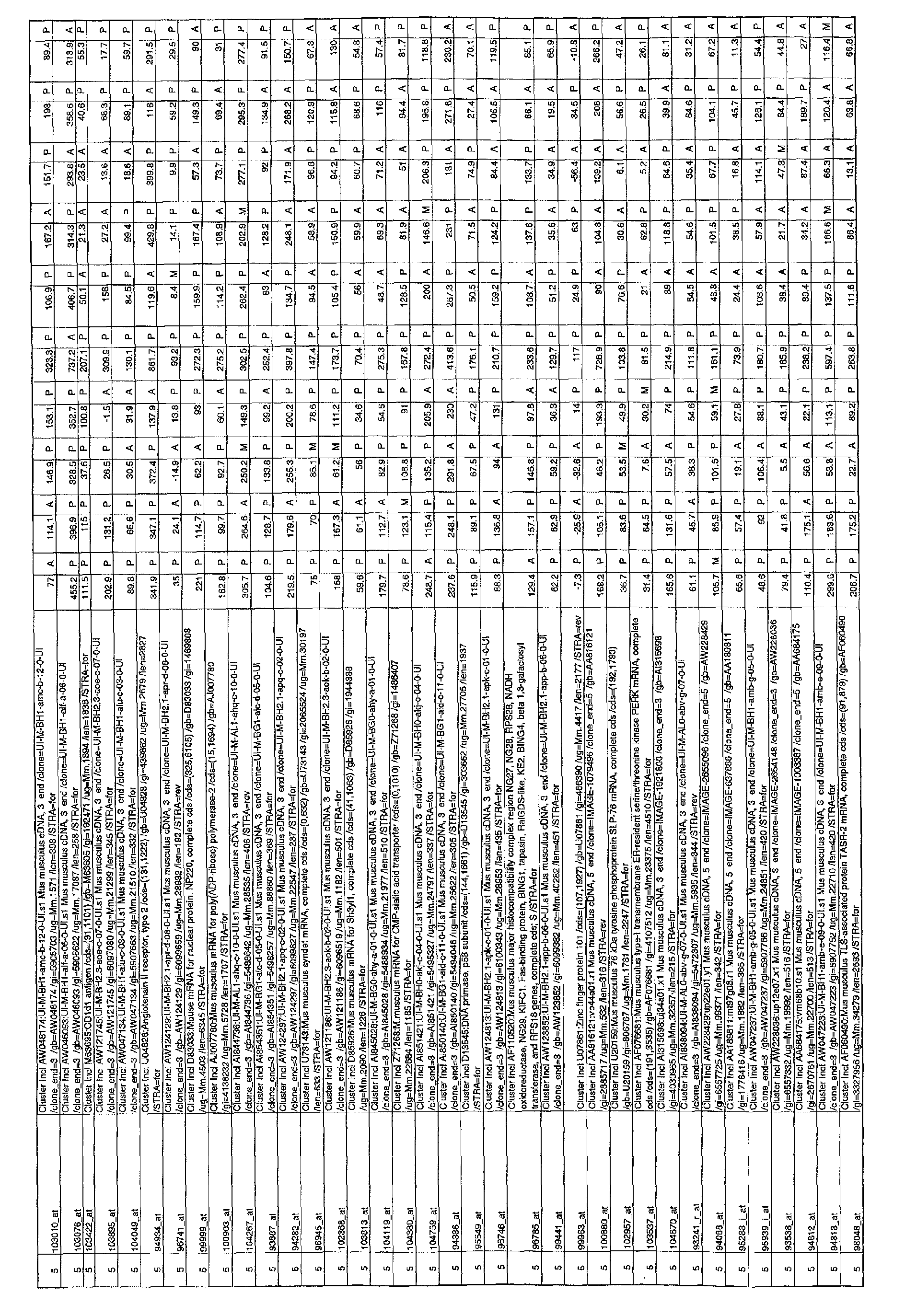

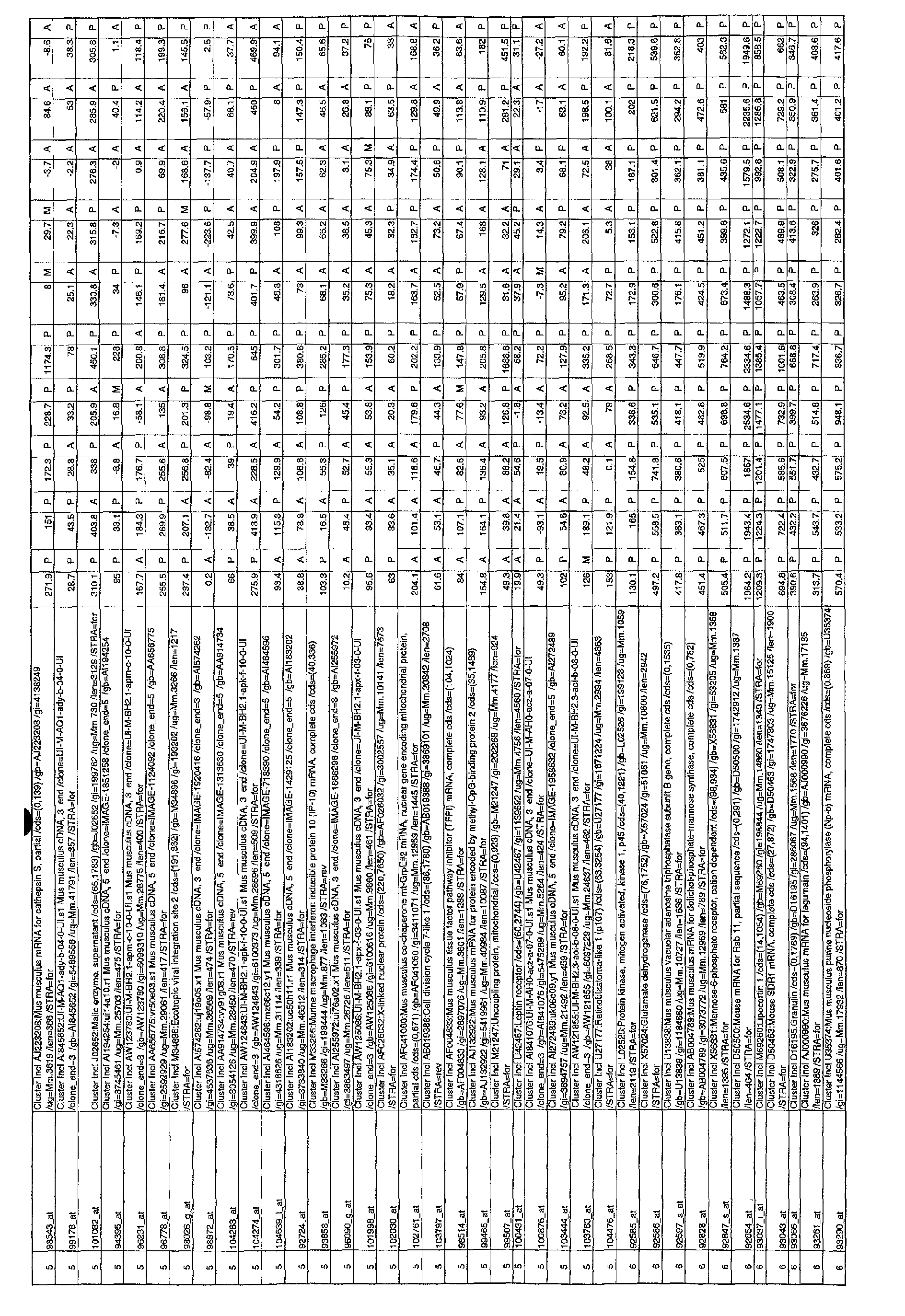

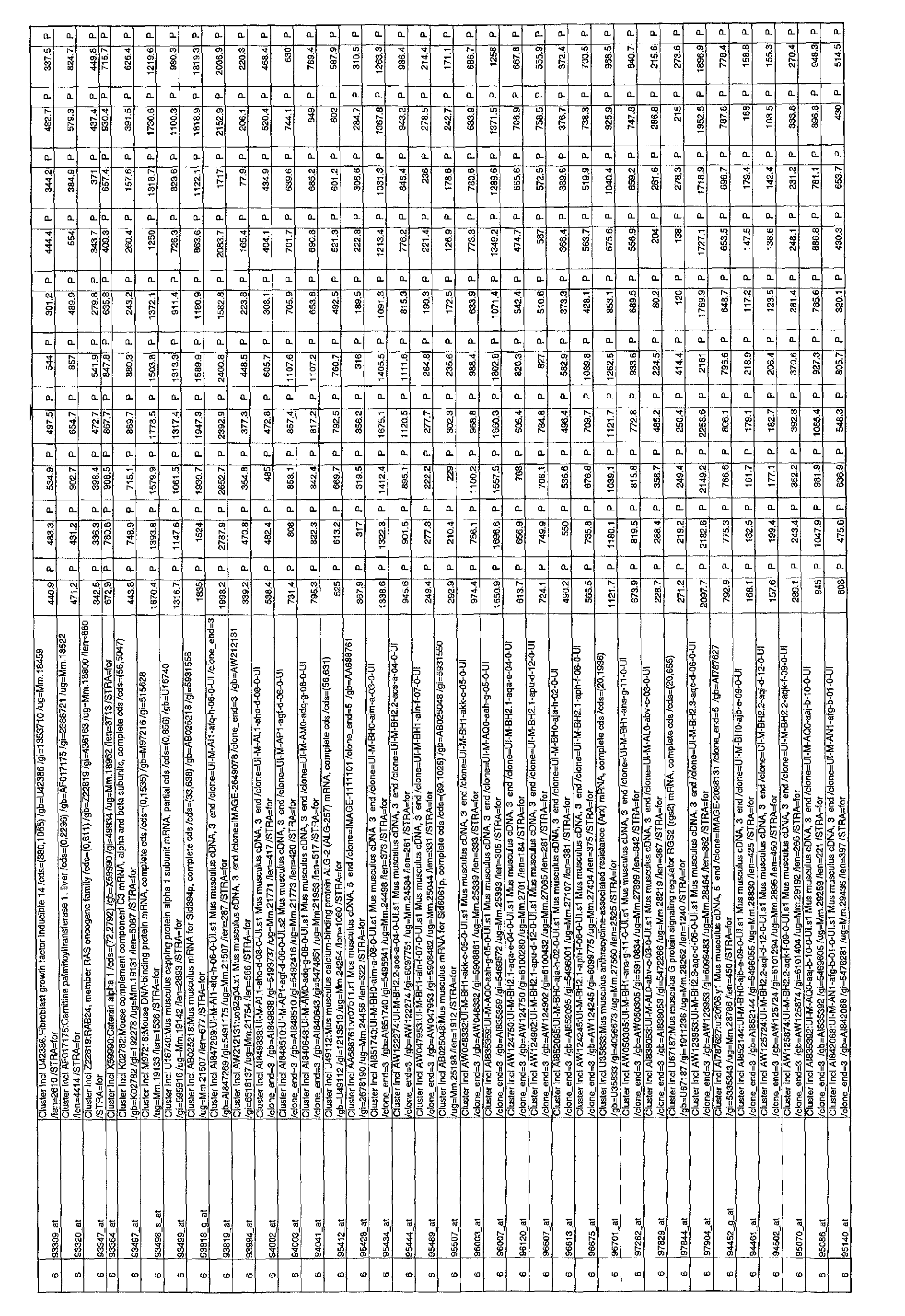

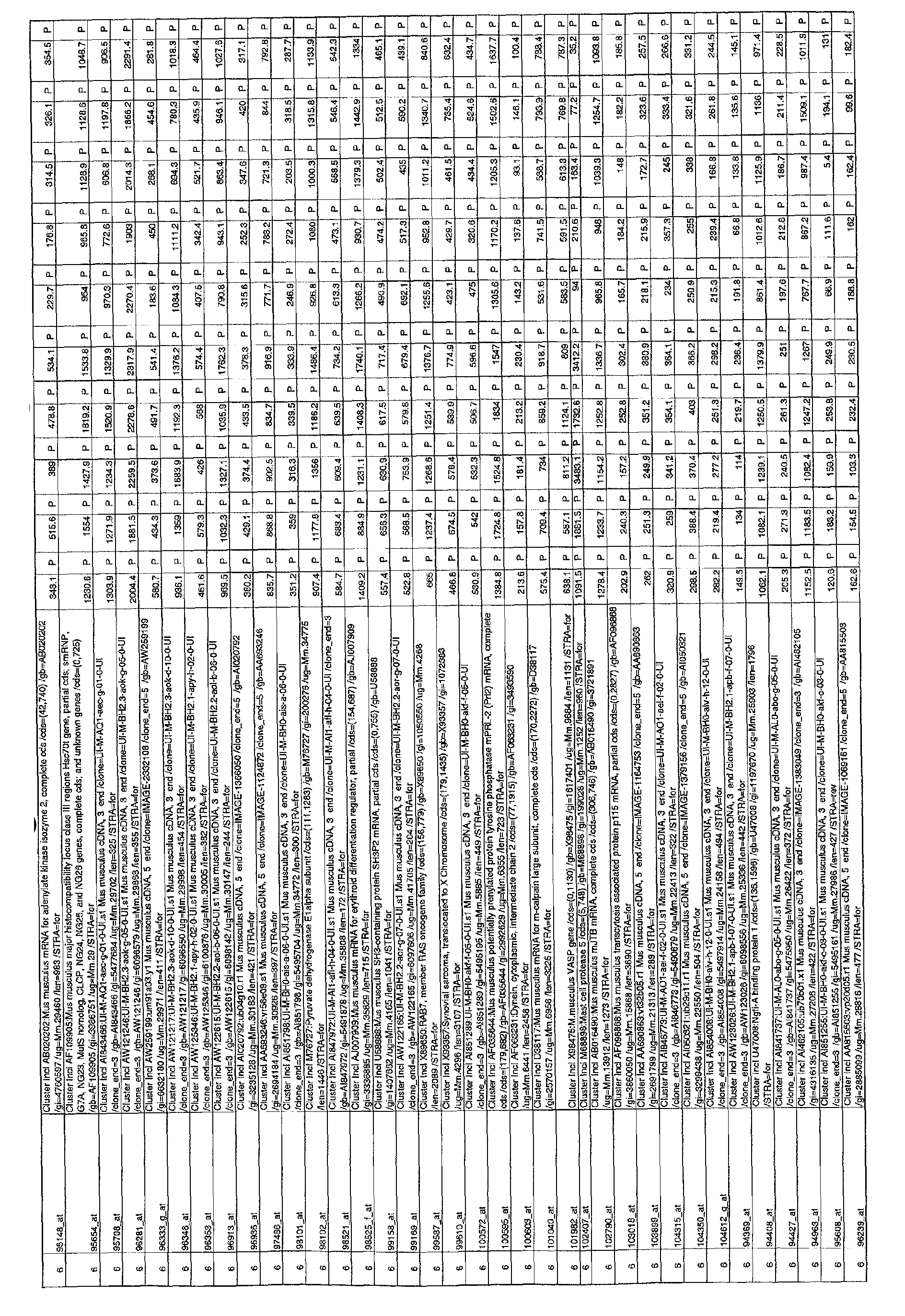

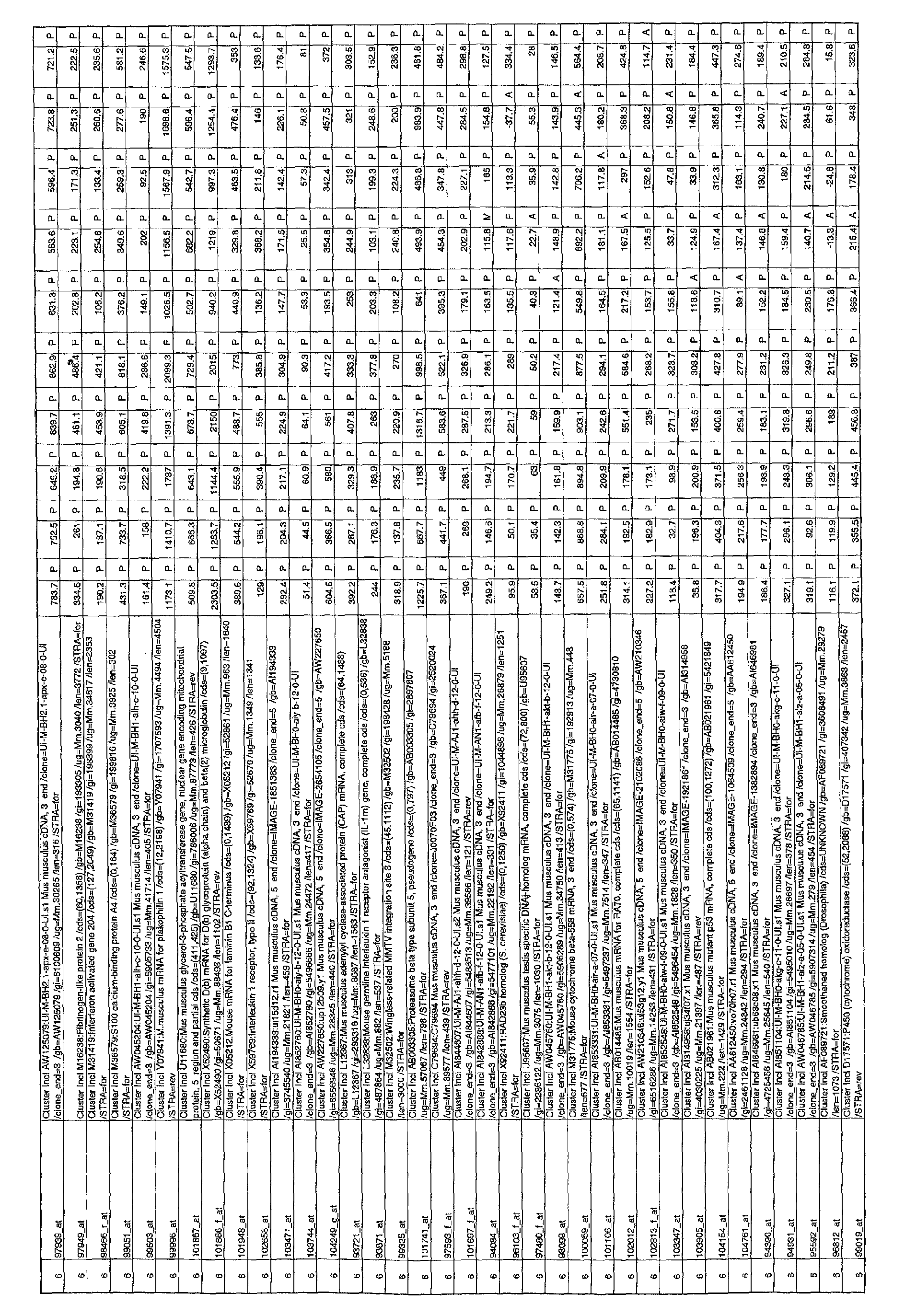

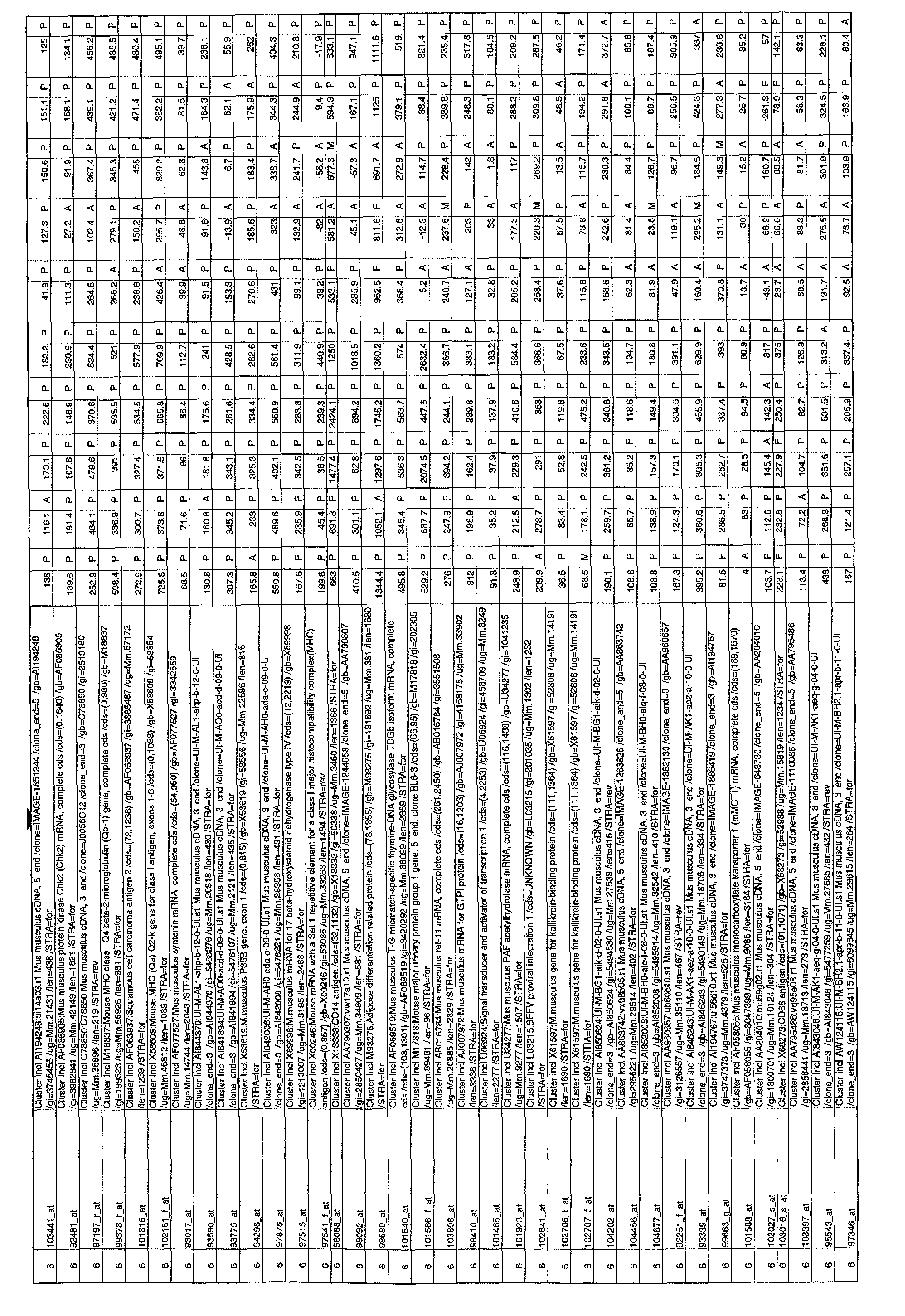

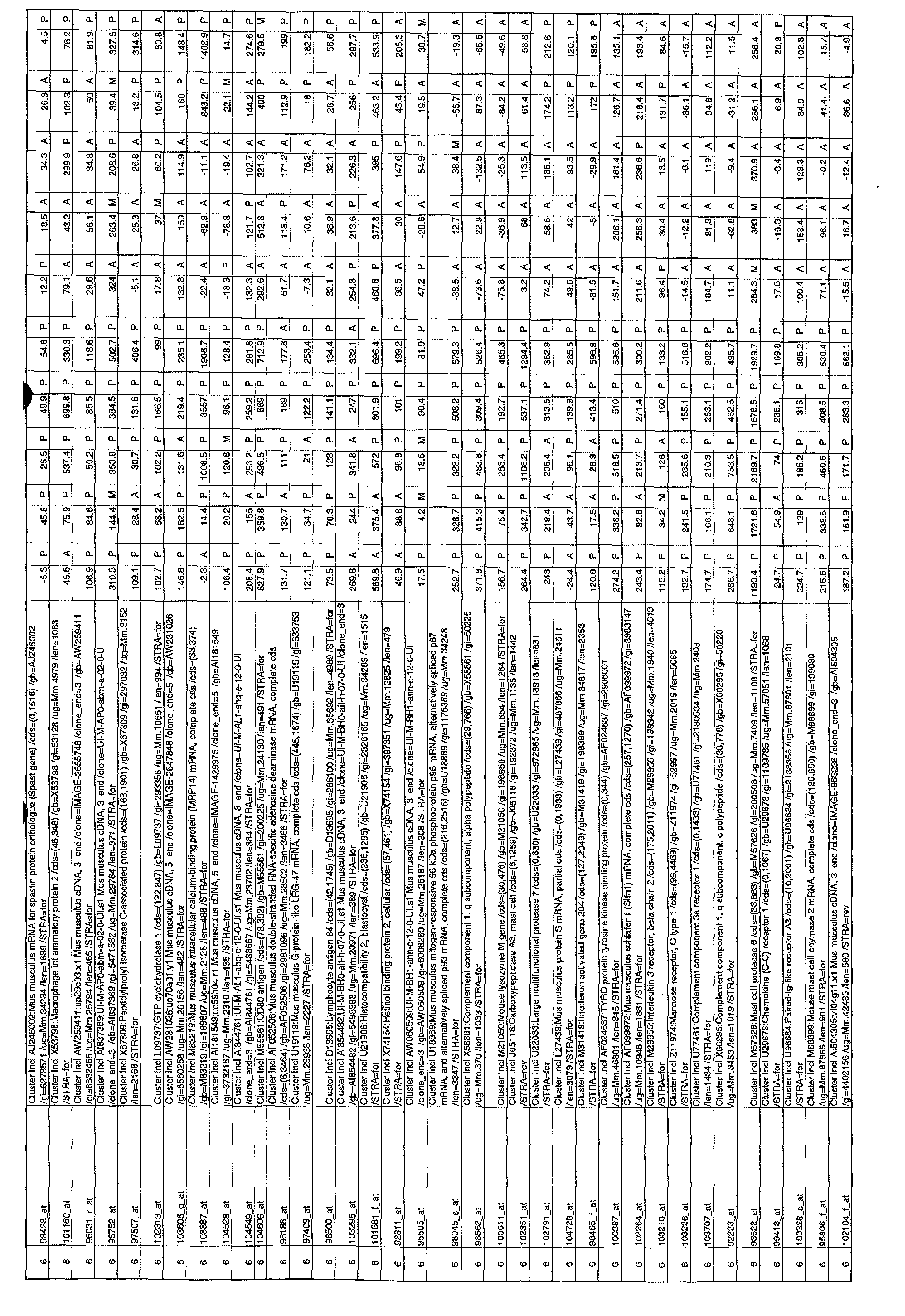

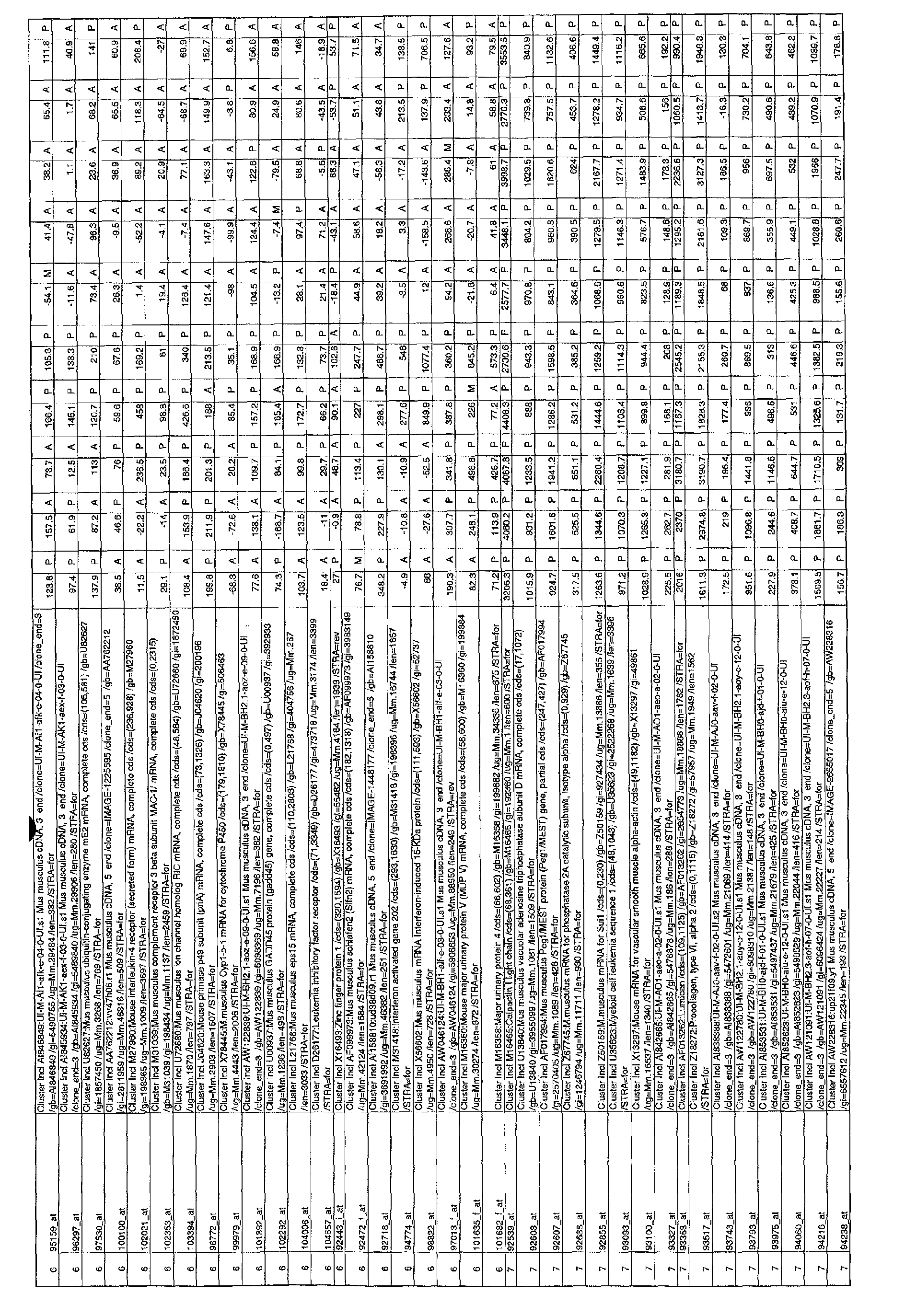

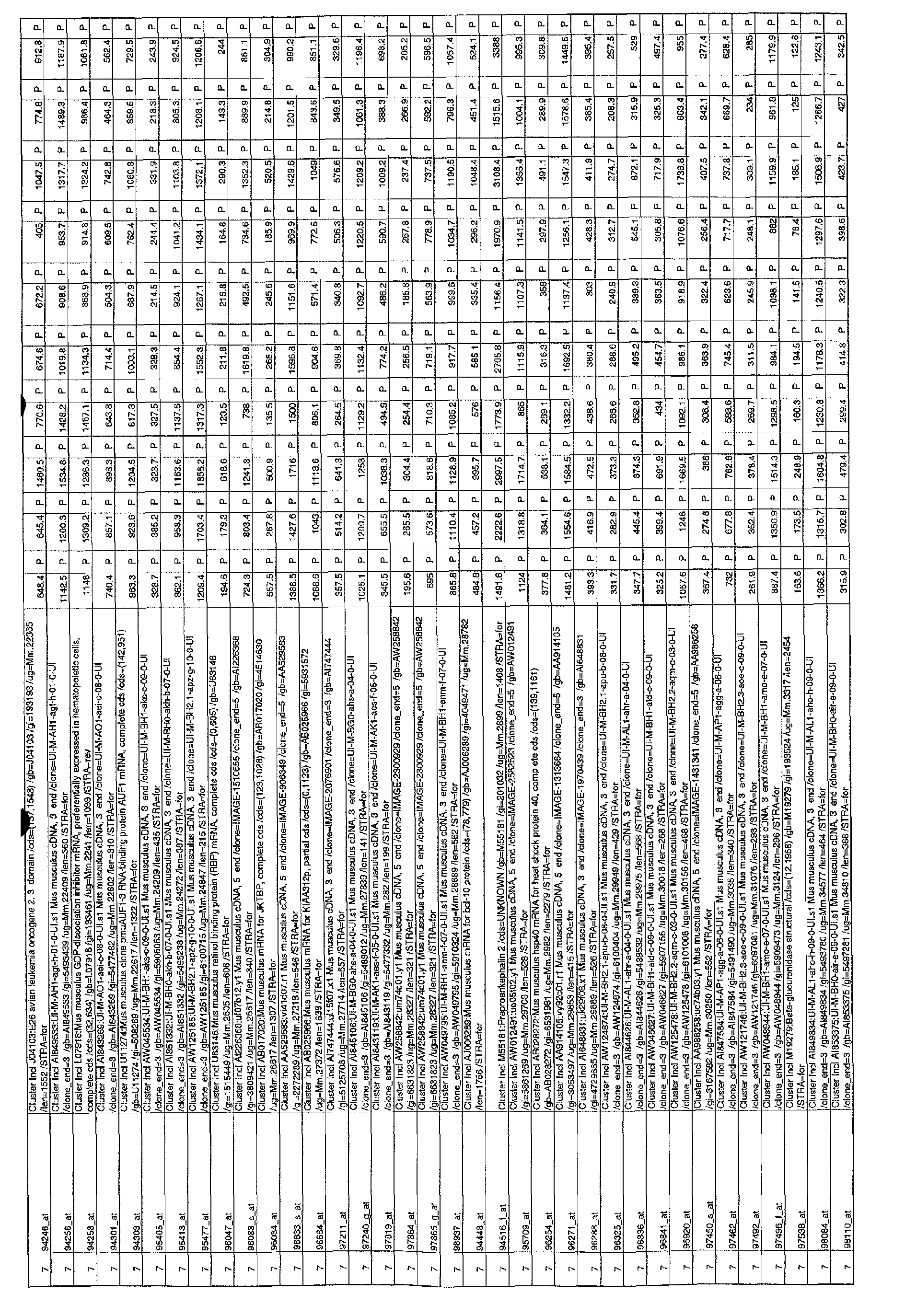

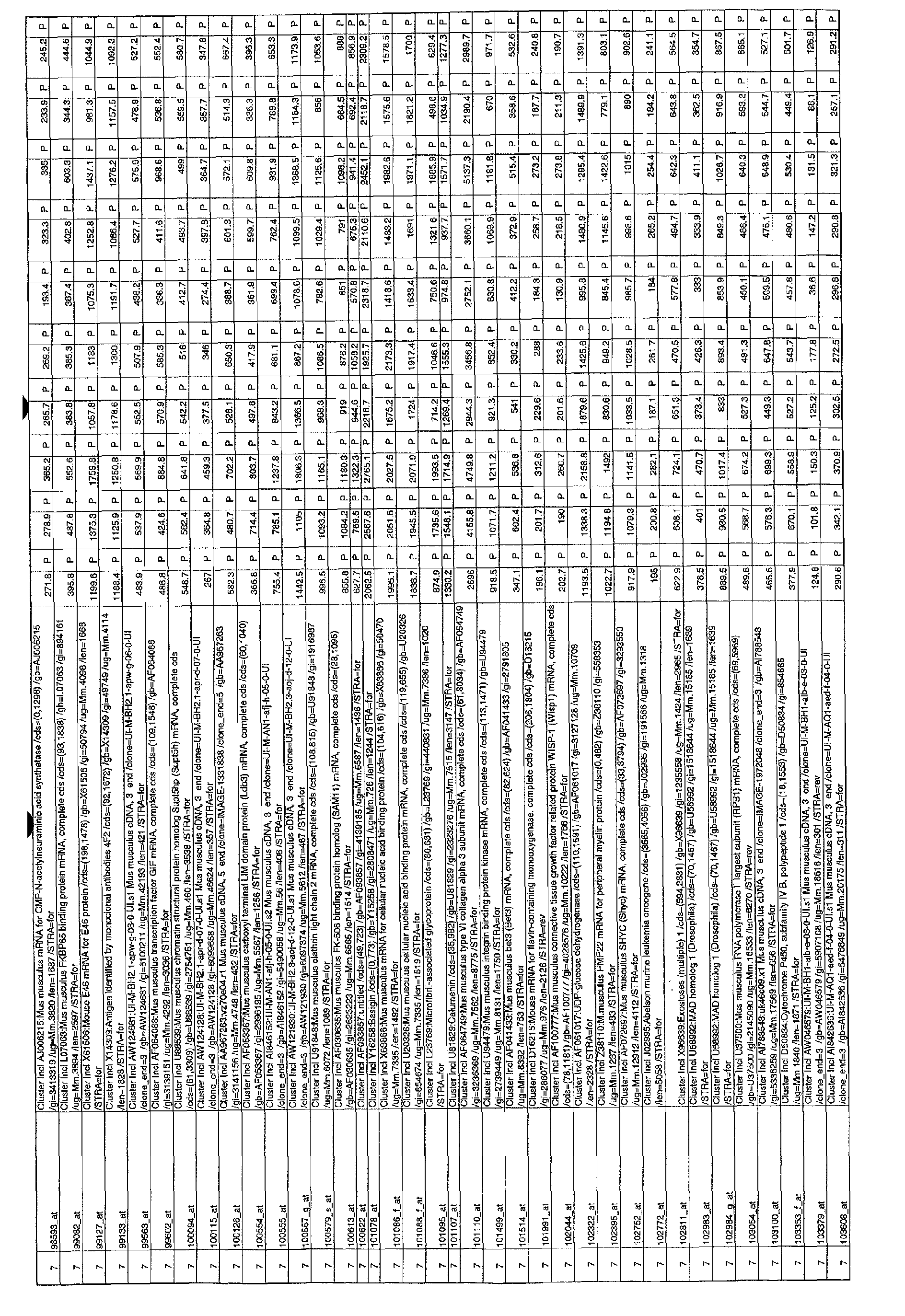

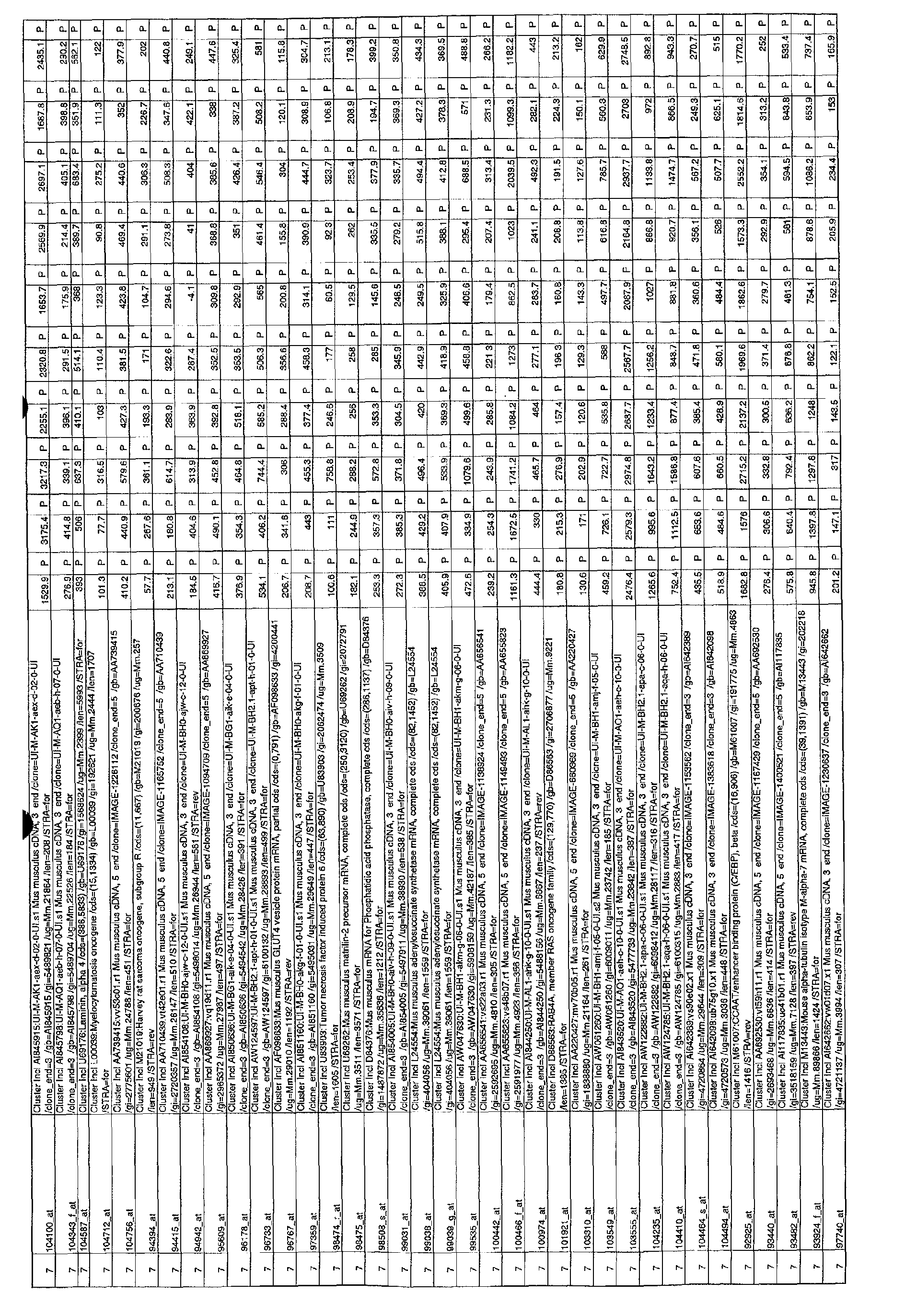

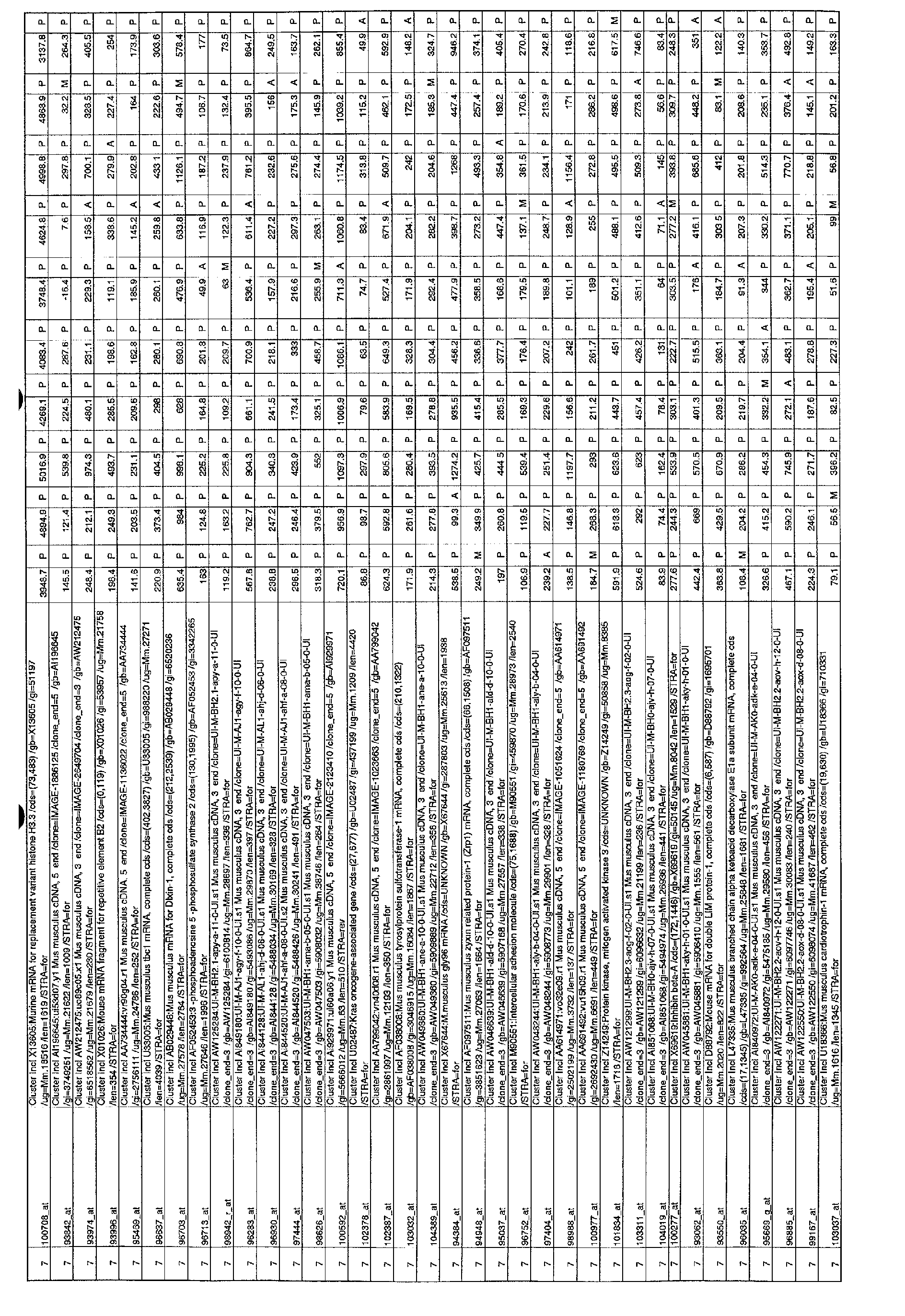

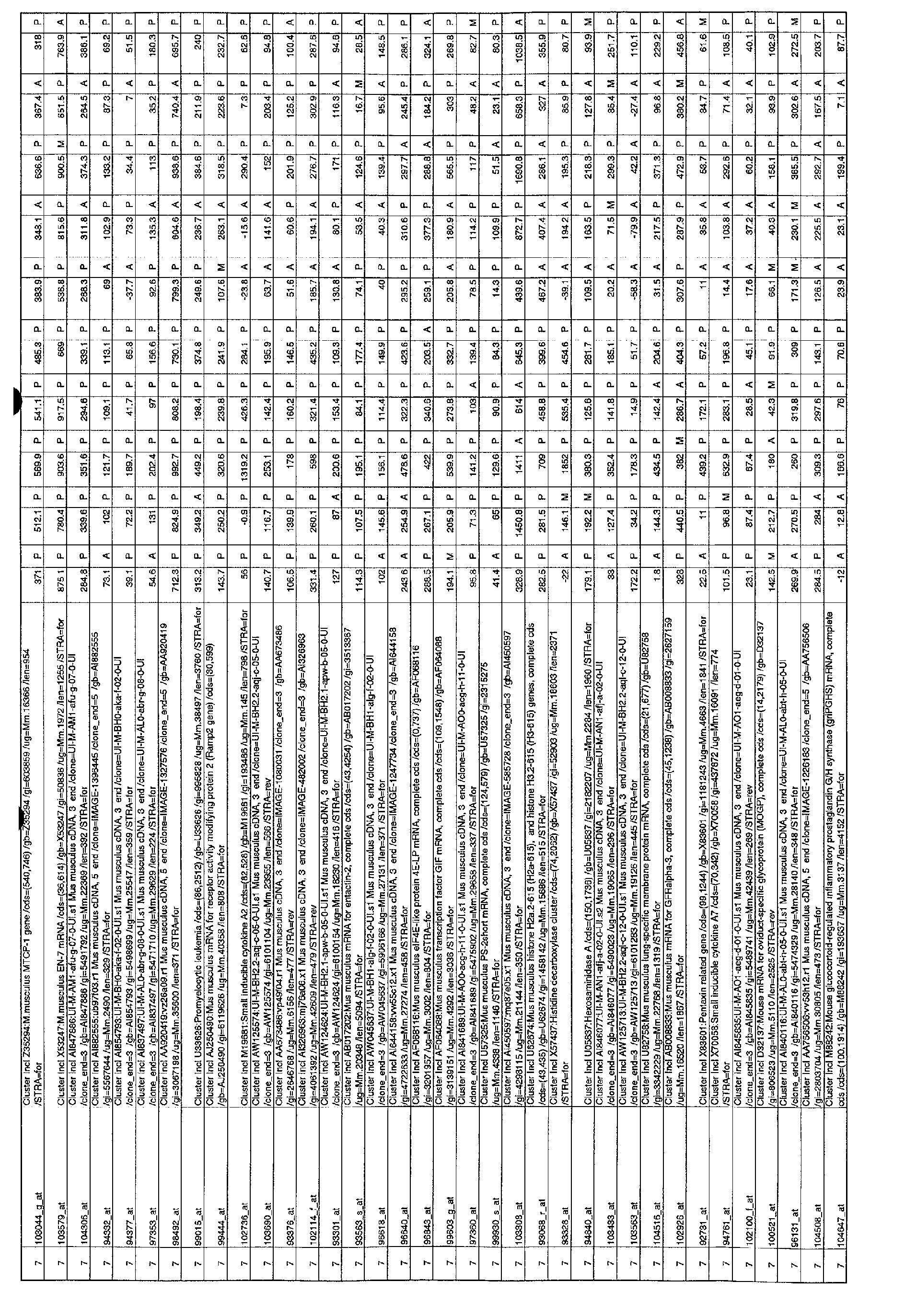

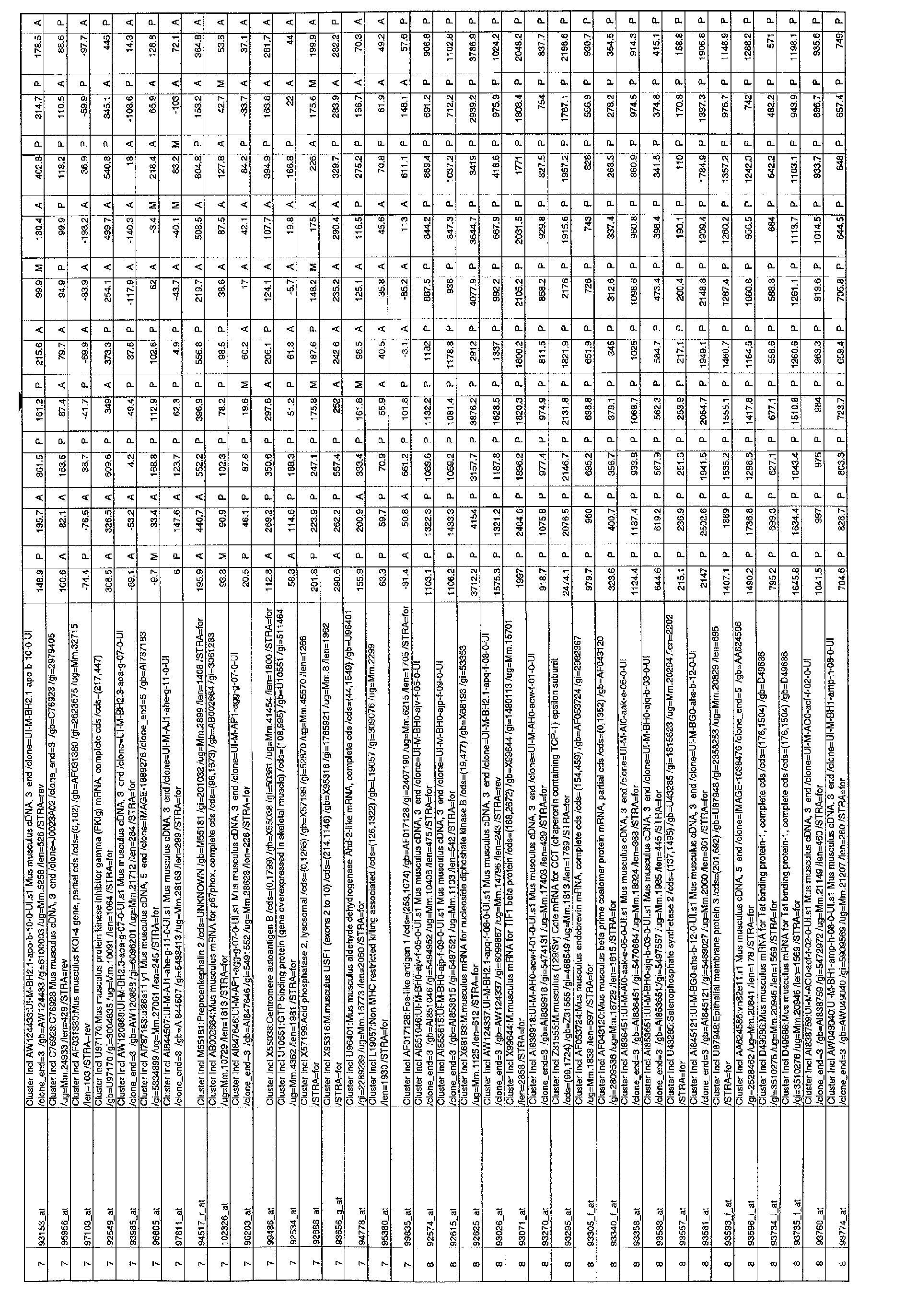

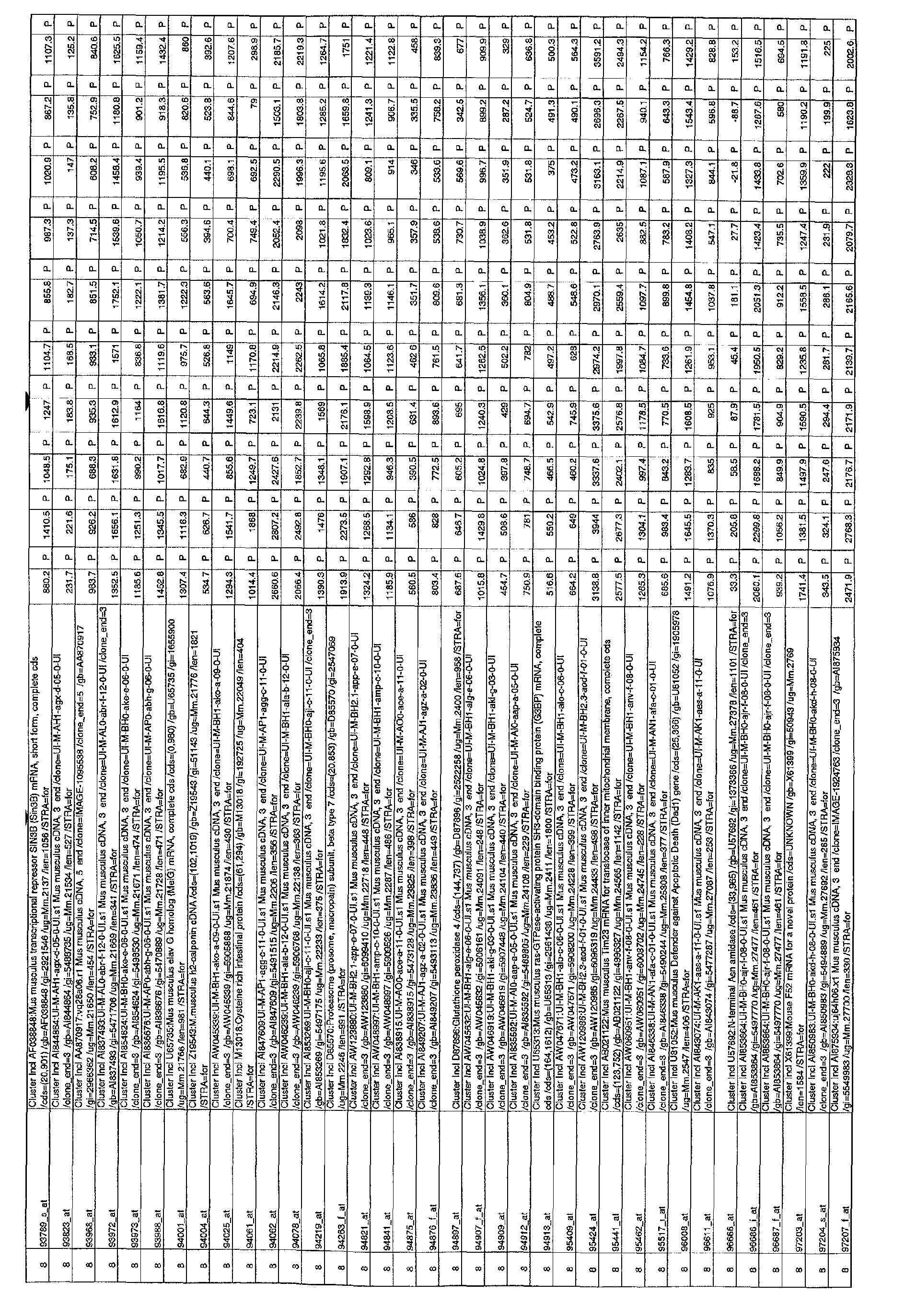

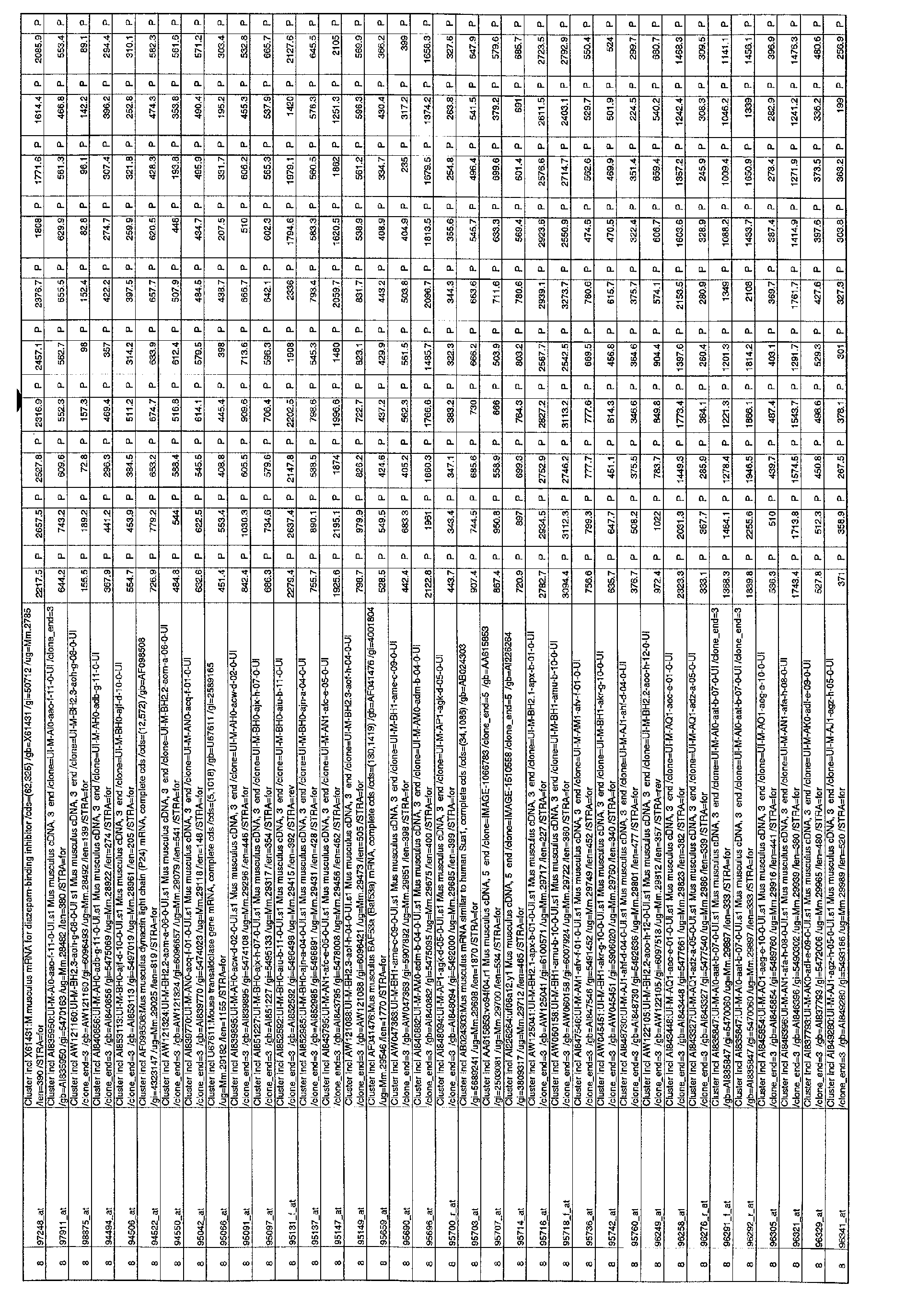

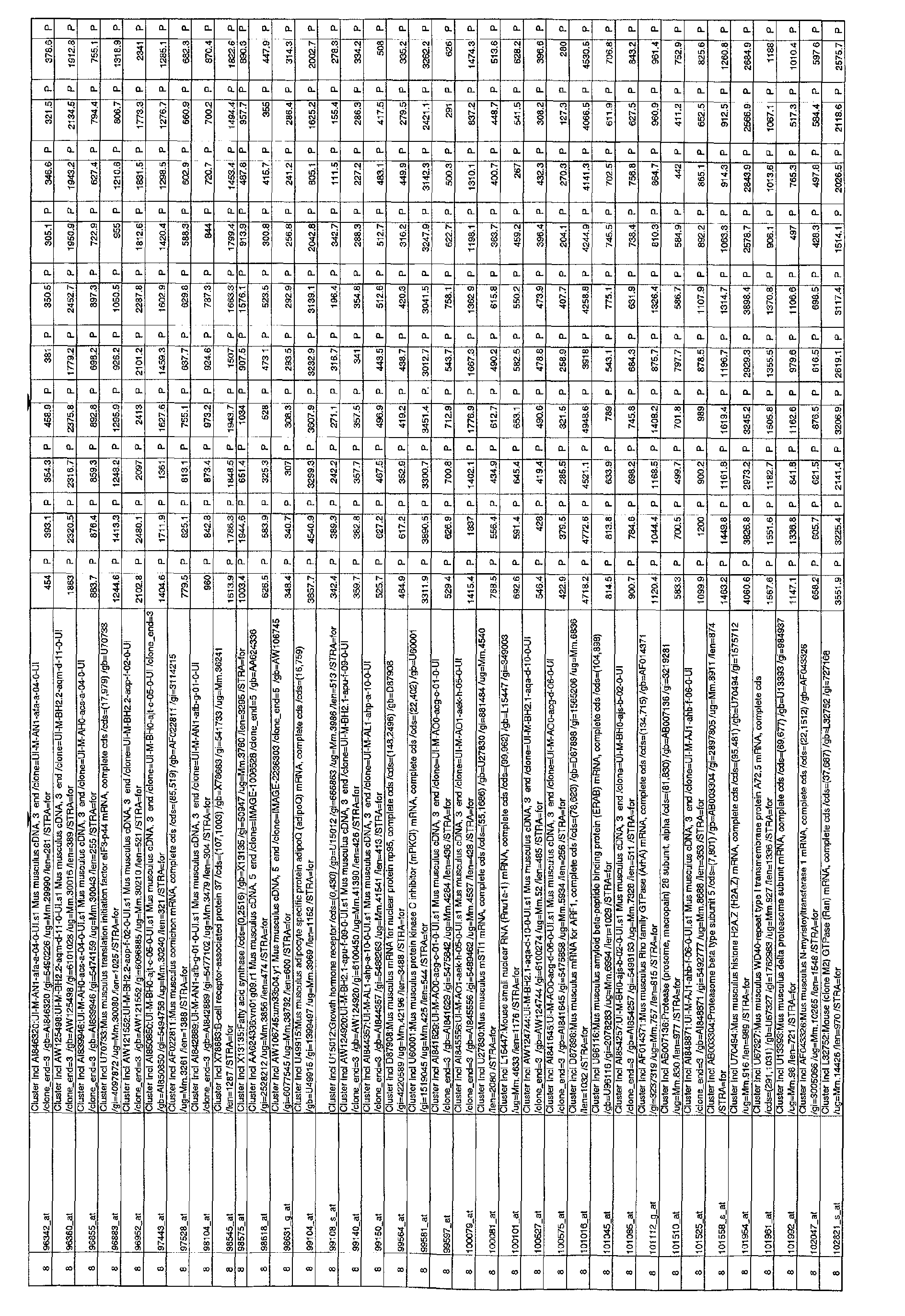

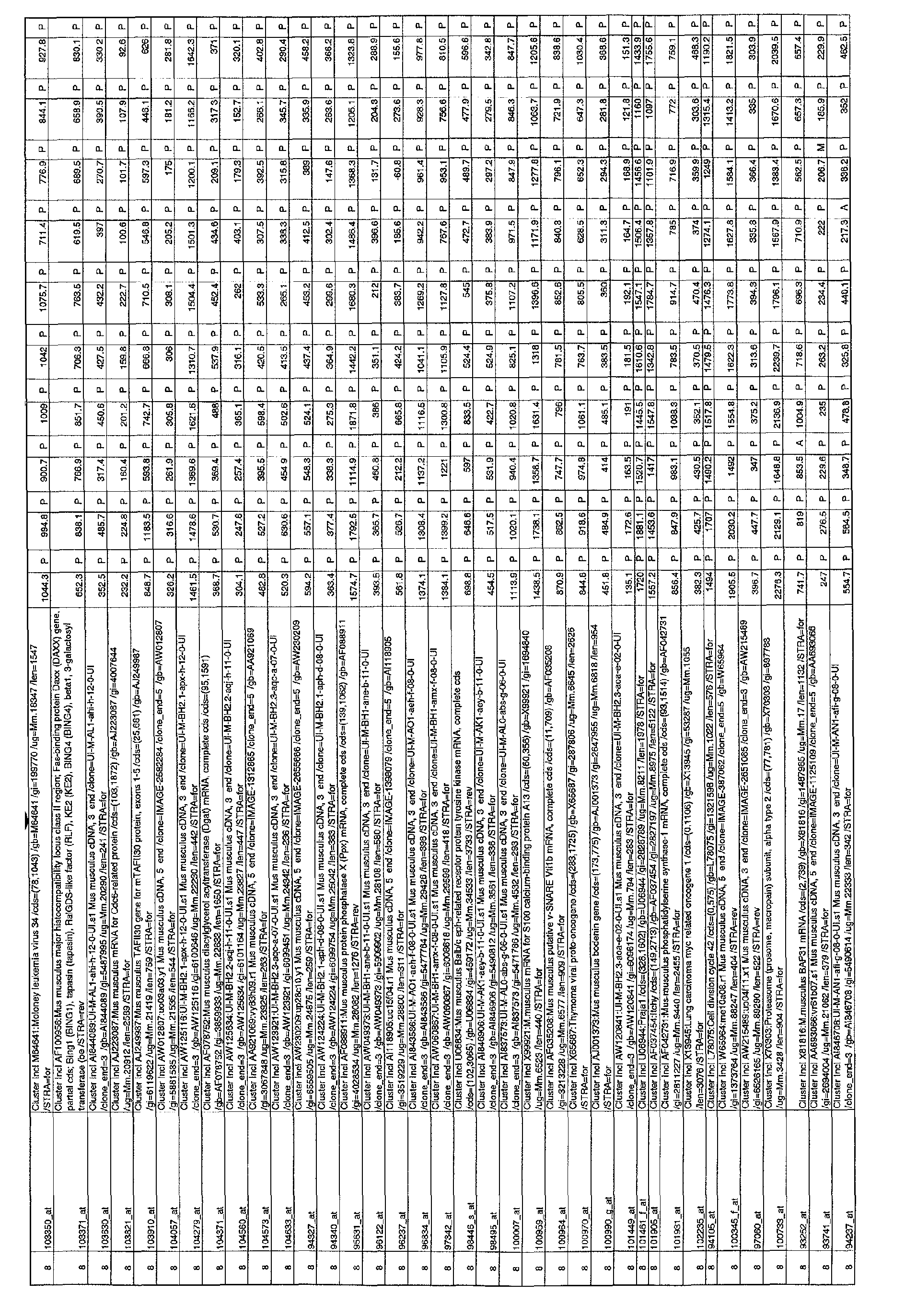

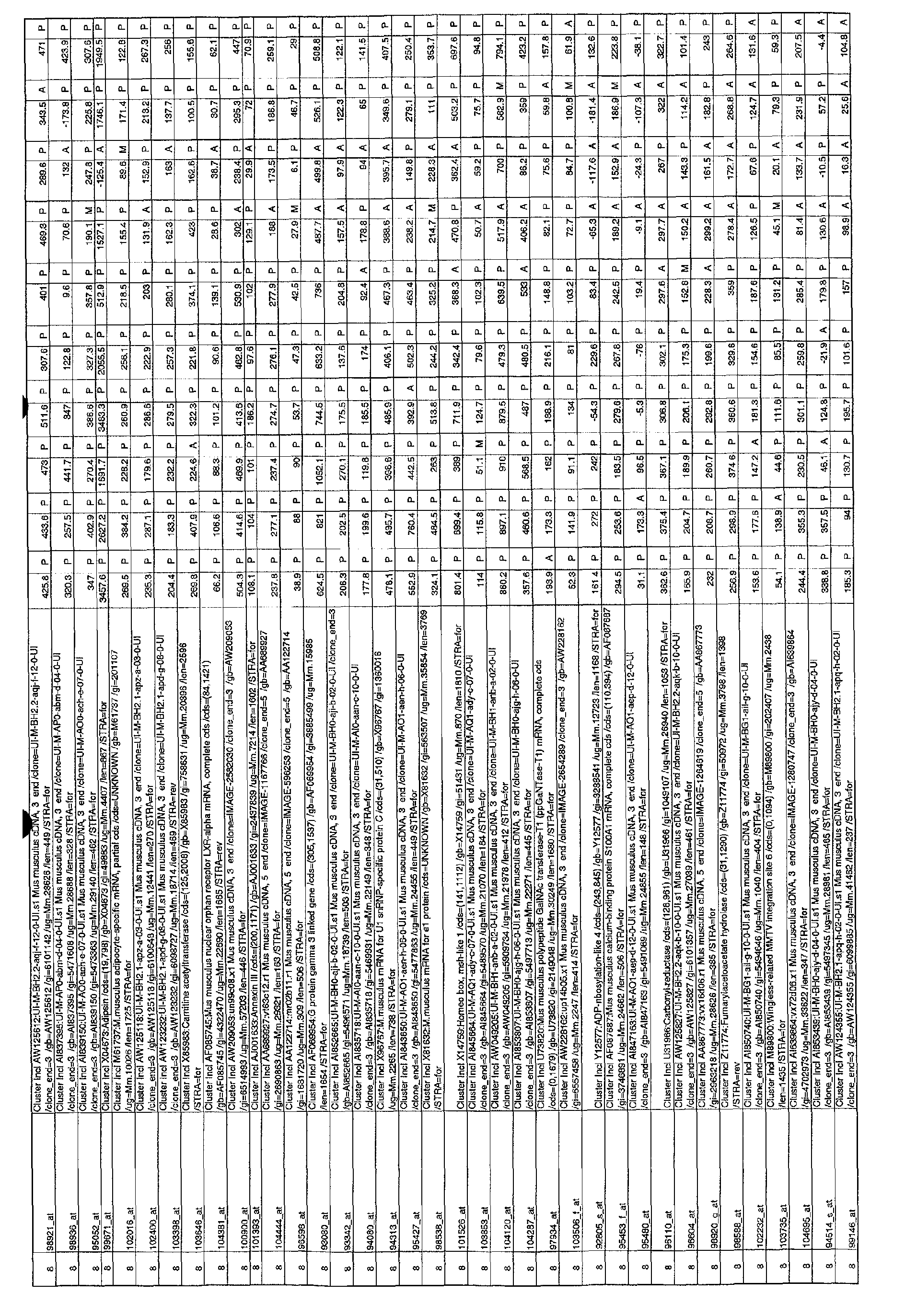

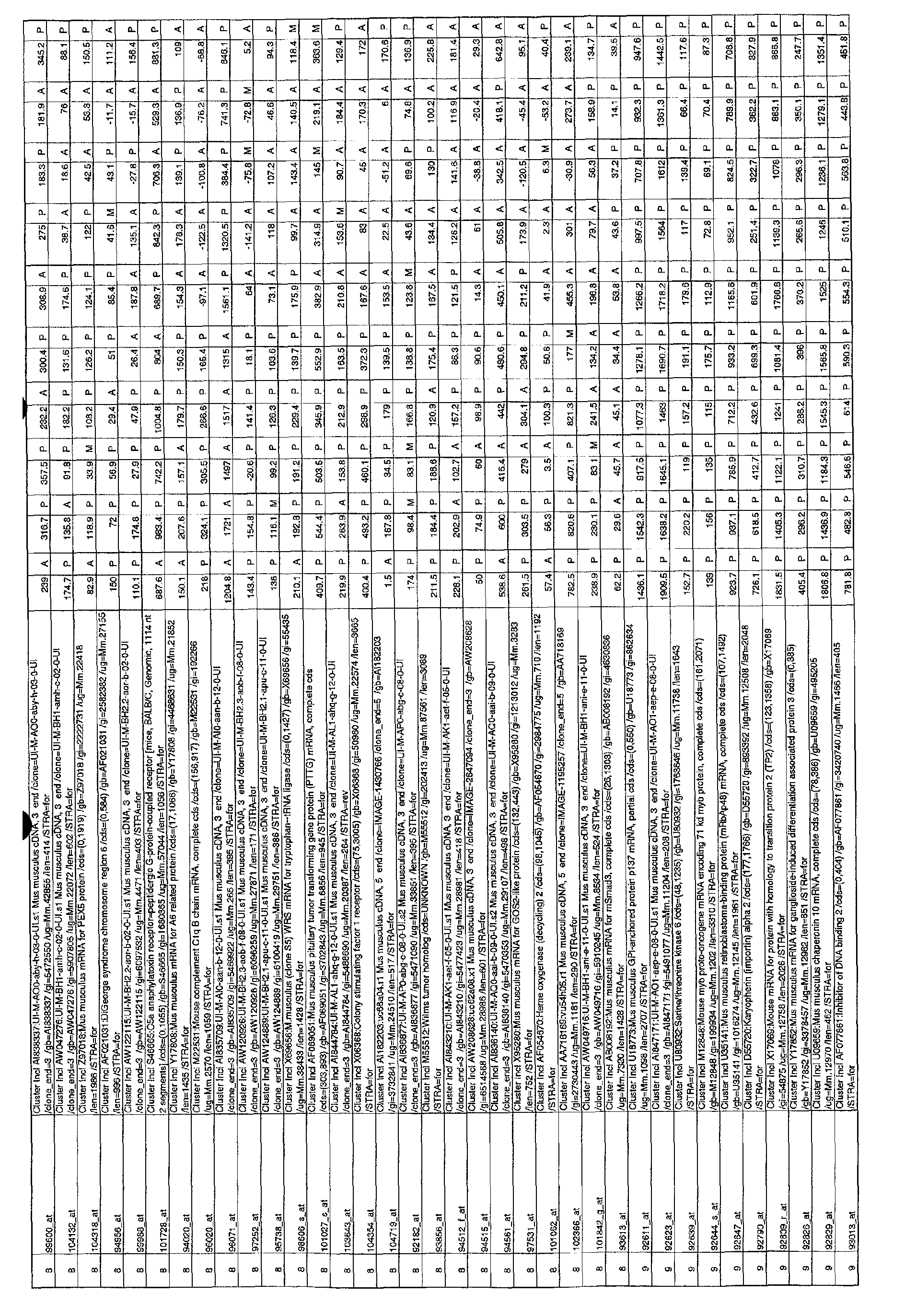

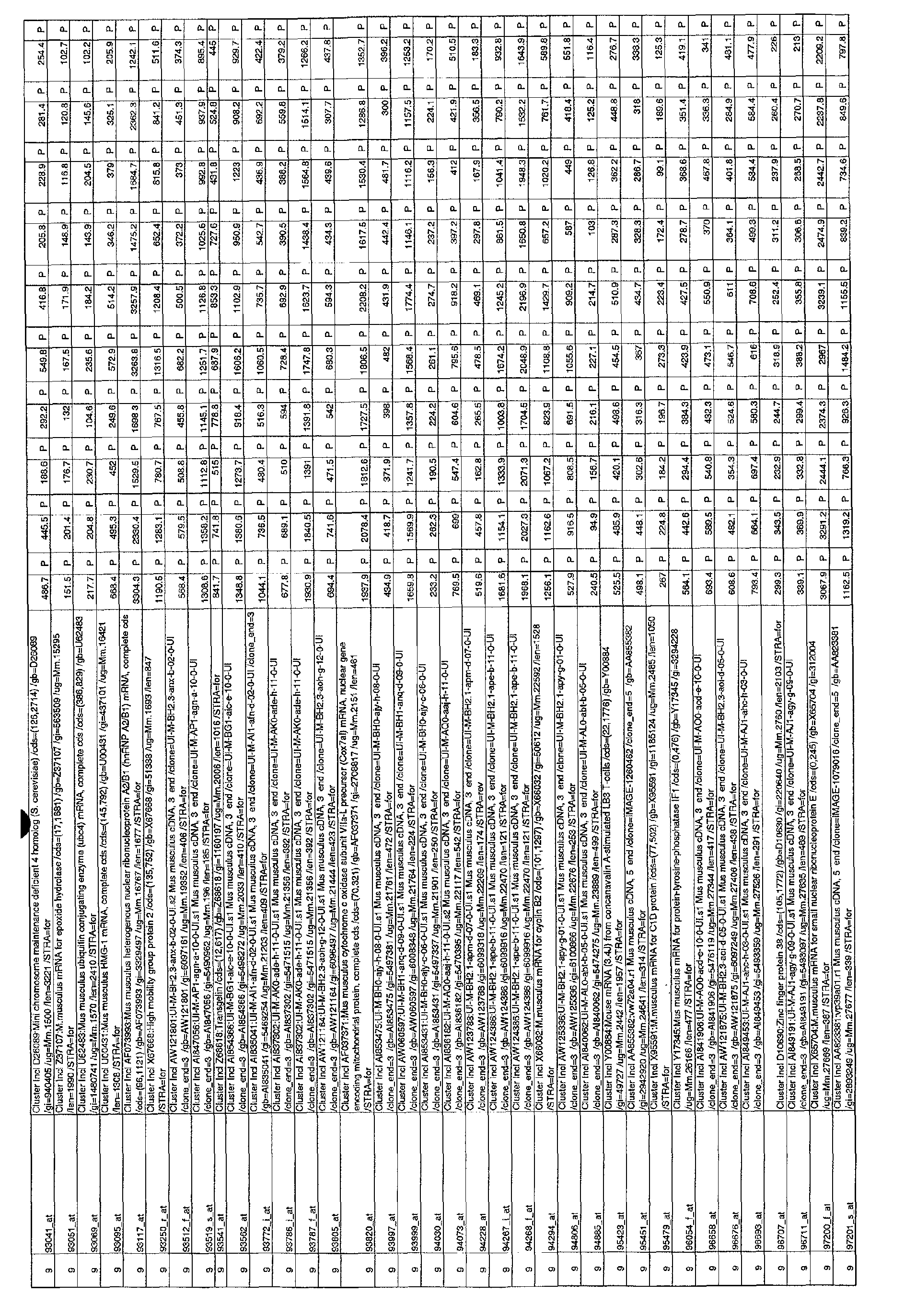

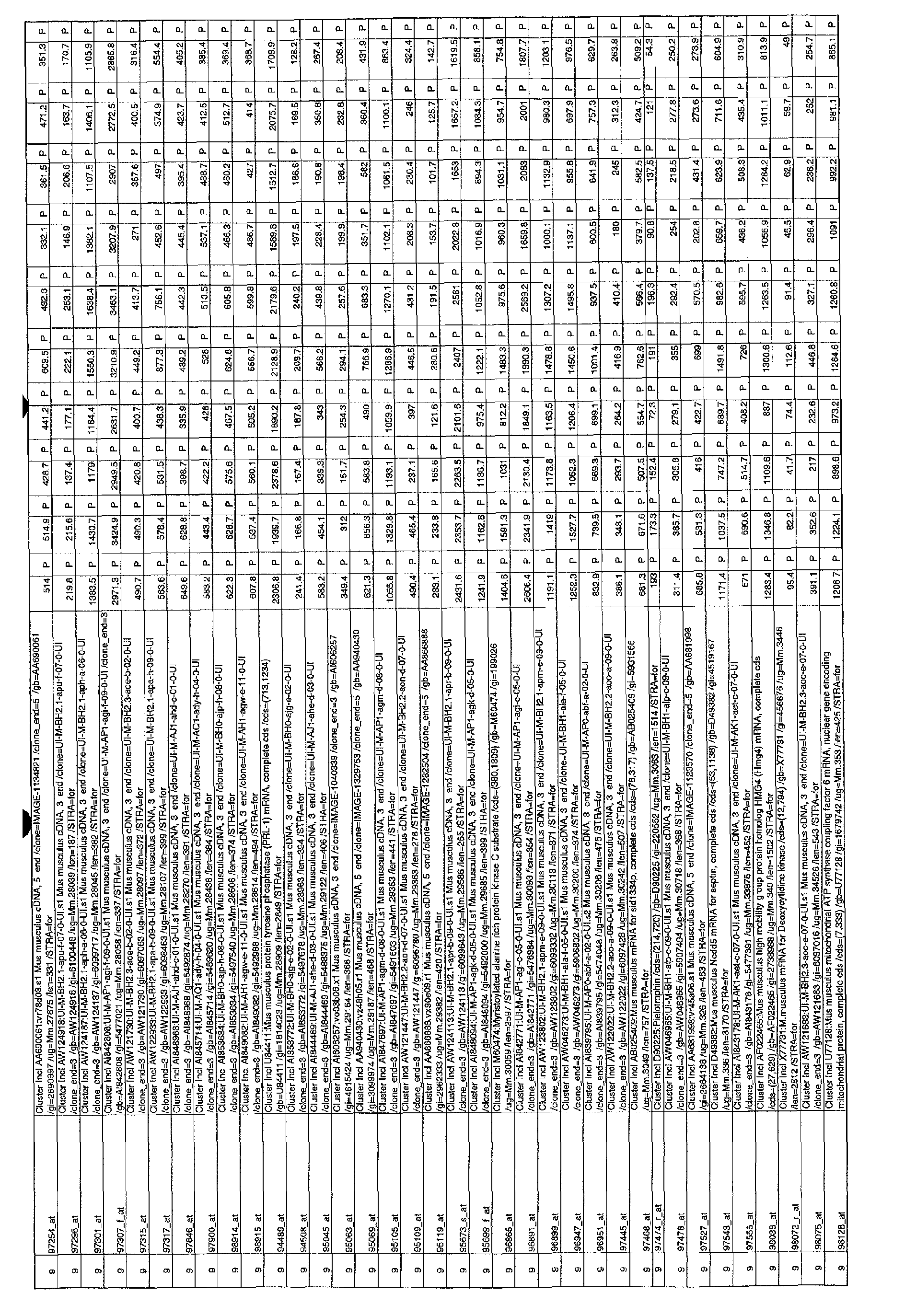

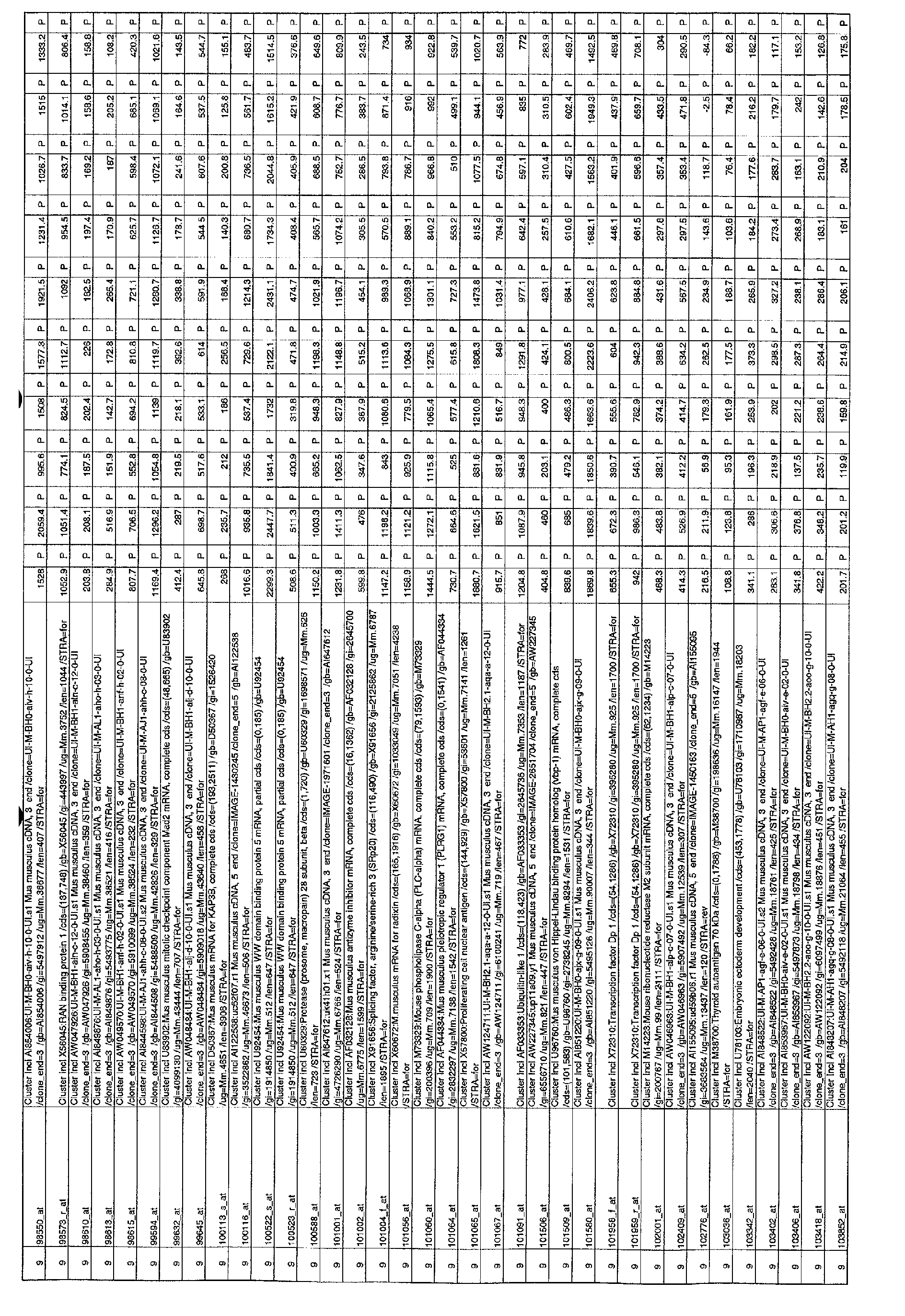

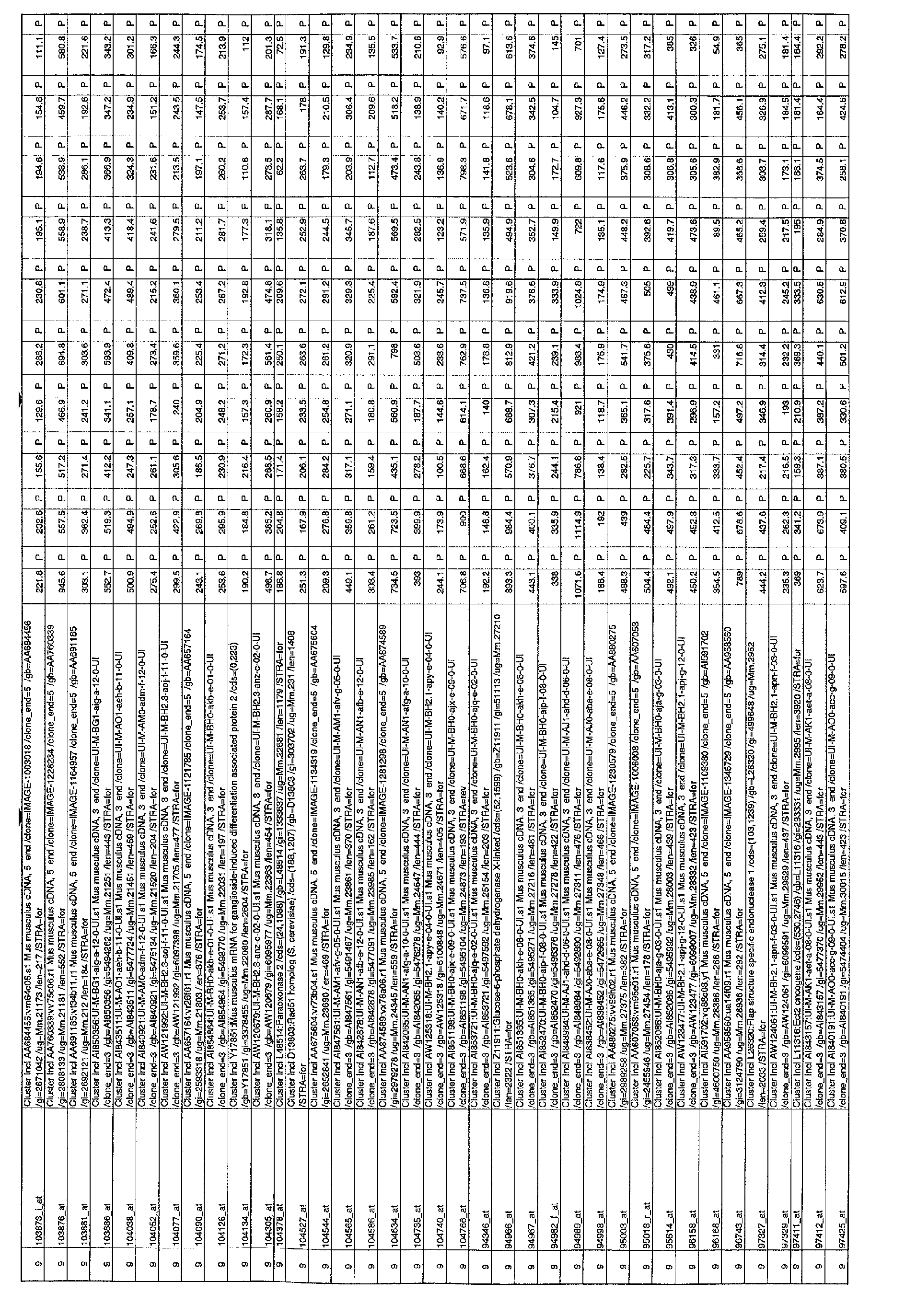

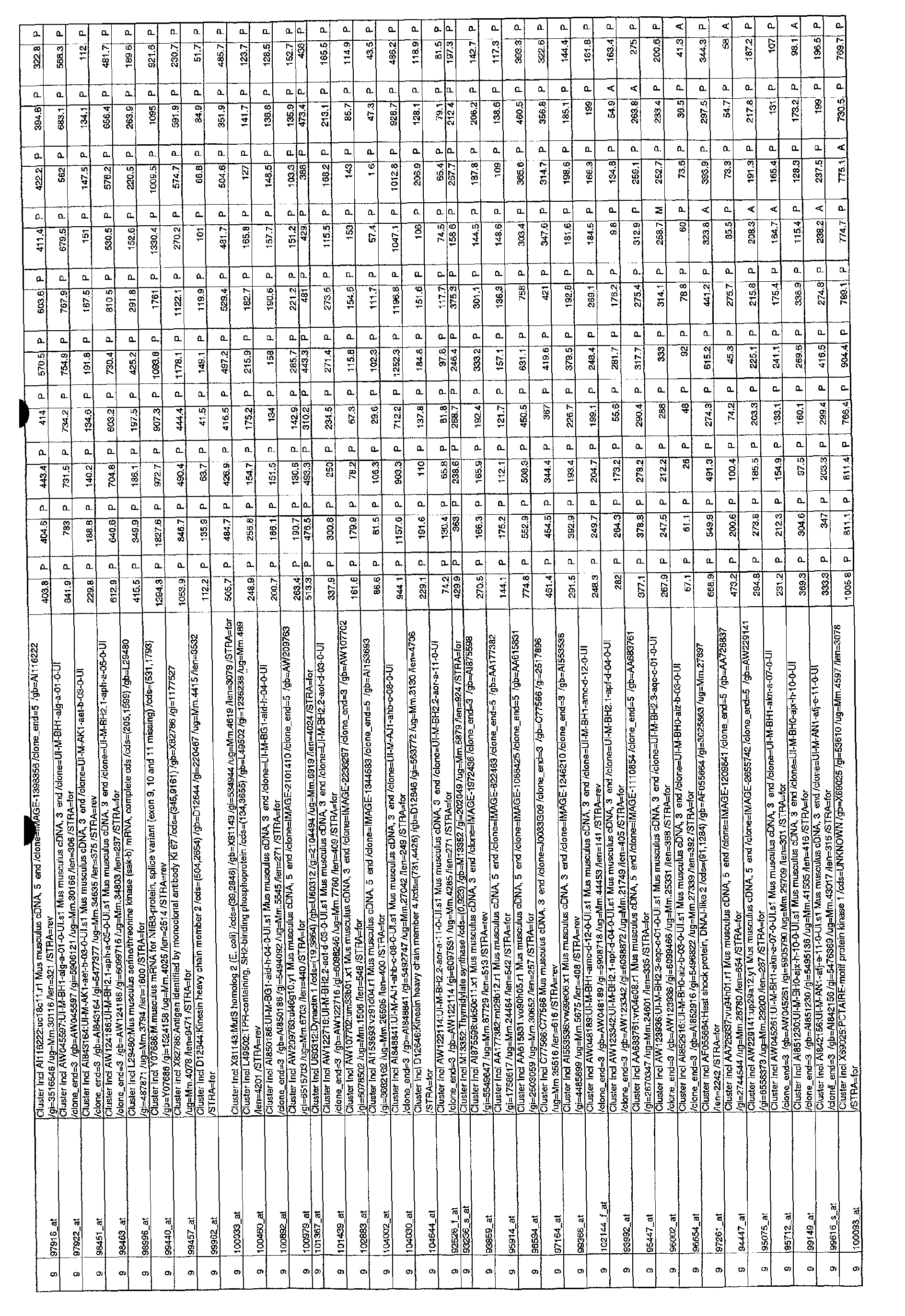

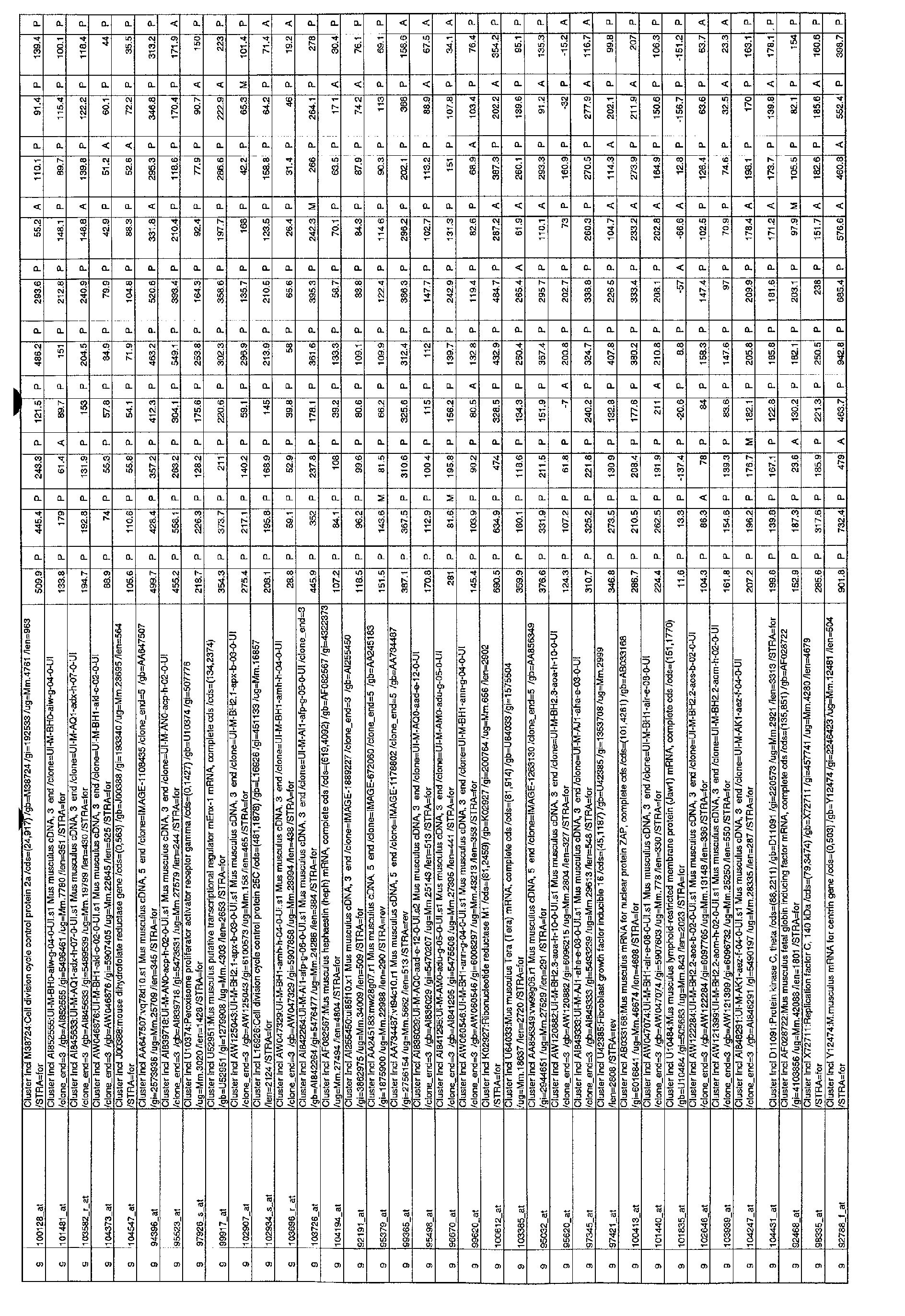

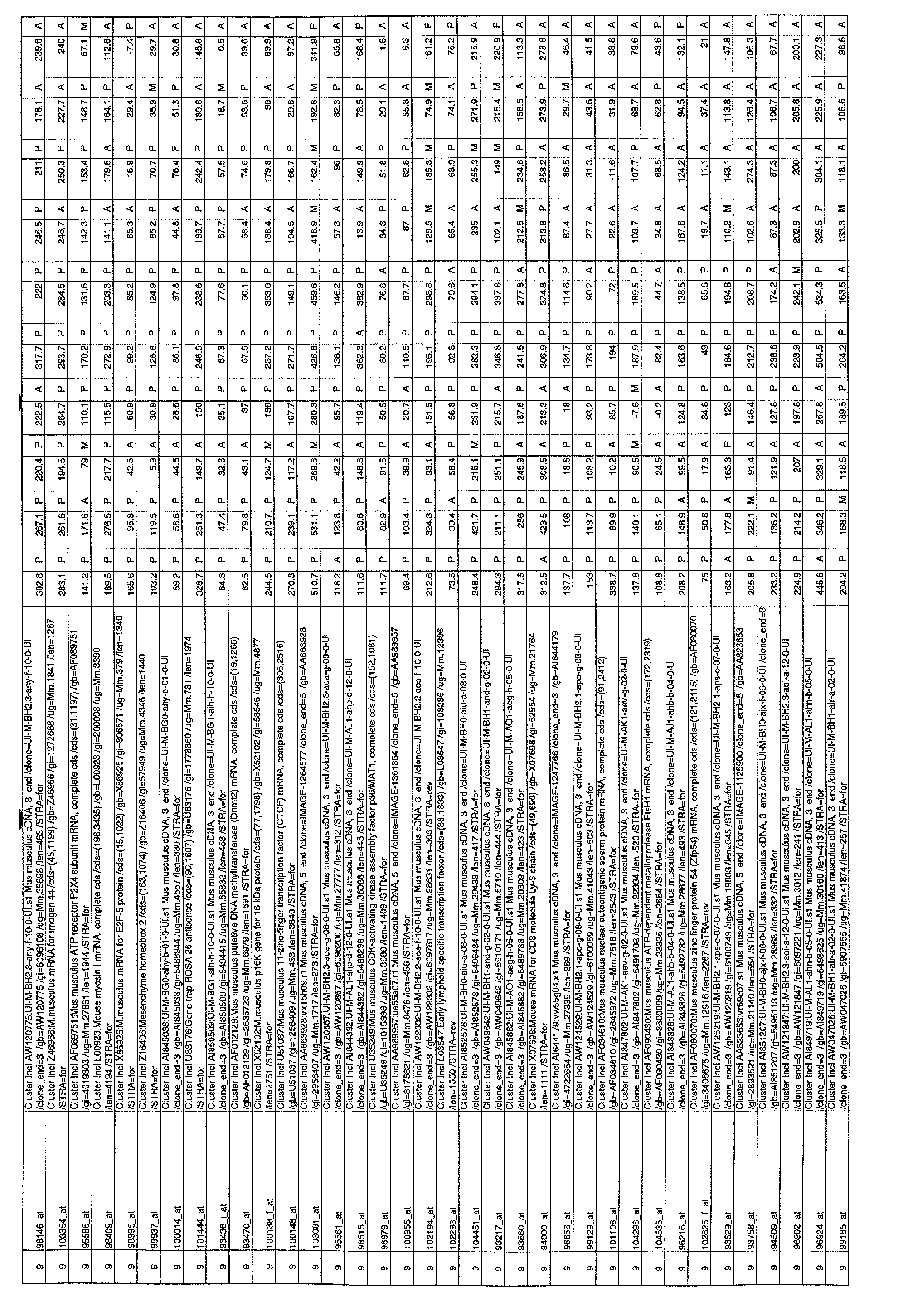

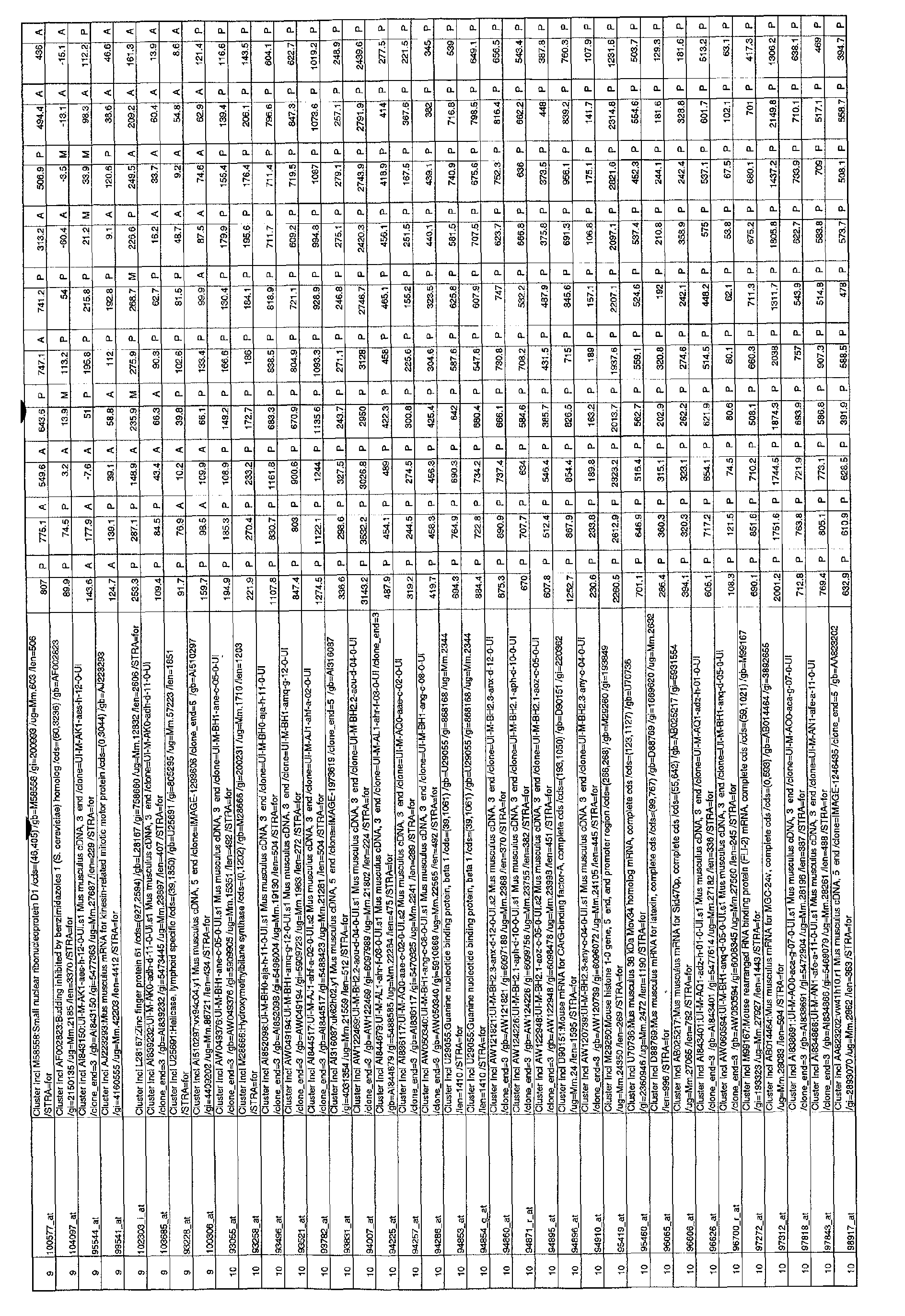

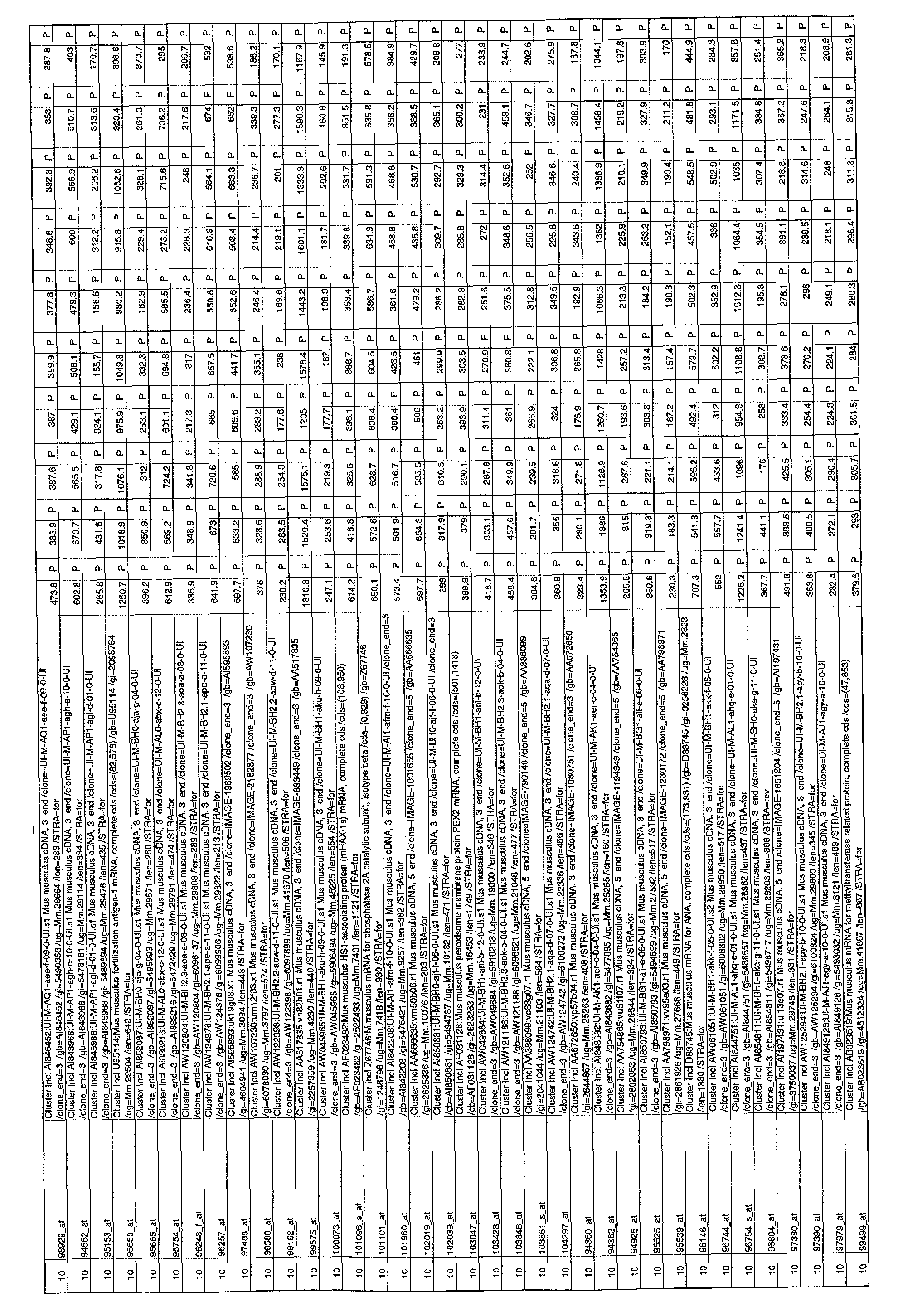

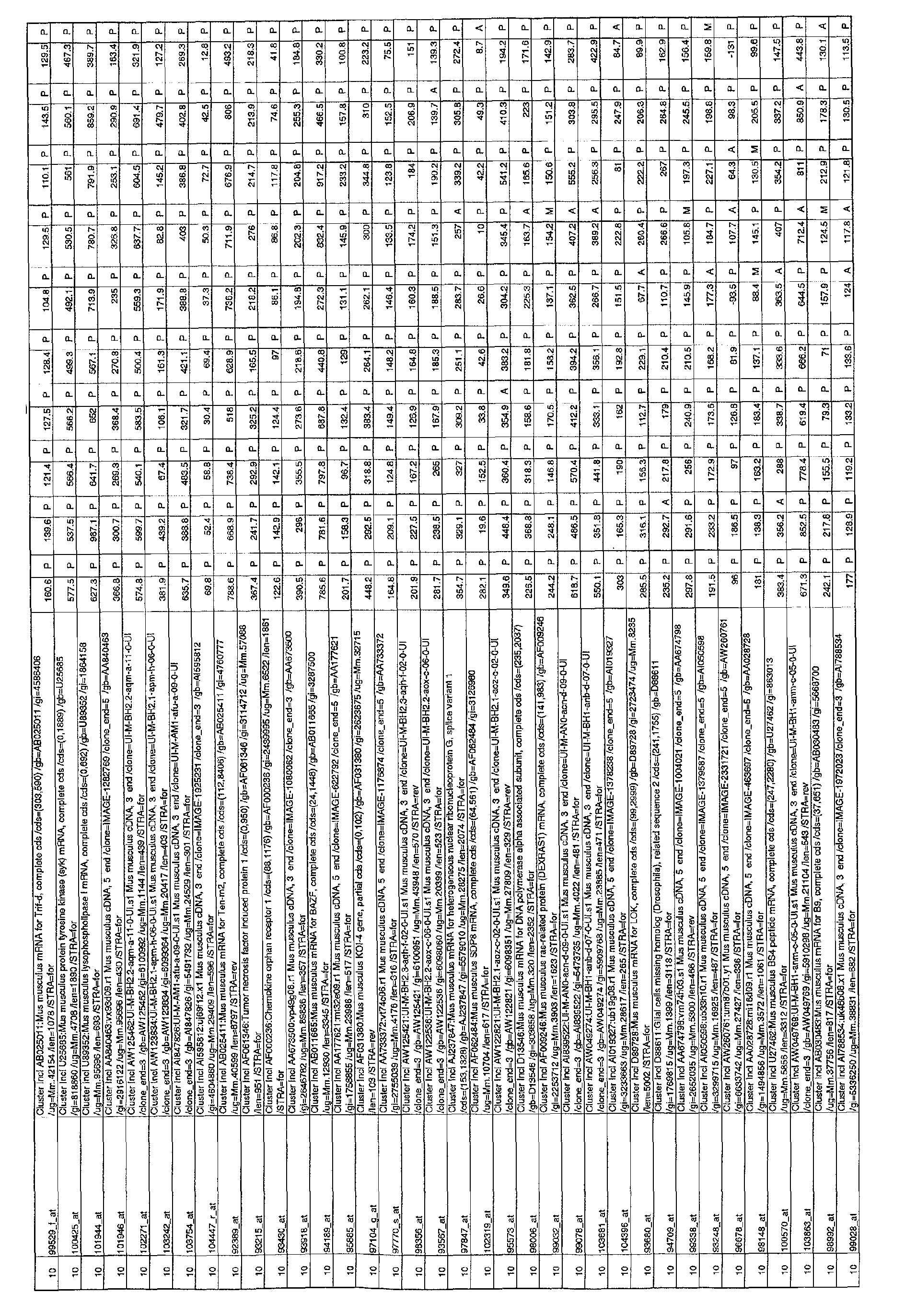

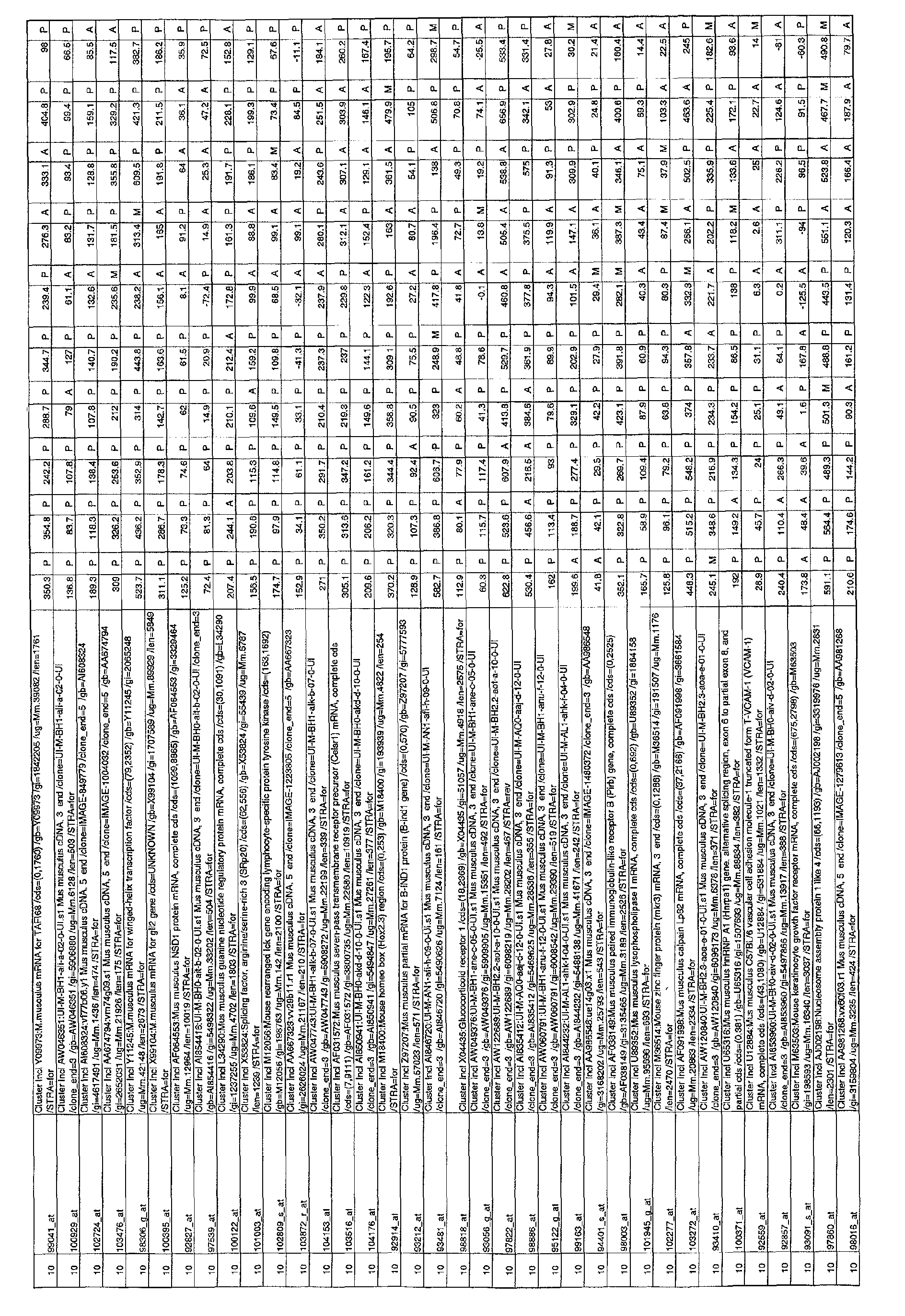

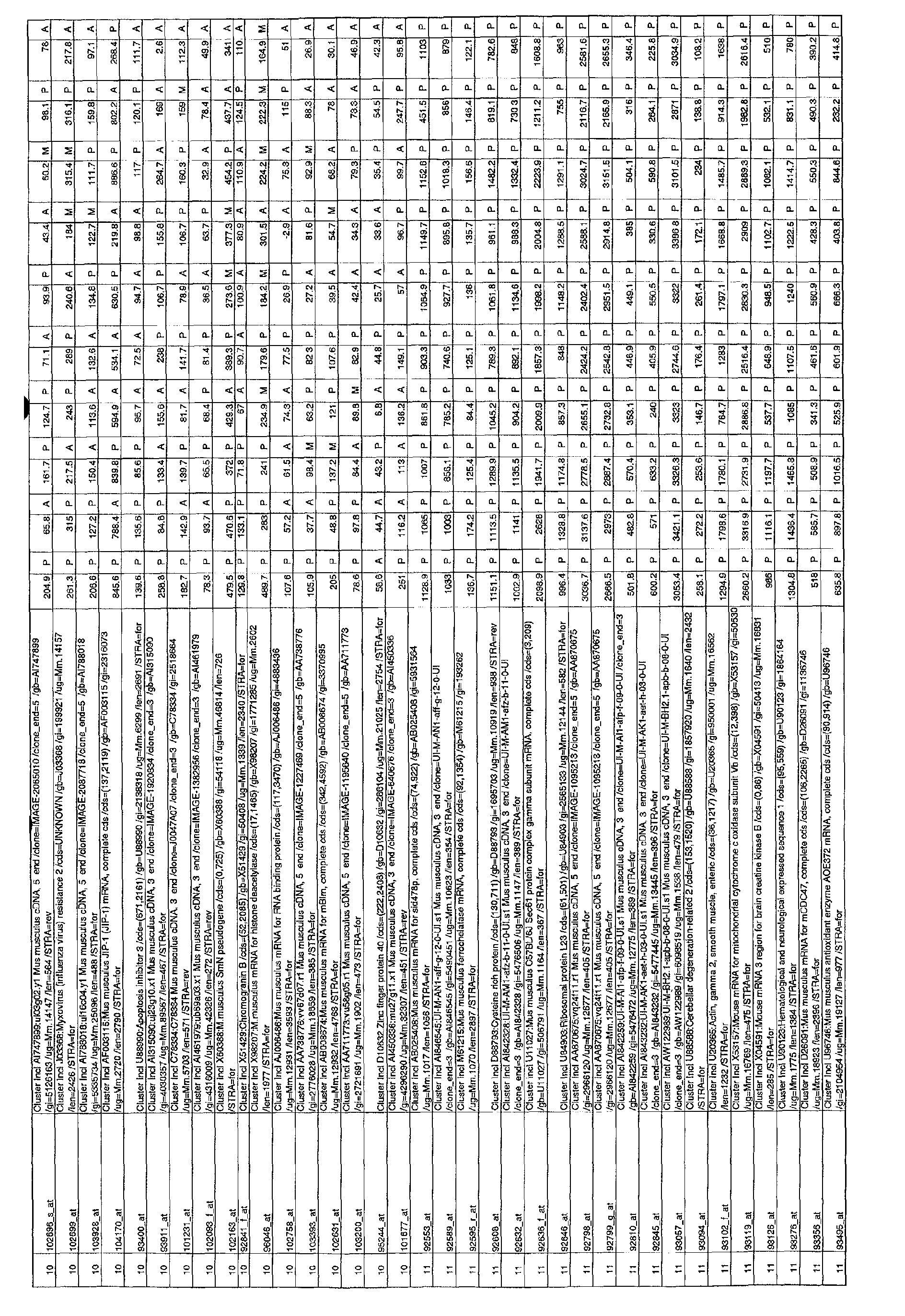

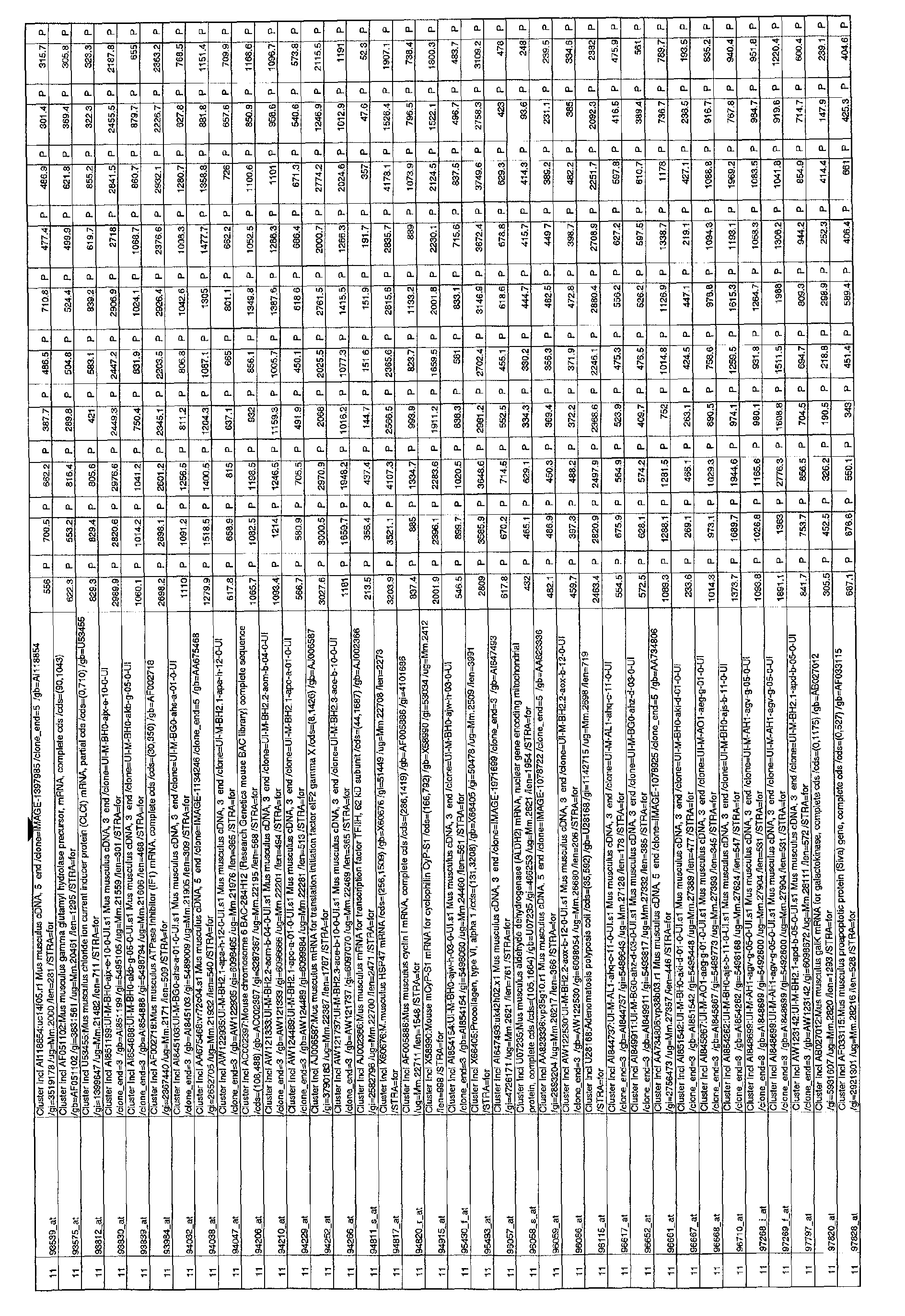

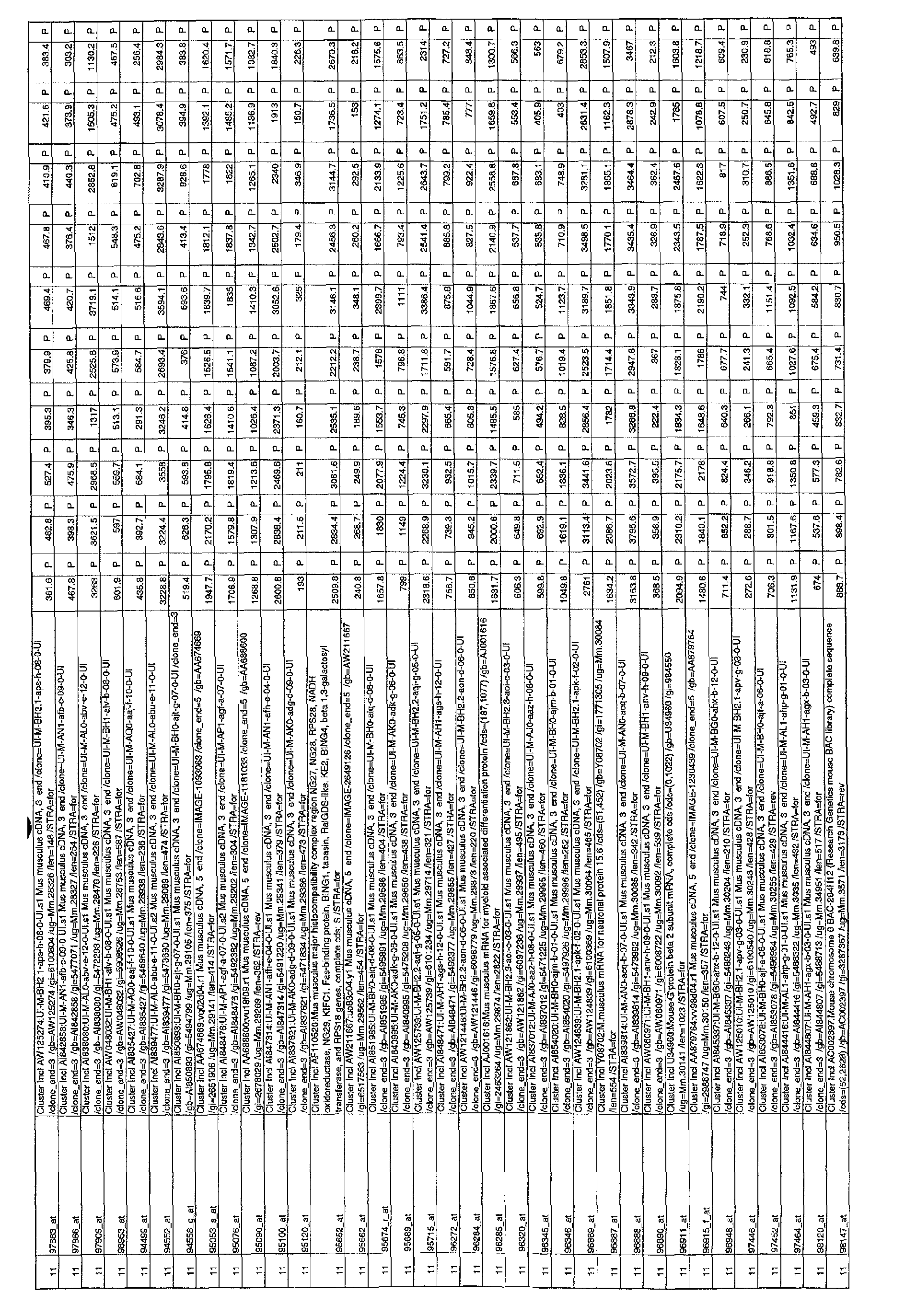

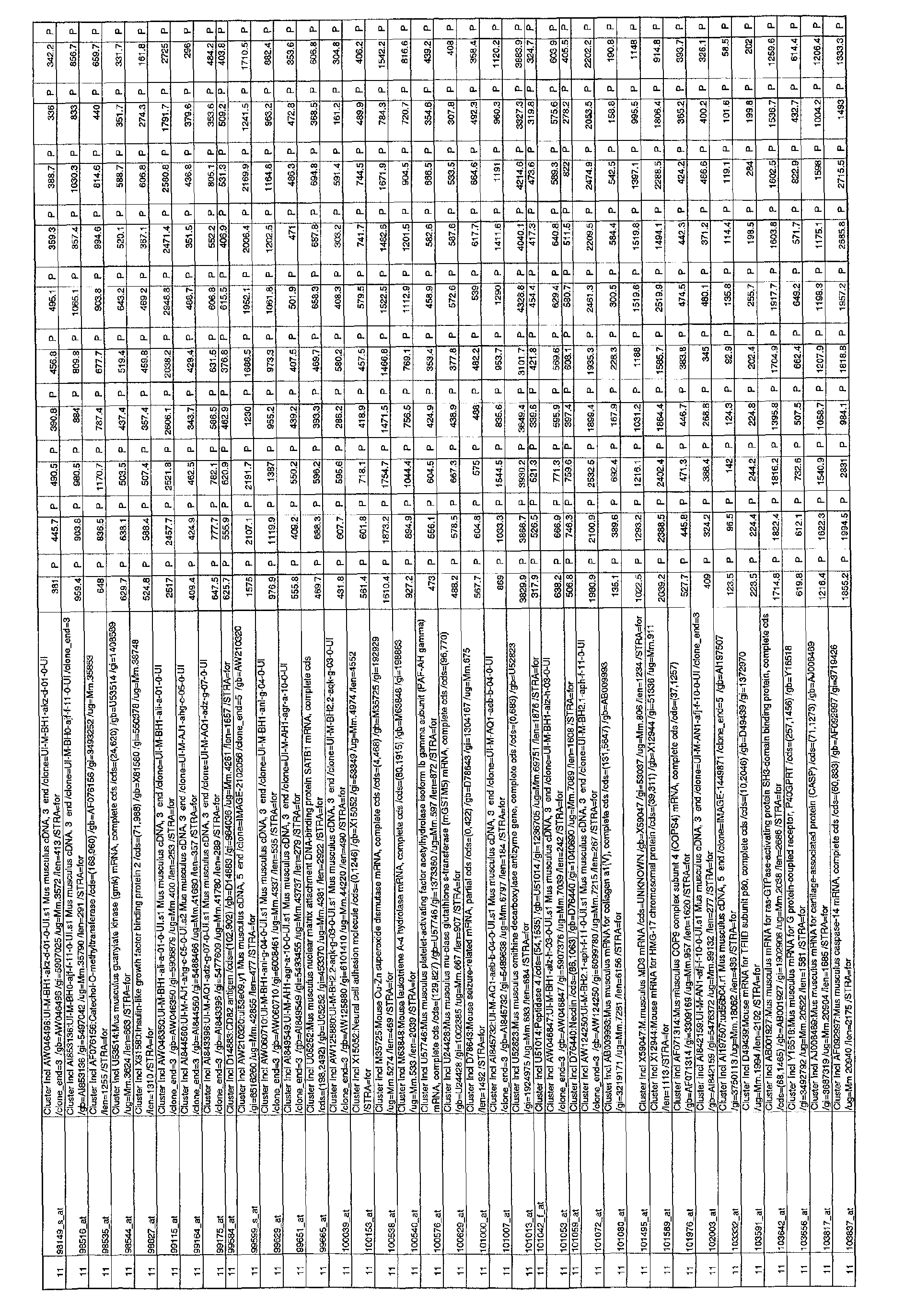

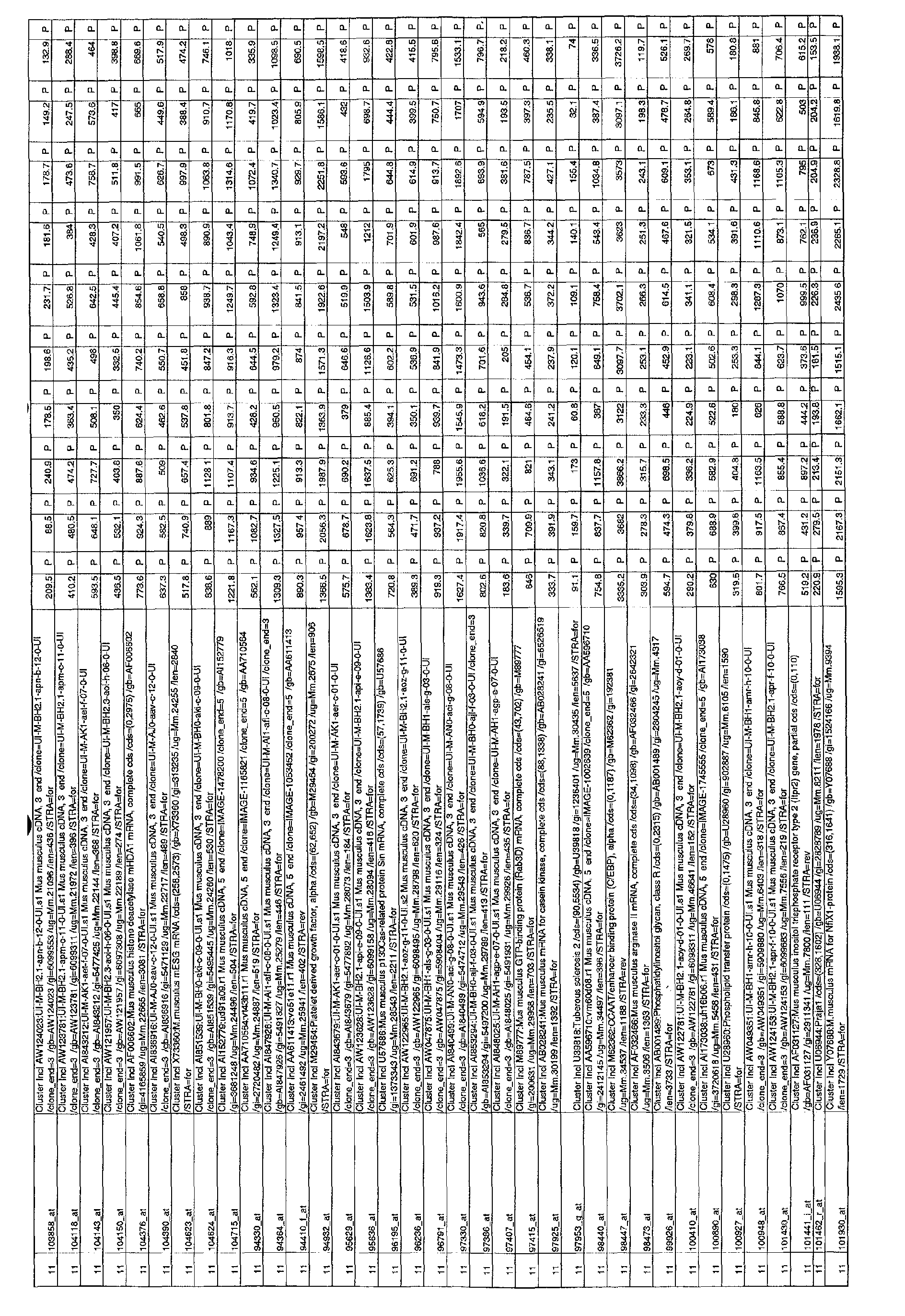

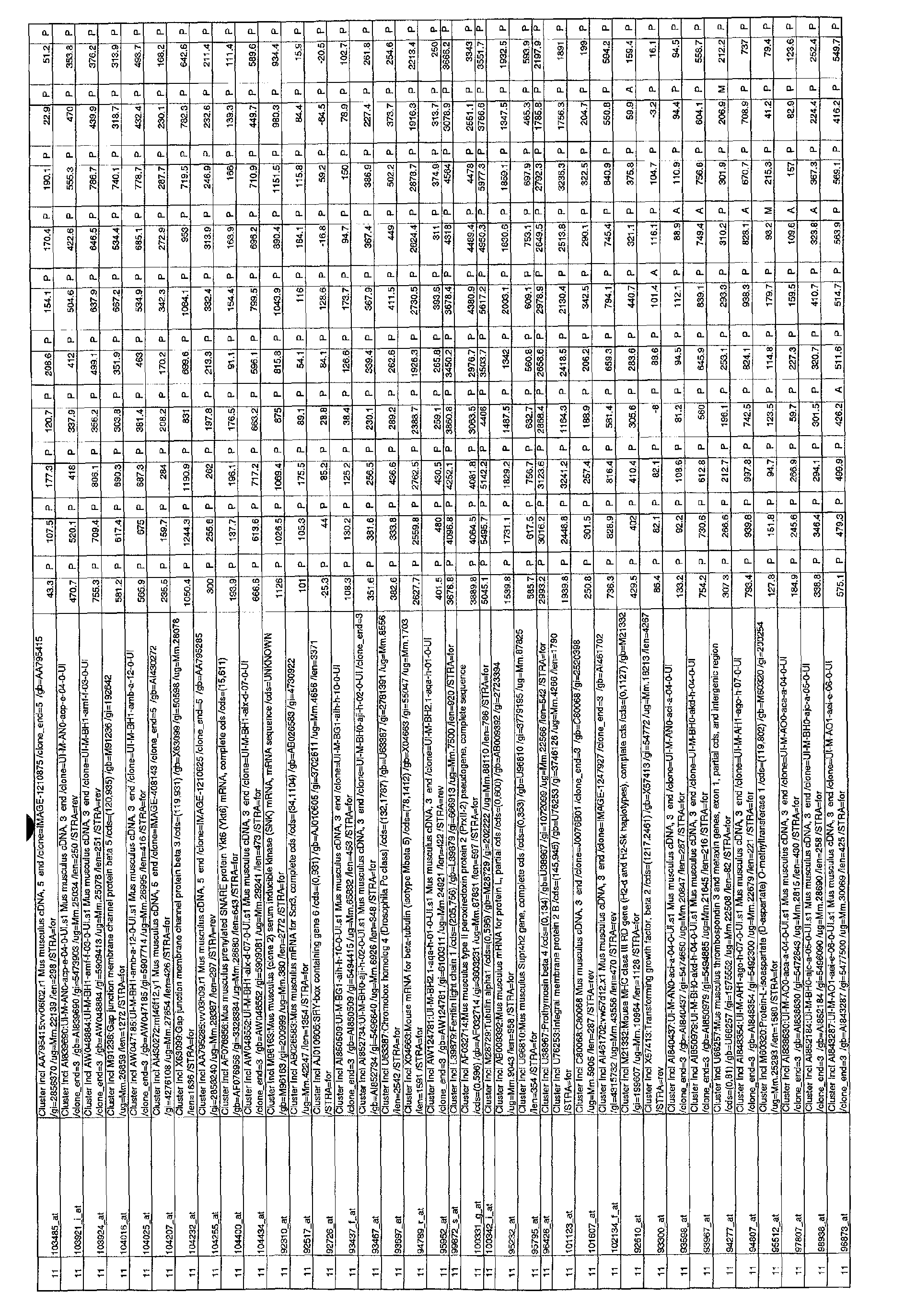

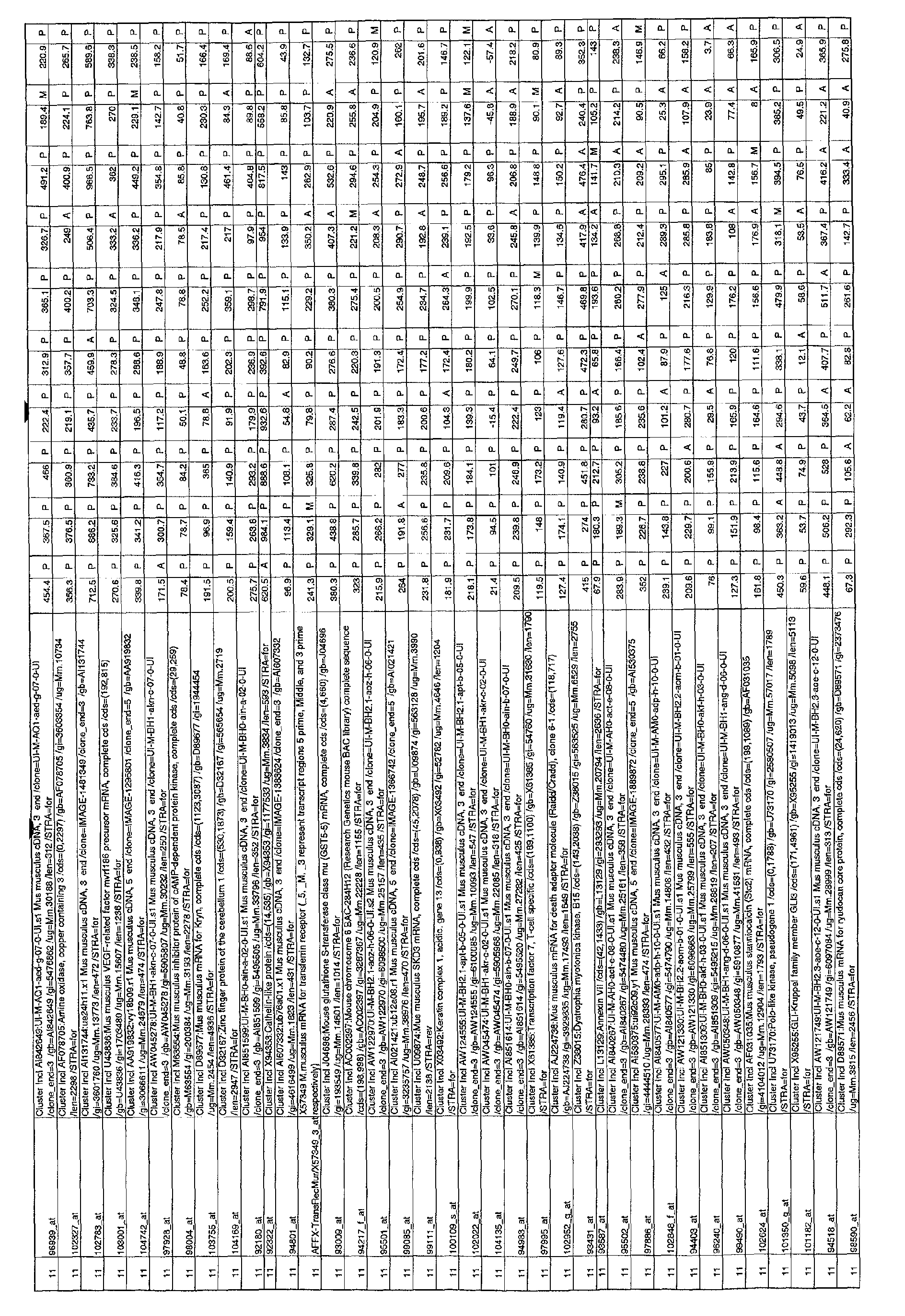

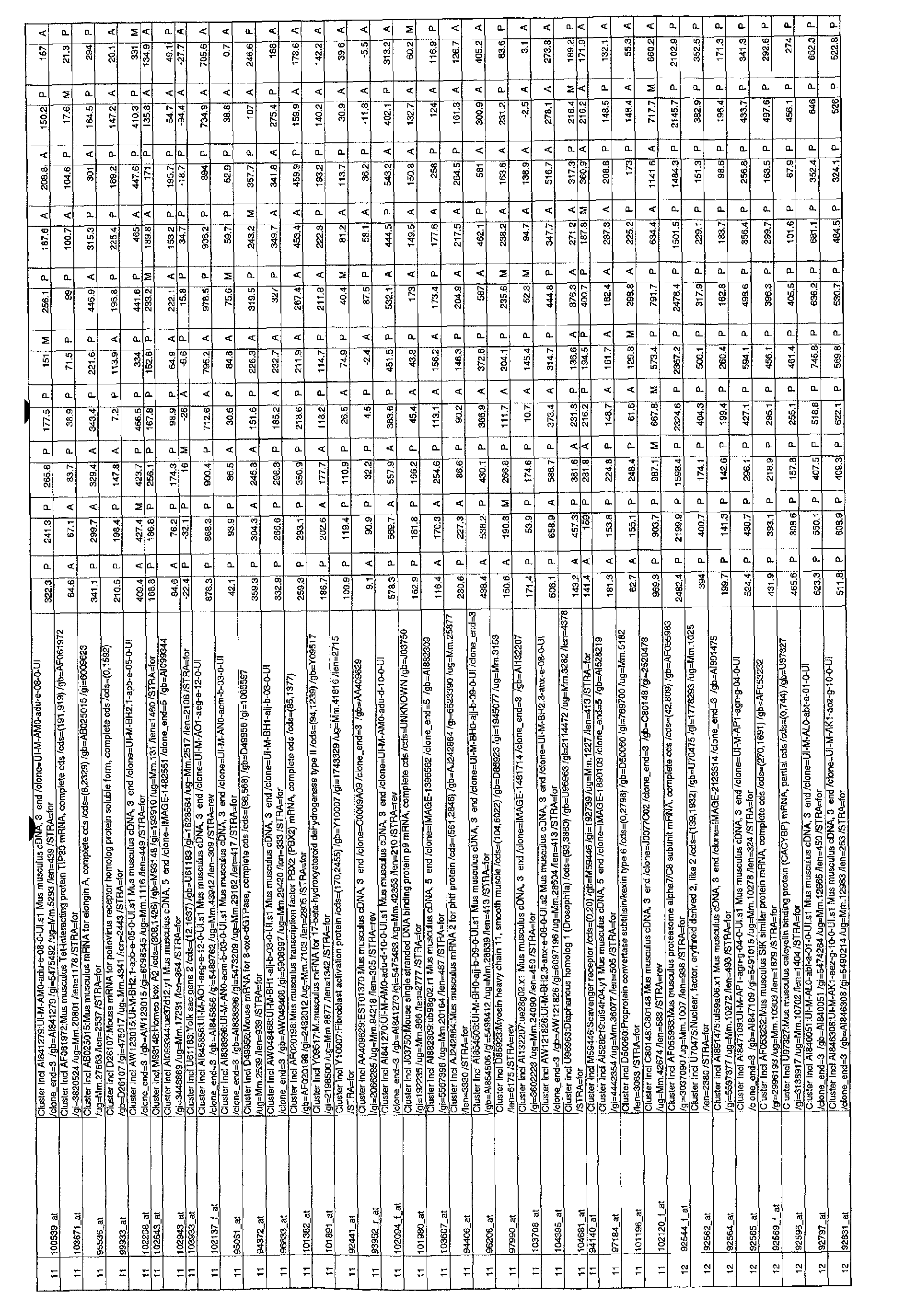

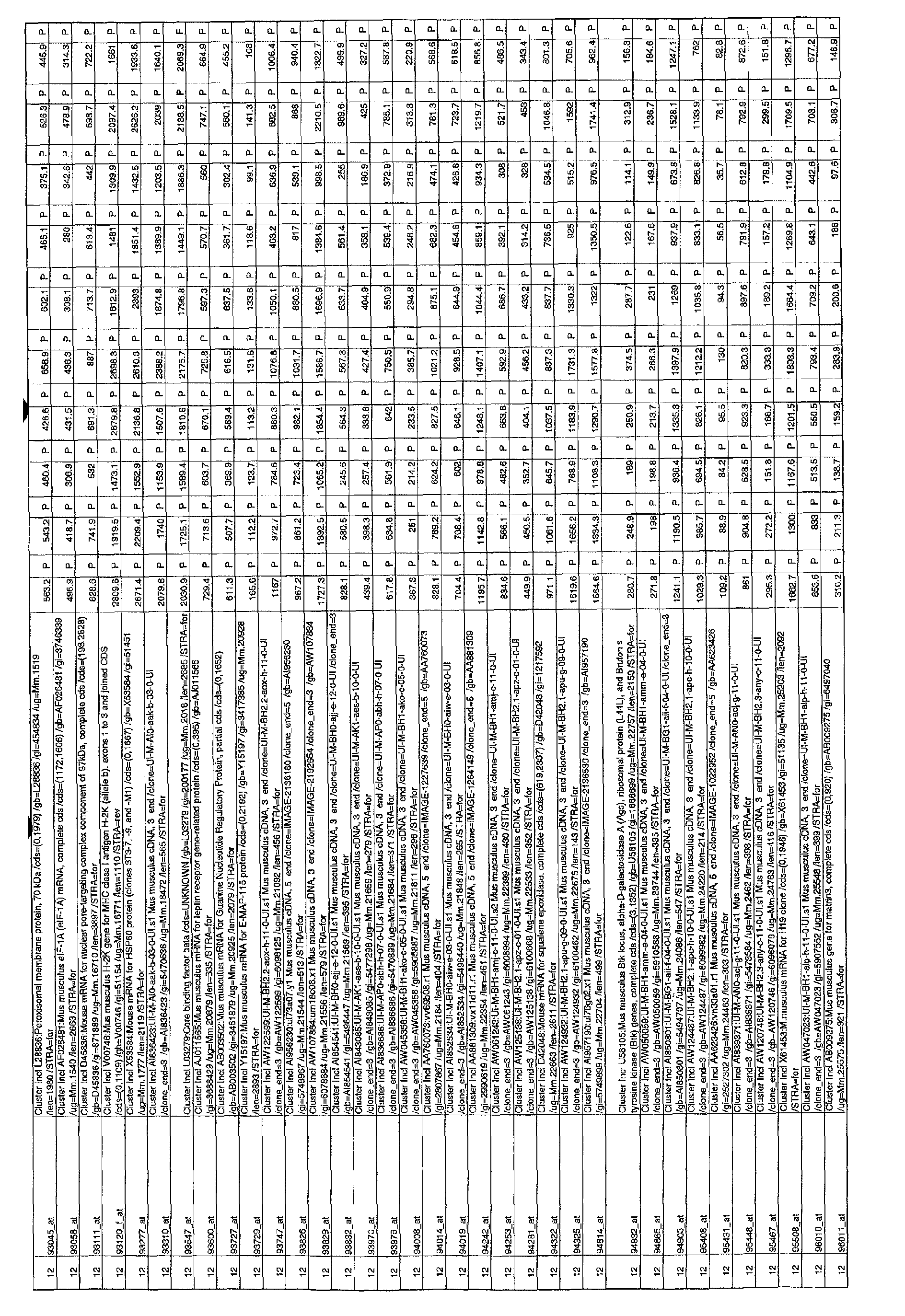

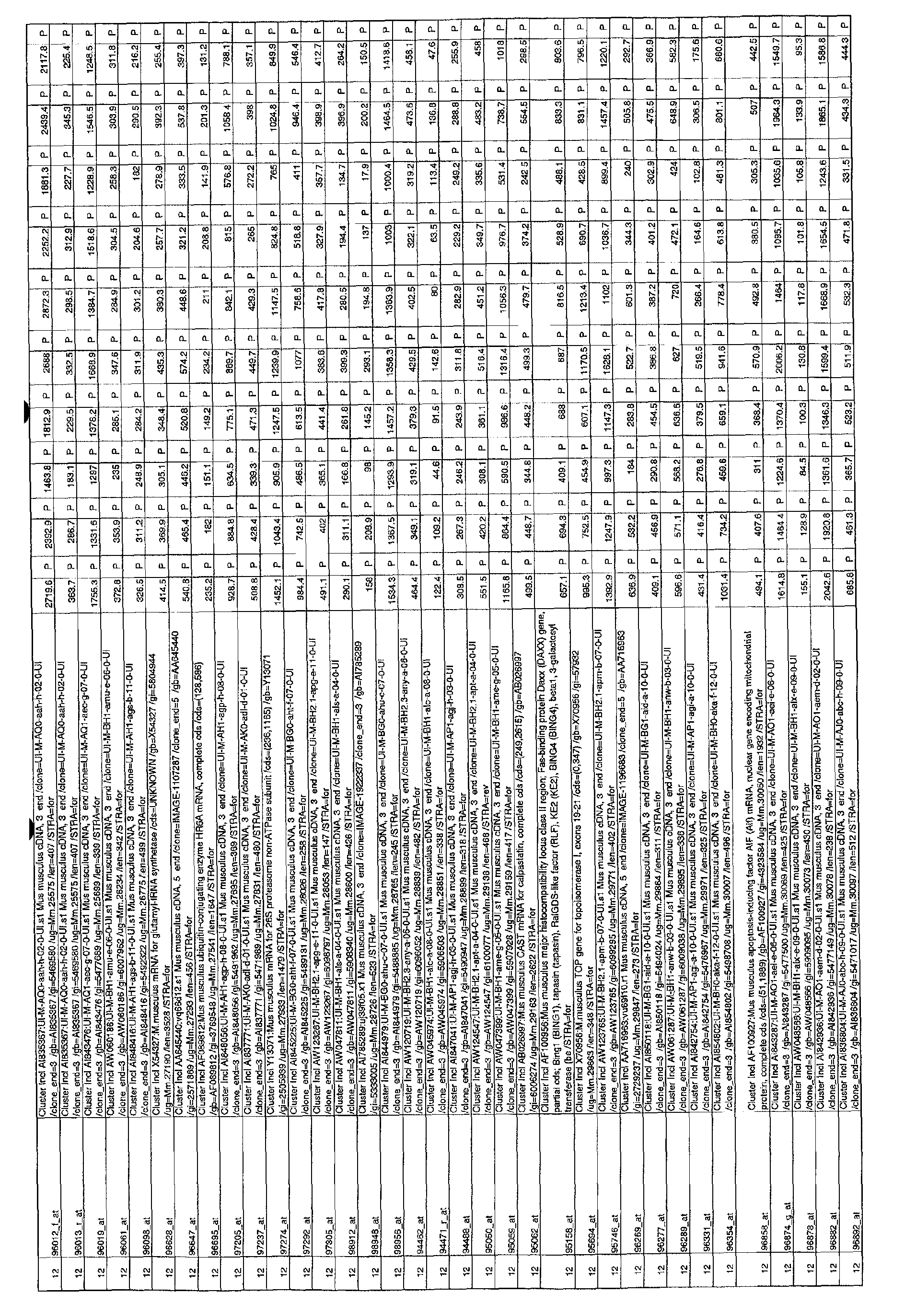

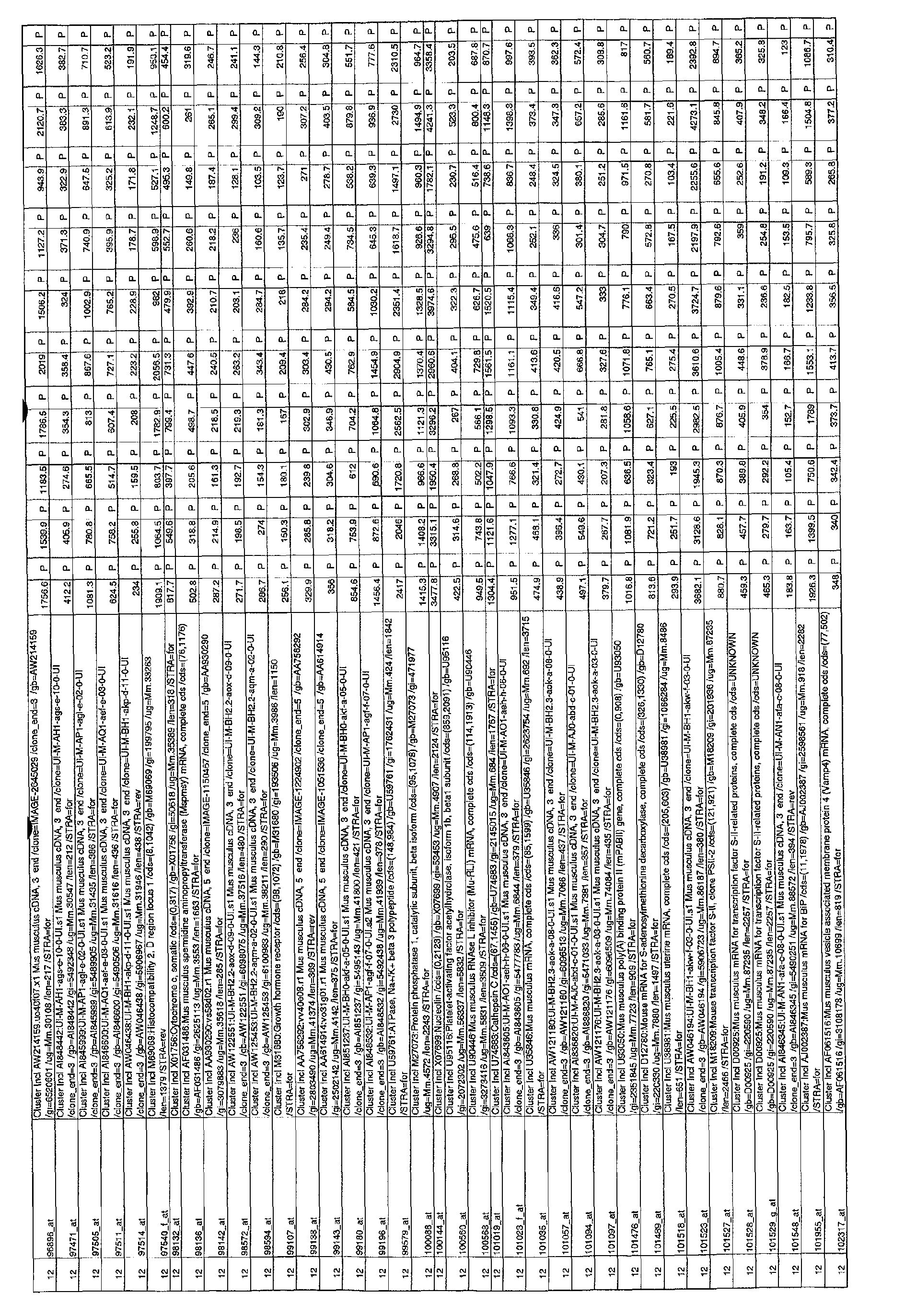

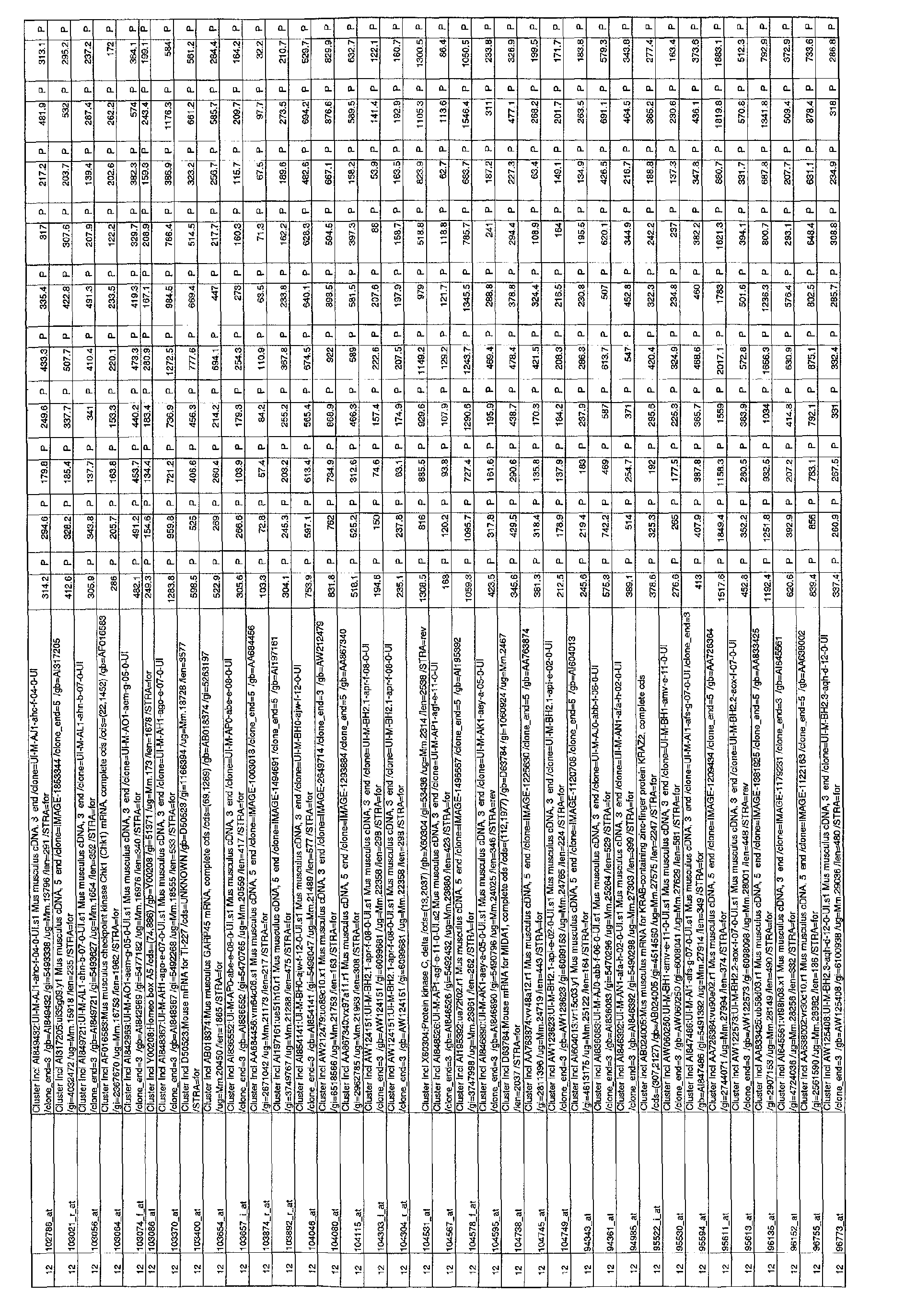

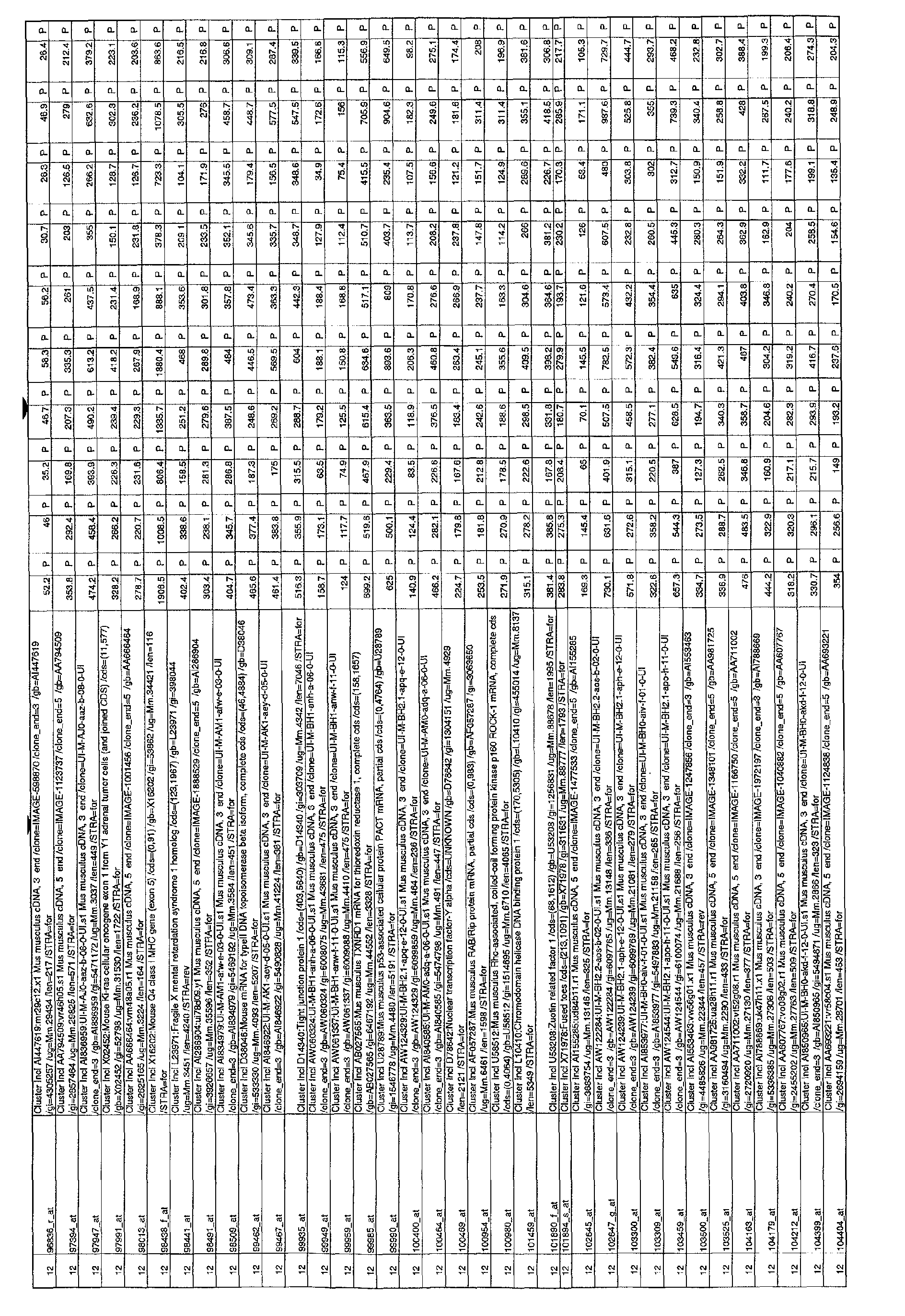

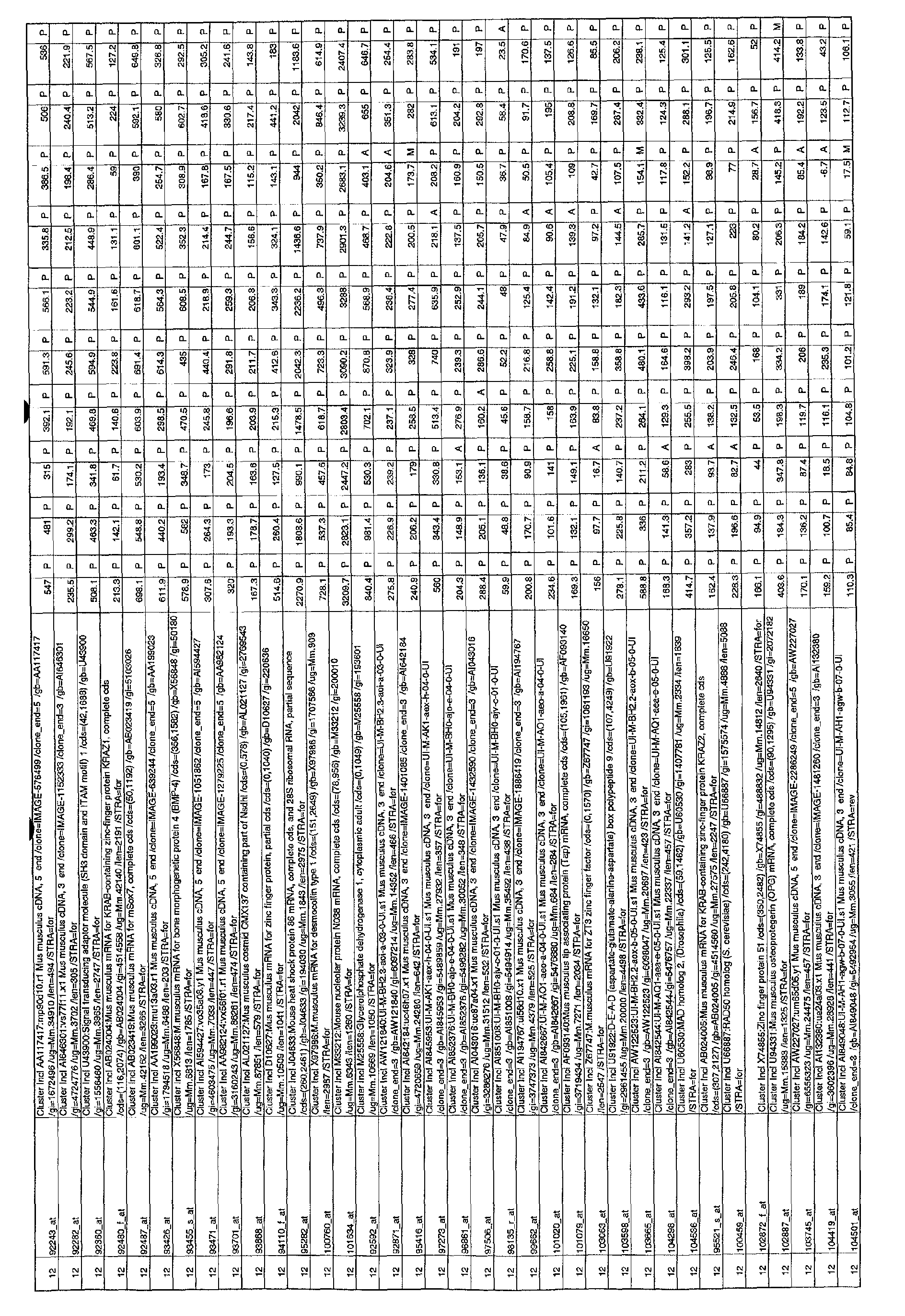

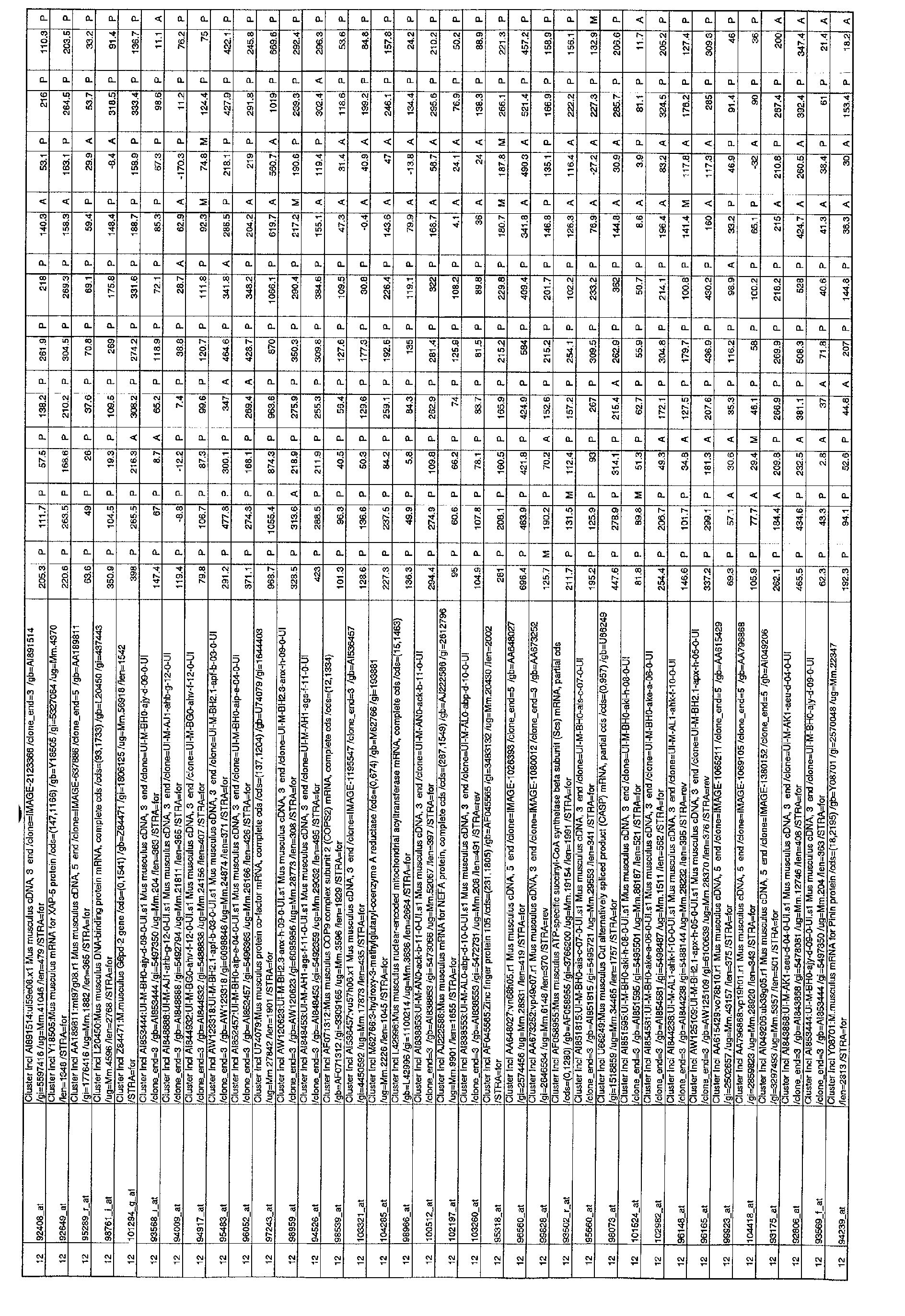

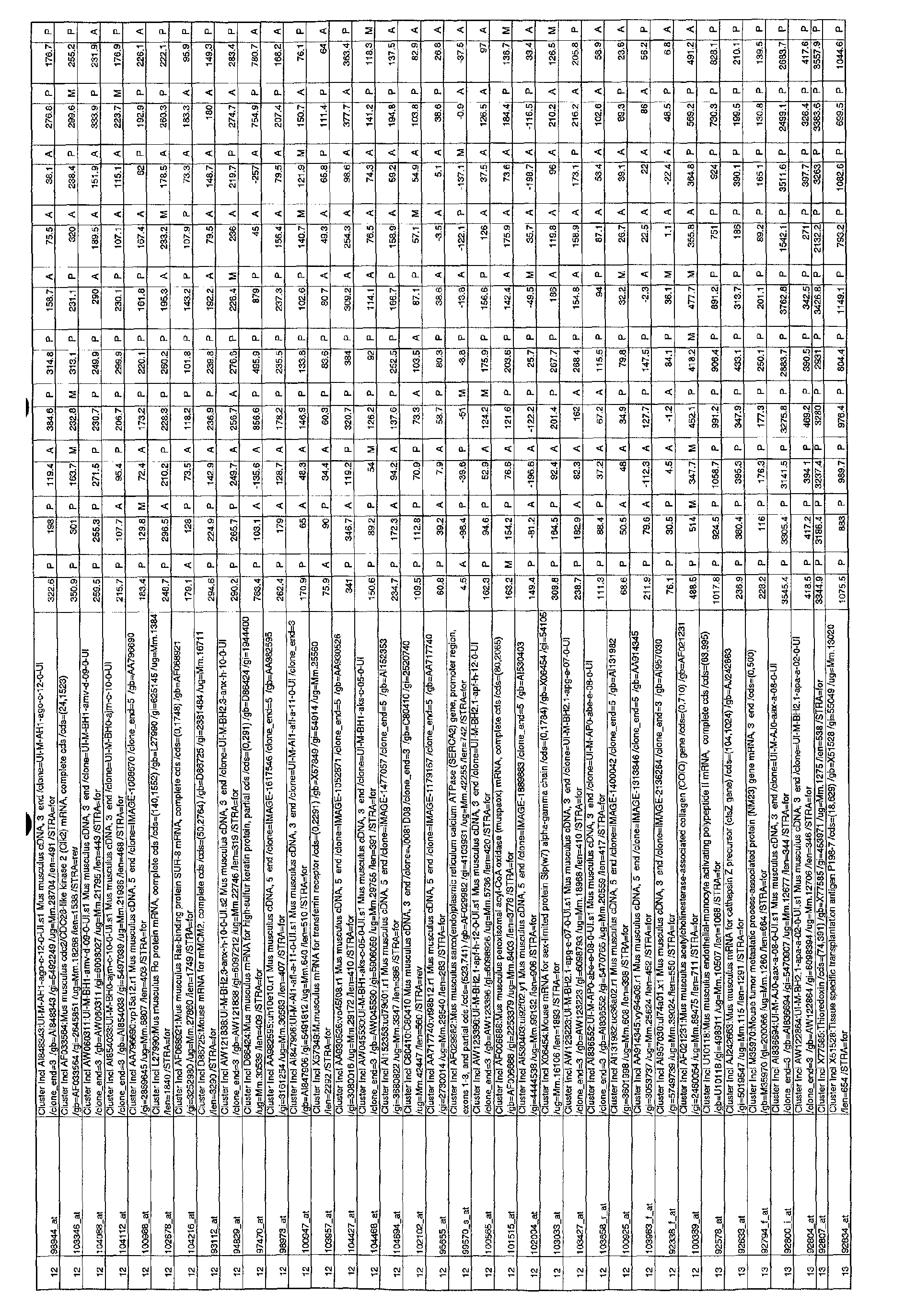

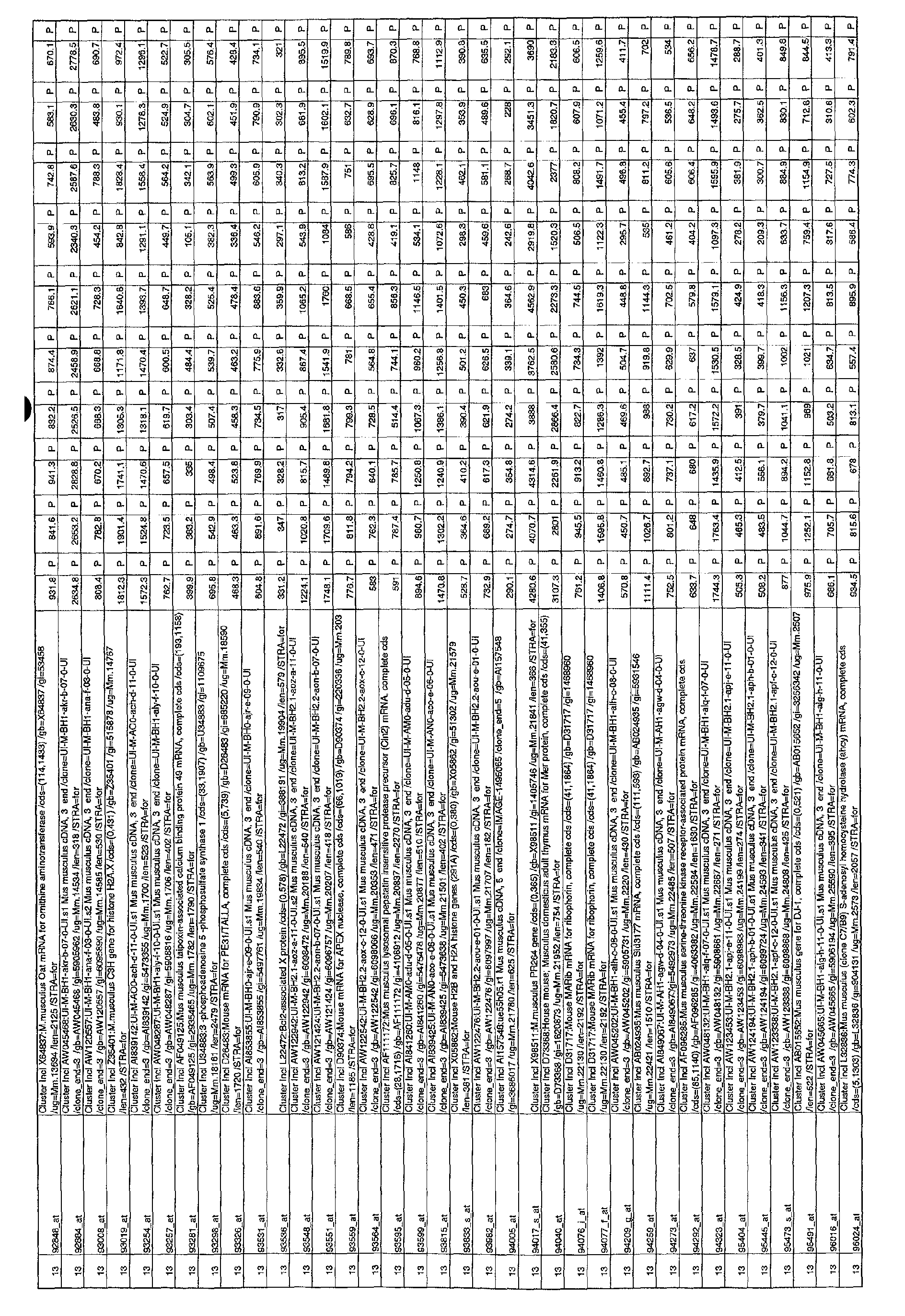

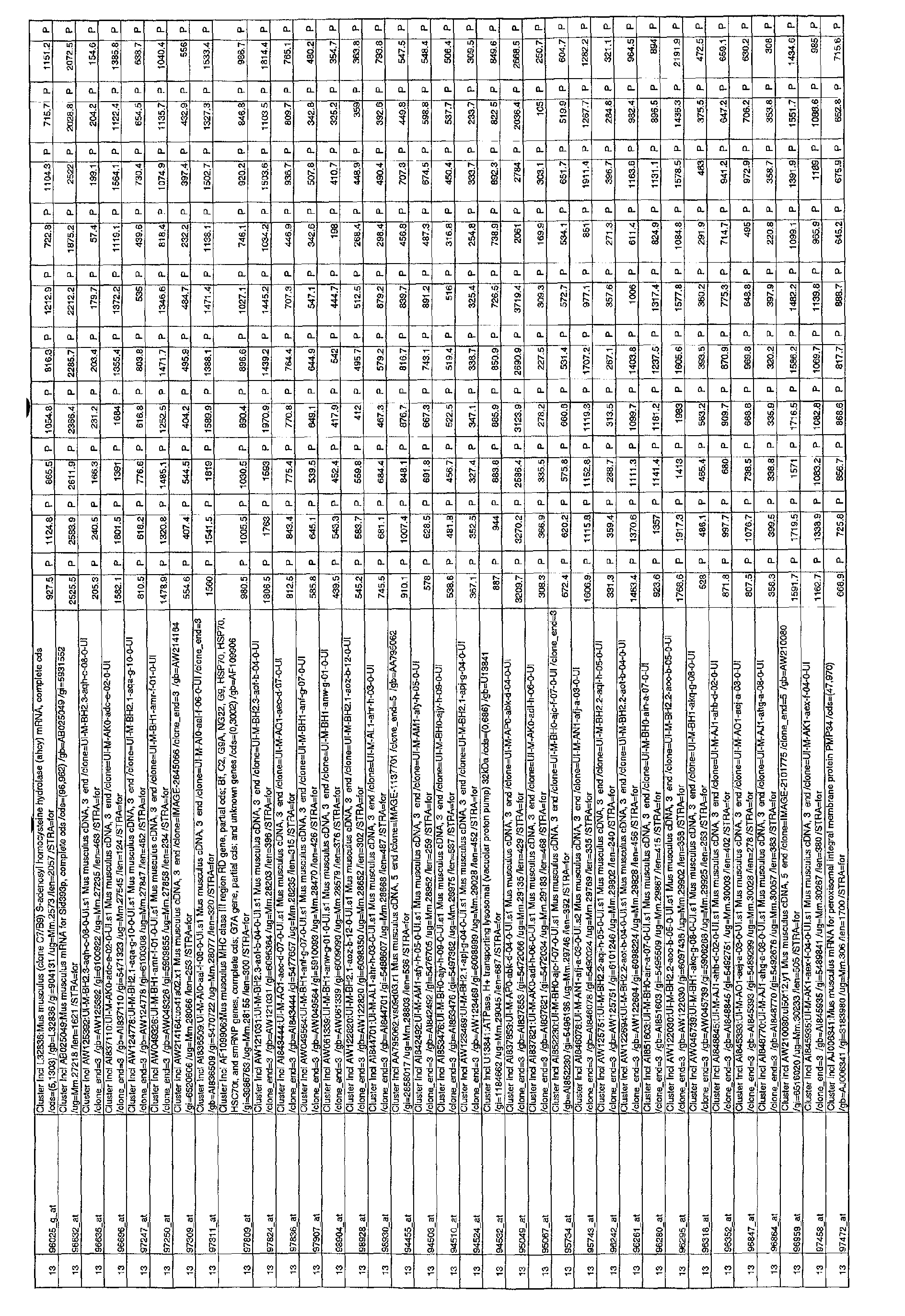

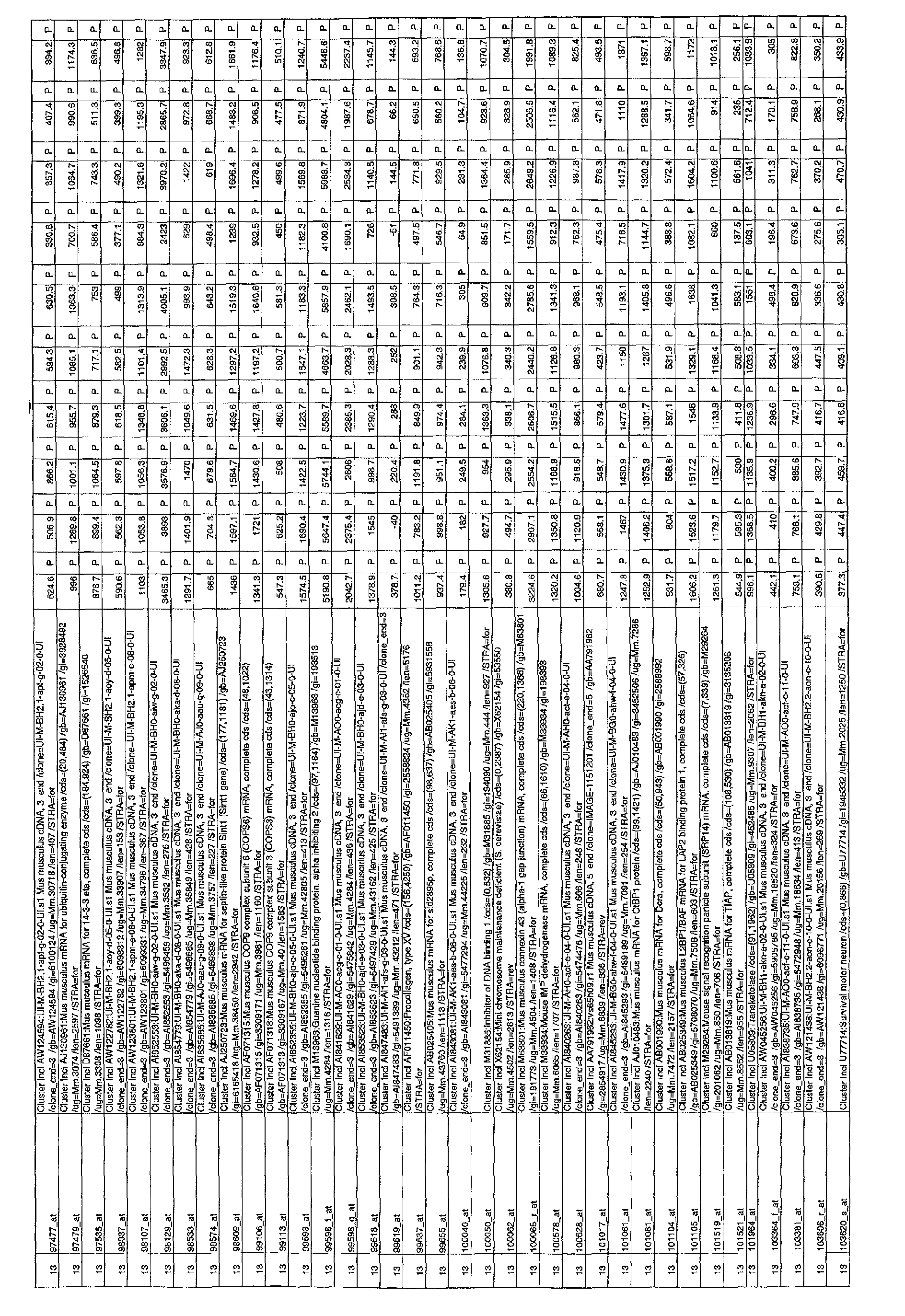

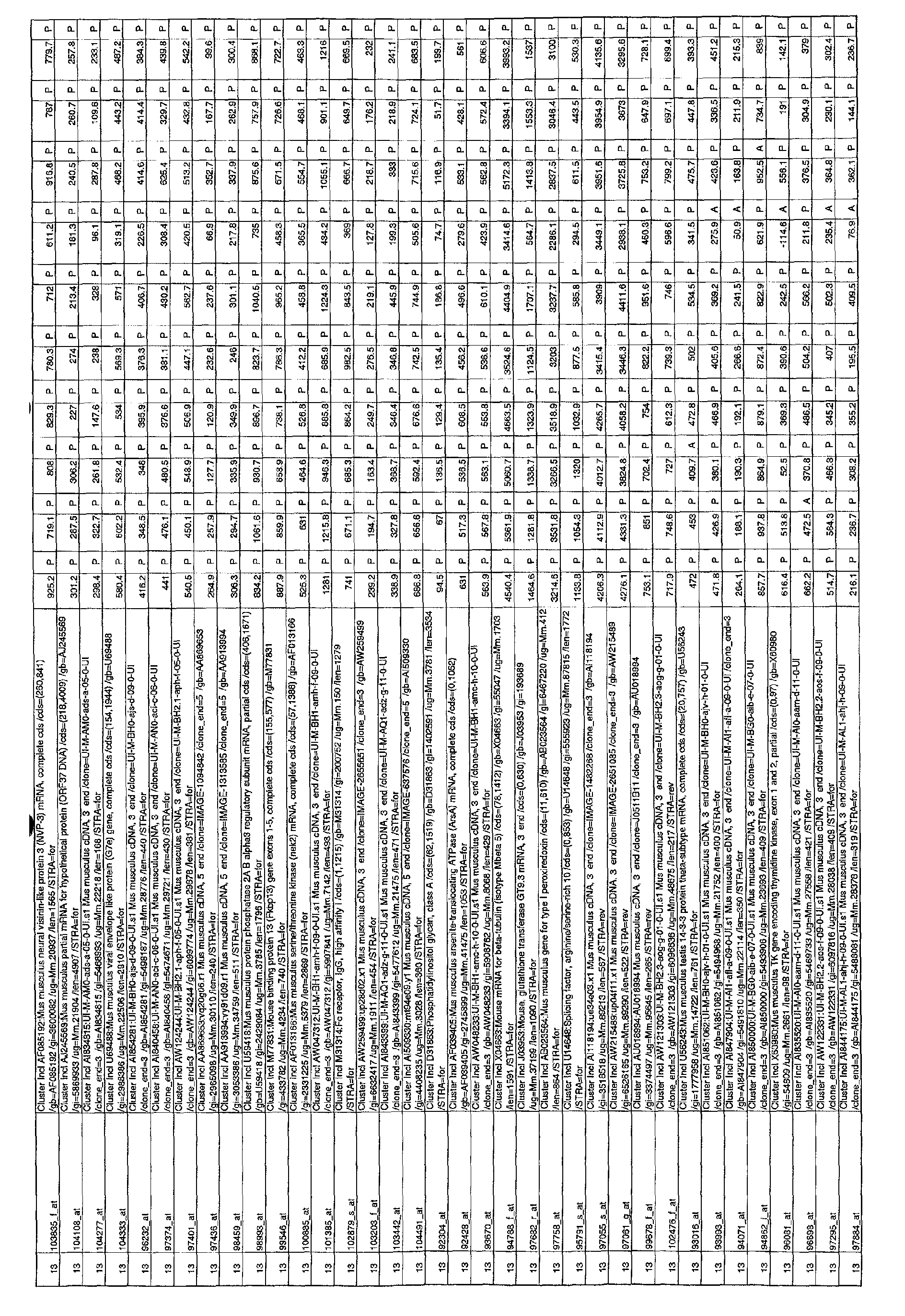

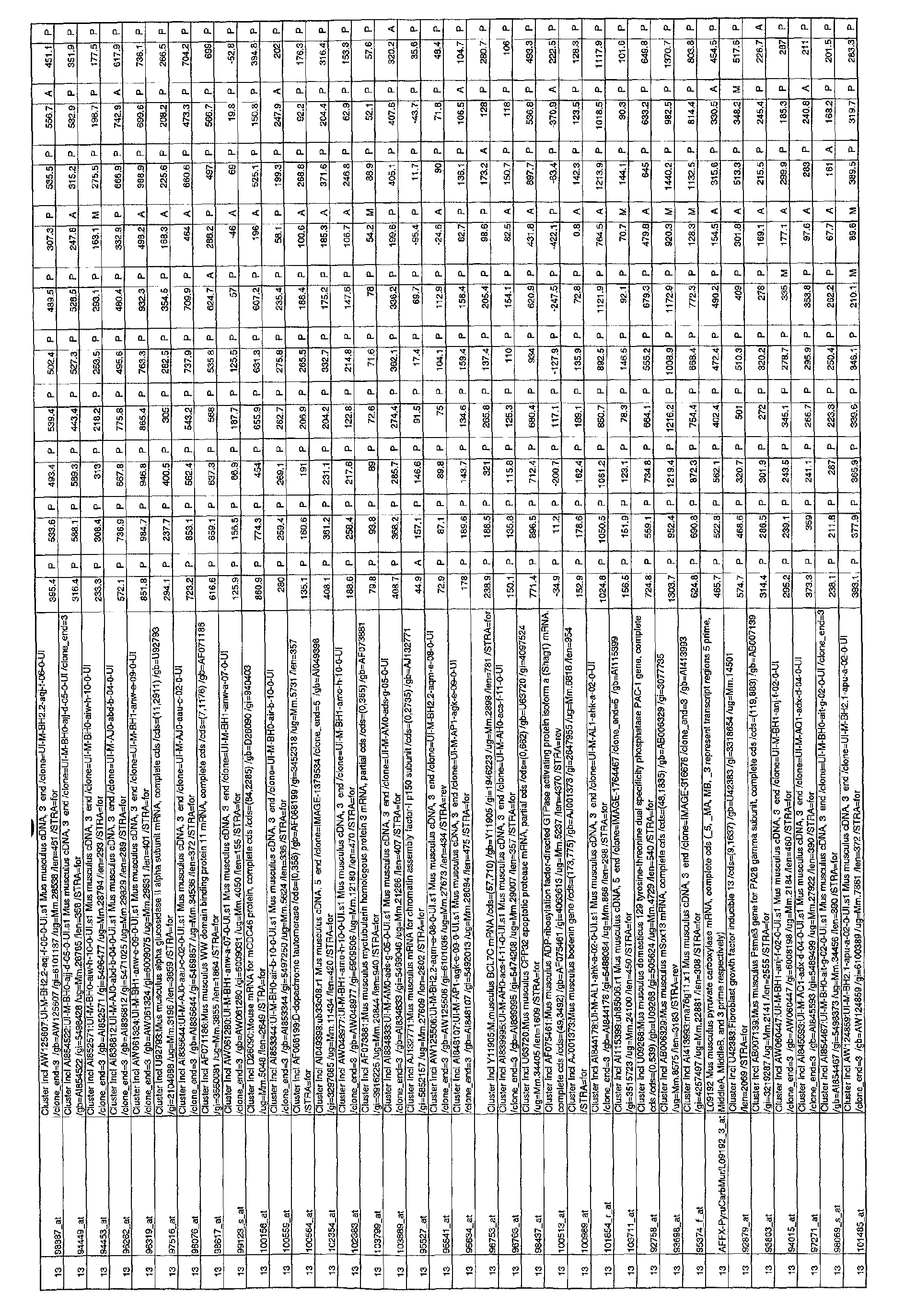

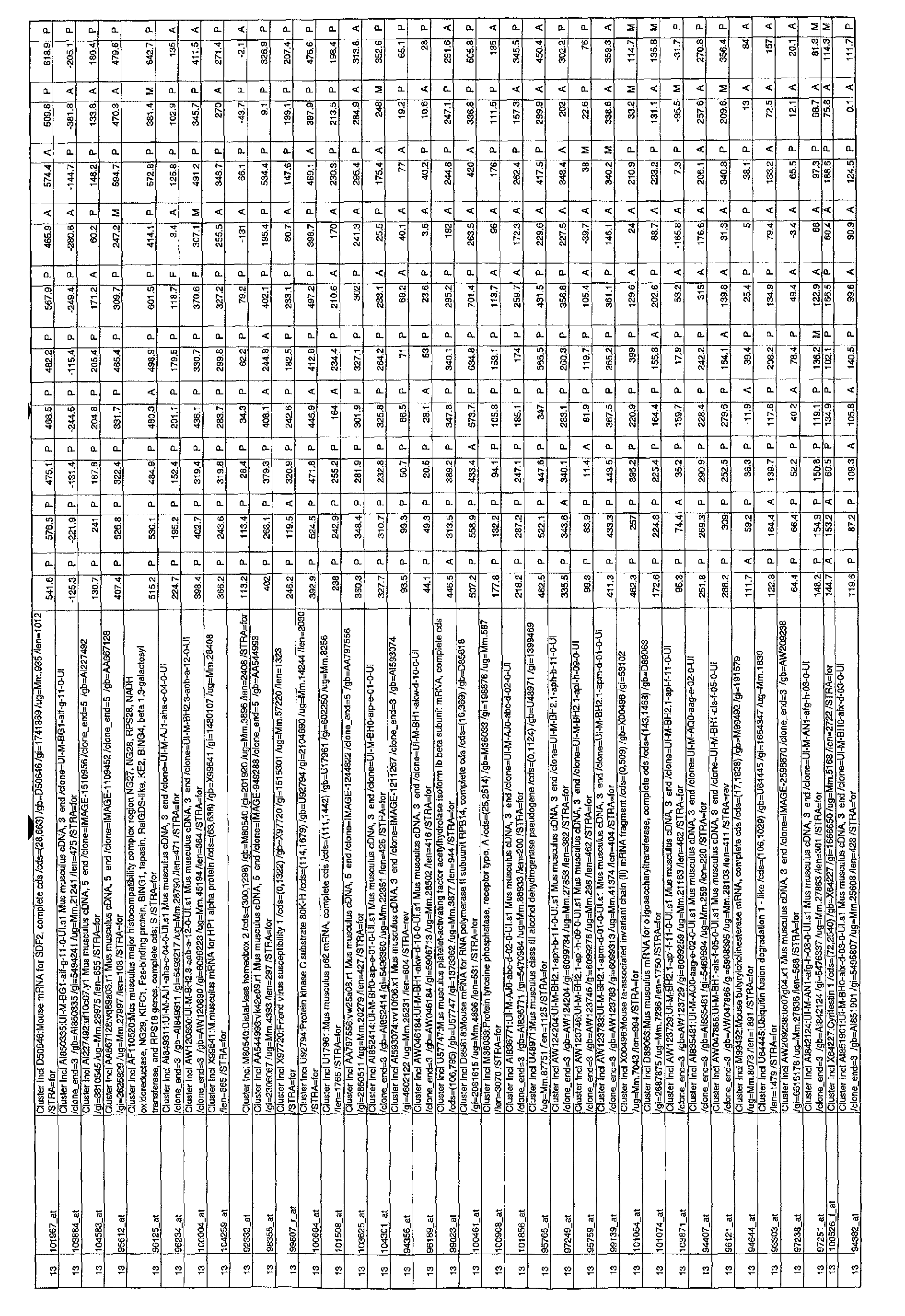

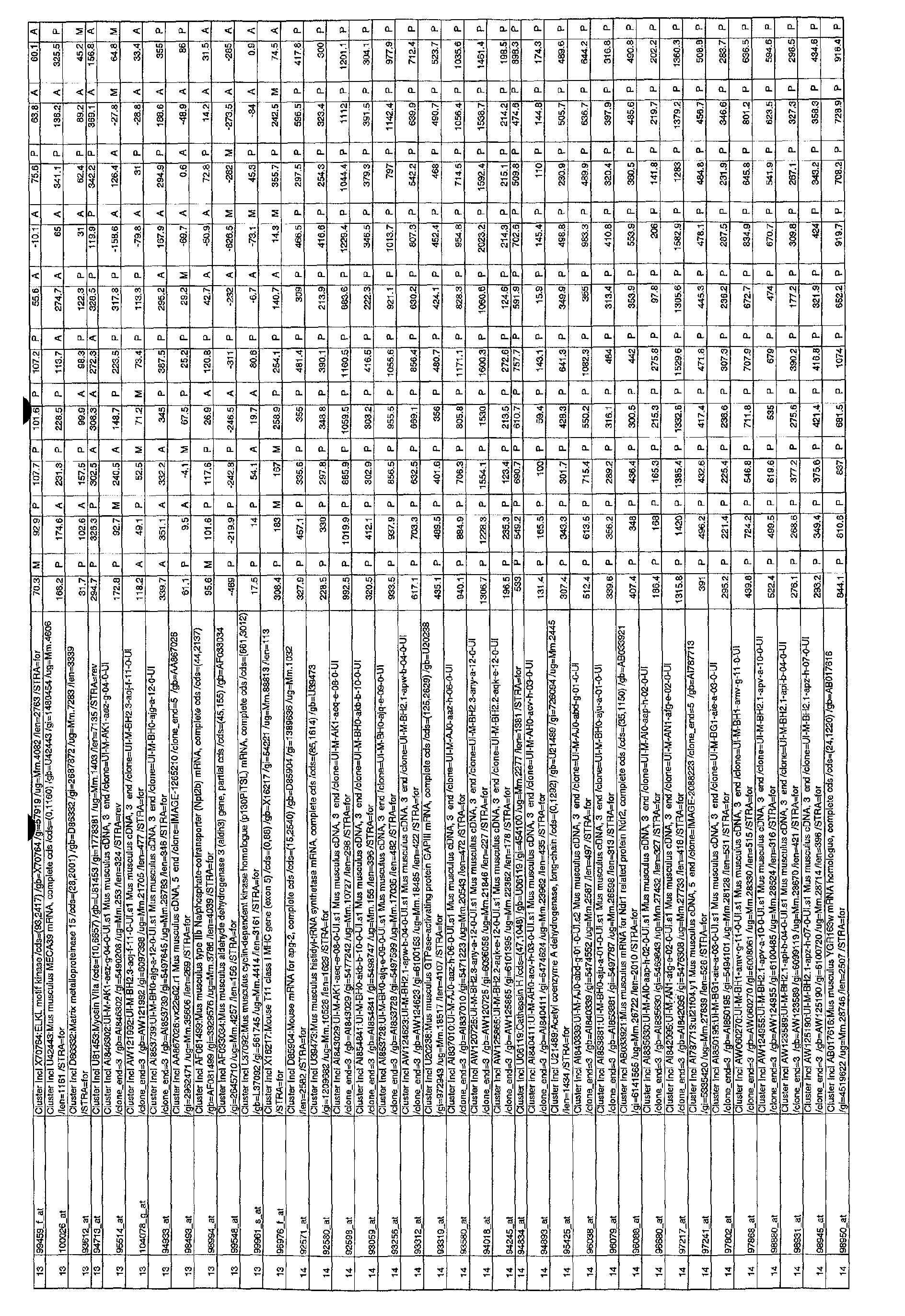

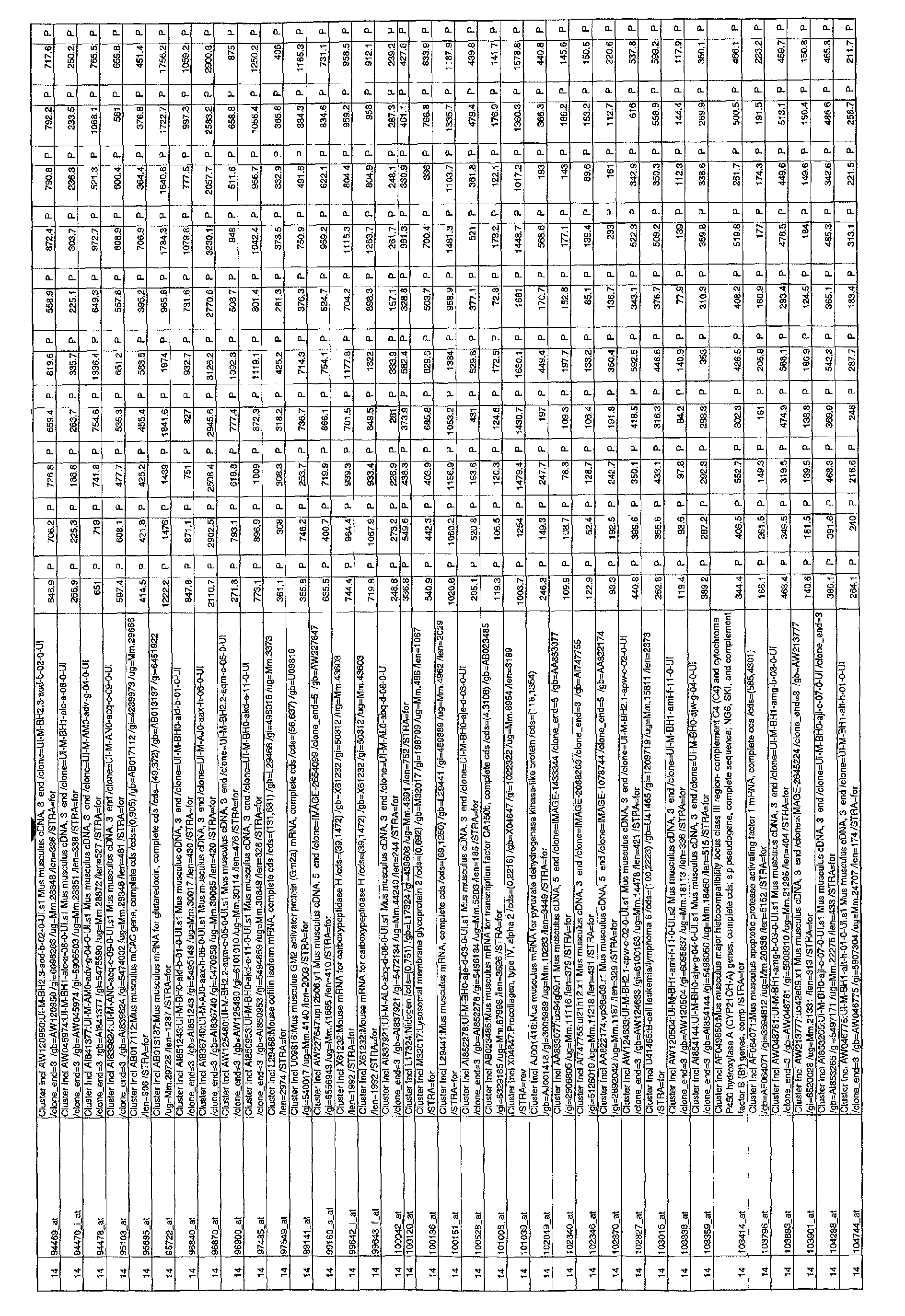

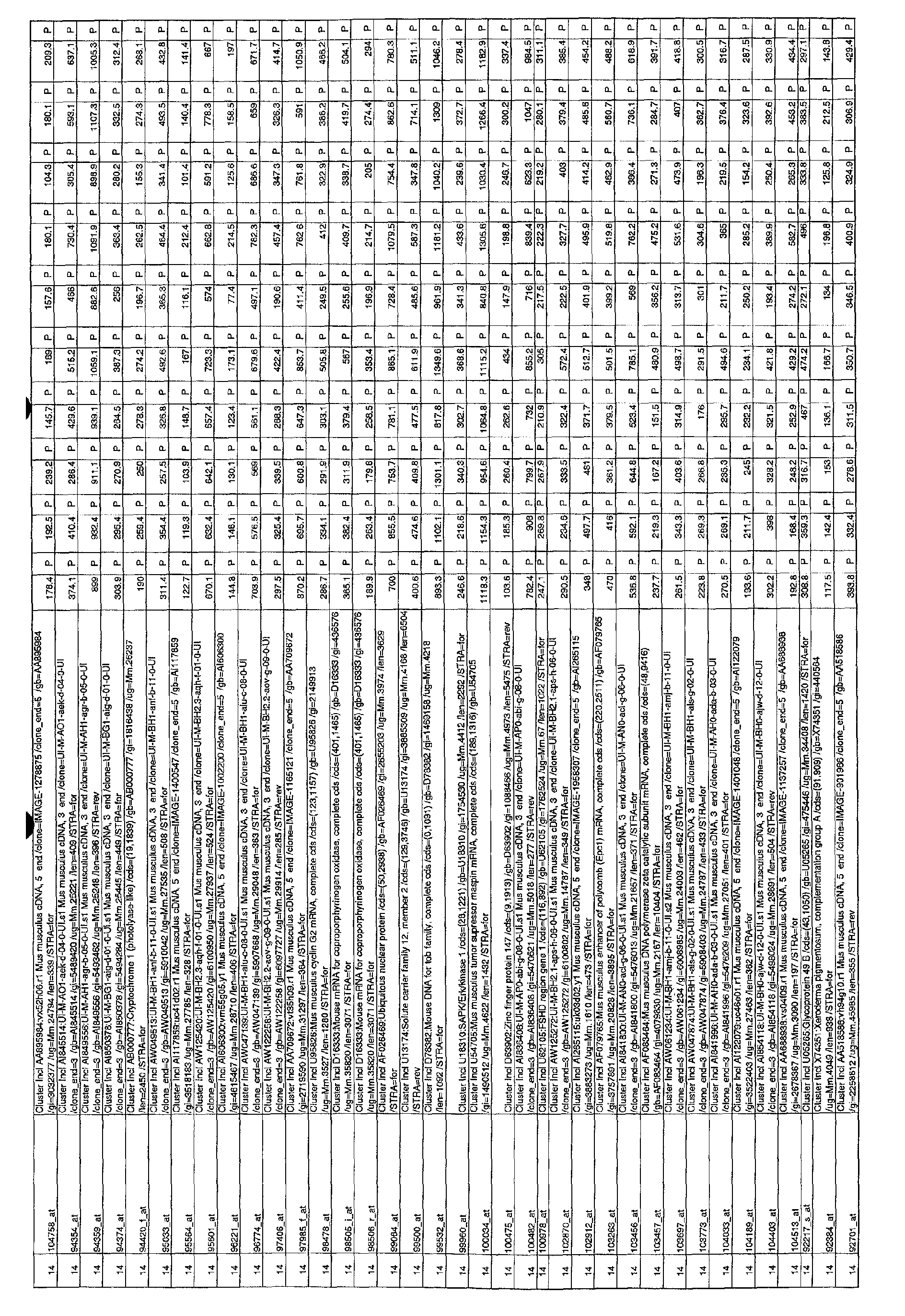

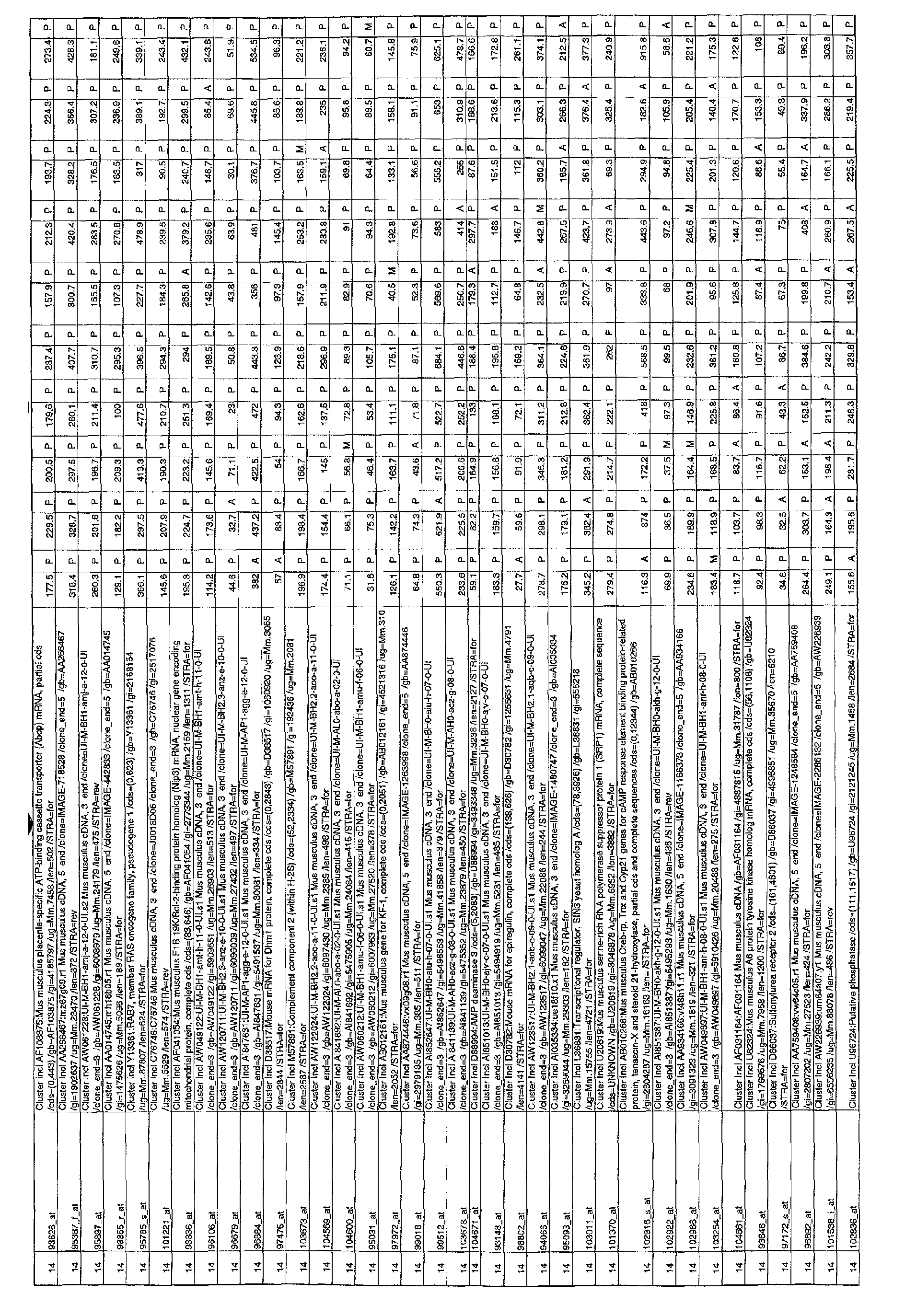

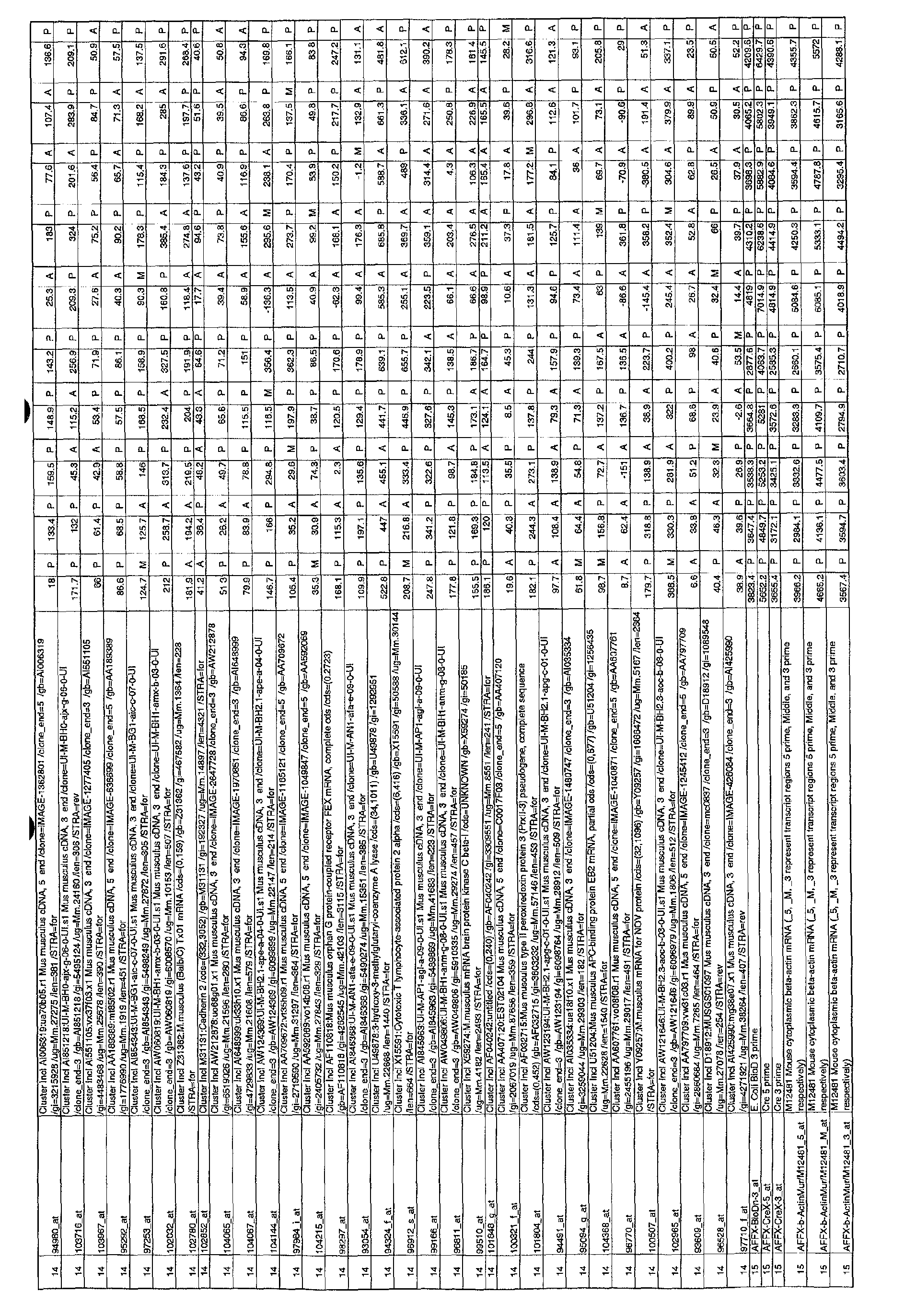

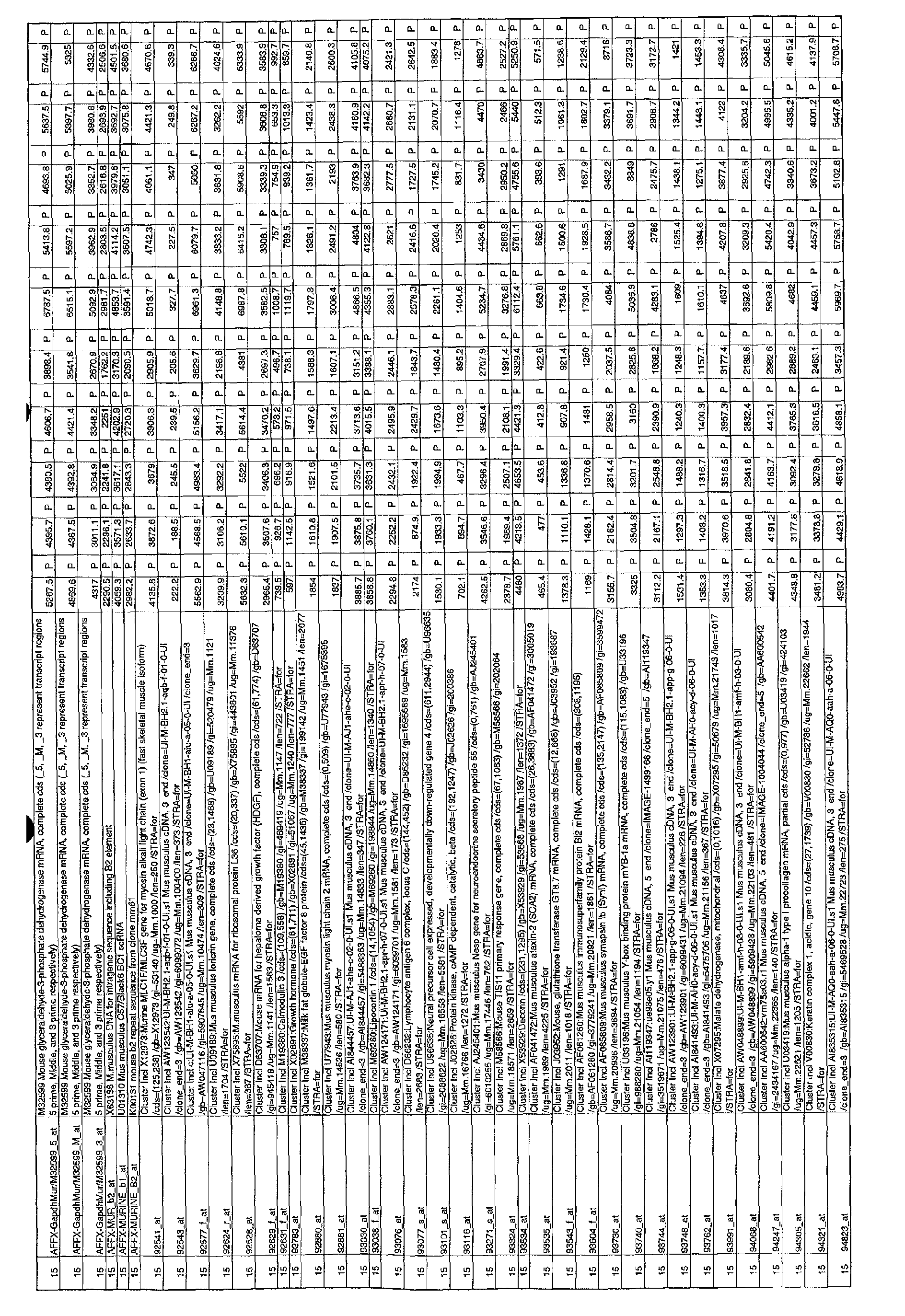

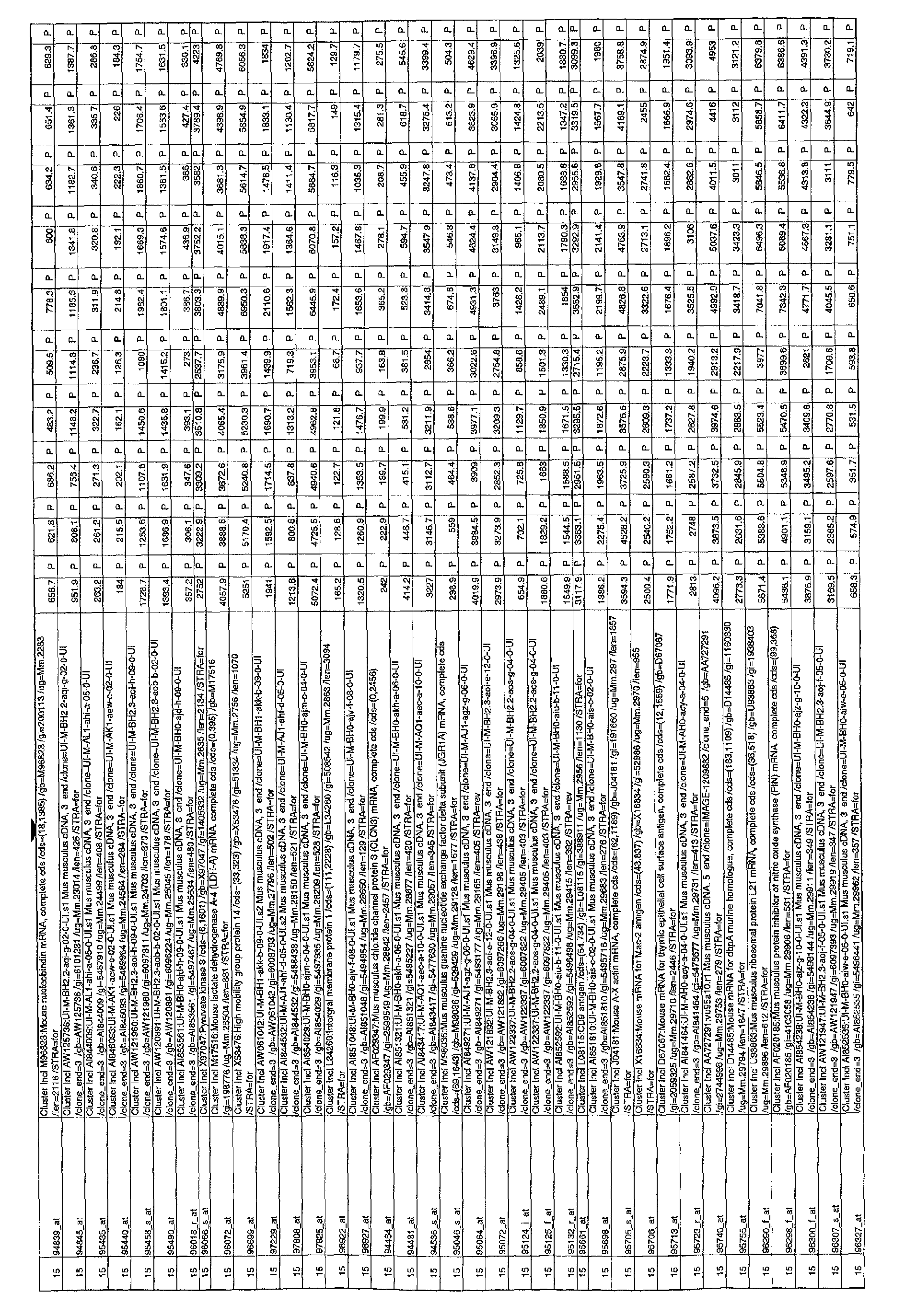

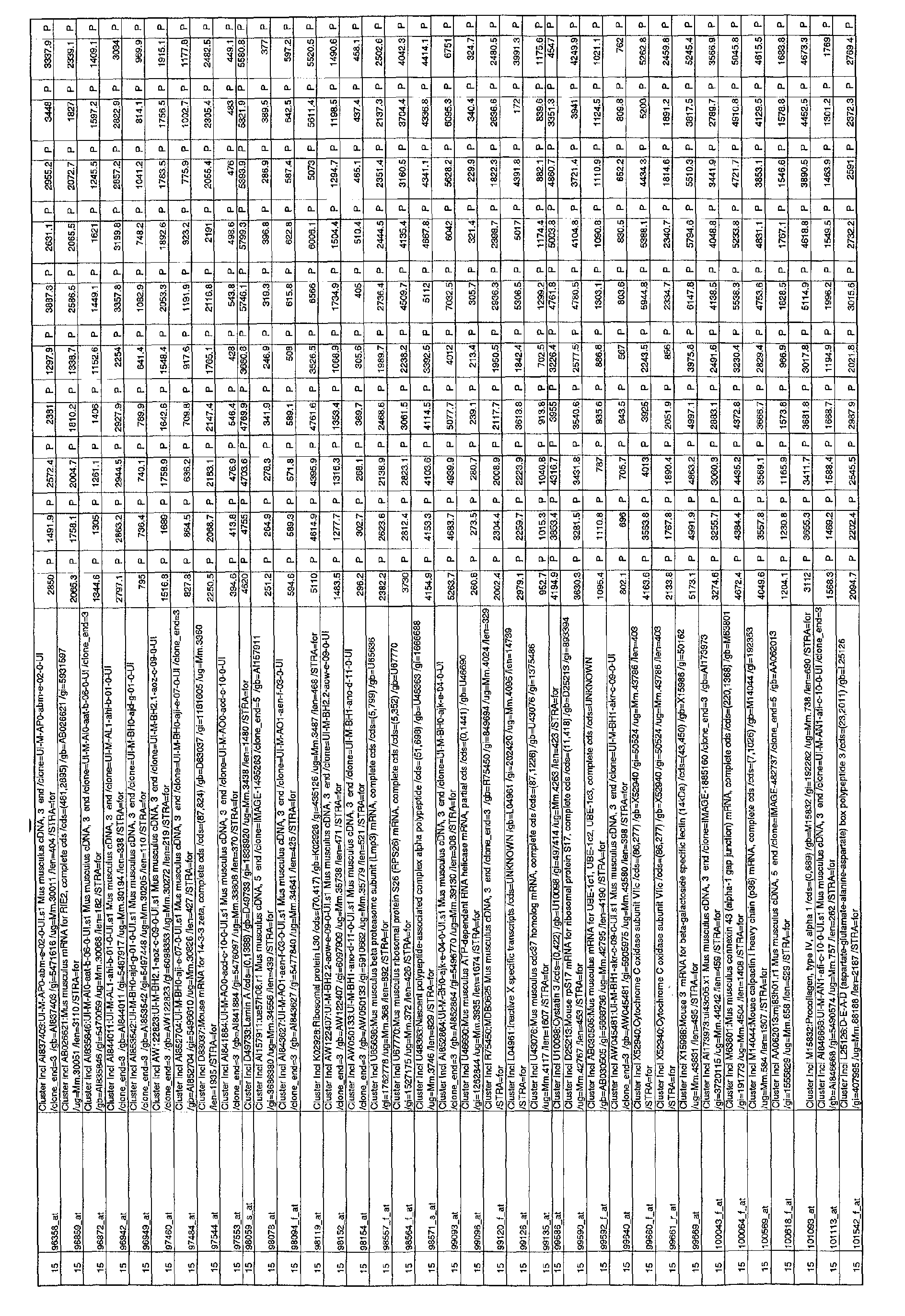

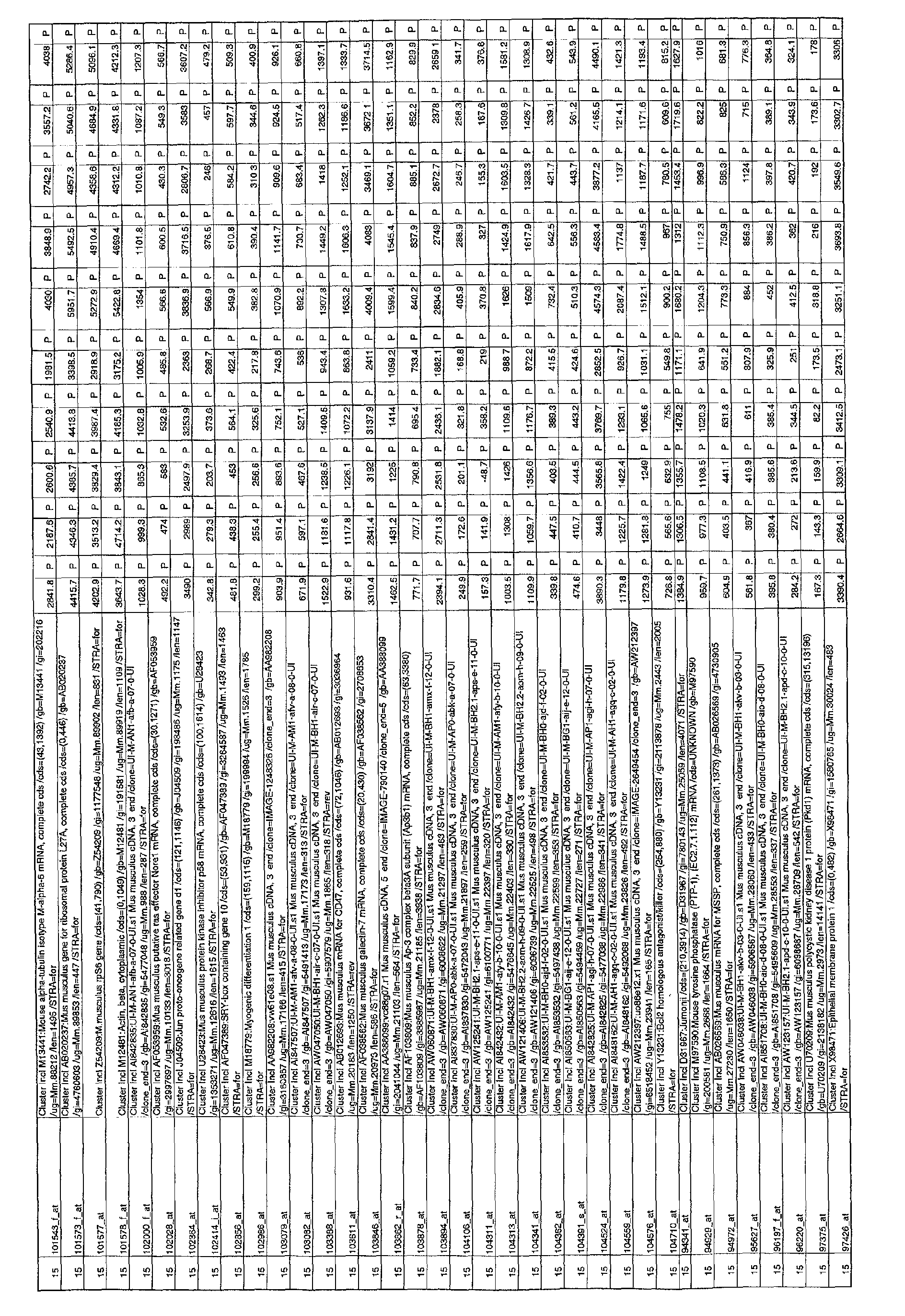

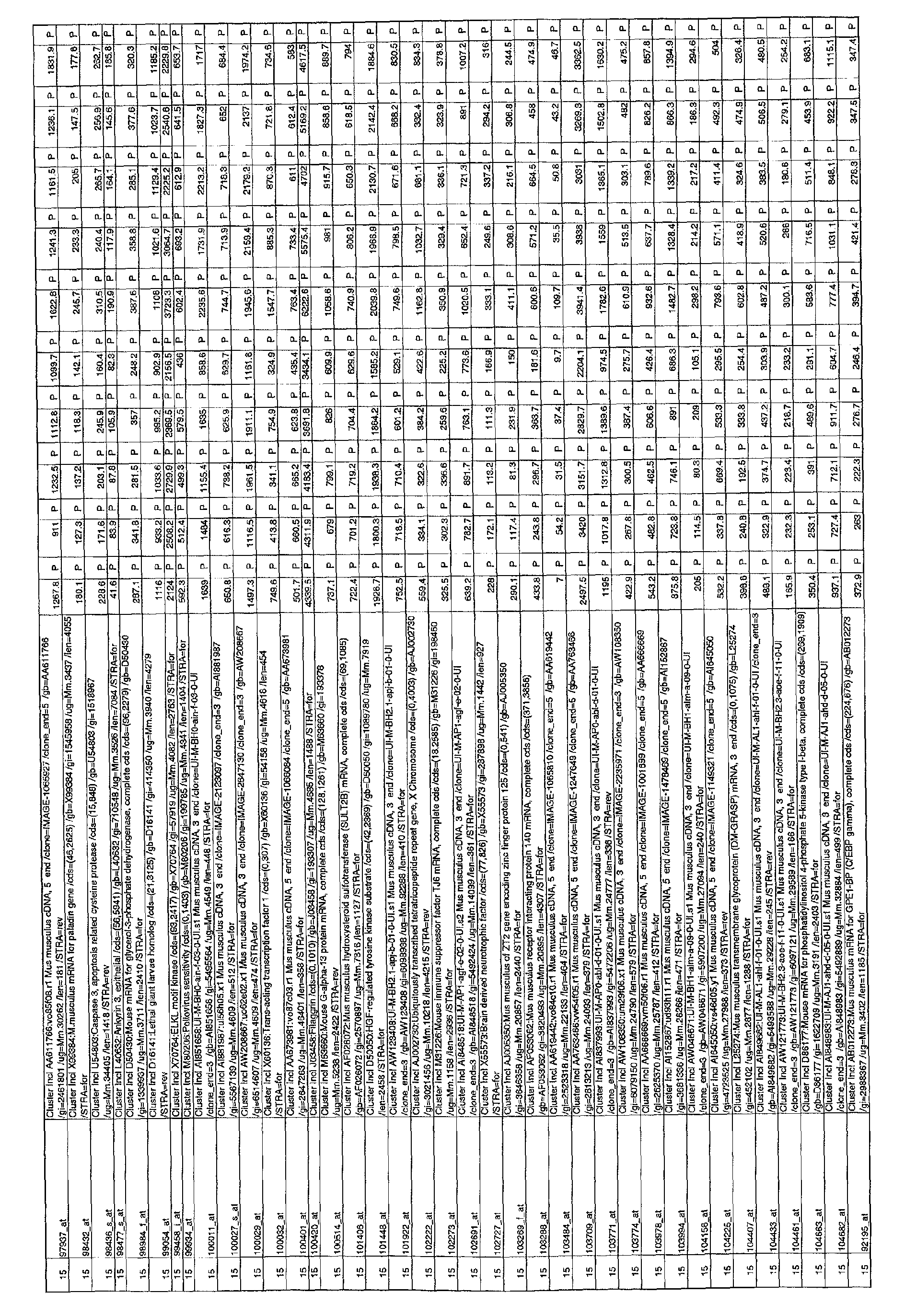

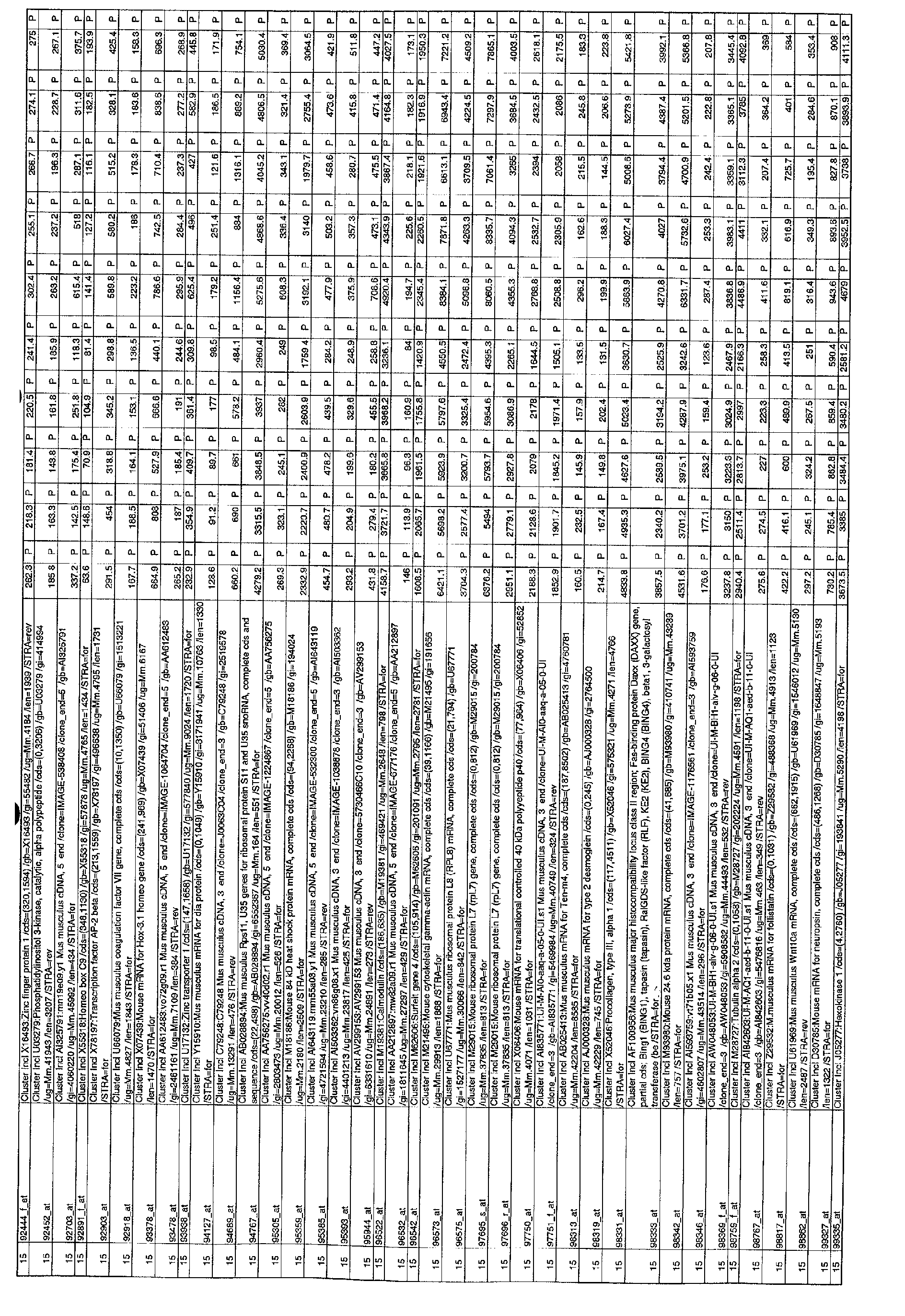

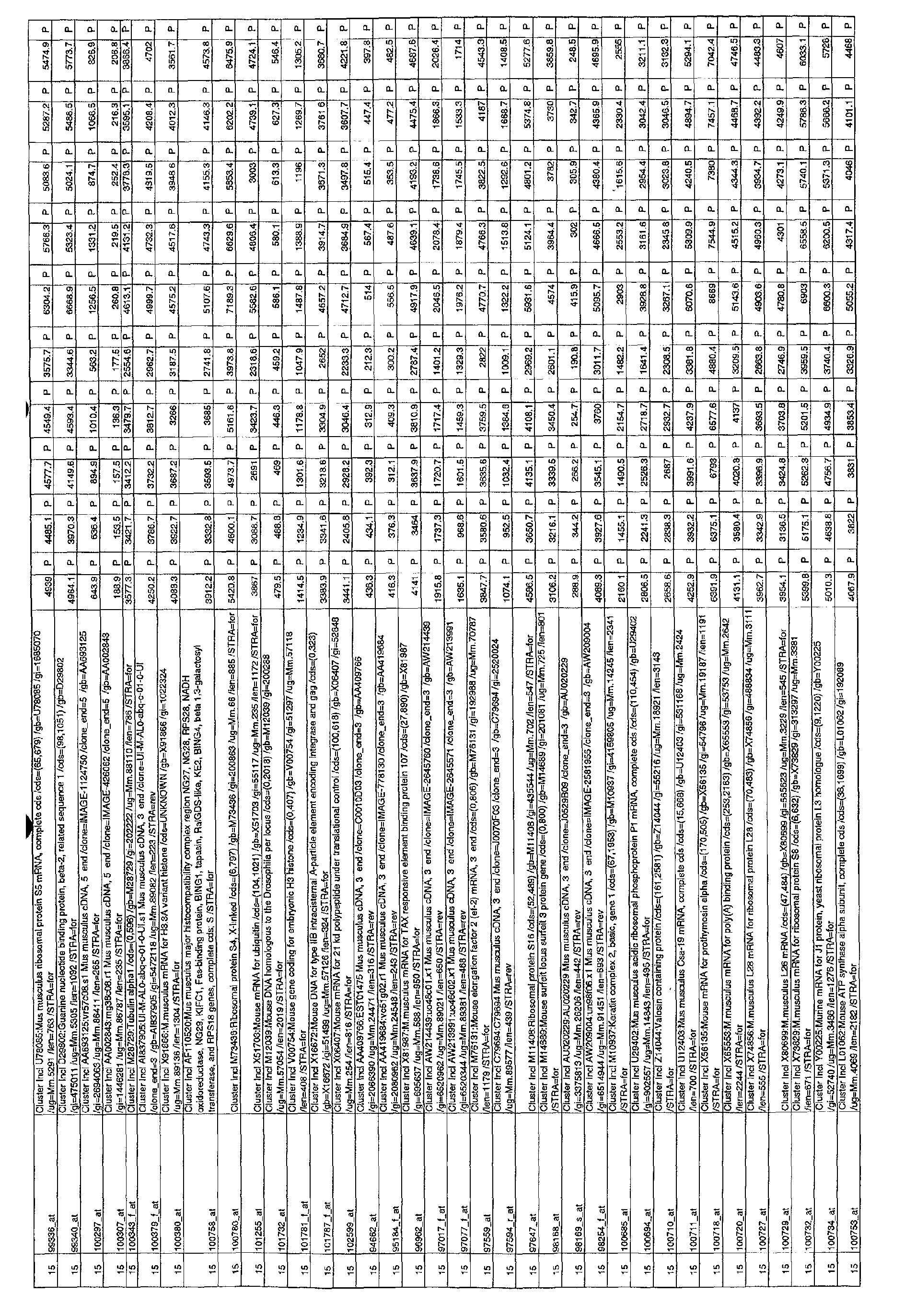

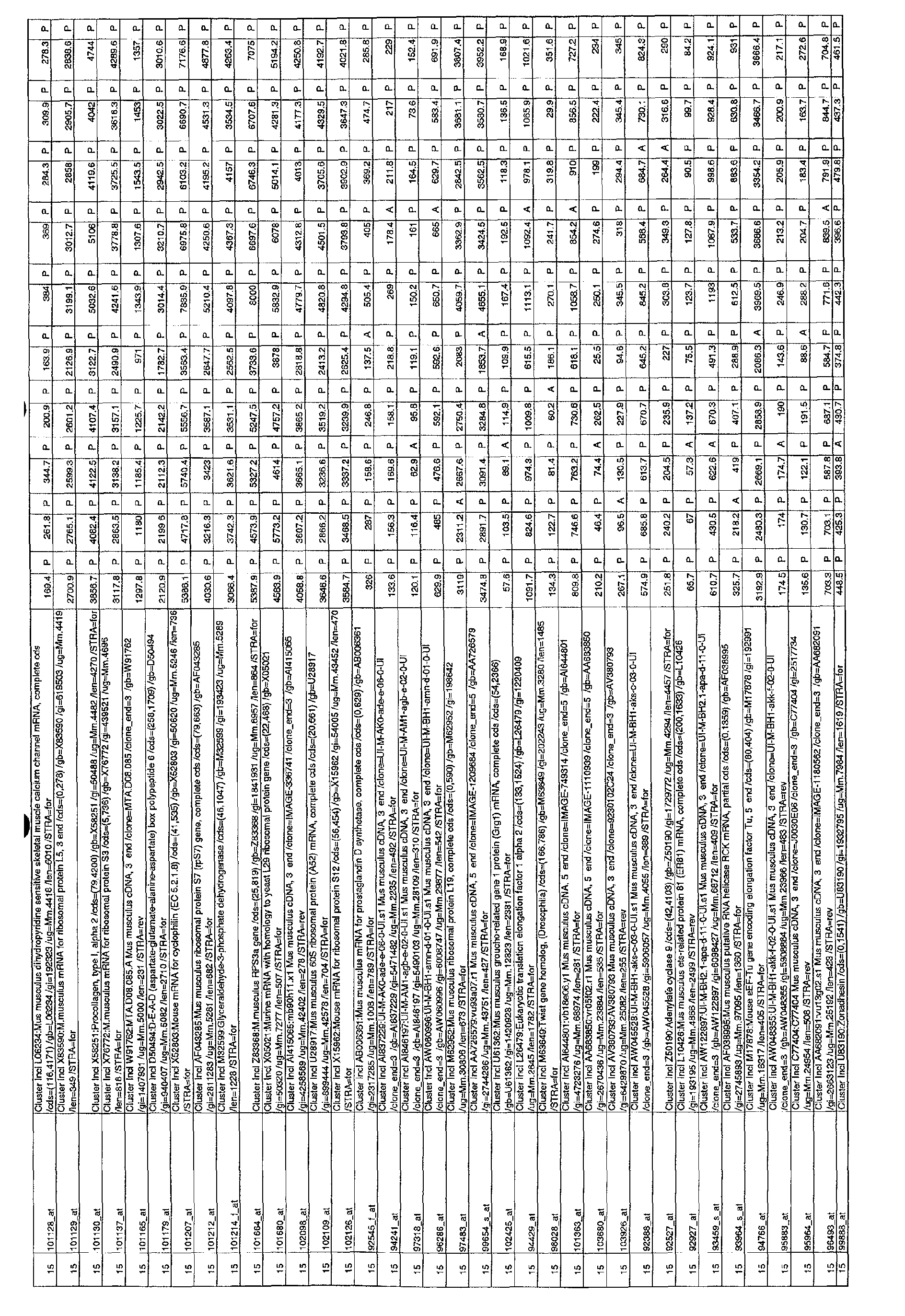

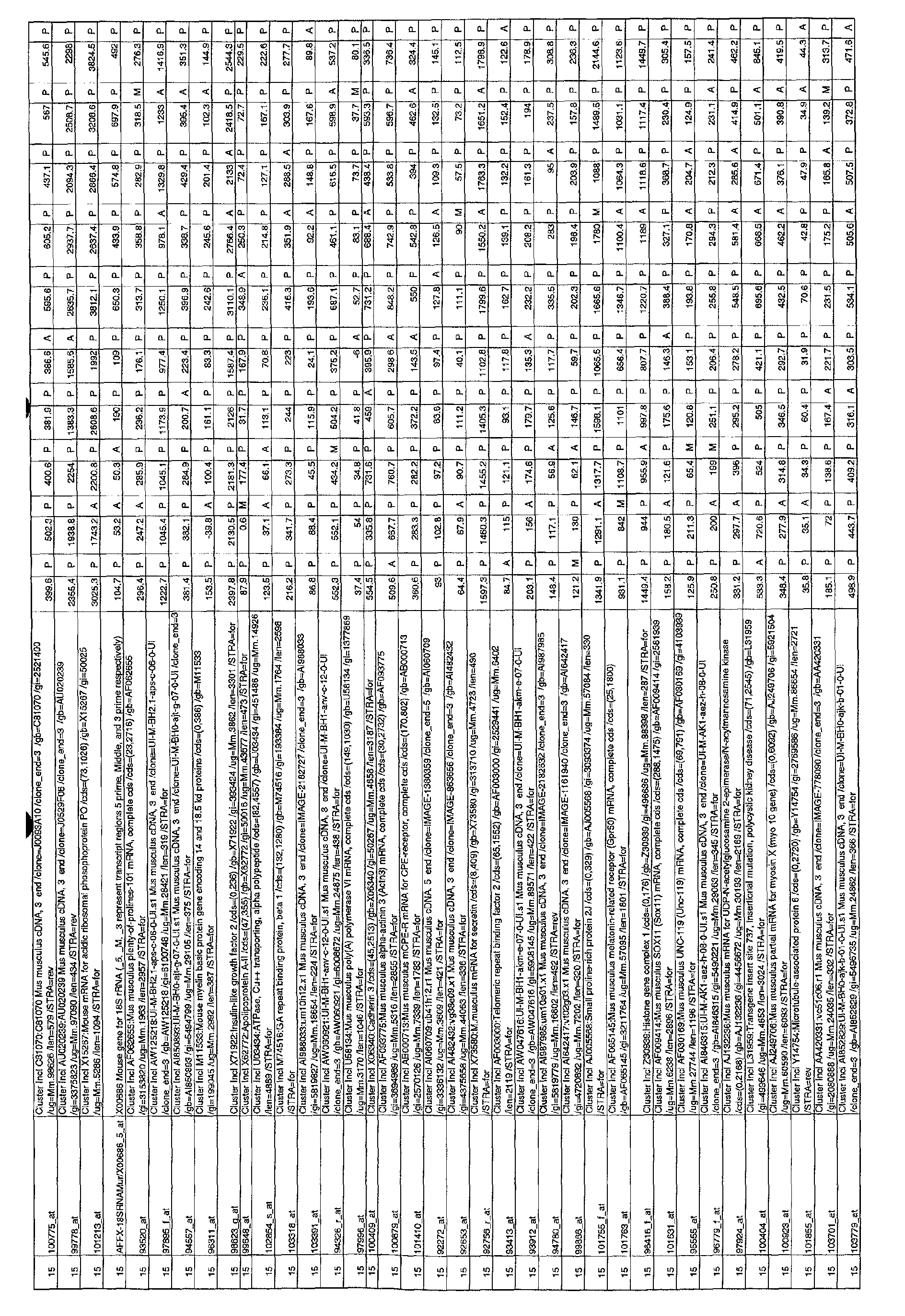

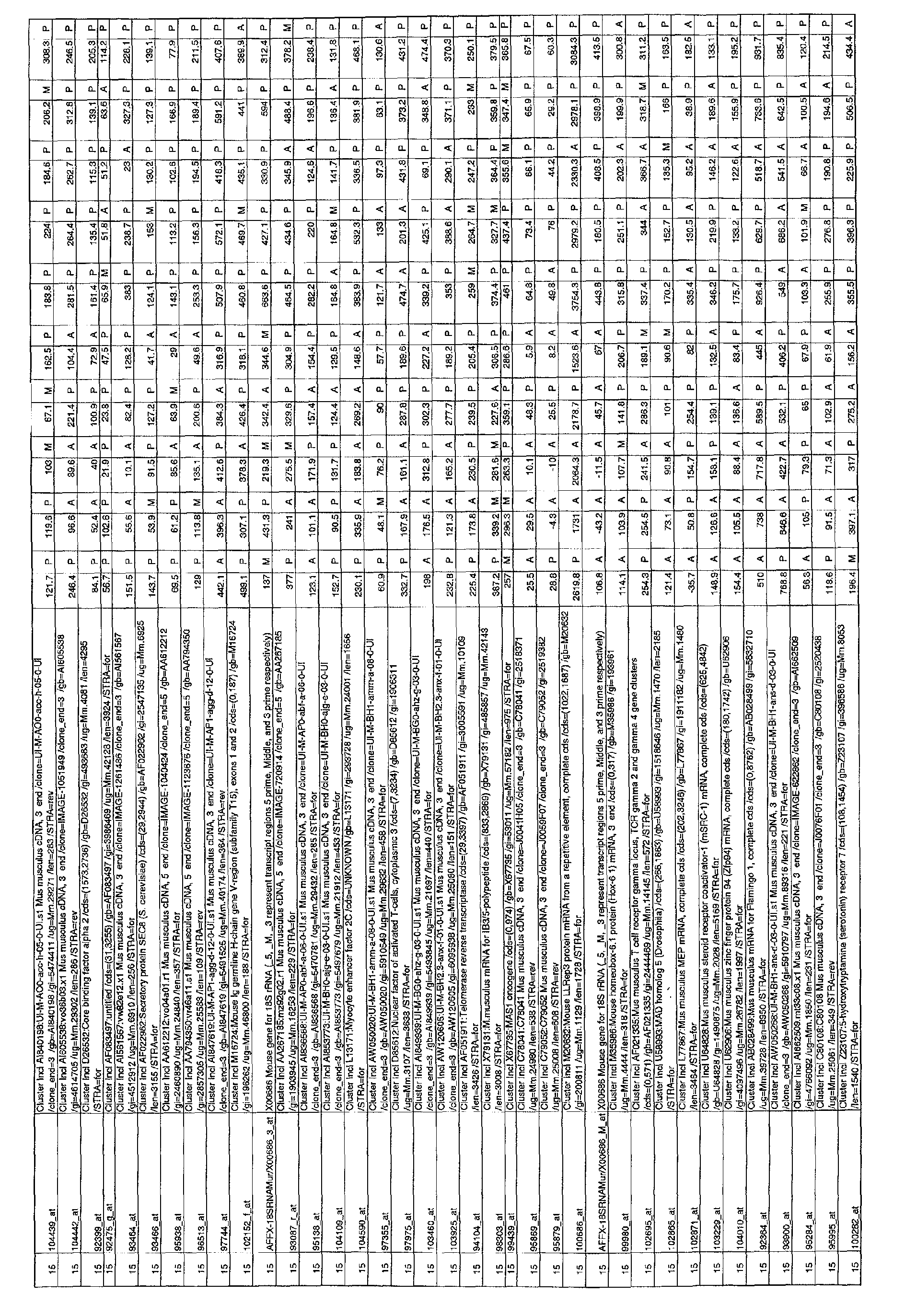

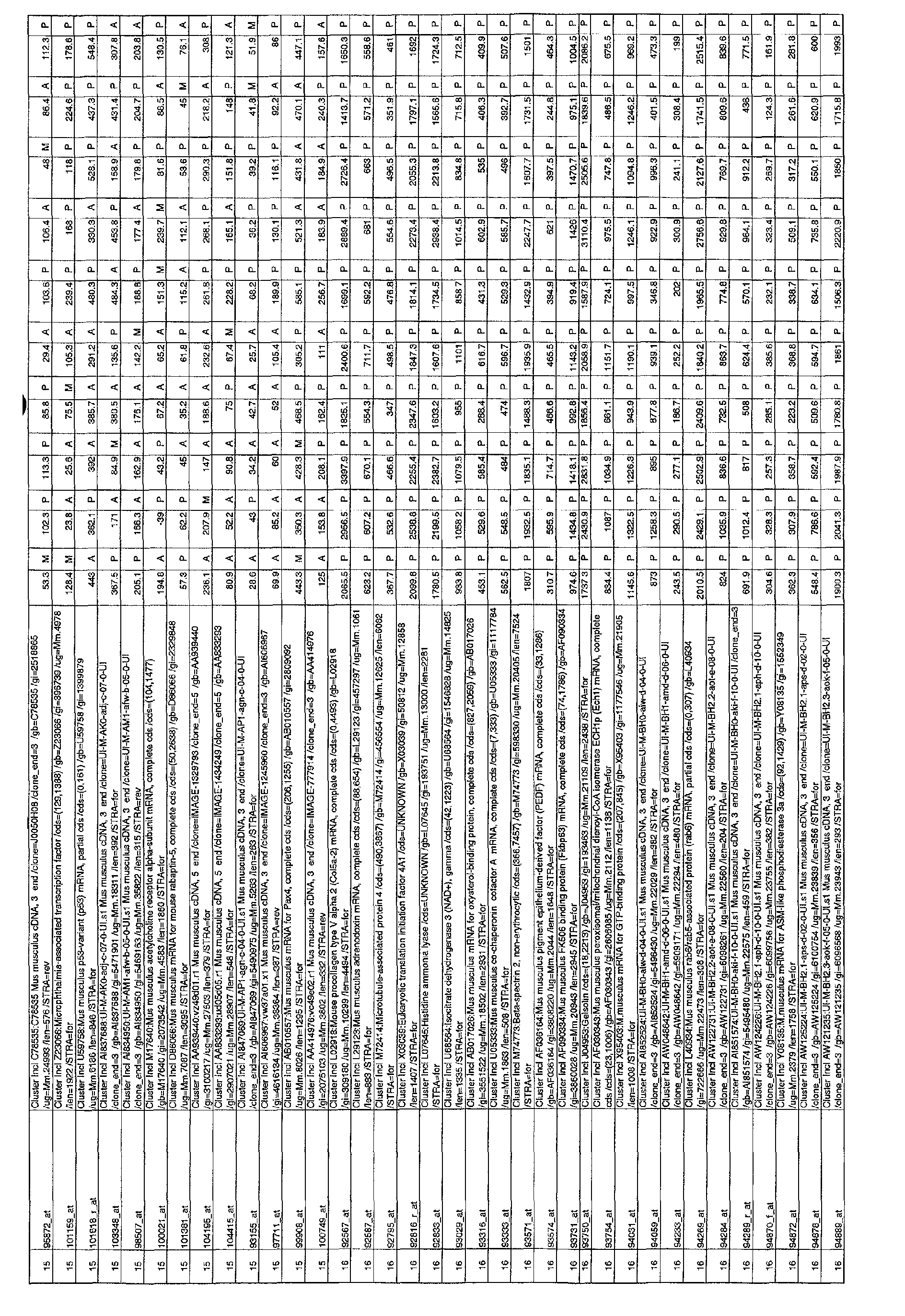

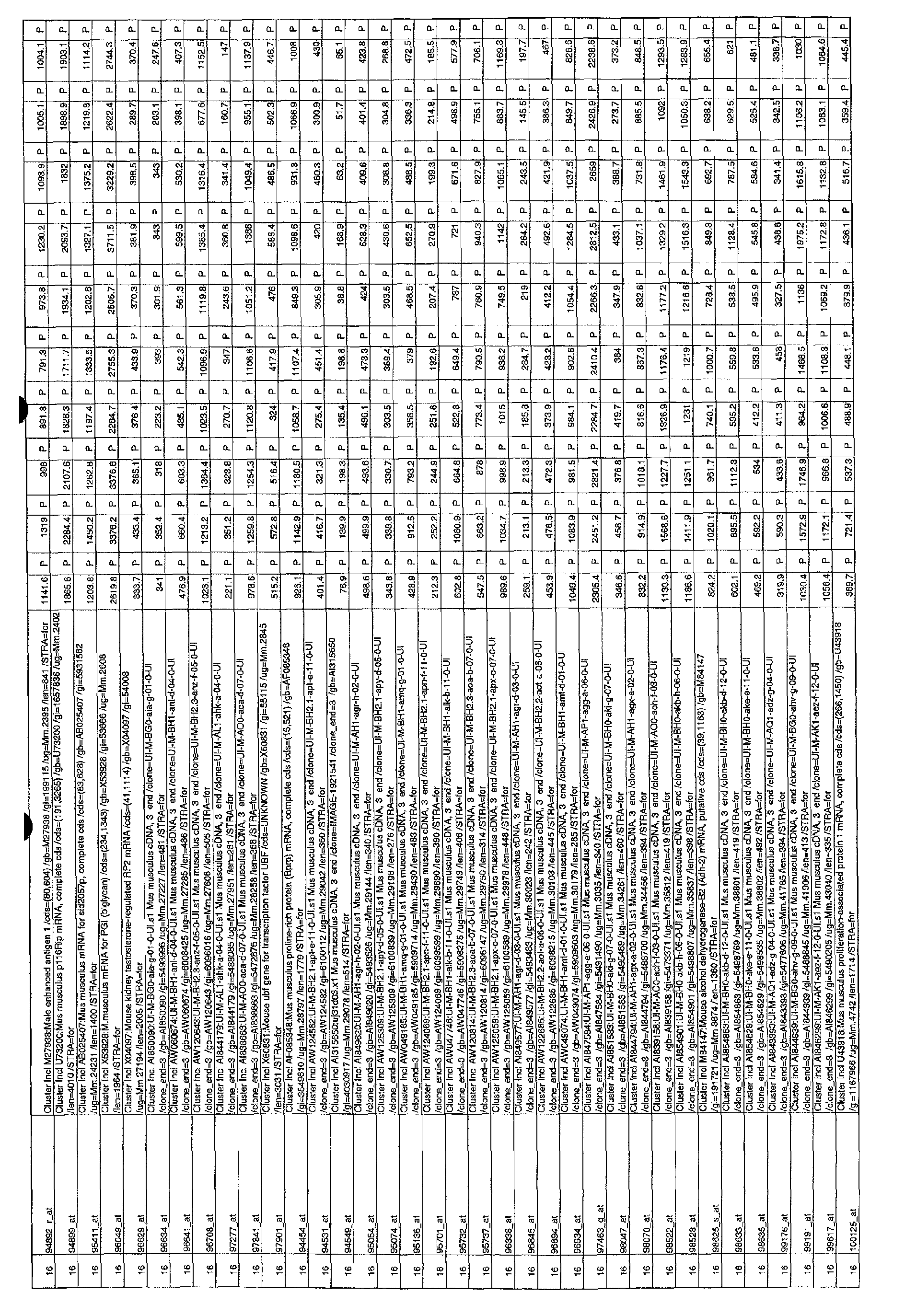

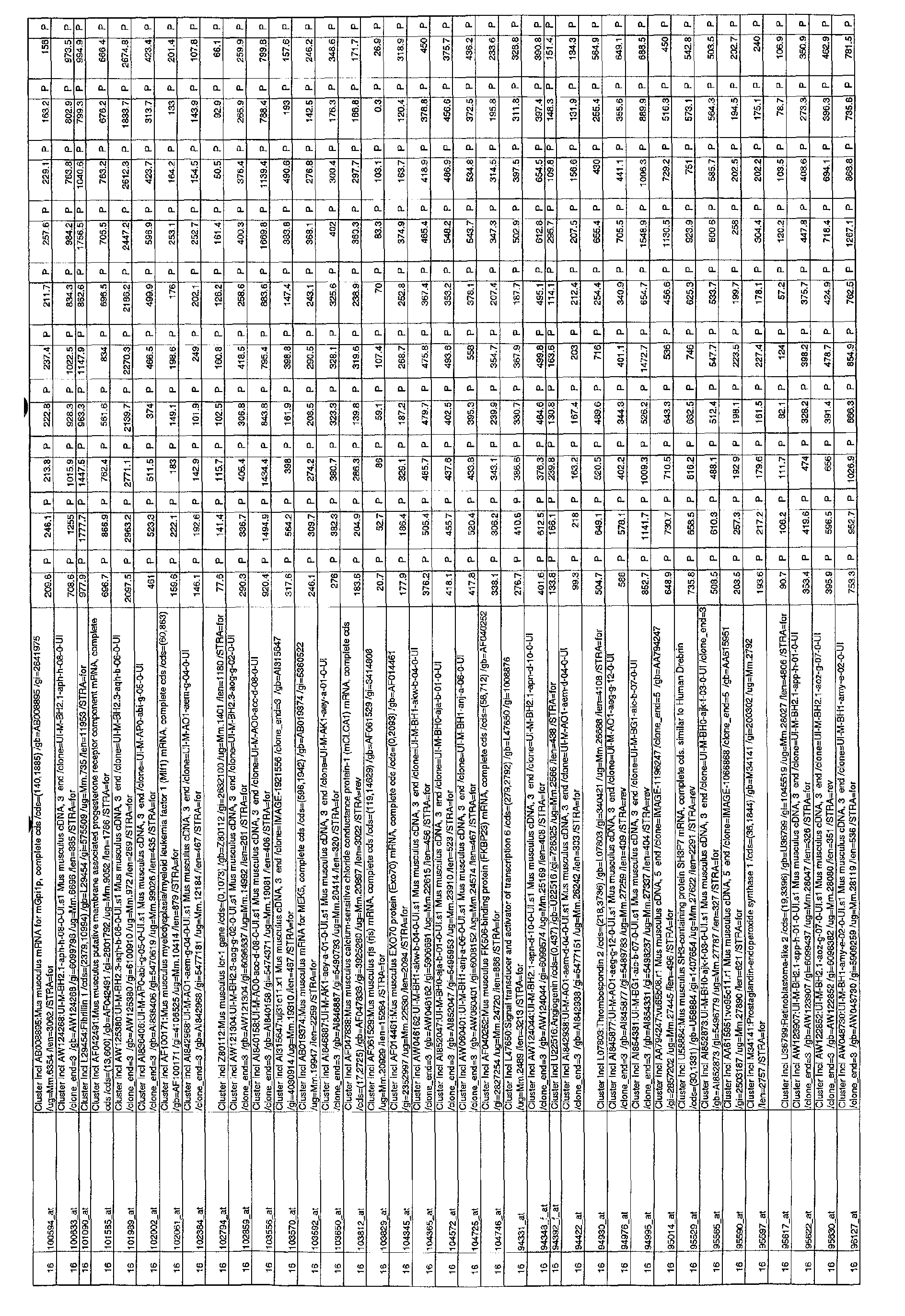

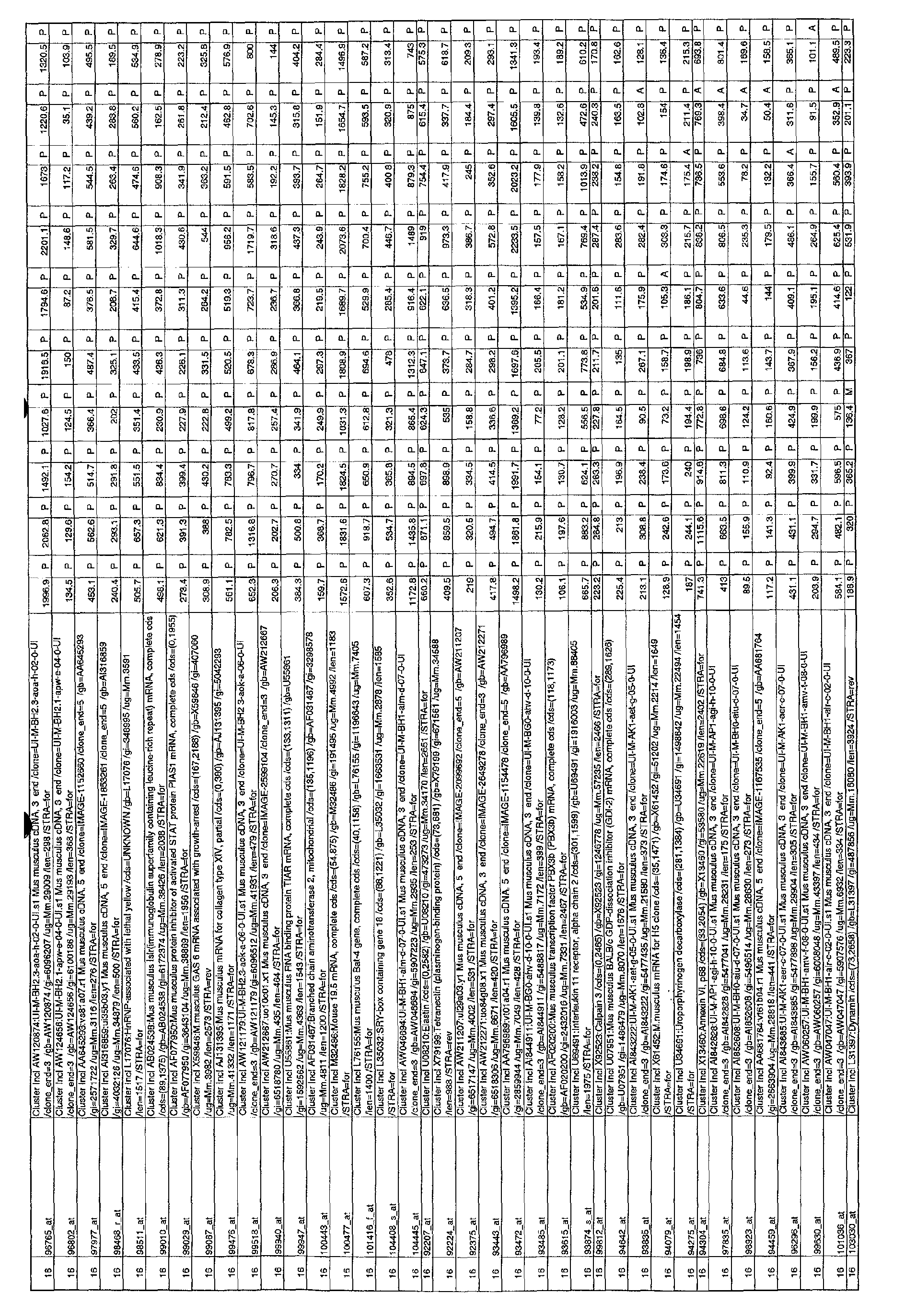

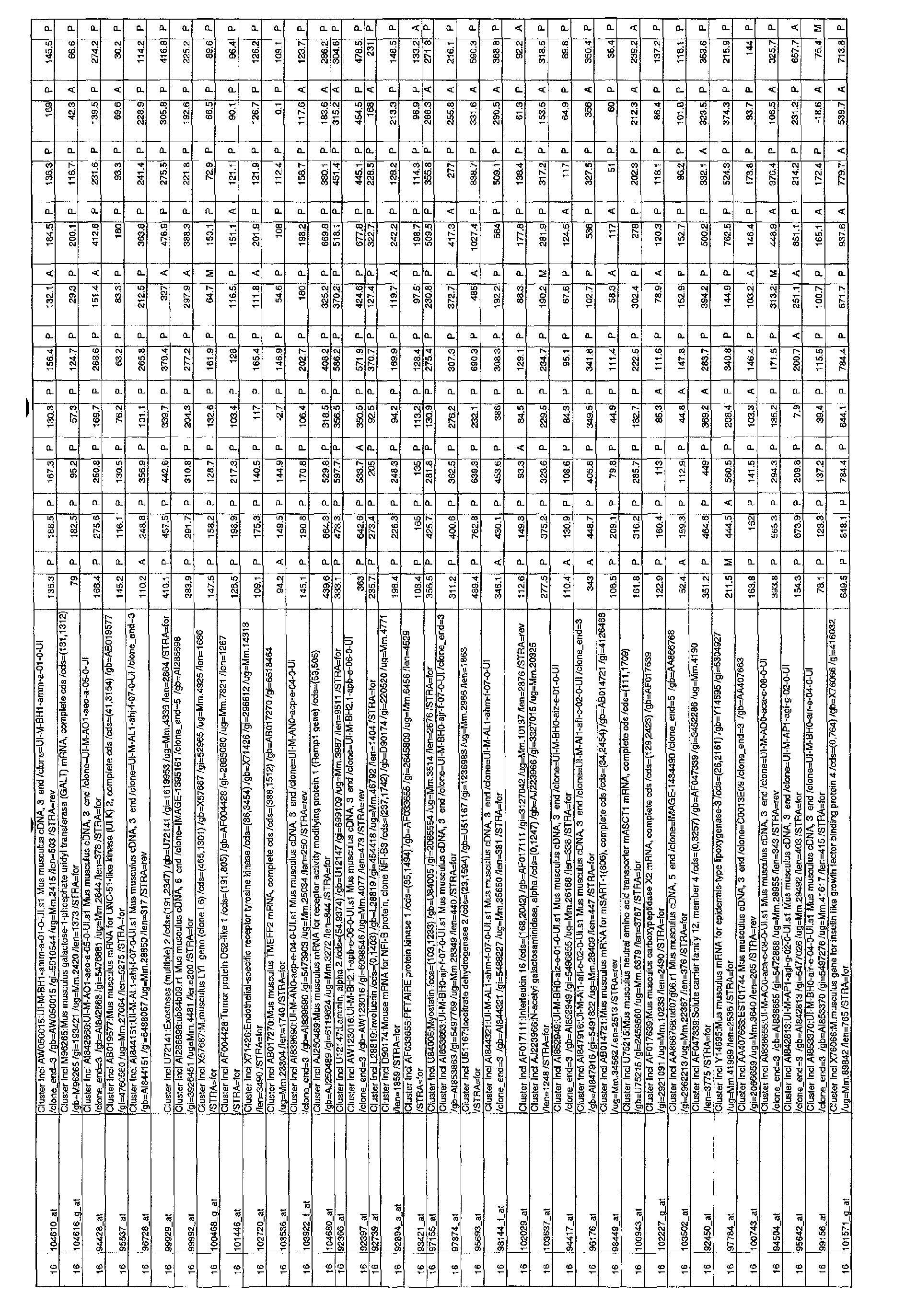

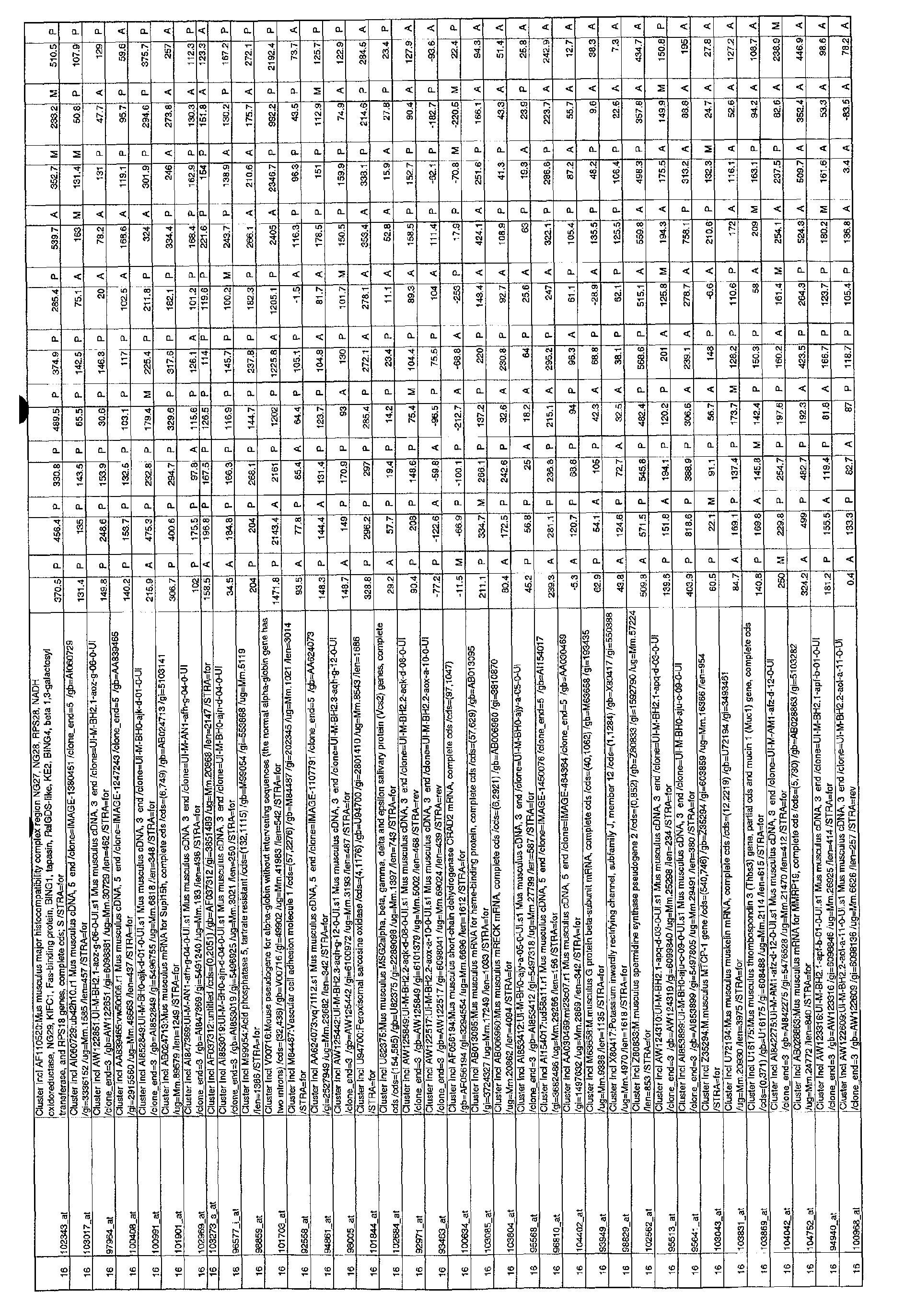

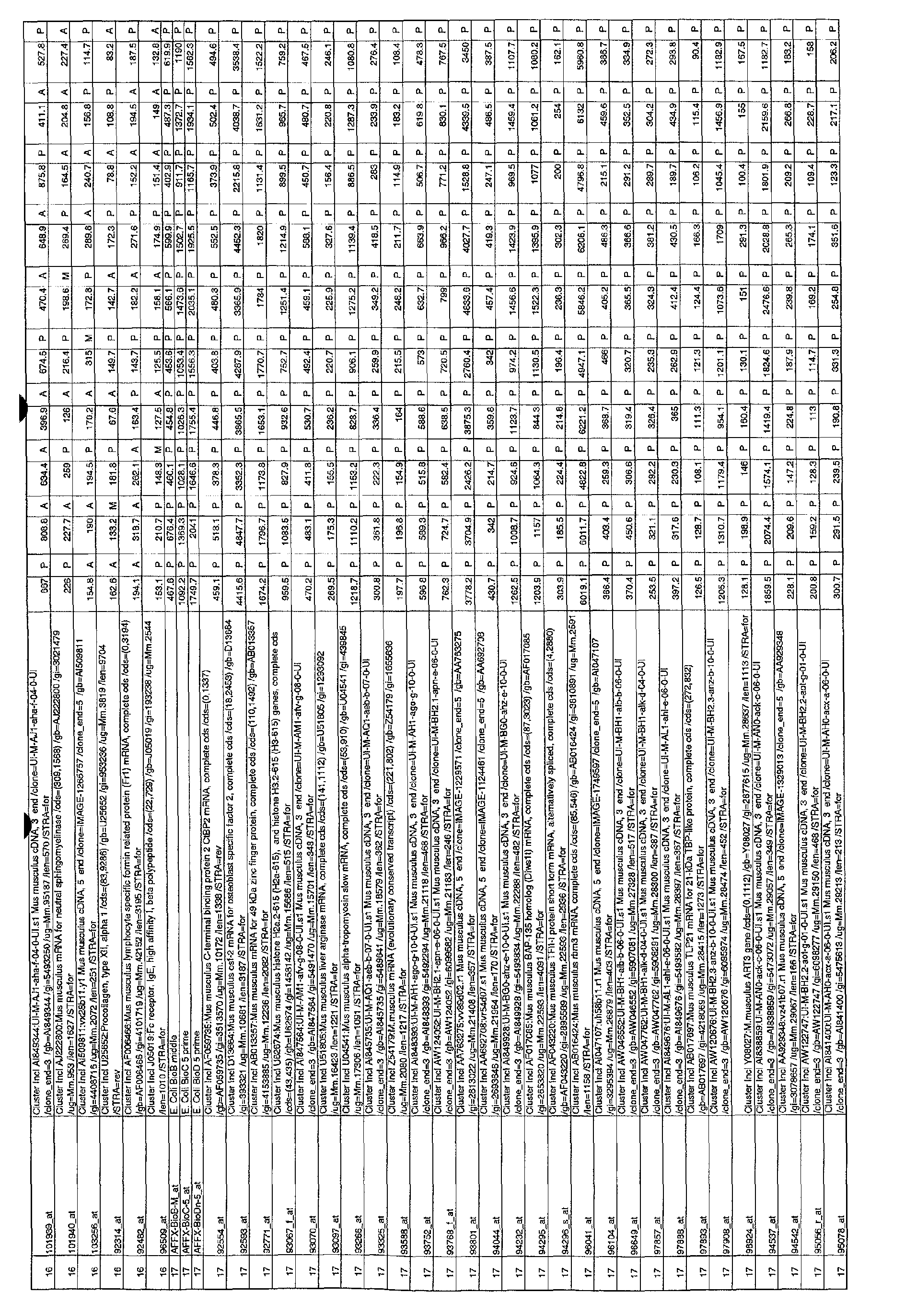

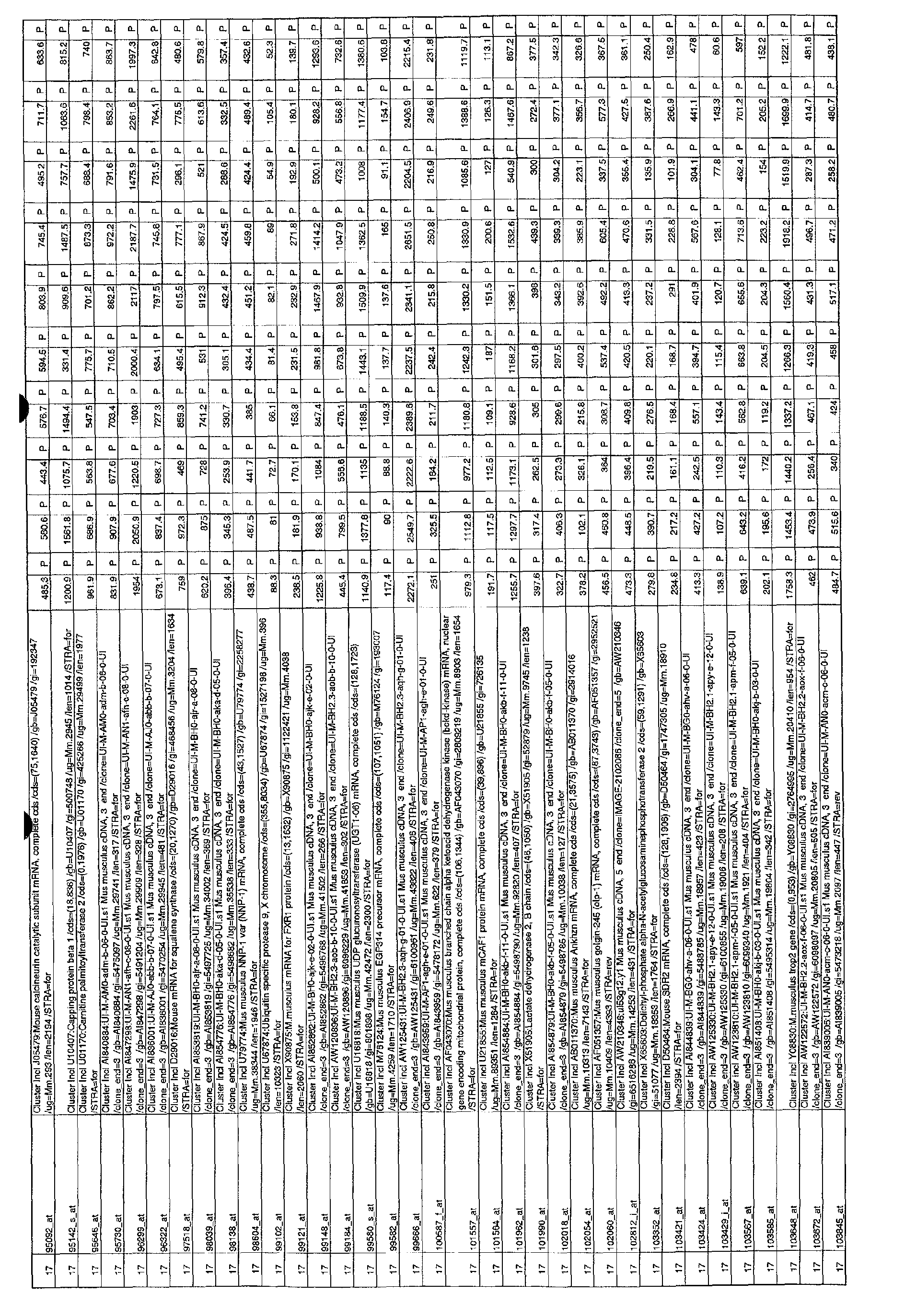

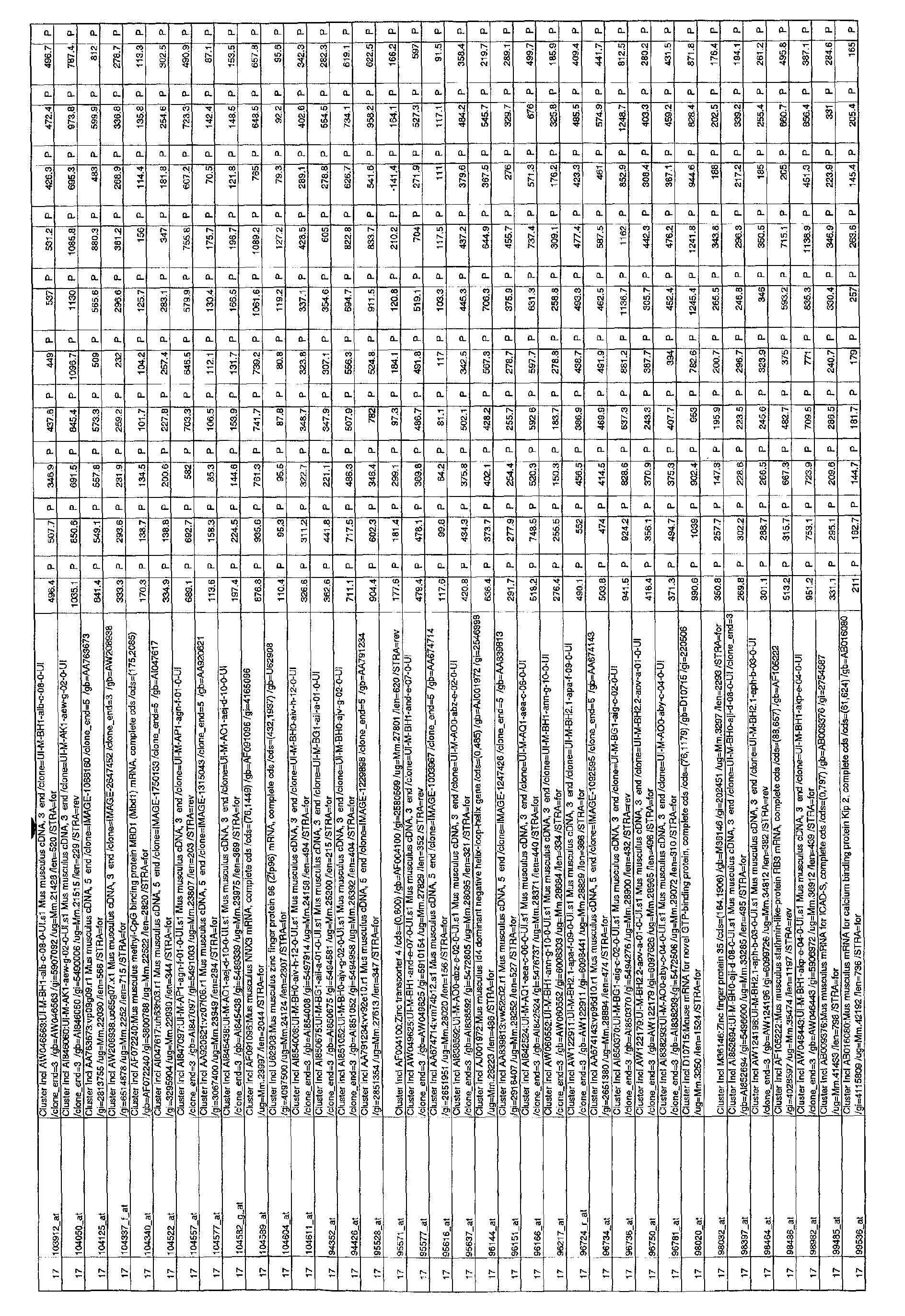

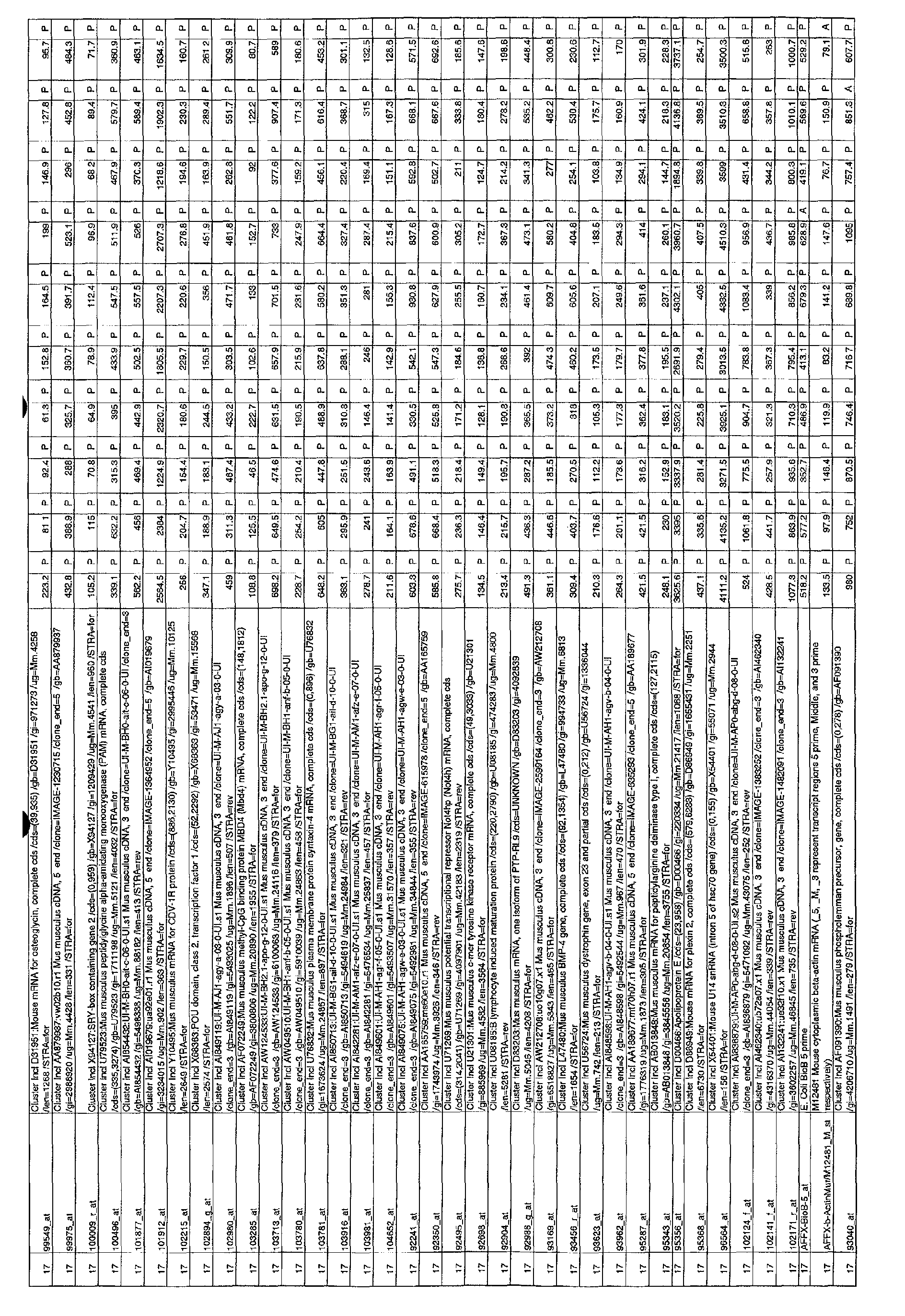

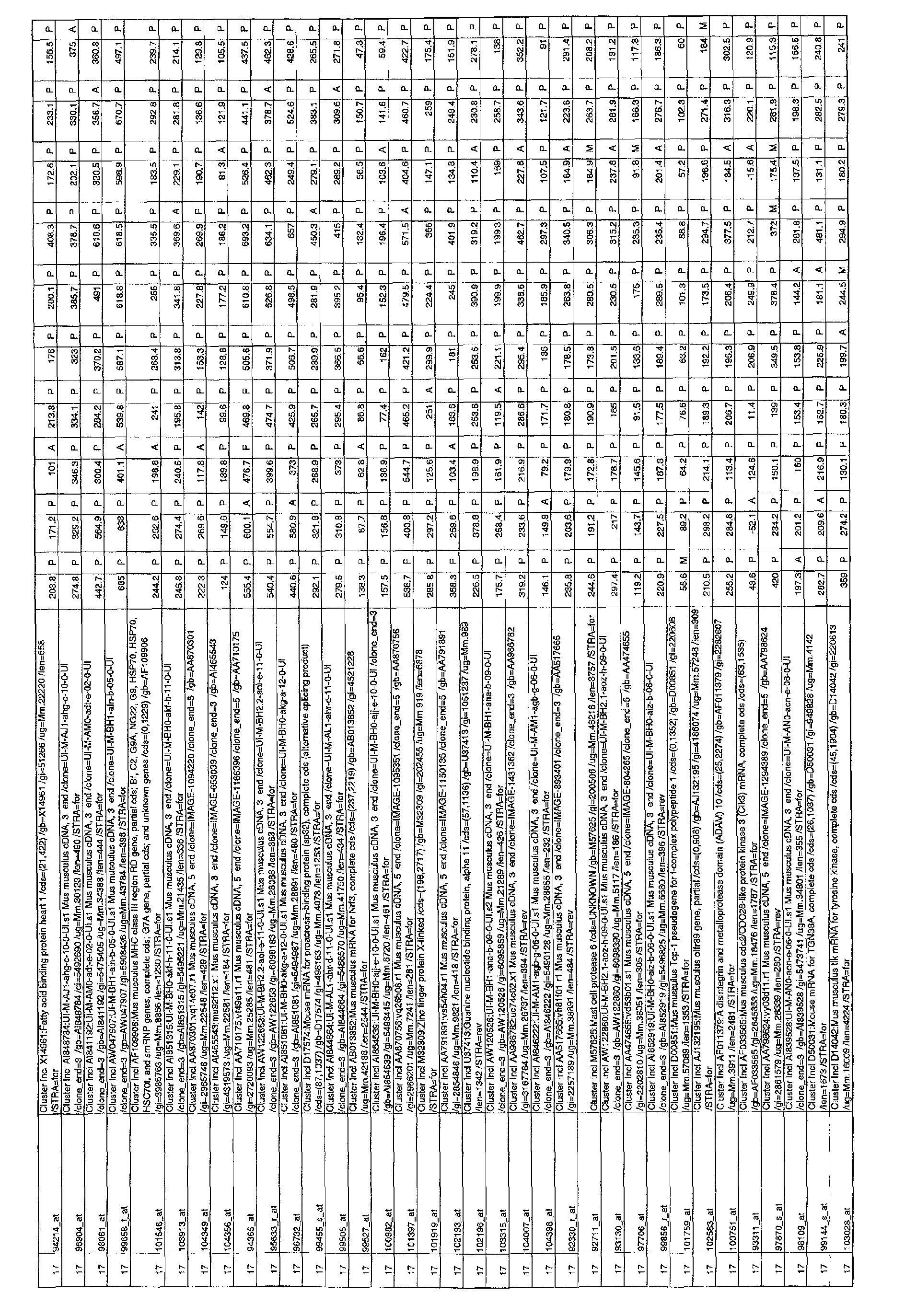

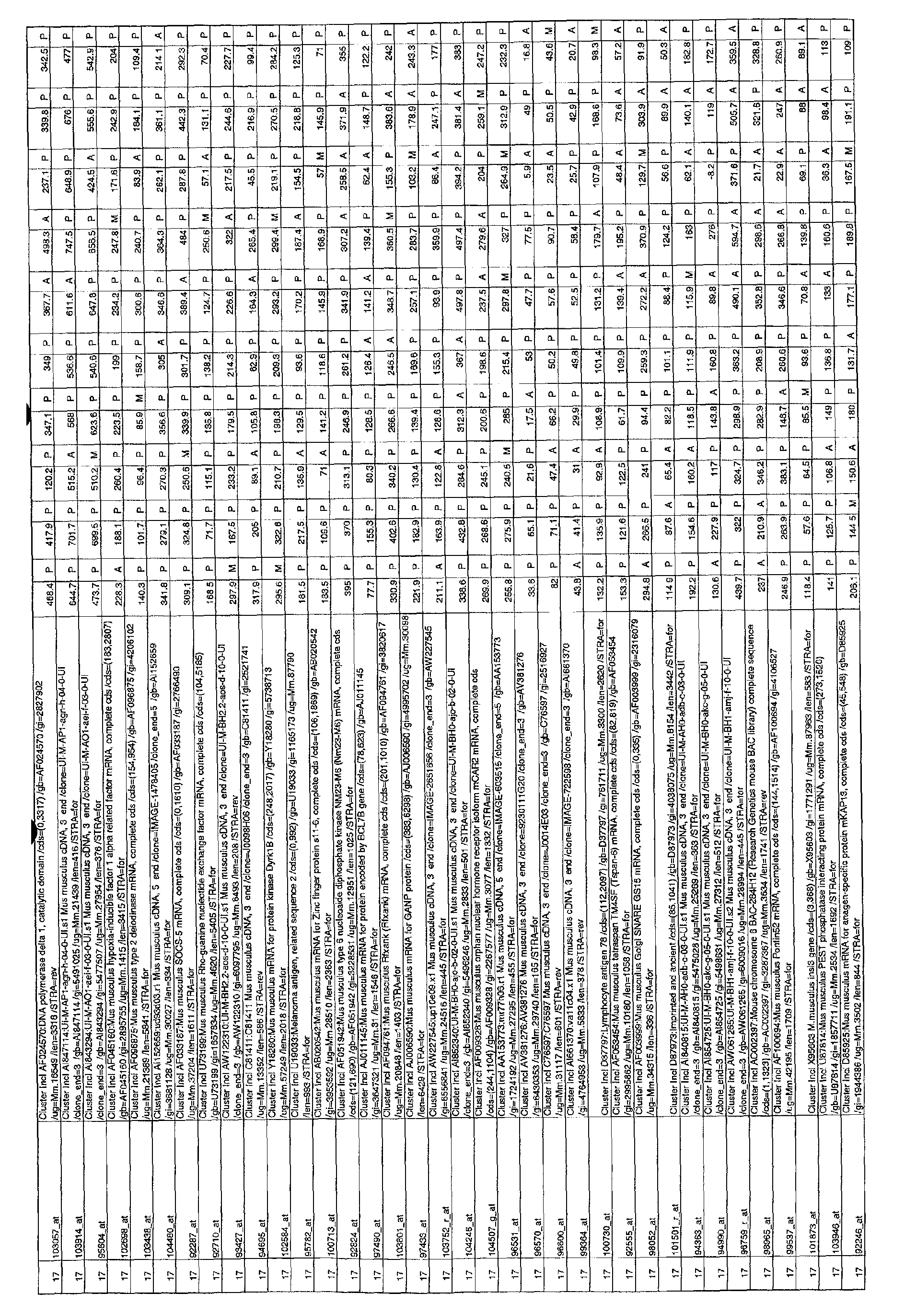

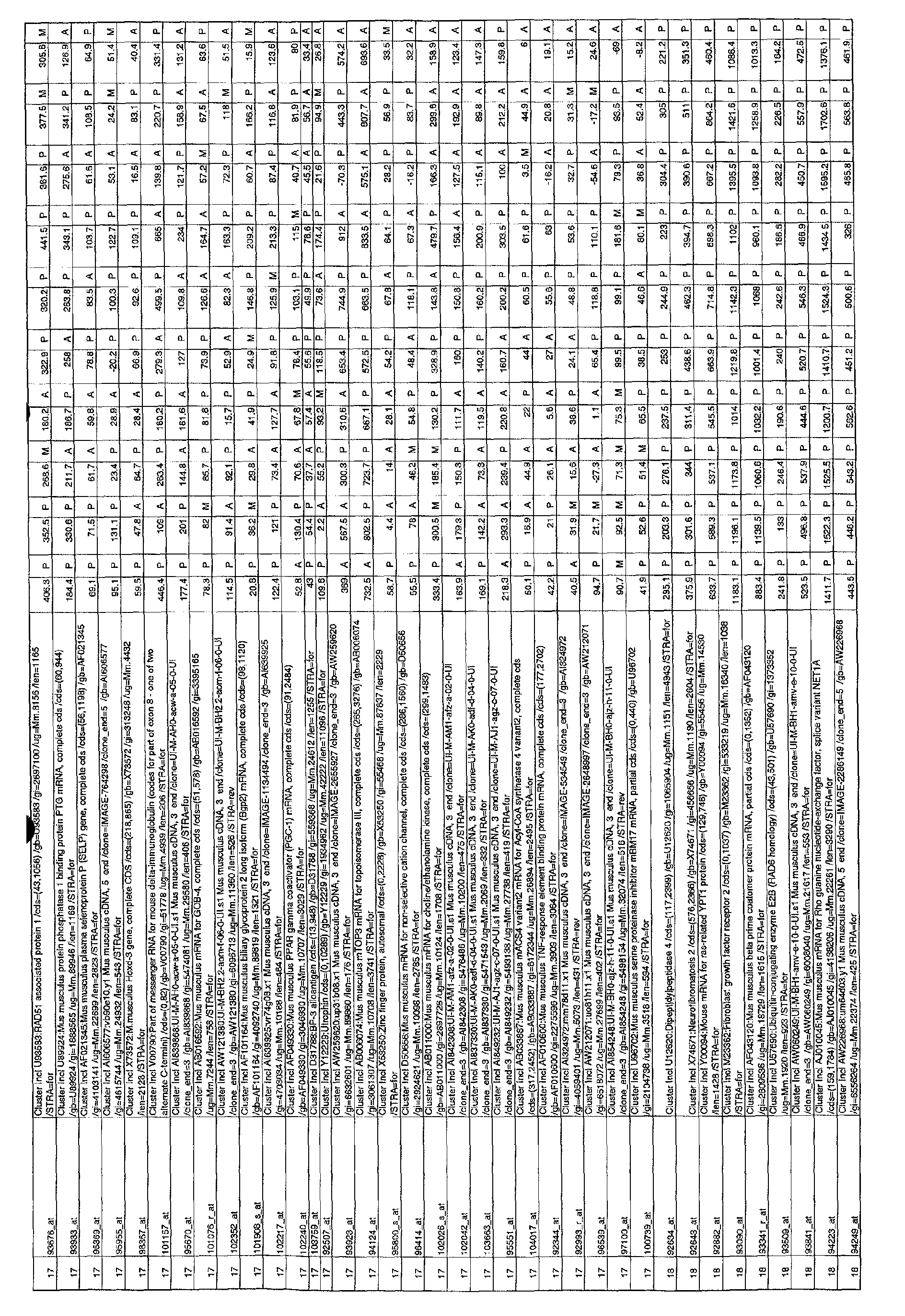

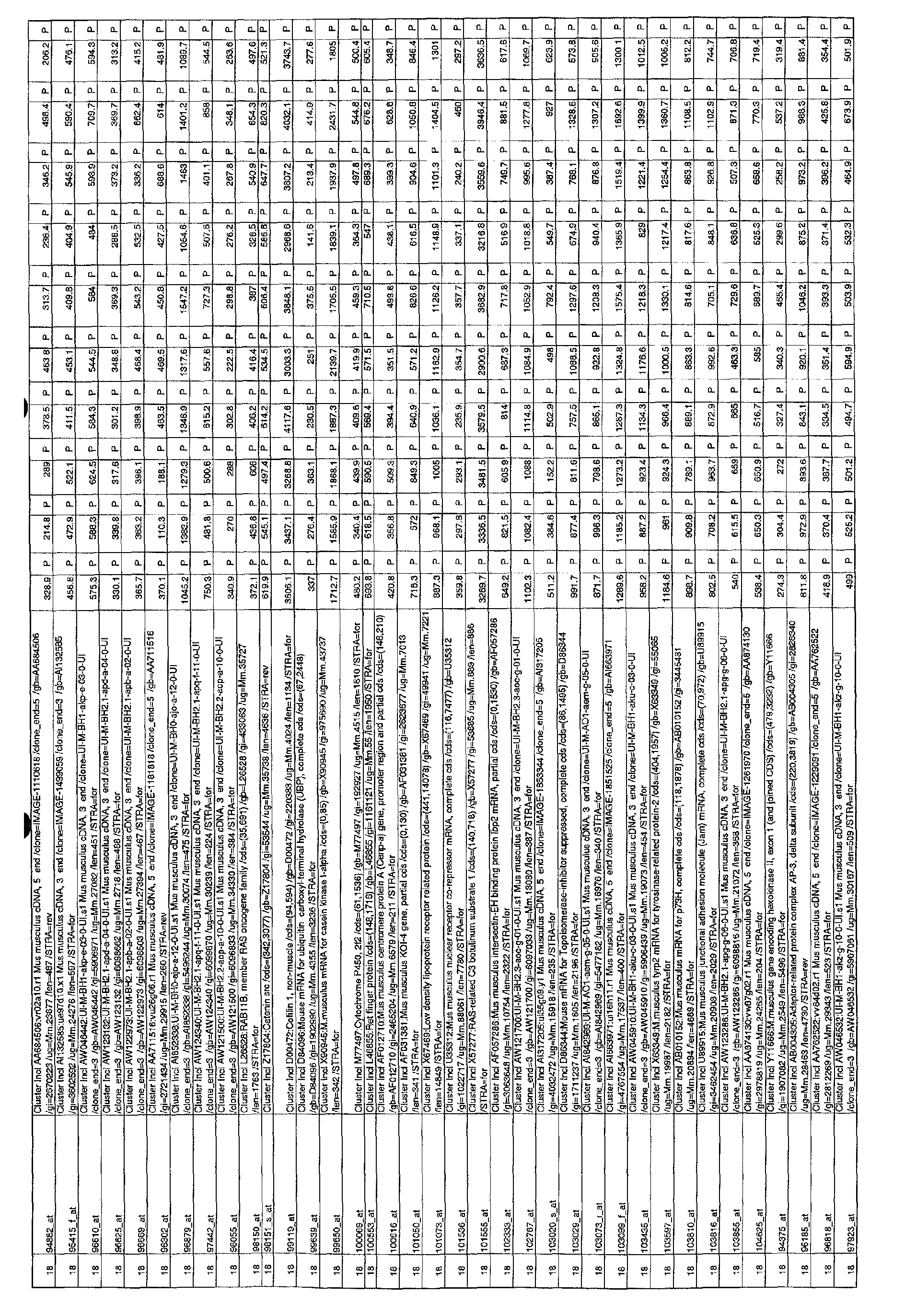

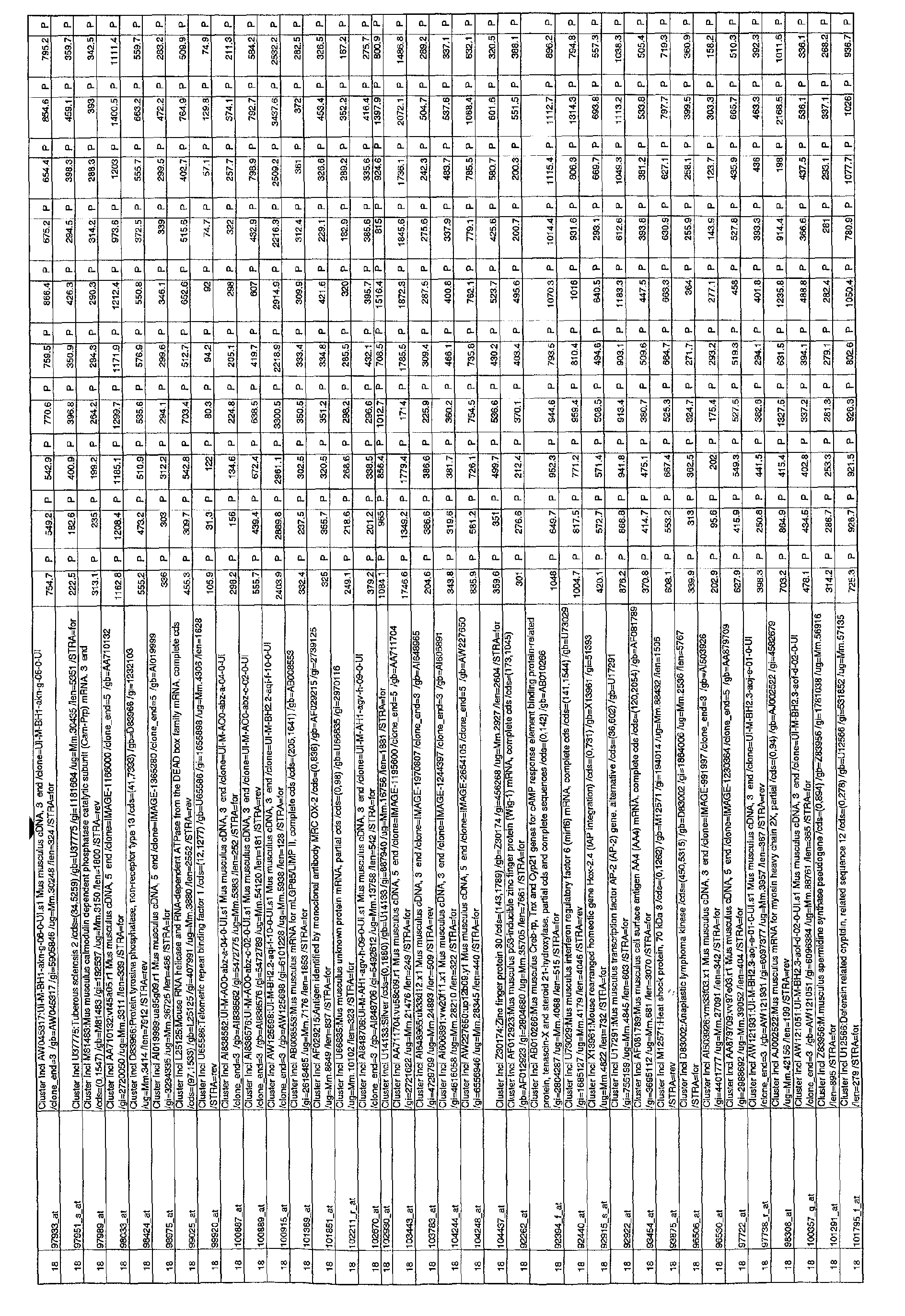

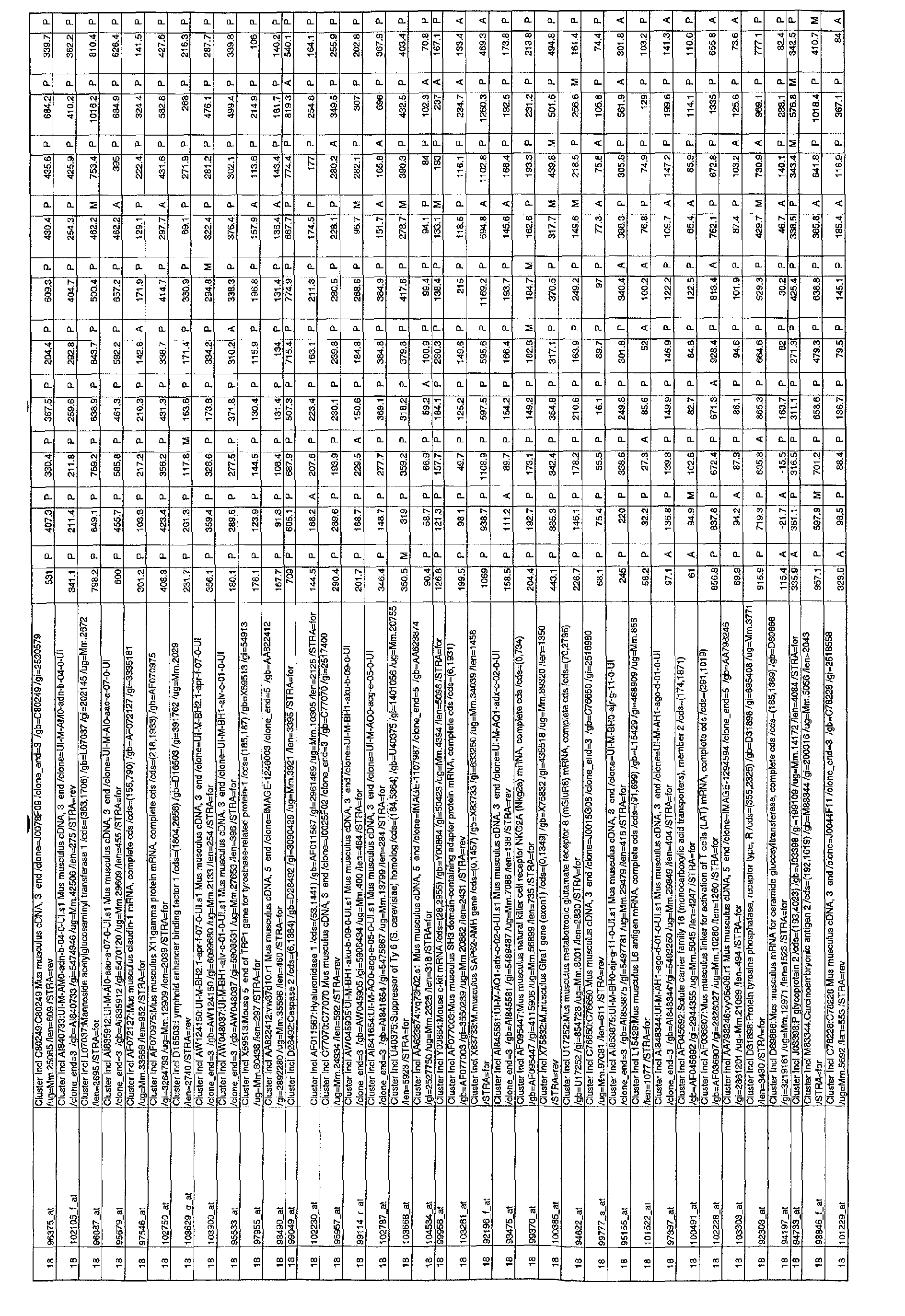

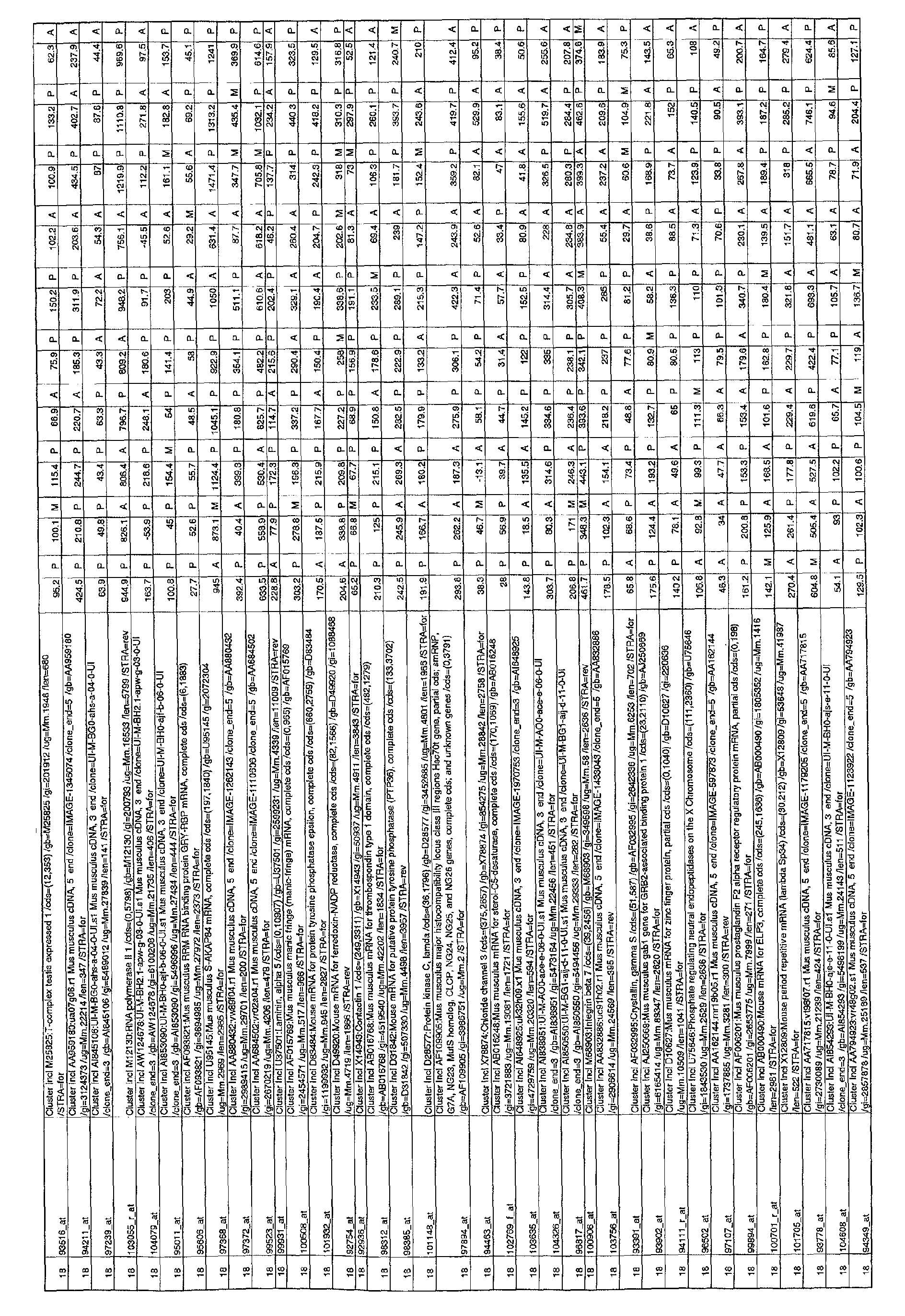

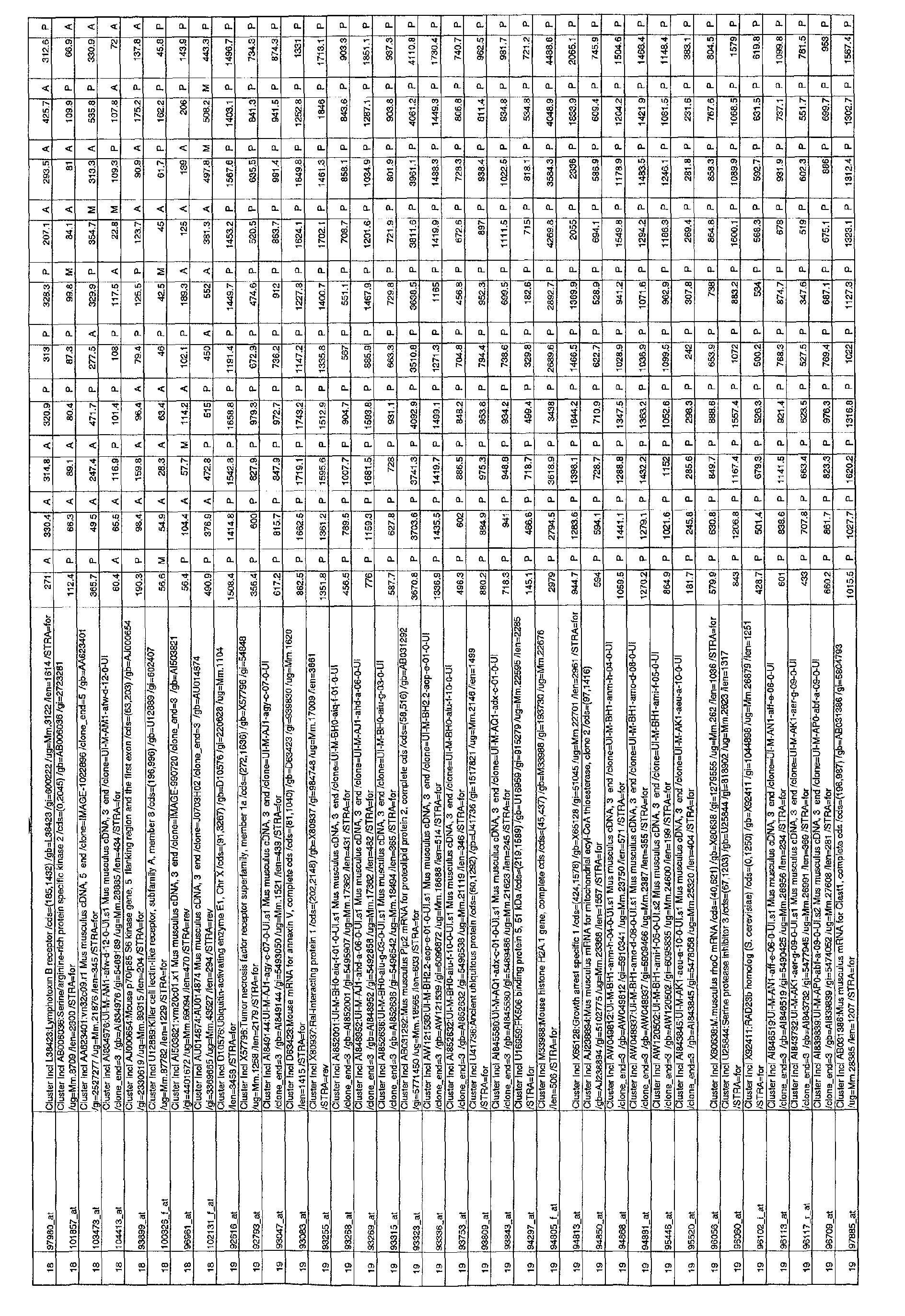

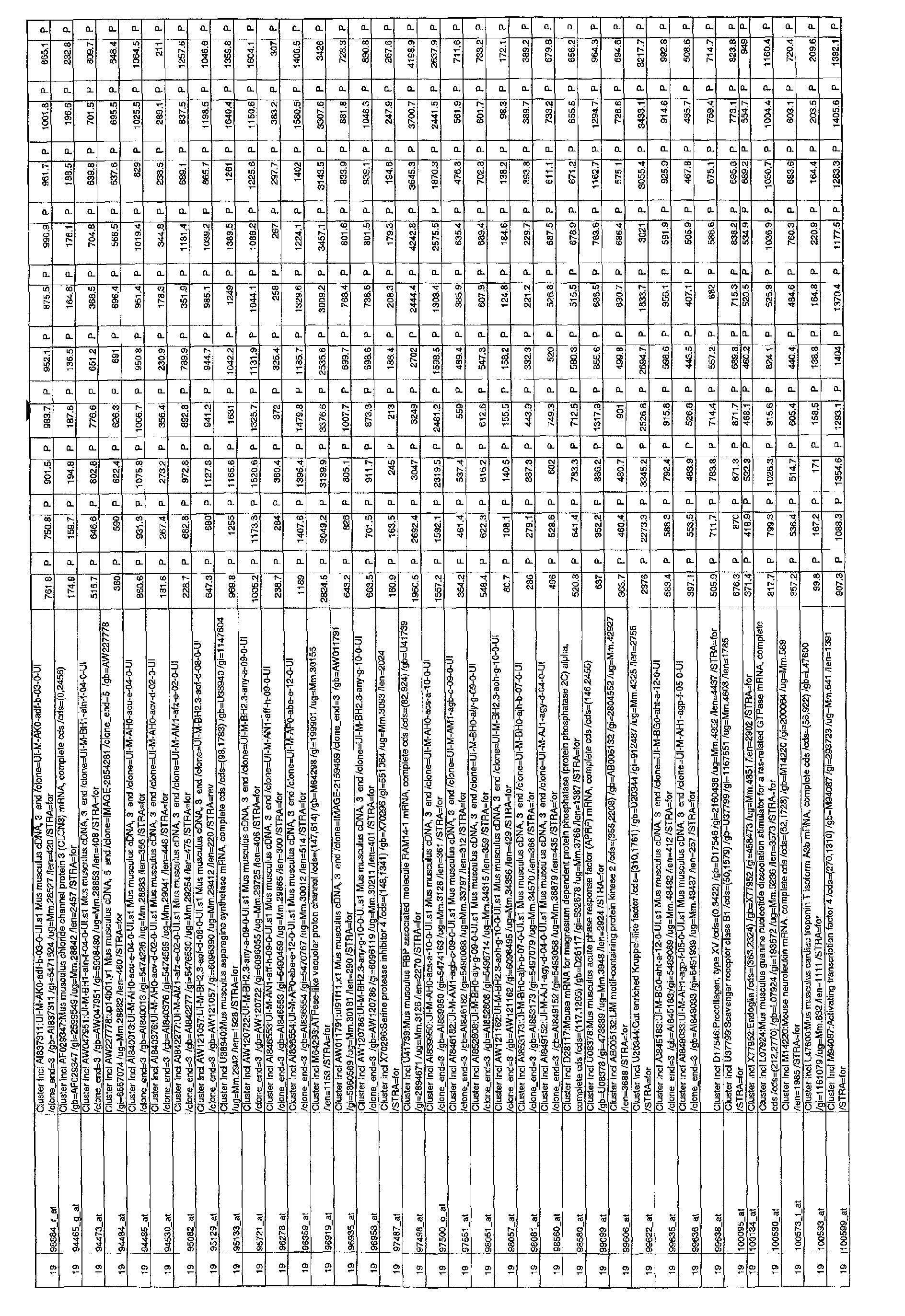

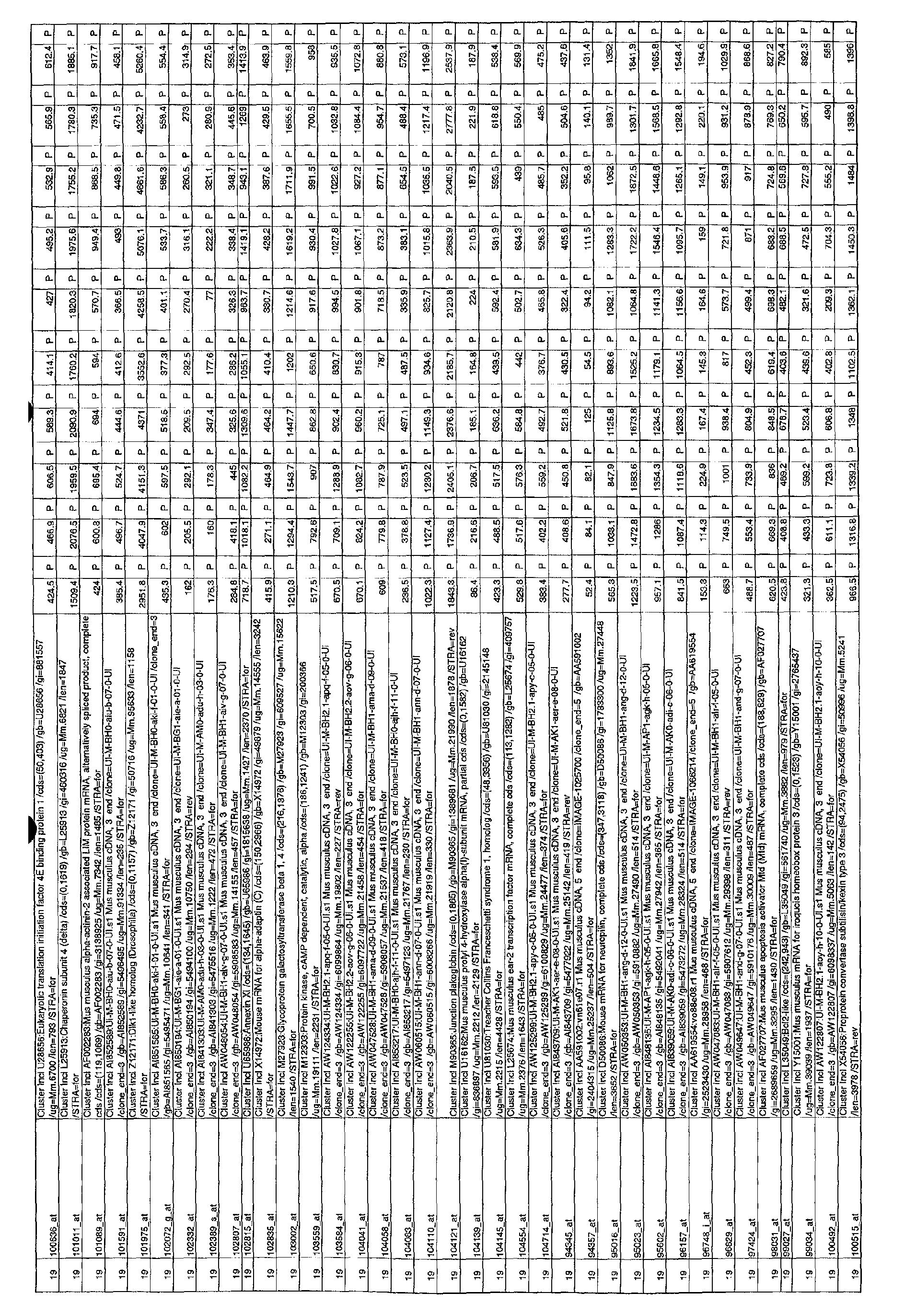

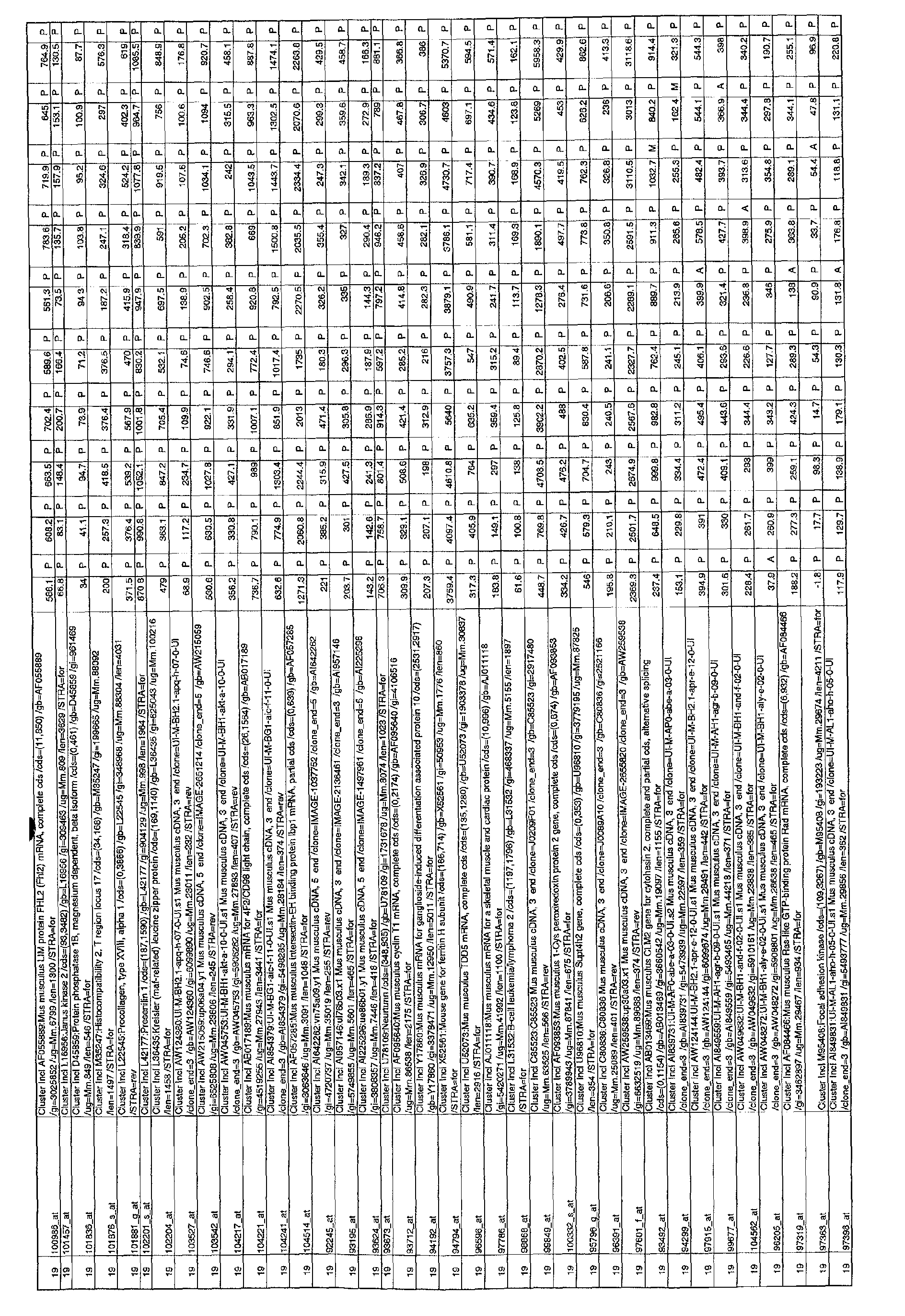

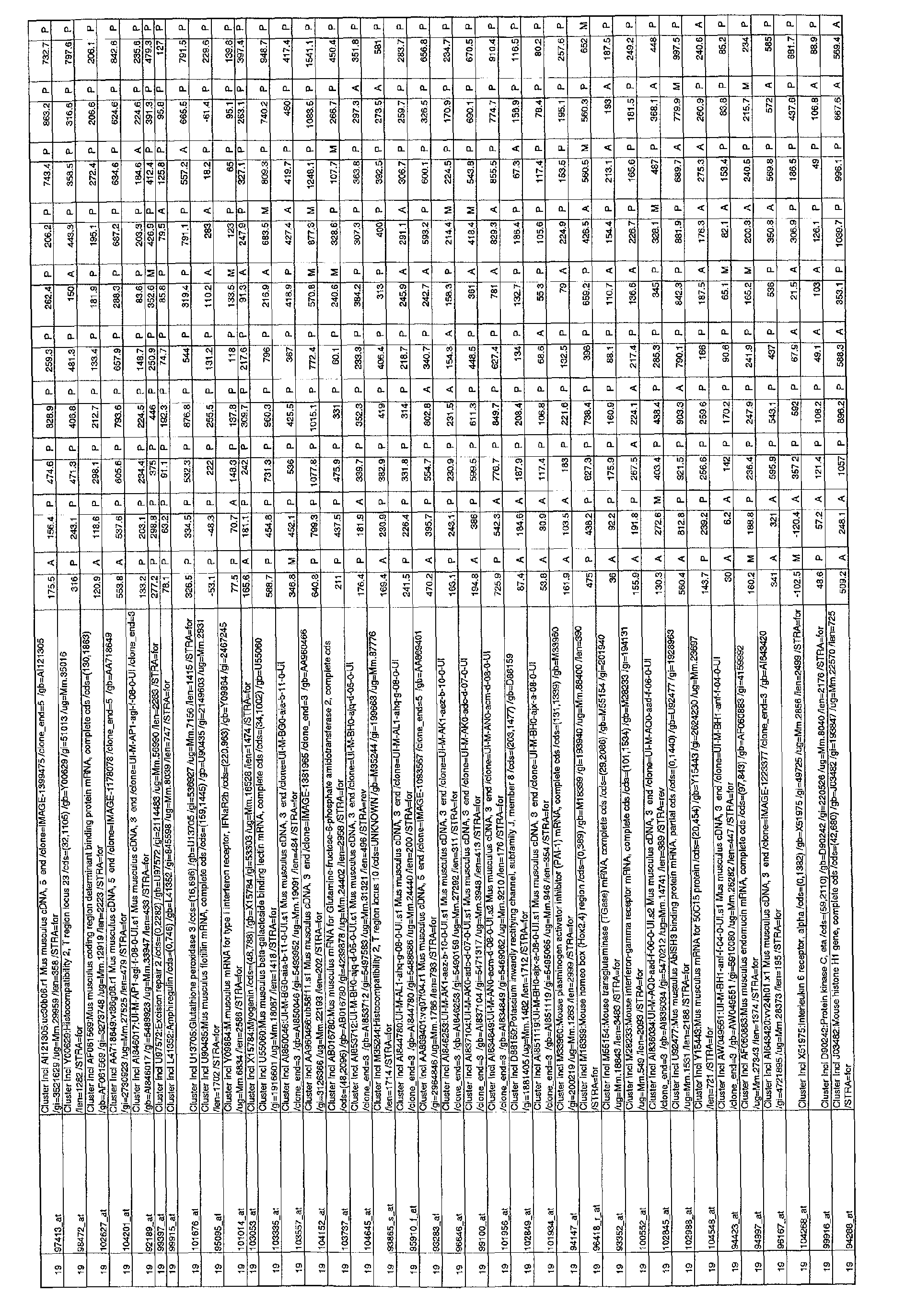

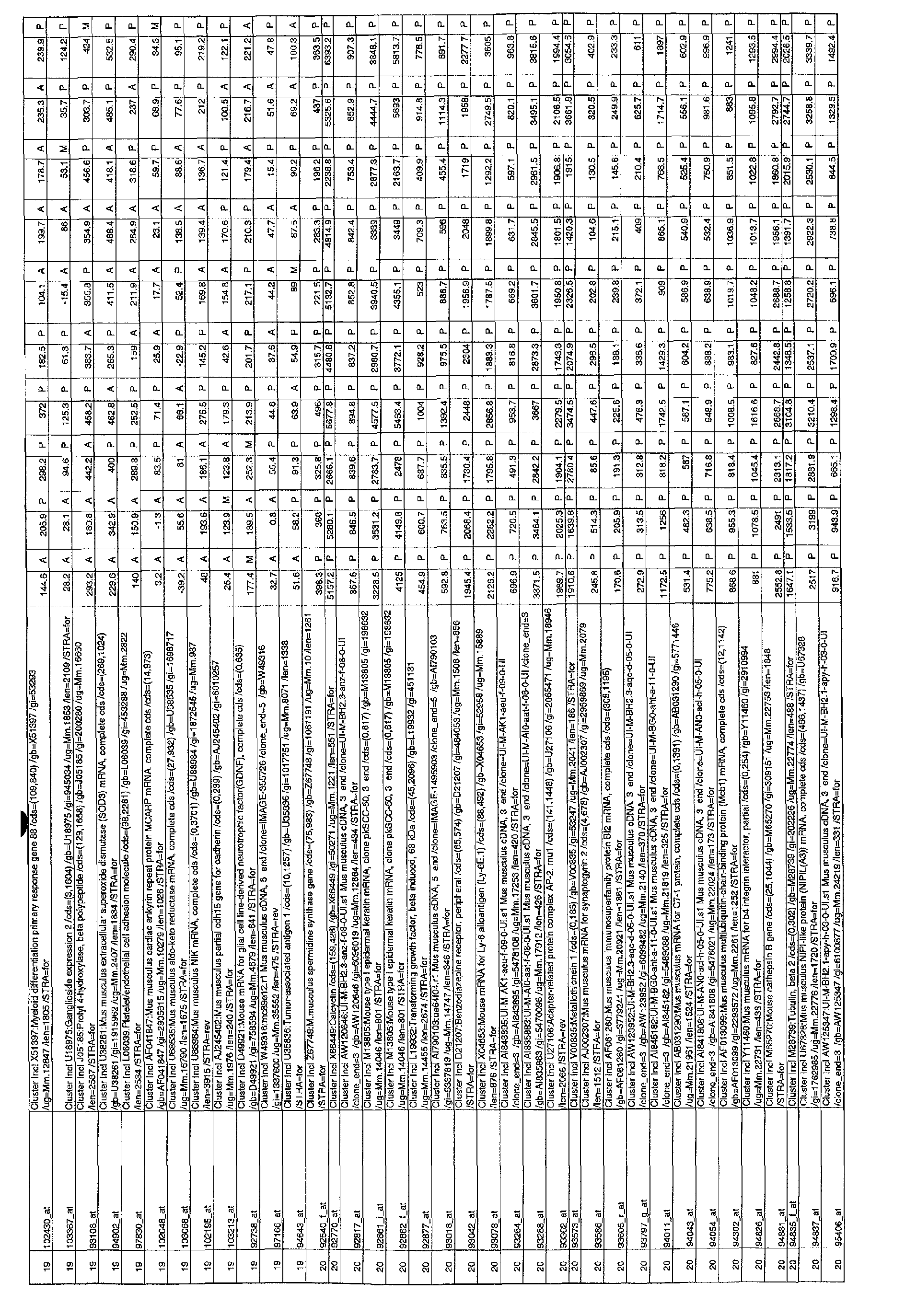

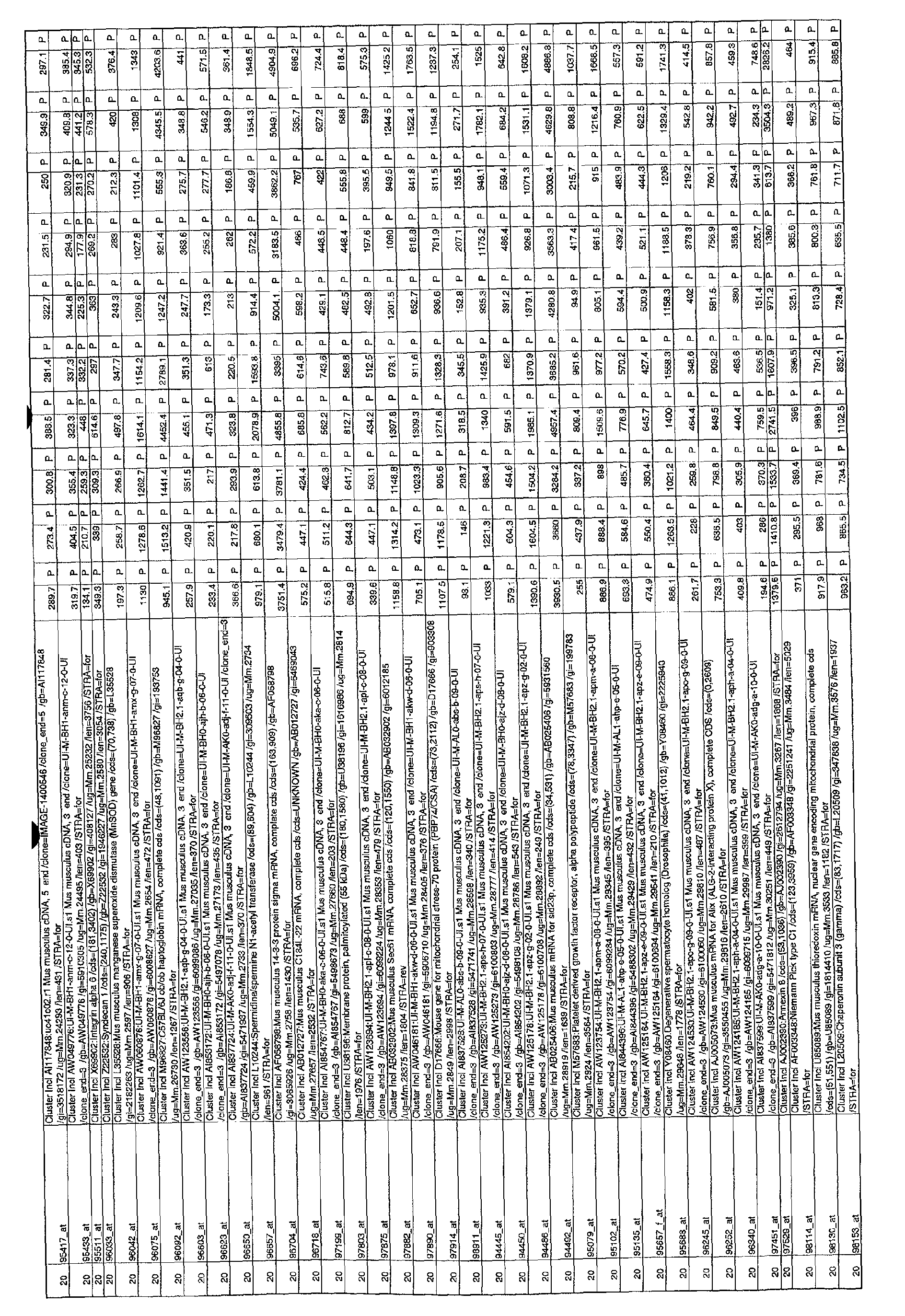

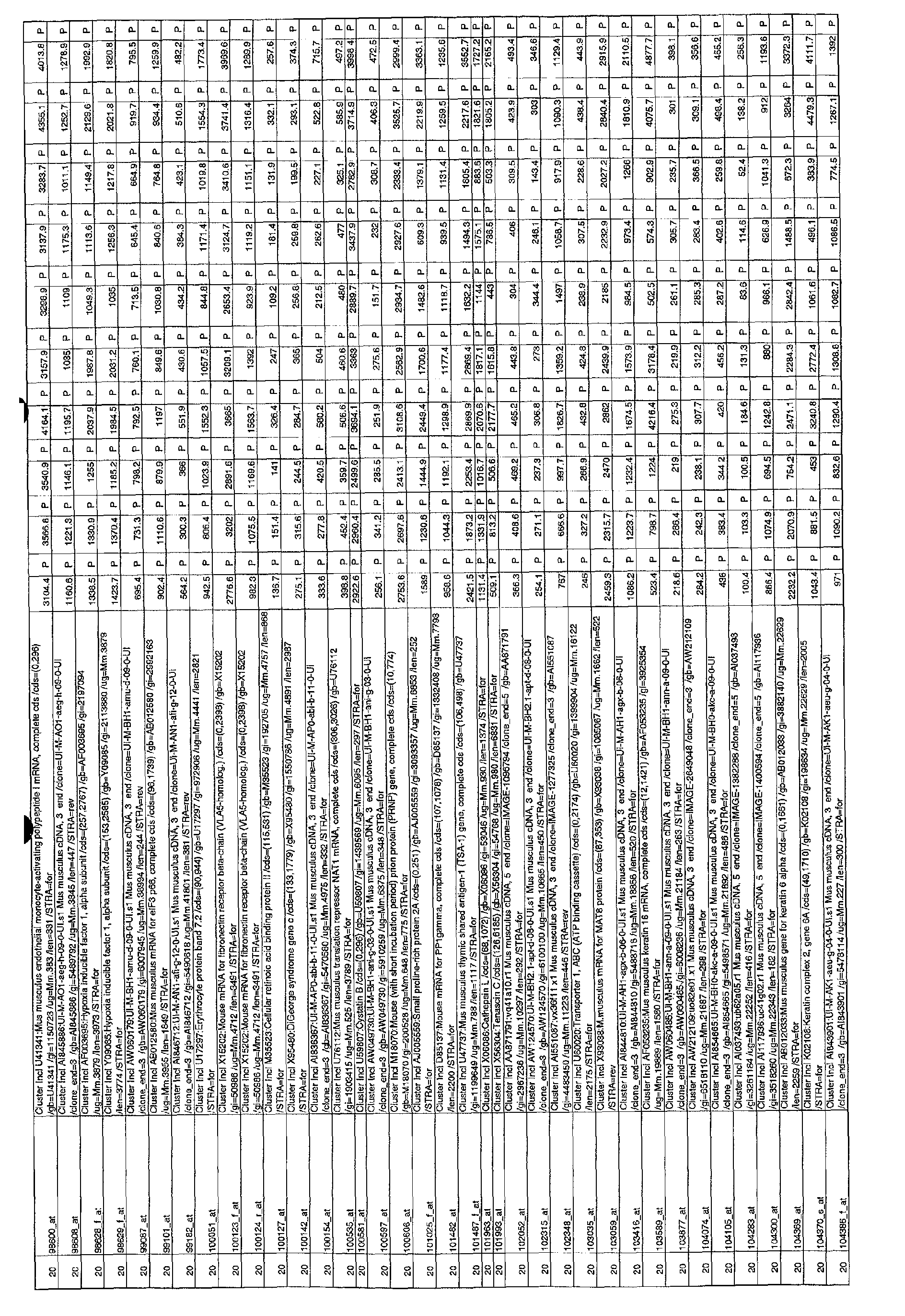

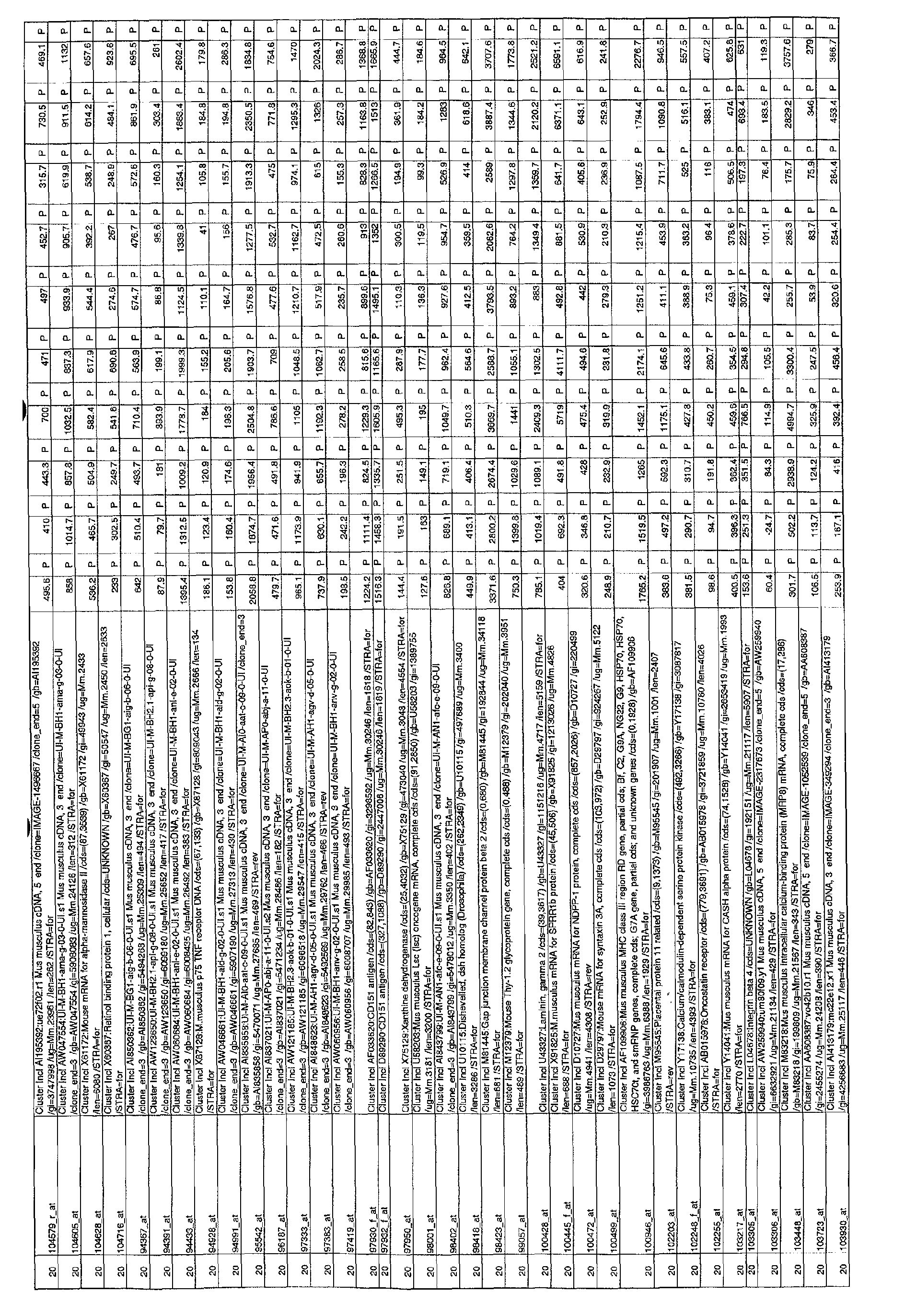

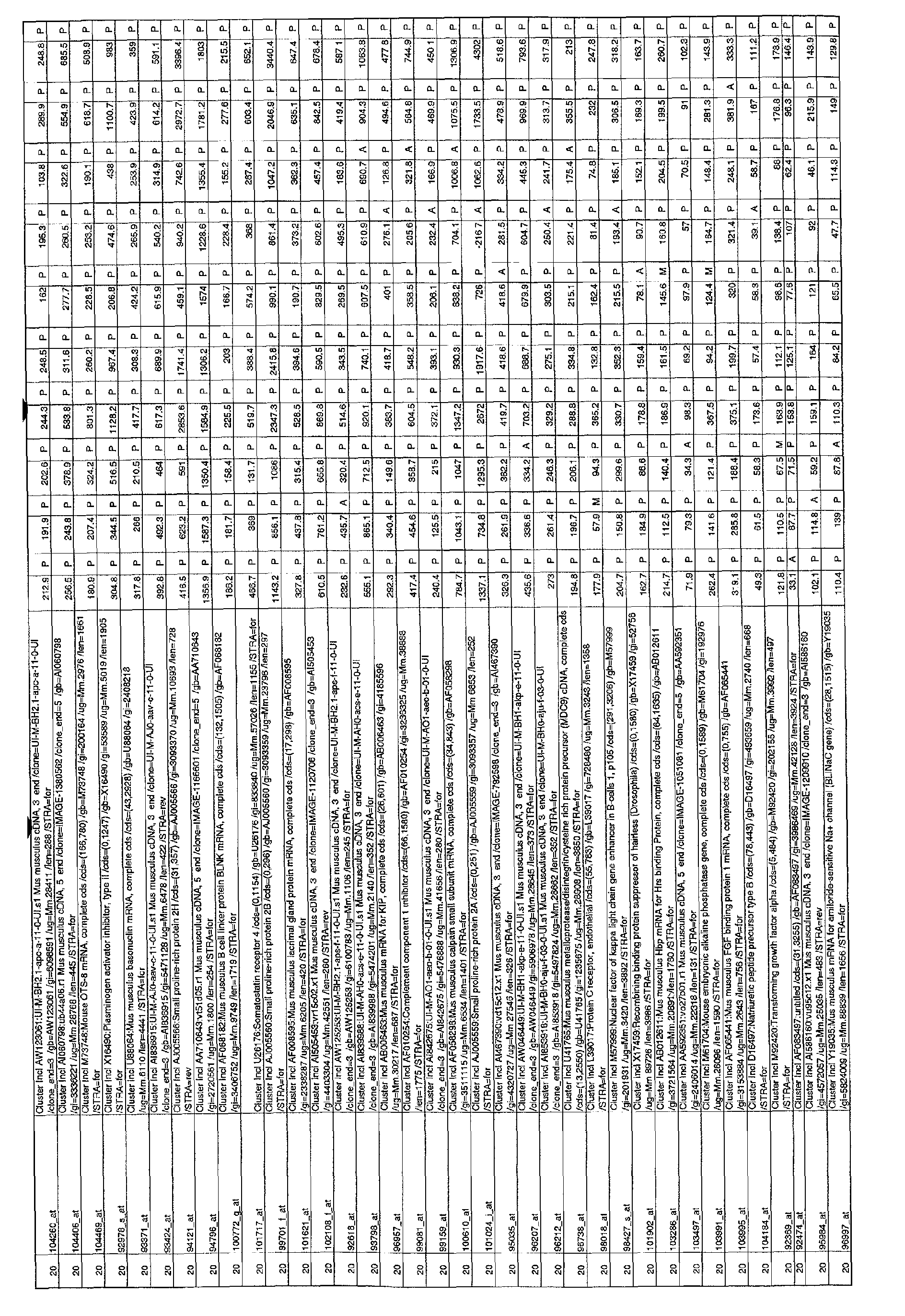

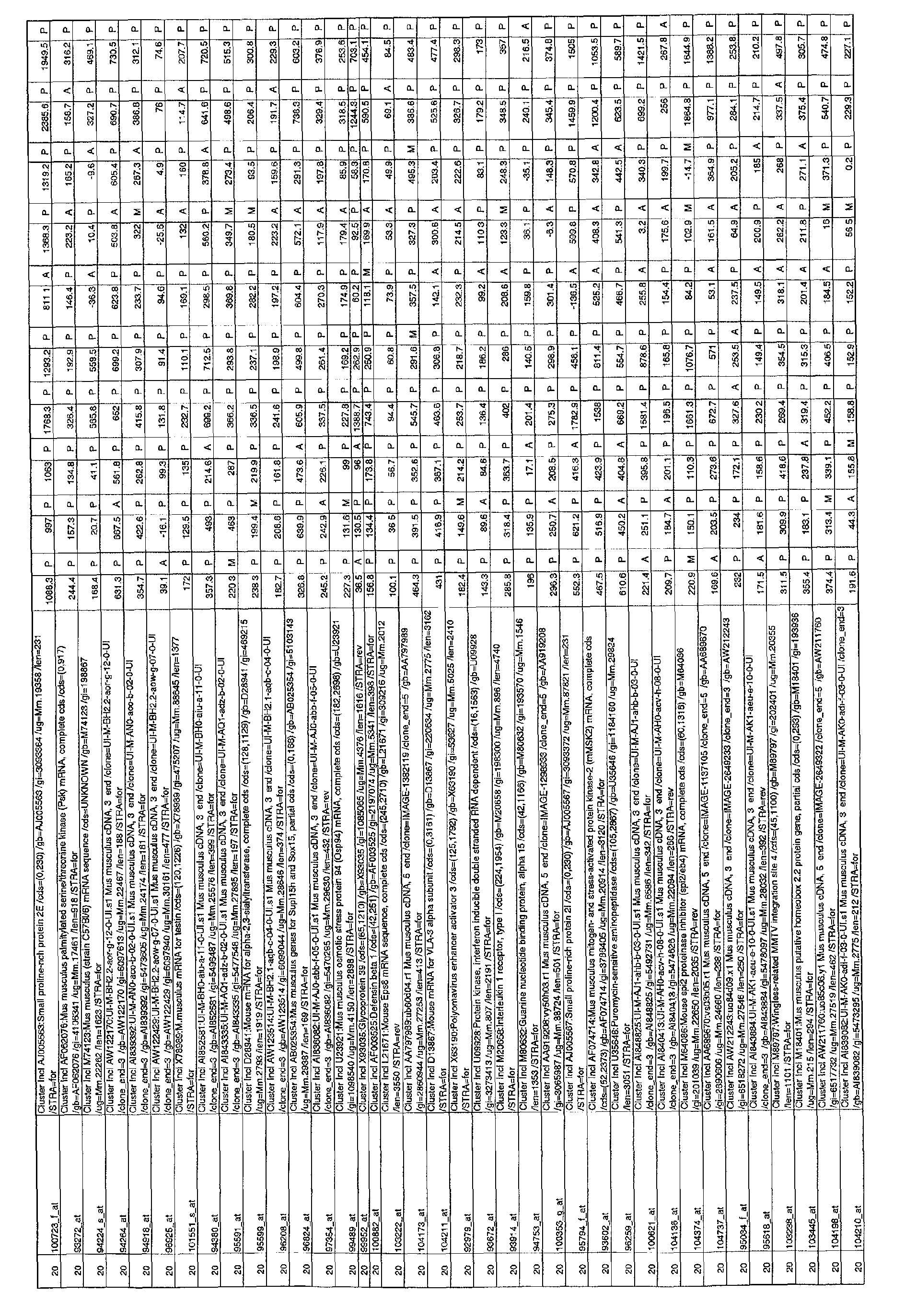

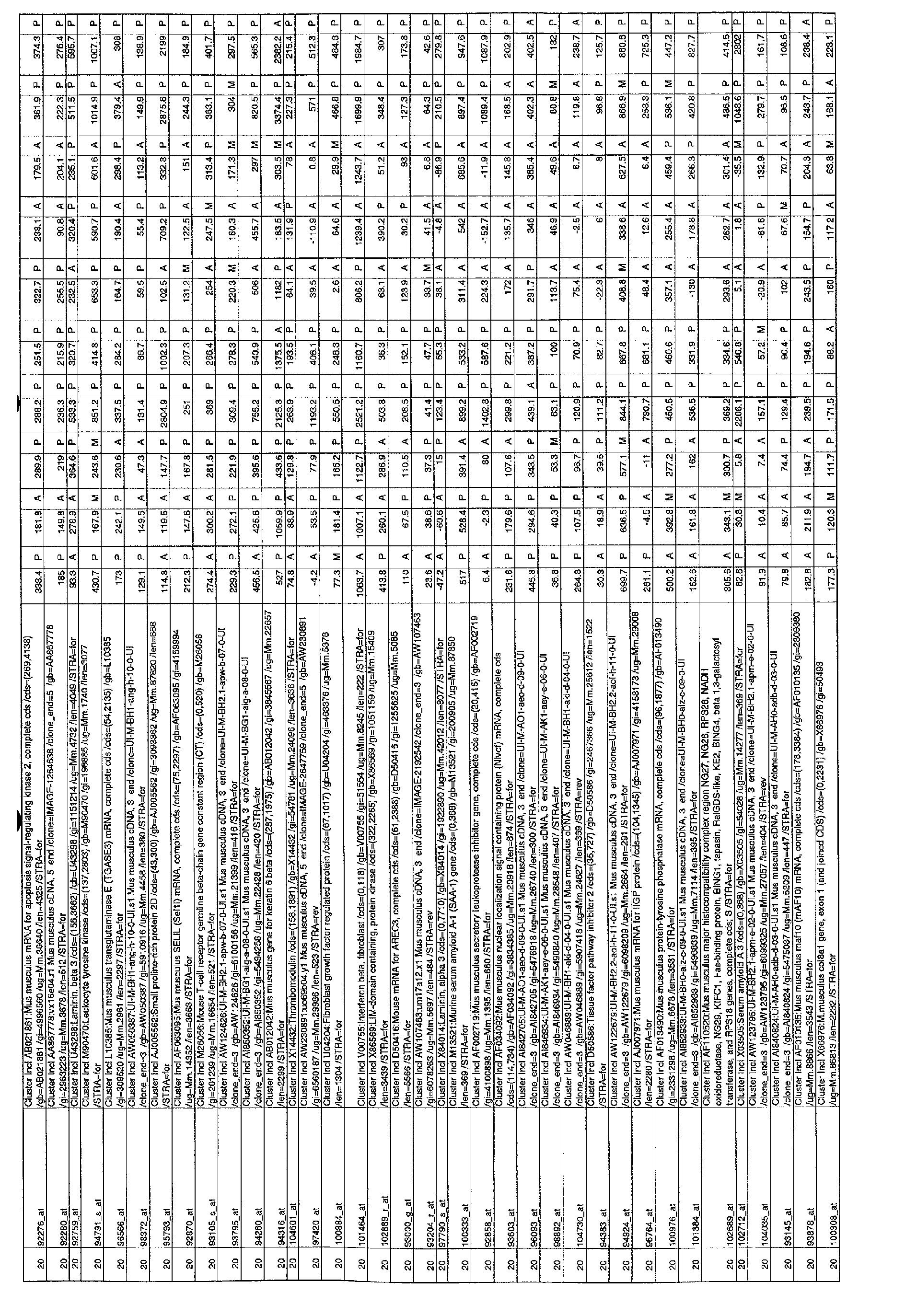

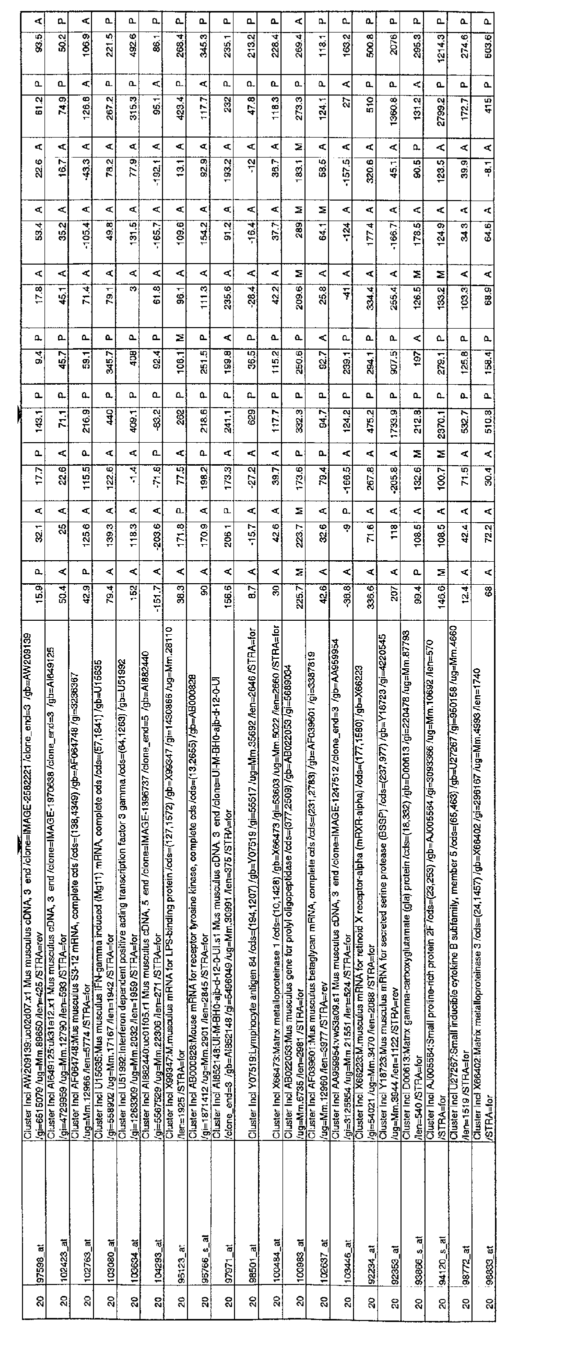

**Table 2:**

| Genes clustering closely to actin | | |
|---|---|---|
| Affymetrix Accession | Accession number | Descriptions |
| 92573_at | AB021743 | Mus musculus mRNA for PR65 (type 2A serine/threonine protein phosphatase A subunit), complete cds |
| 92621_at | X97982 | M.musculus mRNA for poly(C)-binding protein, splice variant E |
| 93346_at | M15668 | Mus musculus X chromosome-linked phosphoglycerate kinase (pgk-1) mRNA |
| 95682_at | AB026432 | Mus musculus mRNA for XPE UV-damaged DNA binding factor, complete cds |
| 96876_at | U34259 | Mus musculus Golgi 4-transmembrane spanning transporter MTP mRNA, complete cds |
| 99668_at | U86405 | Mus musculus brain amphiphysin 2 mRNA, complete cds |
| 101028_i_at | M15501 | Actin, alpha, cardiac /cds=(0,1127) /gb=M15501 |
| 101030_at | X99963 | M.musculus rhoB gene /cds=(0,590) /gb=X99963 |
| 101554_at | U57524 | Mus musculus I kappa B alpha gene, exons 2-6, partial cds |
| 104598_at | X61940 | Mouse mRNA for a growth factor-inducible immediate early gene (3CH134) |

**Table 3:**

| Genes clustering closely to MRP-8 | | |
|---|---|---|
| Affymetrix Accession | Accession number | Descriptions |
| 96092_at | M96827 | C57BL/6J ob/ob haptoglobin mRNA, complete cds |
| 97529_at | AJ002390 | Annexin 8 |
| 103589_at | AF053235 | Mus musculus keratin 16 mRNA, complete cds |
| 104370_s_at | K02108 | Keratin complex 2, gene 6A |
| 103448_at | M83218 | Mus musculus intracellular calcium-binding protein (MRP8) mRNA |
| 94121_at | AJ005566 | Small proline-rich protein 2H |
| 99701_f_at | AJ005560 | Small proline-rich protein |
| 101024_i_at | AJ005559 | Small proline-rich protein 2A |

**Table 4:**

| Genes clustering closely to MMP-3 | | |
|---|---|---|
| Affymetrix Accession | Accession number | Descriptions |
| 100127_at | M35523 | Cellular retinoic acid binding protein II |
| 94391_at | AW123650 | UI-M-BH2.1-api-g-08-0-UI.s1 Mus musculus cDNA, 3 end |
| 97950_at | X75129 | Xanthine dehydrogenase |
| 102255_at | AB015978 | Oncostatin receptor |
| 103723_at | AA608387 | vo42b10.r1 Mus musculus cDNA, 5 end |
| 99159_at | AI842675 | UI-M-AO1-aeo-b-01-0-UI.s1 Mus musculus cDNA, 3 end |
| 98018_at | L39017 | Protein C receptor, endothelial |
| 94224_5_at | M74123 | Mus musculus (strain C57B1/6) mRNA sequence |
| 100882_at | AF003525 | Defensin beta 1 |
| 100884_at | U04204 | Fibroblast growth factor regulated protein |
| 101384_at | AI852933 | UI-M-BHO-aiz-c-09-0-UI.s1 Mus musculus cDNA, 3 end |
| 103080_at | U15635 | Mus musculus IFN-gamma induced (Mg11) mRNA |
| 103634_at | U51992 | Interferon dependent positive acting transcription factor 3 gamma |
| 96123_at | X99347 | M.musculus mRNA for LPS-binding protein |
| 98772_at | U27267 | Small inducible cytokine B subfamily, member 5 |
| 98833_at | X66402 | Matrix metalloproteinase 3 |

**Table 5:**

| Genes clustering closely to Cathepsin-S | | |
|---|---|---|
| Affymetrix Accession | Accession number | Descriptions |
| 93118_at | AI844131 | UI-M-AL1-ahj-d-09-0-UI.s1 Mus musculus cDNA, 3 end |
| 93274_at | M38381 | CDC-like kinase |
| 93806_at | AI848671 | UI-M-AHl-agv-e-02-0-UI.s1 Mus musculus cDNA, 3 end |
| 94081_at | AW124390 | UI-M-BH2.1-ape-c-03-0-UI.s1 Mus musculus cDNA, 3 end |
| 98946_at | AF033186 | Mus musculus WSB-1 mRNA, complete cds /cds=(0,1265) / |
| 98087_at | AW048562 | UI-M-BH1-alx-e-05-0-UI.s1 Mus musculus cDNA, 3 end |
| 99577_at | M57647 | Mouse mast cell growth factor (MGF) mRNA, complete cds |
| 102781_at | U37351 | Mus musculus Paneth cell enhanced expression PCEE mRNA, complete cds |
| 103667_at | AA866655 | ud06h05.r1 Mus musculus cDNA, 5 end |
| 104056_at | AI573367 | ud42d02.y1 Mus musculus cDNA, 5 end |
| 94975_at | AI845607 | UI-M-AQ1-adx-e-10-0-UI.s1 Mus musculus cDNA, 3 end / |
| 102302_at | L16992 | Mus musculus branched-chain alpha-ketoacid dehydrogenase E1 beta-subunit mRNA sequence |
| 103753_at | AI159572 | vz77c07.r1 Mus musculus cDNA, 5 end |
| 104640_f_at | AI464596 | mz66c12.y1 Mus musculus cDNA, 5 end |
| 92502_at | X95504 | M.musculus mRNA for zinc finger protein |
| 92513_at | AJ002636 | Mus musculus mRNA for nuclear protein SA2 / |
| 92542_at | AI849035 | UI-M-AH1-agw-a-06-0-UI.s1 Mus musculus cDNA, 3 end |
| 96616_at | AI891629 | u157e11.x1 Mus musculus cDNA, 3 end |
| 104070_at | AW047728 | UI-M-BH1-alk-a-01-0-UI.s1 Mus musculus cDNA, 3 end |
| 104193_at | AW047583 | UI-M-BH1-akm-b-04-0-UI.s1 Mus musculus cDNA, 3 end |
| 93775_at | AI841894 | UI-M-AO0-acd-d-09-0-UI.s1 Mus musculus cDNA, 3 end / |
| 97869_at | AI844043 | UI-M-AL1-ahi-d-11-0-UI.sl Mus musculus cDNA, 3 end |
| 97541_f_at | X00246 | Mouse mRNA with a Set 1 repetitive element for a class I major histocompatibility complex(MHC) antigen |
| 95028_r_at | C77009 | C77009 Mus musculus cDNA, 3 end |
| 97421_at | U42385 | Fibroblast growth factor inducible 16 |
| 101923_at | U34277 | Mus musculus PAF acetylhydrolase mRNA, complete cds |
| 103202_at | AW047476 | UI-M-BH1-all-g-04-0-UI.s1 Mus musculus cDNA, 3 end |
| 92226_at | AA866971 | vx91g10.r1 Mus musculus cDNA, 5 end |
| 98893_at | AW048644 | UI-M-BH1-amd-d-08-0-UI.s1 Mus musculus cDNA, 3 end |
| 103016_5_at | X68273 | CD68 antigen |
| 94934_at | U04828 | Angiotensin II receptor, type 2 |
| 100880_at | AA816121 | vp44a01.r1 Mus musculus cDNA, 5 end |
| 94818_at | AW047223 | UI-M-BH1-amb-e-09-0-UI.s1 Mus musculus cDNA, 3 end |
| 98045_5_at | U18869 | Mus musculus mitogen-responsive 96 kDa phosphoprotein p96 mRNA, alternatively spliced p67 mRNA, and alternatively spliced p93 mRNA, complete cds |
| 98543_at | AJ223208 | Mus musculus mRNA for cathepsin S, partial |
| 98562_at | X58861 | Complement component 1, q subcomponent, alpha polypeptide |
| 100611_at | M21050 | Mouse lysozyme M gene |
| 98465_f_at | M31419 | Interferon activated gene 204 |
| 100397_at | AF024637 | TYRO protein tyrosine kinase binding protein |
| 103226_at | Z11974 | Mannose receptor, C type 1 |
| 95806_f_at | M68899 | Mouse mast cell chymase 2 mRNA, complete cds |
| 102104_f_at | AI504305 | v104g11.x1 Mus musculus cDNA, 3 end |
| 99507_at | M21247 | Uncoupling protein, mitochondrial |
| 92718_at | AI158810 | ud38d09.r1 Mus musculus cDNA, 5 end |
| 94774_at | M31418 | Interferon activated gene 202 |
| 101635_f_at | M16360 | Mouse major urinary protein V (MUP V) mRNA, complete cds |
| 101682_f_at | M16358 | Major urinary protein 4 |

**Table 6:**

| Genes clustering closely to TGF-beta | | |
|---|---|---|
| Affymetrix Accession | Accession number | Descriptions |
| 92793_at | X57796 | Tumor necrosis factor receptor superfamily, member 1a |
| 92877_at | L19932 | Transforming growth factor, beta induced, 68 kDa / |
| 99604_at | AI848713 | UI-M-AJ1-ahf-a-10-0-UI.sl Mus musculus cDNA, 3 end |
| 101040_at | D38117 | Mus musculus mRNA for m-calpain large subunit, complete cds |
| 101982_at | X98475 | M.musculus VASP gene |
| 96187_at | AI837021 | UI-M-APO-abj-a-11-0-UI.s2 Mus musculus cDNA, 3 end |

Sequence Information

SEQ ID Nos 1 to 12 are DNA sequences corresponding to expressed sequence tags
(ESTs)(mRNA) from Mus musculus found in clusters 2 and 7. The genes that encode
these ESTs encode proteins important in the early wound healing response

SEQ ID Nos 13 to 29 are DNA sequences corresponding to expressed sequence tags
(mRNA) from Mus musculus from clusters 5 and 6, the genes that encode these ESTs
encode proteins that are involved in the inflammatory response and are not required for
wound healing.

SEQ ID NOS 30 to 39 are DNA sequences corresponding to expressed sequence tags
(mRNA) from Mus musculus found in cluster 20, the genes that encode these ESTs
encode proteins important in the late, advanced phase of wound healing, that is genes
involved in cessation of the healing response, maturation and remodelling of the repaired
tissue.

In the list below, the sequence identification number is given, followed by the Accession
number and then the Affymetrix reference number.

SEQ ID NO. 1
>AI846118 98910_at
ttttttttttttttttgaagttagaaaagtcttttaagtttttaaattaaaattgcaccctggtgcgatgactctaatttgggaactcaatccaa
aggcagactctcctgagtgctggtcctctggcctggacacacagagactgggagcctgtcggacagttagaacagggaagggg
cagaagcacccacatcagagggcagccaagtcttgacatctacctggtgacaagg

SEQ ID NO. 2
>AA655823 100466_f_at
ttgaaccgcgggcggcctgggctccttctgccatggcccccgcgctcaggtccttgctgtcgccacggactttactgctgctgctg
ctgagcctggcgctgctgggcgccgccgagcccgccaccgggagcgctgtccccgctcagagccgcccgtgcgtggattgcc
acgagtttgaattcatgcagcgcgcccttcaggacctacggaaaaccgcctacagcctggacgcacggacggagaccctcctgc
tgcaggctgagcgccgggctctgtgtgcctgctggccagctggacgctgaagatctttggccagtgatattccttgtcaataaatgc
ctgctagcatgccgnga

SEQ ID NO. 3
>AI846077 103433_at
tttttttttttttttggttttagcagtatgtttttatttagggaacacagtatttgcaatagttctgacagccgtaaggccctccattttactagc
tgtttgctttgcaataaaatagccagaaattctgatatcaacattaataggatatttcttcatgcaggttgtaggagtatatac
attgtataacactgaagaaattaatacatatgtacattatttacatagtaactatttattgtaaatagaaatgttttctaagataaaaagaaa
tcacaacatggtttctggtctgt

SEQ ID NO. 4
>AI642048 104149_at
gtctgtaaaaatctgtttaataaatatacatcttagaagtaccaaaataattaccaacaaaatacaacatatacaacatttacaagaag
gcgacacagaccttagttgggggcgacttttaagcacatgccactgaacacctggctcttacatgggaggacacactgggctcact
tactaggtctatggtggttcaatcaaaagcacaataaataaaacgtggtcctttcattaggttctggaaaatcacctcccccccccc
aaaaaaaatcccacaaacatgaaccttaagagacattttctttgaatttcagtgatctgtttccccggatttcacaaagacaacagccg
aatcaccccagtaaaatgcctgggtctaggcgctgtgtggtgtggtgctaagtataccctttctcatttttttttctttttct

SEQ ID NO. 5
>AW124785 104410_at
tttttttttttttttttcaggtctcattttcgtttatttgaaattcggtgctcgtgtaagtttttctcttcccctcaaattttatttcagtaaaagga
gacttgggcgaggtggataccccacagccggattcttccccccctgccccccagggtggctaatgctatctggggaagtcgtcat
agggaagagaactatgggtgggctcctgcctgaggcctccaatctcagcccagtggacatatcacaggcagcttaaaaaaaaaa
ccctaaaaaaaacaccccaaaacacacatttaaataggtattcaagacagctttaaaaaatgcacccactcacaccccccctccctttt
ctttttggaaaaaaaaataggaaaaaaaaaaaaaccaaaccgaattctcgcttggcctcta

SEQ ID NO. 6
>AW047643 104477_at
tttttttttttttttttaaattcaagtgcctccatcttgtttacctatcactttaaaaagtactttaagtataagcttcaagagttttgagttctag
agttttgcctggctgttgagatttggtgcaaattctcatgtgtgtttatataggaaggtgagctgaccatccactgcagaggatggagg
taattaacatgatctgtgctctttacaaaagagaatccagtgacctggcctgtgtgtgtgtgtgcgcgcgcgtgcacacacgctcgct
agcgctcacattatgcatgaaaacttgattttcctgaagcatccatttccaaattggatagggatgaaaaagtaaaatttccattggatt
caattagaagc

SEQ ID NO. 7

>AA764261 104533_at
ttttttaagacaaaaggtttttattcaaaaaatgccaagtataaaaaaaaaaaataagatctcttttattcccctgtacagtatactttact
cagataaaaccagcaggctgcacgctgctcaaagcacagccccagagaggatccggaagcacaccacaccggaactgtcttat
gtacagtcagcagaccctagacccactccagcaagcagccaagggggcccctggagaagggggctacagcagctgtcccacc
ggagccccaacaccccagcaagcgtcacagccctcccctaaaaggtccccaagtcaggagga

SEQ ID NO. 8
>AI845237 94255_g_at
tttttttttttttctgacacagtagtaatccttccagactccttacacaatattacaactcccccagtacataaatgttcctataccatgca
cacagcaacatggggtccactgatgtcgcaggcgacttttctaatggtggaacatagcacctcaagttctgccatctacacagtgaa
gggacgtgatggtcggggctccagagtgacagcaaactgcctcttgggtgacacgcttgtggcgaagtgcctcc

SEQ ID NO. 9
>AI845579 94555_at
tttttttttttttttcatccacaaaaaaaaaccaattttattaatccaagaaatgaaaaccatgtccctatggcttctagccccctcaaaa
ggatgaggctgggttcatgaacctggggtagaatgtcccctatccctcaccctcagcttattagactggaaaaagagtttgtgcaaa
taggacggtcaccagaggtaccaagtatgggggtgtcaggccagggcagcagatgaaggaggtgagggatctgt

SEQ ID NO. 10
>AB023957 96132_at
ctgacaaggagatctgtggagtttttctccaagtgaaccaatcccctgtgtcctggctcacactgtggttagggtgggcacatccact
ctgccatctttaacacacagaaaccaaacatcagtgctccagtagcttactaatggaaagaggttggggtgagactcacccacaga
tgagccactctgctaggagccagctgtccctgtgctccttggtggggtctaggaggagtcaccctgttactagaattaccagcttgat
tcggcttcctctctgcatttccctggttctgccccacctgcgaggtccagagccatctcattcagggttcgccgcttcacagtcatccg
aagggaggaggggatggccgccacccattcctttgtattattgcaactggtgctataaacaccaagtgctattgctcacagagacc
catgaaggtaggtggaggagagacaaaaaacctagctggaactgaaagcagacatggagaccaggacccaggagaagagtc
agtcgtgtatttaagagttcactttgtgaagcagtagctttcagtgcggtgttaaattataacgaaggcatggagtaccagggtgcttt
atatttagattatattttaagtcatggtgcatacttgaggggggtttattaaaaatgaaagtactaacttaatgtctactaagtttaataaaca
aagaatttggaagtaaaaaaaaaaagct

SEQ ID NO. 11
>AW125478 96920_at

tttttttttttttttatgaagaagtattttaatagctgaaactcaaagtcactgtactccgggttccaggattcctaagaggcatttttggcca
tctcagaagccagggtcacccacctgtggtctcggggcatcaaattcctctgagtgccgactcggaggaaaagtgtaaacacgcc
caagaccatggctgcgaggaccttcctcttgcctacgcatccgtcaactgcattagtcagacaacttaggctaagggaagggaca
aatgttgactgtgtaagcaggaacagcccaagcttgtaaggaaaagcacgctgcatacatagcatacagaaggcaggacttctgc
ctgtgcacctgcaacattacatttgttttgcccgcaaactatcaagaaggattctgtcgggaggggtgttccctgaacaagcaaaatg
ttggacaaaggtcccgggtgtcctgcgtcac

SEQ ID NO. 12
>AW049031 98083_at
ttttttttttttttttgctgataagaattctttttatgttattcgaataaaaaatacattcatacagaaatataacaatctcgcaaaaaacaatt
tcaaataaaatcttgtaaaacaaaattttacaaaaatcttacaaagattctttagataacagggtgcttcaaaaaaaaagaaataaaga
aatttcactaatagaaattttttttttaatttcaagcaaaagttcctgcttgattgaggctcagttgtcacctgaccagaatggactgctta
gtattaaagttacagcatcgacacggacggcacccagccccagccagtccagcaacgtcgctgtgtttcataagtgagacgcgcc
agcacaagtttcctctctcttctgtttaccttcttacttaatggaattgctatggataagcacacagcagggccaaaaaaggagttttcc
aaaatccagcaaatcaagtg

SEQ ID NO. 13
>AA816121 100880_at
cataaaacagctgaatacatacaaaactgtggttattgatgagctgttgcttttgttgcctttcttgctgtttattaactgacaaagaccat
gctaacaatcatctttatgaatgggtgctagctgatgggcagatcatttgtatgggctagataaaaatgttaataatgaagccatgtgc
tcaggtttgaattgaaaacaattaaaaaaaatcacttgctattttgttttcctttgaagaatataaagttatttcagttagatcatcagtaaa
gttttatctgggctaagaaaatttcccttgagttcttgtgct

SEQ ID NO. 14
>AI255271 102096_f_at
tttttttttttttttttttttttttttttttttttttttttttaagggcaggggtaatctttttttgacccggaagaggaaaaaattcatgaaaaattgc
cgtgggtttttcaaaaaaaaatttaaagaactctttgtttcttgatgaggtatcctaggtaccatggggatagactggaattggccttctc
agggagacaggatggaatgcaaaccccaaaatttaaactggggatgctgtttggataggaagggatgatgggggacccctggg
gagaagcctccactcaacactggaggctcaggccattcttcattctcggccctggaggcaacgatgggcattggataggccaatg
atattttctttaaggattccatgctcctcacatagttgggcaaacctttccttgatgtctgacctcaaatctggttctcggccatagagcc
ccatcagcttgaaggttcccccatcctttccgtaatgagatgagccataagaaagttatcatagtctgcctaagtataggaaatggatt
gaatcatcatcccgcacagatattcaccagctttttgtttgcagcaccctagataat

SEQ ID NO. 15
>AI504305 102104_f_at
tttttgtctttcccatgcttttattggttgatagagaacaaaagaatatcaaacaaatgtgtttacatatcacaggggacttgatctgtgca
taaaaacaaaataacaacaaaagcccttacatatagaacatacatncaaaaaagatttnttttngaaccaacagcatgaanacttctt
gagattcattgcatgccactagtttctatgcactagtgaataaaactgatatgataaaagacccacatcatagcagagattctgaatac
aaagactgctaatggttaactgttgctgttaattgcagggggcaagaaaatgtccctttgctccactacaatggcctgaaacattgnt
nggntttctttctttctttctttttttattacgtattttcttcaattacatttccaatgcgtatcccccaaagcccatacctcccccccactct
cctacccacccattcccatttttttggcccctgcgttcccctgactggggcatataaaagttgcctgaccaatggccttctcttccagtg
atggccgactaggtcatcttttgatacatatgccgctag

SEQ ID NO. 16
>AA866655 103667_at
ggtaactacagcttagaaacccaatatcagttgtacttaacatctttaagagcatgatcataaaggctattttgacagttcattttaacat
ttagagttgataagggtttatatctcatctgtccatattgtttgcaaaggaacaaggttttatgtaggtgagagaaagcatttgtacga
agttagatttgaacatagataagataggttattccagttgagaatattgttcgaagaatgtctgtgaatgcagacatgggtcctgcttag
tgccatgggtagactgagtcttctgtaagtcatcataacatagtaattttggtcctgaattttacattaaattatttgaatatatacaga
cctaaattttaaaatctgtacatatataattttgatgtattaagatggataatattgctgatttaaatttaattatgcacatacttaaaaggac
cgaaatgtctggtaaagtaaattgtaaataaggatatgtagcctttaaccttttaaataattagatttttttaatgtgggtctcctcag

SEQ ID NO 17
>AI159572 103753_at
gcttaacgtgtttgatacctaacttcatgatcacgatgacctgtccttaccaagagcaaagcaagacatagctgtggctttgcttctgt
aaccagctcatggggtgtgaatgtcatgaagttgtctcgctgatatatgtttaatgtcatgttatatgagatgatcatgtgatgtgtacag
actatgttgagaagtgccagtggtagtaactgtaaagtctgactcacaccattaatccctctaaagtacacagccttctgcctctgtgt
ggagttgtcagtacagctcgtcaaagaaaactgcctaatataaaaatcatatatatggtgataattttccctcttttgtagtctgcacaa
gatccataaaagattgtatttttattactatttaacgagtgattaaatgtagtctgcacagtgagcaagagttcacaattctttttatactgc
tggattttgttgtgcatcatttaa

SEQ ID NO. 18
>AI573367 104056_at
ggaacttcaatagaatatcgtcccttgtctgaggaaagaccacctcaaccacatgcctatctagagattgaataaaactgtgaggag
acactgtgtggatgccttaagatggctggcgctggctgcagggaacaatctatttcagctcatcttcataaagcccaagatgctccc

gtgcataaccttgttgaagaaatgagaaatgcggtgctggctactttgatgtgtctggggaggctggcgtcattggttaccatcatta
agtcactgtccagtgcccaagccttccttcactaccactcatatgtctacatccattcattcatctatgcaagcttgtgtttttgtggtttgt
attttacacctggacatcaggtttaacattggtgttttaaataatttttaaaccaaagtattttatgagtaagtctatgtgctctgtttttgt

SEQ ID NO. 19
>AI153421 96215_f_at
cttcggcacaggcgcacggtnaggagtttgcctctgcctcccgagtgctgggatgaaaggtgtgcaccaccacgcccgactcca
attttttctttattatttttttgtgggtgtgaattgaaagtctaatacgtgtttctctgtttatggacccggatcccacgccagcgtccact
ggtcactgccgctgcccacagcgacgtcgcccacgagagcacacacgtagacccagacgccgtggtcaagaatctctgccatc
cccacgggacggacggacggacggactccacaaggtgcgcgtgtcaccgaggccgccagaatggagccactctcacgattct
caacagggcctgaccgcgtacagaaatgtccccctcaataaa

SEQ ID NO. 20
>AW047728 104070_at
tttttttttttttttcacattctacttctgctttattgcacaatatctataagaaaatattaattttaaattagaaaacataaaaatgctttcaat
atagaagagaccatctgattttaaagacaatactctttaaggatgtctacccaggaaataacaaactttcacaggataccaaactgga
cagacgcttcagcgaaatcggtacaaagctgttttaaaaccagtctgagacacttaatgcagcctctattatcactcttcattaatagtc
tagataccactcaaagtgttccttacatttcccaatgttgaggcatcttcagtgtgatcttcagtgaacactatgaacatacagcatgcc
ttccatgtcagaagcagcactcggga

SEQ ID NO. 21
>AW047583 104193_at
tttttttttttttttcaacacaaagctttcggcatattttattgtgaaatattggcttagaaaaagtacaaaatgccaacagtcagatgta
tacaggtacagtattttgttaatctatacatgtgaccagcttaacaggctcatcggaaaatgcatatttagaccaatatactctaaaaat
gactgtaatattcacaggctattaatacatgataaactggagtctatatgaccctcactgggatatgctaactgcagcaaacaggcta
aaagcacaatacagcacaccatagtacctctggacaggtccacgtttaaagagtatcaattcattagatcaatacactacagatctg
ggactgagactcaatcactaatttatacaattgcaaaaaaaatttaggttataaaataaagttatgg

SEQ ID NO. 22
>AI464596 104640_f_at
ggcatcgttggtttcttggctttgtgcatgttaaacctggtatttctaatgatacagtttttcatatgtacataaatttttgtatgtaggtgtgt
cggggagggcagaacaacttgcaagtttgatctcaatgtgggggtcttaaactacctggtcttaaagctttctcttcttcctagg

gtgacacgagtgaaccctaccctaaaatggtttaaagataacatgaagggtccttaaaataaattgaagaaatcctcaaatcttctgg
tgacctggatggacagcaaaggaatcaaatccctcaaataatttatttgacttaaattaaaaataaataaa

SEQ ID NO. 23
>AI848671 93806_at
tttttttttttttttcacttaatcagttttattgaaattcatcagatgaaaagaatcagtgttccatagaaaaggattaatgttgtactttgtaa
ttttaaacaccaacaactgcttaacaaaaattcatgtgagttttacattttgttgttctttttacttaaggagtgaagtctgatttcaccaaa
actccctgtactcagtctttcccagtaaaagtaatctttgtttcaaaattccctattcacatatacttagggtcccaatgaagtgtccacat
ttagcagtacaattaacaatttacagtgtttttgaaagtggaactctttctaaagaacaaaatttctaaagggcaaatcagctcttaaatg
tgaaatttacaa

SEQ ID NO. 24
>AW124390 94081_at
ttttttttttttttttgacttttaaaggatttttatactatattaaaaaaacacaaaataaaaaagggatccatcaacatatattttagaagtc
catccaagagtttcagtgtccaccagccatggacacaacagccatgcctgtgtcattgttcagtctgtagctcatgtaaggatcaag
gaggtgactttaagttacaatcaaacttgctctg

SEQ ID NO. 25
>AI845607 94975_at
tttttttttttttgtgataatacaattttctttagtaaaaataatttacagcatcaataacatatacacaatttcataactgaactttacctcca
atatatttctatacaatacttaacattattgaacttaaaactgttatgctattttgttggctttaaataacaatgatttgtagttacttaagaga
tatataaggttttacagtcacatattatgcataaaattgataaaacaaacaaatcagcacagtcaccttgaaagggaaaataacccac
tggaaaaaagccaaagttcatttccttccccgtttttactccccaaatcaaatacagtatcattgtccagtaccttagcatcttctctc

SEQ ID NO. 26
>C77009 95028_r_at
gggaggaaanggtttatttggcttacactttcatactgcttactgttcatcaccaaggaagtngggactggaactcaagcaggtcaa
gaagcagaactgatgcagaggccacagaaggatgtttctttattagcttgcttcacctggcttgctcagactgctgtcttagagaacc
caagactaccagtgcagagagatggtcccacccacaaggggcctttccccttgatcactaattgagaaaatgtcttacatctggatc
tcatggaggcatttcccaactgaagctcctttctctgtgataacttcagcttgtgtcaagttgacacaaaattagccagtataattgaca
ccttgtcaatttgaggcgnnnncacatcactagtaatgctcaactcttagtttcttattcatgcccaagatatacacaactttgaaagcc
ccacagactttacatattaaaagttcaatgcttttaaaatatccaatatncgtg

SEQ ID NO. 27
>AI891629 96616_at
ttcacatagcatcaagtgactacagatacaacactagaattttgatgtgtattatttgaacacgtttatatatgagaaaaatttgaaattta
agttacaaagtgagtatttttttagcacatttagtagtgactatttatataaactatcattttgtttgggtgaccaaatgaaatttgaacataa
ttaaagaacatataactttcaaacaacattaatacagttacagacctgttacactgagaattatgtacacgtattaaagaggactgagg
tttcttaaacccattcttttgaaaggtttttaatccttcttaatgtaattcagtgtaaaacttcaaaaagtctactcggtgtcacagcaggat
gaggtggaataatccctgctgatcactgagcaaactgtgtttgtcatctgattctcctgacaaccactatacttga

SEQ ID NO. 28
>AA684508 97838_at
gttcatctcaaagccctttacactttggagccaggcctgttcaattttacaaataacacgttttgcttgtagtcagggtgctgtgacaac
atgaagagttcgagctacctcccaggatgagtgcaggttttcctggtgacaaatctcaggcattcaaaggttctgttagcagtgccat
acttgtgggtttacagagatggtgtgtctggctgagaacaaaggagccgtaccagggaagttgaagcctatcttcattttcaagtgc
aggctaaaataaggtctgaggaaaagaatgagcagcgtgtgaaggataggaacagaaatcatggaagatctgtttgtccaggcca
gtgtttcaccataagagatcactttaaaataaatgagatgaaataatt

SEQ ID NO. 29
>AW048644 98893_at
tttttttttttttttcactttgcaaactgttgtttttatttggattaaaaagctttgatgaacaaacatatgatacatacatggtatagaacca
gttaccacttaaaacctcagatgatatagtacagaaattagaataaaccaaattttaatcaggtctggaaacaaacgttcaagctgaa
agctgtaacatccatttggagattttacacacctgtcccttacccactcagttcttccttagaaggggaaggcagacttagtaaaacca
atgtttttaagtcagctgactgacattctattttaaacctataggtccccaacccaatataaacaaaattcttcacaggaacttacatttt
gaaaacaattaggccattatatttcctaagccacattactacagttcttaaaagtcatgagaaag

SEQ ID NO. 30
>AI844810 103416_at
tttttttttttttttattcatgcttgcctagggatggggaatagatcattcaataaaaacatacagtaaaaacagggggtggggagggg
gaaggcttatcatgtacaatgtttaaactacaatagtgatgtaccttaattacttccatgcacacaagtctaacattacaagtttttaaaa
aataaacaccattaagacttctaggagcattttataataaattcctaatttttctttgtagatagatcaagcacctccaaaatacagattc
ctatacacagtgagcactttacttaacgtacatggacagcctcaggacgagctgacgtctcggatcagctcggcaggcaacaaac
catagtgccaaatggaaagaagggcagttgcaaataaacttaaaactaagttaacttttataattaaatacagaaaatatactgatttg
ctaaaaataaataagatgtgatgtatttaacacttcactataaagaatgaacaccatgacatcctcgtgccg

SEQ ID NO. 31
>AA608387 103723_at
ttcgtcgacatgcaatagcagggggaaaggggacaggtaacaggagtacctttgttttaacagacaaaagtaatctccaggcctta
gggactcataaagatatgagtacattgcttattggaaacttgtaggttcacatattattttgactaaggtatatggatttccccccactta
cataagtaaacccaacataaggaggaccattccttttctttcattcagatgaccttttctgaatctgggcactgaagggatgcataaa
ataatgttaatgttttcagtaatgtcttcaagtagaagactttctgttgaactacttataagaaagtaaaataaatattgtctttttgtatgtc
aaaaaaaaaaaaaaaaaaaaaaaaaagg

SEQ ID NO. 32
>AA689670 104737_at
aattcgtcgacatggcacagcttcctccctaggcgtgagactccggctccttcactatgagacttctagccctttccggtctgctctgc
atgctgctcctctgtttctgcattttctcctcagaagggagaagacatcctgccaagtccttgaaactcaggcgctgctgtcacctatc
tcctagatccaagctgacaacctggaaaggaaaccacacaaggccctgcagactctgcag

SEQ ID NO. 33
>AW060684 94433_at
tttttttttttttttgattaatcatgtatttattttagttcagttaaaacaaacatacattgtttcattgaaaccggtgtagcatttactctctgc
caacaggccacagaggcatgttggcctctaacagtacagctgggacatttacactatatacatacaaggagtcctctttcattgagat
cacacttgacatgttaccaaatagccatgtggtagctactgcattttgaaaatttagacctcatttaaagctttagattatctgaacttaa
gttgatgtccaaccagtttattgcaaatacaatttaaacaagttattttcaattttagtattatacacaatgtcaatttcttaagattgaattct
ctgtacaagta

SEQ ID NO. 34
>AW125178 94486_at
tttttttttttttttgaccacacagacatggagttaccaattgtagaaatcgaaaaaattggcatcaactctcgaagtcagagtgaactc
ttgcctgcgggttggcttgactacgcccaggcactgagctgcctcaaccagctagggagctatgatgatgctgagtcctgttttcat
gatgtcaccagatgtatgtagtaggtaatttgtctcacaaaggcctttgtaccaatggctgtgaagcttg

SEQ ID NO. 35
>AI835858 95542_at
tttttttttttttttaattacgcaaaaatttatttgaccaaaatgtagaaaaagtgatactattacgtatgatacaatagcaagaacataaa
gaacagagtagattttatcaattgcagtttgctcaaagttcatctccctggtagtgtgatgcttaataaataggagtgaggggcag

ggttcagataagctagaagcagggtgactttcagtcaggactgtaaacttgagttggccccgcactgctggggaatgtggaatgttt
cagtgtgagatgttaactgagaaaggaaaactacaaagccaaaaaatgtgcagcctacagagcactccacatccagttcaatcag
gaggtcttggtcttttattagtgtcattcccgaaggaattcaagtcttggcaaagtcaagcttccatttgttgttccctgaacaatggaga
tgaagttaagccaatttctttttcta

SEQ ID NO. 36
>AW121246 96281_at
tttttttttcctactagactaattaaaaaaaaaagtcaacattgatatcgattacttgcatgaagacacaggaaaacaggcgacataca
ctccttaaagacacacaagataagaagatgaaacttcaggtacataagaagtctgtacaaaaagctagatttgatactctcaaaaag
tgaagggtcctacaacagcatagagaaataatttaatgccttccagaacagaactccagctctgtggaggttttcctattctatgggg
gcagatctcatgccaacccacagagcaggcgcttccacctcctatccgtttatgcggtagttttcatggatttccggccggcctcgtg
ccg

SEQ ID NO. 37
>AW214159 96896_at
atgttgaaaaatcttatttattttcttaattaaaaagaagtatatttgtgttaaatttagaatatttagctttccattgatcttcccaatacttgtc
agatagatactaatagtgctcagaactgtgaaagttaatatccaataccatattttctaagaagaccctcaaggcattgggga
ttttaatttaaaaataaaagaattttaattagcattg

SEQ ID NO. 38
>AW123953 97904_at
tttttttttttttttttgaaaaagaaatttttgcctttattagaatggcattaggccttaaatatgccaattttggtaatcacattattgttttaat
aagaaacgactctacagaattgcaatactggtccaacagtcttgtccttcttttaaagcaagaaacagaatgtaagtaaccagaaag
cagggcaggcatcggctaacccaggagactagcttcttagatccaagcatttgcagagagaaccgttgggctggggaggggtgg
agcagctcgagataactggaacccagagtgtgcgccaagtcccatgaggctgcttgttgaactcatcttttccttggtcacattgtttc
cctc

SEQ ID NO. 39
>AI842675 99159_at
tttttttttttttttttcaagagaaaataggatttatttatcacatttgagatgaacacacaggactgtcccagaaatcttttacatatatattat
tgatagattggatttattcagcatacagaaaaagcaaagcctgccataagacacttccctcttgagaacagcaagatgtcaggcagt
gtctgtaagttttcaccacgggaattcacattgagcaaaaagaccgggctcacaggtccgttcccaggtcctgctgcttctatttcaa
aggctttca

## Claims

1. A gene comprising a sequence according to any one of SEQ ID Nos. 1 to 39.

2. A gene comprising a sequence according to SEQ ID No. 32.

3. A gene comprising a sequence according to SEQ ID No. 19.

4. An isolated and purified protein encoded by a gene according to any one of claims 1 to 3.

5. A human homologue of a gene according to any one of claims 1 to 3 or of a protein according to claim 4.

6. A biomarker derived from a gene according to any one of claims 1 to 4.

7. A method for identification of a compound useful in the treatment of wounds or of conditions **characterised by** an excessive or impaired wound healing response, which comprises contacting a compound with a protein encoded by a gene comprising a sequence identified by one of the accession numbers of cluster 2, 7, 11, 16, or 20 or a mammalian homologue thereof and detecting specific binding of the chemical compound to the protein.

8. A method for identification of a compound useful for the treatment or reduction of inflammation which comprises contacting a compound with a protein encoded by a gene comprising a sequence identified by one of the accession numbers of cluster 5 or 6 or a mammalian homologue thereof and detecting specific binding of the chemical compound to the protein.

9. An antagonist or inhibitor of a protein encoded by a gene comprising a sequence identified by one of the accession numbers of cluster 2, 7, 11, 16, or 20 or a mammalian homologue thereof, for use in the treatment of a disease or disorder **characterised by** excessive wound healing response, such as scarring, fibrosis, restenosis post angioplasty, psoriasis, post-traumatic/surgical adhesions of the peritoneal cavity, joints and ligaments, benign prostatic hyperplasia, glaucoma, or peripheral nerve injury.

10. The use of an antagonist or inhibitor of a protein encoded by a gene comprising a sequence identified by one of the accession numbers of cluster 2, 7, 11, 16, or 20 or a mammalian homologue thereof, in the manufacture of a medicament for the treatment of a disease or disorder **characterised by** excessive wound healing response, such as scarring, fibrosis, restenosis post angioplasty, psoriasis, post-traumatic/surgical adhesions of the peritoneal cavity, joints and ligaments, benign prostatic hyperplasia, glaucoma, or peripheral nerve injury.

11. A method for treatment of a disease or disorder **characterised by** excessive wound healing response, such as scarring, fibrosis, restenosis post angioplasty, psoriasis, post-traumatic/surgical adhesions of the peritoneal cavity, joints and ligaments, benign prostatic hyperplasia, glaucoma, or peripheral nerve injury, comprising administering to a mammal in need of such treatment a therapeutically effective amount of an antagonist or inhibitor of a protein encoded by a gene comprising a sequence identified by one of the accession numbers of cluster 2, 7, 11, 16, or 20 or a mammalian homologue thereof.

12. An agonist or activator of a protein encoded by a gene comprising a sequence identified by one of the accession numbers of cluster 2, 7, 11, 16, or 20 or a mammalian homologue thereof, for use in the treatment of a disease or disorder **characterised by** an impaired healing response, such as chronic dermal ulcers, oral mucocystis, emphysema, ulcerative diseases of the gastro-intestinal (GI) tract, or cystitis.

13. The use of an agonist or activator of a protein encoded by a gene comprising a sequence identified by one of the accession numbers of cluster 2, 7, 11, 16, or 20 or a mammalian homologue thereof, in the manufacture of a medicament for the treatment of a disease or disorder **characterised by** an impaired healing response, such as chronic dermal ulcers, oral mucocystis, emphysema, ulcerative diseases of the gastro-intestinal (GI) tract, or cystitis.

14. A method for treatment of a disease or disorder **characterised by** an impaired healing response, such as chronic dermal ulcers, oral mucocystis, emphysema, ulcerative diseases of the gastro-intestinal (GI) tract, or cystitis, comprising administering to a mammal in need of such treatment a therapeutically effective amount of an agonist or activator of a protein encoded by a gene comprising a sequence identified by one of the accession numbers of cluster 2, 7, 11, 16, or 20 or a mammalian homologue thereof.

15. An antagonist or inhibitor of a protein encoded by a gene comprising a sequence identified by one of the accession numbers of cluster 5 or 6 or a mammalian homologue thereof for the treatment or prevention of inflammation.

16. The use of an antagonist or inhibitor of a protein encoded by a gene comprising a sequence identified by one of the accession numbers of cluster 5 or 6 or a mammalian homologue thereof, in the manufacture of a medicament for the treatment or prevention of inflammation.

17. A method for treatment or prevention of inflammation comprising administering to a mammal in need of such treatment a therapeutically effective amount of an antagonist or inhibitor of a protein encoded by a gene comprising a sequence identified by one of the accession numbers of cluster 5 or 6 or a mammalian homologue thereof for the treatment or prevention of inflammation.

18. A method for investigating wound repair in a mammal comprising: taking a wound tissue sample at one or more time points, isolating mRNA from the sample (or samples), determining for the, or each time point, the level of mRNA expression from one or more of the genes including the sequence of one or more of the accession numbers grouped in clusters 2, 7, 11, 16 and 20 or a homologue thereof; comparing the level of mRNA expression of the, or each, gene from the wound tissue sample at a given time point with the level of mRNA expression of that gene in a control sample of mRNA taken from non wounded tissue and determining whether the level of mRNA expression of the, or each, gene differs in the wound tissue sample relative to a control sample taken from unwounded skin.

19. A method for identification of a mammal with an impaired wound healing response comprising: taking a tissue sample from a test subject, isolating mRNA from the sample, determining the level of mRNA expression from one or more of the genes including the sequence of one or more of the accession numbers grouped in clusters 2, 7, 11, 16 and 20 and comparing the mRNA expression level detected in the test subject with the mRNA expression level of the or each gene a control subject with a normal wound healing response; wherein a difference in mRNA expression levels of one or more of said genes indicates that the test subject has an impaired wound healing response.


SEQUENCE LISTING

<110> Pfizer Inc (US, JP, CA, EP except EP(UK))
      Pfizer Ltd (EP(UK))

<120> Wound Healing Biomarkers

<130> PCS 22024

<150> GB0114869.1
<151> 2001-06-18

<150> US60/305346
<151> 2001-07-13

<160> 39

<170> FastSEQ for Windows Version 4.0

<210> 1
<211> 234
<212> DNA
<213> Mus musculus

<400> 1
tttttttttt tttttttgaa gttagaaaag tcttttaagt ttttaaatta aaattgcacc 60
ctggtgcgat gactctaatt tgggaactca atccaaaggc agactctcct gagtgctggt 120
cctctggcct ggacacacag agactgggag cctgtcggac agttagaaca gggaaggggc 180
agaagcaccc acatcagagg gcagccaagt cttgacatct acctggtgac aagg       234

<210> 2
<211> 356
<212> DNA
<213> Mus musculus

<400> 2
ttgaaccgcg ggcggcctgg gctccttctg ccatggcccc cgcgctcagg tccttgctgt 60
cgccacggac tttactgctg ctgctgctga gcctggcgct gctgggcgcc gccgagcccg 120
ccaccgggag cgctgtcccc gctcagagcc gcccgtgcgt ggattgccac gagtttgaat 180
tcatgcagcg cgcccttcag gacctacgga aaaccgccta cagcctggac gcacggacgg 240
agaccctcct gctgcaggct gagcgccggg ctctgtgtgc ctgctggcca gctggacgct 300
gaagatcttt ggccagtgat attccttgtc aataaatgcc tgctagcatg ccgnga     356

<210> 3
<211> 296
<212> DNA
<213> Mus musculus

<400> 3
tttttttttt tttttggttt tagcagtatg tttttattta gggaacacag tatttgcaat 60
agttctgaca gccgtaaggc cctccatttt actagctgtt tgctttgcaa taaaatagcc 120
agaaattctg atatcaacat taataggata tttcttcatg caggttgtag gagtatatac 180
attgtataac actgaagaaa ttaatacata tgtacattat ttacatagta actatttatt 240
gtaaatagaa atgttttcta agataaaaag aaatcacaac atggtttctg gtctgt     296

<210> 4
<211> 434
<212> DNA
<213> Mus musculus

<400> 4
gtctgtaaaa atctgtttaa taaatataca tcttagaagt accaaaataa ttaccaacaa 60
aatacaacat atacaacatt tacaagaagg cgacacagac cttagttggg ggcgactttt 120

aagcacatgc cactgaacac ctggctctta catgggagga cacactgggc tcacttacta 180
ggtctatggt ggttcaatca aaagcacaat aaataaaacg tggtcctttc attaggttct 240
ggaaaatcac ctcccccccc cccaaaaaaa atcccacaaa catgaacctt aagagacatt 300
ttctttgaat ttcagtgatc tgtttccccg gatttcacaa agacaacagc cgaatcaccc 360
cagtaaaatg cctgggtcta ggcgctgtgt ggtgtggtgc taagtatacc ctttctcatt 420
tttttctttt ttct 434

<210> 5
<211> 417
<212> DNA
<213> Mus musculus

<400> 5
tttttttttt tttttttcag gtctcatttt cgtttatttg aaattcggtg ctcgtgtaag 60
tttttctctt tcccctcaaa ttttatttca gtaaaaggag acttgggcga ggtggatacc 120
ccacagccgg attcttcccc ccctgccccc cagggtggct aatgctatct ggggaagtcg 180
tcatagggaa gagaactatg ggtgggctcc tgcctgaggc ctccaatctc agcccagtgg 240
acatatcaca ggcagcttaa aaaaaaaacc ctaaaaaaaa caccccaaaa cacacattta 300
aataggtatt caagacagct ttaaaaaatg cacccactca cacccccctc cctttctttt 360
ttggaaaaaa aaataggaaa aaaaaaaaaa ccaaaccgaa ttctcgcttg gcctcta 417

<210> 6
<211> 375
<212> DNA
<213> Mus musculus

<400> 6
tttttttttt tttttttaaa ttcaagtgcc tccatcttgt ttacctatca ctttaaaaag 60
tactttaagt ataagcttca agagttttga gttctagagt tttgcctggc tgttgagatt 120
tggtgcaaat tctcatgtgt gtttatatag gaaggtgagc tgaccatcca ctgcagagga 180
tggaggtaat taacatgatc tgtgctcttt acaaaagaga atccagtgac ctggcctgtg 240
tgtgtgtgtg cgcgcgcgtg cacacacgct cgctagcgct cacattatgc atgaaaactt 300
gattttcctg aagcatccat ttccaaattg gatagggatg aaaaagtaaa atttccattg 360
gattcaatta gaagc 375

<210> 7
<211> 320
<212> DNA
<213> Mus musculus

<400> 7
tttttttaag acaaaaggtt tttattcaaa aaatgccaag tataaaaaaa aaaaataaga 60
tctcttttat tcccctgtac agtatacttt actcagataa aaccagcagg ctgcacgctg 120
ctcaaagcac agccccagag aggatccgga agcacaccac accggaactg tcttatgtac 180
agtcagcaga ccctagaccc actccagcaa gcagccaagg gggcccctgg agaagggggc 240
tacagcagct gtcccaccgg agccccaaca ccccagcaag cgtcacagcc ctcccctaaa 300
aggtccccaa gtcaggagga 320

<210> 8
<211> 255
<212> DNA
<213> Mus musculus

<400> 8
tttttttttt tttttctgac acagtagtaa tccttccaga ctccttacac aatattacaa 60
ctcccccagt acataaatgt tcctatacca tgcacacagc aacatggggt ccactgatgt 120
cgcaggcgac ttttctaatg gtggaacata gcacctcaag ttctgccatc tacacagtga 180
agggacgtga tggtcggggc tccagagtga cagcaaactg cctcttgggt gacacgcttg 240
tggcgaagtg cctcc 255

<210> 9
<211> 254
<212> DNA
<213> Mus musculus

<400> 9
tttttttttt tttttttcat ccacaaaaaa aaaccaattt tattaatcca agaaatgaaa 60
accatgtccc tatggcttct agccccctca aaaggatgag gctgggttca tgaacctggg 120
gtagaatgtc ccctatccct caccctcagc ttattagact ggaaaaagag tttgtgcaaa 180
taggacggtc accagaggta ccaagtatgg gggtgtcagg ccagggcagc agatgaagga 240
ggtgagggat ctgt 254

<210> 10
<211> 728
<212> DNA
<213> Mus musculus

<400> 10
ctgacaagga gatctgtgga gtttttctcc aagtgaacca atcccctgtg tcctggctca 60
cactgtggtt agggtgggca catccactct gccatcttta acacacagaa accaaacatc 120
agtgctccag tagcttacta atggaaagag gttggggtga gactcaccca cagatgagcc 180
actctgctag gagccagctg tccctgtgct ccttggtggg gtctaggagg agtcaccctg 240
ttactagaat taccagcttg attcggcttc ctctctgcat ttccctggtt ctgccccacc 300
tgcgaggtcc agagccatct cattcagggt tcgccgcttc acagtcatcc gaagggagga 360
ggggatggcc gccacccatt cctttgtatt attgcaactg gtgctataaa caccaagtgc 420
tattgctcac agagacccat gaaggtaggt ggaggagaga caaaaaacct agctggaact 480
gaaagcagac atggagacca ggacccagga gaagagtcag tcgtgtattt aagagttcac 540
tttgtgaagc agtagctttc agtgcggtgt taaattataa cgaaggcatg gagtaccagg 600
gtgctttata tttagattat attttaagtc atggtgcata cttgaggggg tttattaaaa 660
atgaaagtac taacttaatg tctactaagt ttaataaaca aagaatttgg aagtaaaaaa 720
aaaaagct 728

<210> 11
<211> 468
<212> DNA
<213> Mus musculus

<400> 11
   tttttttttt tttttatgaa gaagtatttt aatagctgaa actcaaagtc actgtactcc 60
   gggttccagg attcctaaga ggcatttttg gccatctcag aagccagggt cacccacctg 120
   tggtctcggg gcatcaaatt cctctgagtg ccgactcgga ggaaaagtgt aaacacgccc 180
   aagaccatgg ctgcgaggac cttcctcttg cctacgcatc cgtcaactgc attagtcaga 240
   caacttaggc taagggaagg gacaaatgtt gactgtgtaa gcaggaacag cccaagcttg 300
   taaggaaaag cacgctgcat acatagcata cagaaggcag gacttctgcc tgtgcacctg 360
   caacattaca tttgttttgc ccgcaaacta tcaagaagga ttctgtcggg aggggtgttc 420
   cctgaacaag caaaatgttg gacaaaggtc ccgggtgtcc tgcgtcac 468

<210> 12
<211> 473
<212> DNA
<213> Mus musculus

<400> 12
tttttttttt tttttttgct gataagaatt ctttttatgt tattcgaata aaaaatacat 60
tcatacagaa atataacaat ctcgcaaaaa acaatttcaa ataaaatctt gtaaaacaaa 120
attttacaaa aatcttacaa agattcttta gataacaggg tgcttcaaaa aaaaagaaat 180
aaagaaattt cactaataga aatttttttt tttaatttca agcaaaagtt cctgcttgat 240
tgaggctcag ttgtcacctg accagaatgg actgcttagt attaaagtta cagcatcgac 300
acggacggca cccagcccca gccagtccag caacgtcgct gtgtttcata agtgagacgc 360
gccagcacaa gtttcctctc tcttctgttt accttcttac ttaatggaat tgctatggat 420
aagcacacag cagggccaaa aaaggagttt tccaaaatcc agcaaatcaa gtg 473

<210> 13
<211> 316
<212> DNA
<213> Mus musculus

<400> 13
cataaaacag ctgaatacat acaaaactgt ggttattgat gagctgttgc ttttgttgcc 60
tttcttgctg tttattaact gacaaagacc atgctaacaa tcatctttat gaatgggtgc 120

tagctgatgg gcagatcatt tgtatgggct agataaaaat gttaataatg aagccatgtg 180
ctcaggtttg aattgaaaac aattaaaaaa aatcacttgc tattttgttt tcctttgaag 240
aatataaagt tatttcagtt agatcatcag taaagtttta tctgggctaa gaaaatttcc 300
cttgagttct tgtgct 316

<210> 14
<211> 598
<212> DNA
<213> Mus musculus

<400> 14
tttttttttt tttttttttt tttttttttt tttttttttt ttttaagggc aggggtaatc 60
tttttttgac ccggaagagg aaaaaattca tgaaaaattg ccgtgggttt ttcaaaaaaa 120
aatttaaaga actctttgtt tcttgatgag gtatcctagg taccatgggg atagactgga 180
attggccttc tcagggagac aggatggaat gcaaacccca aaatttaaac tggggatgct 240
gtttggatag gaagggatga tgggggaccc ctggggagaa gcctccactc aacactggag 300
gctcaggcca ttcttcattc tcggccctgg aggcaacgat gggcattgga taggccaatg 360
atattttctt taaggattcc atgctcctca catagttggg caaacctttc cttgatgtct 420
gacctcaaat ctggttctcg gccatagagc cccatcagct tgaaggttcc cccatccttt 480
ccgtaatgag atgagccata agaaagttat catagtctgc ctaagtatag gaaatggatt 540
gaatcatcat cccgcacaga tattcaccag cttttttgtt tgcagcaccc tagataat 598

<210> 15
<211> 580
<212> DNA
<213> Mus musculus

<400> 15
tttttgtctt tcccatgctt ttattggttg atagagaaca aaagaatatc aaacaaatgt 60
gtttacatat cacaggggac ttgatctgtg cataaaaaca aaataacaac aaaagccctt 120
acatatagaa catacatnca aaaaagattt nttttngaac caacagcatg aanacttctt 180
gagattcatt gcatgccact agtttctatg cactagtgaa taaaactgat atgataaaag 240
acccacatca tagcagagat tctgaataca aagactgcta atggttaact gttgctgtta 300
attgcagggg gcaagaaaat gtccctttgc tccactacaa tggcctgaaa cattgntngg 360
ntttctttct ttctttcttt ttttattacg tattttcttc aattacattt ccaatgcgta 420
tcccccaaag cccatacctc cccccccact ctcctaccca cccattccca ttttttggcc 480
cctgcgttcc cctgactggg gcatataaaa gttgcctgac caatggcctt ctcttccagt 540
gatggccgac taggtcatct tttgatacat atgccgctag 580

<210> 16
<211> 542
<212> DNA
<213> Mus musculus

<400> 16
ggtaactaca gcttagaaac ccaatatcag ttgtacttaa catctttaag agcatgatca 60
taaaggctat tttgacagtt cattttaaca tttagagttg ataagggttt atatctcatc 120
tgtccatatt gtttgcaaag gaacaaggtt ttatgtaggt gagagaaagc atttgtacga 180
agttagattt gaacatagat aagataggtt attccagttg agaatattgt tcgaagaatg 240
tctgtgaatg cagacatggg tcctgcttag tgccatgggt agactgagtc ttctgtaagt 300
catcataaca tagtaatttt ggtcctgaat tttacattaa attatttgaa tatatacaga 360
cctaaatttt aaaatctgta catatataat tttgatgtat taagatggat aatattgctg 420
atttaaattt aattatgcac atacttaaaa ggaccgaaat gtctggtaaa gtaaattgta 480
aataaggata tgtagccttt aaccttttaa ataattagat tttttaatgt gggtctcctc 540
ag 542

<210> 17
<211> 476
<212> DNA
<213> Mus musculus

<400> 17
gcttaacgtg tttgatacct aacttcatga tcacgatgac ctgtccttac caagagcaaa 60
gcaagacata gctgtggctt tgcttctgta accagctcat ggggtgtgaa tgtcatgaag 120
ttgtctcgct gatatatgtt taatgtcatg ttatatgaga tgatcatgtg atgtgtacag 180

actatgttga gaagtgccag tggtagtaac tgtaaagtct gactcacacc attaatccct 240
ctaaagtaca cagccttctg cctctgtgtg gagttgtcag tacagctcgt caaagaaaac 300
tgcctaatat aaaaatcata tatatggtga taattttccc tcttttgtag tctgcacaag 360
atccataaaa gattgtattt ttattactat ttaacgagtg attaaatgta gtctgcacag 420
tgagcaagag ttcacaattc tttttatact gctggatttt gttgtgcatc atttaa 476

<210> 18
<211> 448
<212> DNA
<213> Mus musculus

<400> 18
ggaacttcaa tagaatatcg tcccttgtct gaggaaagac cacctcaacc acatgcctat 60
ctagagattg aataaaactg tgaggagaca ctgtgtggat gccttaagat ggctggcgct 120
ggctgcaggg aacaatctat ttcagctcat cttcataaag cccaagatgc tcccgtgcat 180
aaccttgttg aagaaatgag aaatgcggtg ctggctactt tgatgtgtct ggggaggctg 240
gcgtcattgg ttaccatcat taagtcactg tccagtgccc aagccttcct tcactaccac 300
tcatatgtct acatccattc attcatctat gcaagcttgt gtttttgtgg tttgtatttt 360
acacctggac atcaggttta acattggtgt tttaaataat ttttaaacca aagtatttta 420
tgagtaagtc tatgtgctct gtttttgt 448

<210> 19
<211> 385
<212> DNA
<213> Mus musculus

<400> 19
cttcggcaca ggcgcacggt naggagtttg cctctgcctc ccgagtgctg ggatgaaagg 60
tgtgcaccac cacgcccgac tccaattttt ttctttatta ttttttttgt gggtgtgaat 120
tgaaagtcta atacgtgttt ctctgtttat ggacccggat cccacgccag cgtccactgg 180
tcactgccgc tgcccacagc gacgtcgccc acgagagcac acacgtagac ccagacgccg 240
tggtcaagaa tctctgccat ccccacggga cggacggacg gacggactcc acaaggtgcg 300
cgtgtcaccg aggccgccag aatggagcca ctctcacgat tctcaacagg gcctgaccgc 360
gtacagaaat gtccccctca ataaa 385

<210> 20
<211> 392
<212> DNA
<213> Mus musculus

<400> 20
tttttttttt tttttttcac attctacttc tgctttattg cacaatatct ataagaaaat 60
attaatttta aattagaaaa cataaaaatg ctttcaatat agaagagacc atctgatttt 120
aaagacaata ctctttaagg atgtctaccc aggaaataac aaactttcac aggataccaa 180
actggacaga cgcttcagcg aaatcggtac aaagctgttt taaaaccagt ctgagacact 240
taatgcagcc tctattatca ctcttcatta atagtctaga taccactcaa agtgttcctt 300
acatttccca atgttgaggc atcttcagtg tgatcttcag tgaacactat gaacatacag 360
catgccttcc atgtcagaag cagcactcgg ga 392

<210> 21
<211> 426
<212> DNA
<213> Mus musculus

<400> 21
tttttttttt tttttttcaa cacaaagctt tcggcatatt ttattgtgaa atattggctt 60
agaaaaaagt acaaaatgcc aacagtcaga tgtatacagg tacagtattt tgttaatcta 120
tacatgtgac cagcttaaca ggctcatcgg aaaatgcata tttagaccaa tatactctaa 180
aaatgactgt aatattcaca ggctattaat acatgataaa ctggagtcta tatgaccctc 240
actgggatat gctaactgca gcaaacaggc taaaagcaca atacagcaca ccatagtacc 300
tctggacagg tccacgttta aagagtatca attcattaga tcaatacact acagatctgg 360
gactgagact caatcactaa tttatacaat tgcaaaaaaa atttaggtta taaaataaag 420
ttatgg 426

<210> 22

<211> 339
<212> DNA
<213> Mus musculus

<400> 22
ggcatcgttg gtttcttggc tttgtgcatg ttaaacctgg tatttctaat gatacagttt 60
ttcatatgta cataaatttt tgtatgtagg tgtgtcgggg agggcagaac aacttgcaag 120
tttgatctca atgtgggggt cttaaactac ctggtcttaa agctttctct tcttcctagg 180
gtgacacgag tgaaccctac cctaaaatgg tttaaagata acatgaaggg tccttaaaat 240
aaattgaaga aatcctcaaa tcttctggtg acctggatgg acagcaaagg aatcaaatcc 300
ctcaaataat ttatttgact taaattaaaa ataaataaa 339

<210> 23
<211> 385
<212> DNA
<213> Mus musculus

<400> 23
tttttttttt tttttttcac ttaatcagtt ttattgaaat tcatcagatg aaaagaatca 60
gtgttccata gaaaaggatt aatgttgtac tttgtaattt taaacaccaa caactgctta 120
acaaaaattc atgtgagttt tacattttgt tgttcttttt acttaaggag tgaagtctga 180
tttcaccaaa actccctgta ctcagtcttt cccagtaaaa gtaatctttg tttcaaaatt 240
ccctattcac atatacttag ggtcccaatg aagtgtccac atttagcagt acaattaaca 300
atttacagtg tttttgaaag tggaactctt tctaaagaac aaaatttcta aagggcaaat 360
cagctcttaa atgtgaaatt tacaa 385

<210> 24
<211> 217
<212> DNA
<213> Mus musculus

<400> 24
tttttttttt tttttttgac ttttaaagga tttttatact atattaaaaa aaacacaaaa 60
taaaaaaggg atccatcaac atatatttta gaagtccatc caagagtttc agtgtccacc 120
agccatggac acaacagcca tgcctgtgtc attgttcagt ctgtagctca tgtaaggatc 180
aaggaggtga ctttaagtta caatcaaact tgctctg 217

<210> 25
<211> 370
<212> DNA
<213> Mus musculus

<400> 25
tttttttttt tttttgtgat aatacaattt tctttagtaa aaataattta cagcatcaat 60
aacatataca caatttcata actgaacttt acctccaata tatttctata caatacttaa 120
cattattgaa cttaaaactg ttatgctatt ttgttggctt taaataacaa tgatttgtag 180
ttacttaaga gatatataag gttttacagt cacatattat gcataaaatt gataaaacaa 240
acaaatcagc acagtcacct tgaaagggaa aataacccac tggaaaaaag ccaaagttca 300
tttccttccc cgttttttac tccccaaatc aaatacagta tcattgtcca gtaccttagc 360
atcttctctc 370

<210> 26
<211> 496
<212> DNA
<213> Mus musculus

<400> 26
gggaggaaan ggtttatttg gcttacactt tcatactgct tactgttcat caccaaggaa 60
gtngggactg gaactcaagc aggtcaagaa gcagaactga tgcagaggcc acagaaggat 120
gtttctttat tagcttgctt cacctggctt gctcagactg ctgtcttaga gaacccaaga 180
ctaccagtgc agagagatgg tcccacccac aaggggcctt tccccttgat cactaattga 240
gaaaatgtct tacatctgga tctcatggag gcatttccca actgaagctc ctttctctgt 300
gataacttca gcttgtgtca agttgacaca aaattagcca gtataattga caccttgtca 360
atttgaggcg nnnncacatc actagtaatg ctcaactctt agtttcttat tcatgcccaa 420
gatatacaca actttgaaag ccccacagac tttacatatt aaaagttcaa tgcttttaaa 480

atatccaata tncgtg 496

<210> 27
<211> 443
<212> DNA
<213> Mus musculus

<400> 27
ttcacatagc atcaagtgac tacagataca acactagaat tttgatgtgt attatttgaa 60
cacgtttata tatgagaaaa atttgaaatt taagttacaa agtgagtatt ttttagcaca 120
tttagtagtg actatttata taaactatca ttttgtttgg gtgaccaaat gaaatttgaa 180
cataattaaa gaacatataa ctttcaaaca acattaatac agttacagac ctgttacact 240
gagaattatg tacacgtatt aaagaggact gaggtttctt aaacccattc ttttgaaagg 300
tttttaatcc ttcttaatgt aattcagtgt aaaacttcaa aaagtctact cggtgtcaca 360
gcaggatgag gtggaataat ccctgctgat cactgagcaa actgtgtttg tcatctgatt 420
ctcctgacaa ccactatact tga 443

<210> 28
<211> 398
<212> DNA
<213> Mus musculus

<400> 28
gttcatctca aagcccttta cactttggag ccaggcctgt tcaattttac aaataacacg 60
ttttgcttgt agtcagggtg ctgtgacaac atgaagagtt cgagctacct cccaggatga 120
gtgcaggttt tcctggtgac aaatctcagg cattcaaagg ttctgttagc agtgccatac 180
ttgtgggttt acagagatgg tgtgtctggc tgagaacaaa ggagccgtac cagggaagtt 240
gaagcctatc ttcattttca agtgcaggct aaaataaggt ctgaggaaaa gaatgagcag 300
cgtgtgaagg ataggaacag aaatcatgga agatctgttt gtccaggcca gtgtttcacc 360
ataagagatc actttaaaat aaatgagatg aaataatt 398

<210> 29
<211> 430
<212> DNA
<213> Mus musculus

<400> 29
tttttttttt tttttttcac tttgcaaact gttgttttta tttggattaa aaagctttga 60
tgaacaaaca tatgatacat acatggtata gaaccagtta ccacttaaaa cctcagatga 120
tatagtacag aaattagaat aaaccaaatt ttaatcaggt ctggaaacaa acgttcaagc 180
tgaaagctgt aacatccatt tggagatttt acacacctgt cccttaccca ctcagttctt 240
ccttagaagg ggaaggcaga cttagtaaaa ccaatgtttt ttaagtcagc tgactgacat 300
tctattttaa acctataggt ccccaaccca atataaacaa aattcttcac aggaacttac 360
attttgaaaa caattaggcc attatatttc ctaagccaca ttactacagt tcttaaaagt 420
catgagaaag 430

<210> 30
<211> 520
<212> DNA
<213> Mus musculus

<400> 30
tttttttttt tttttttatt catgcttgcc tagggatggg gaatagatca ttcaataaaa 60
acatacagta aaaacagggg gtggggaggg ggaaggctta tcatgtacaa tgtttaaact 120
acaatagtga tgtaccttaa ttacttccat gcacacaagt ctaacattac aagtttttaa 180
aaaataaaca ccattaagac ttctaggagc attttataat aaattcctaa ttttttcttt 240
gtagatagat caagcacctc caaaatacag attcctatac acagtgagca ctttacttaa 300
cgtacatgga cagcctcagg acgagctgac gtctcggatc agctcggcag gcaacaaacc 360
atagtgccaa atggaaagaa gggcagttgc aaataaactt aaaactaagt taacttttat 420
aattaaatac agaaaatata ctgatttgct aaaaataaat aagatgtgat gtatttaaca 480
cttcactata aagaatgaac accatgacat cctcgtgccg 520

<210> 31
<211> 390
<212> DNA

<213> Mus musculus

<400> 31
ttcgtcgaca tgcaatagca gggggaaagg ggacaggtaa caggagtacc tttgttttaa 60
cagacaaaag taatctccag gccttaggga ctcataaaga tatgagtaca ttgcttattg 120
gaaacttgta ggttcacata ttattttgac taaggtatat ggatttcccc ccacttacat 180
aagtaaaccc aacataagga ggaccattcc ttttctttca ttcagatgac ctttttctga 240
atctgggcac tgaagggatg cataaaataa tgttaatgtt ttcagtaatg tcttcaagta 300
gaagactttc tgttgaacta cttataagaa agtaaaataa atattgtctt tttgtatgtc 360
aaaaaaaaaa aaaaaaaaaa aaaaaaaagg 390

<210> 32
<211> 238
<212> DNA
<213> Mus musculus

<400> 32
aattcgtcga catggcacag cttcctccct aggcgtgaga ctccggctcc ttcactatga 60
gacttctagc cctttccggt ctgctctgca tgctgctcct ctgtttctgc attttctcct 120
cagaagggag aagacatcct gccaagtcct tgaaactcag gcgctgctgt cacctatctc 180
ctagatccaa gctgacaacc tggaaaggaa accacacaag gccctgcaga ctctgcag 238

<210> 33
<211> 383
<212> DNA
<213> Mus musculus

<400> 33
tttttttttt tttttttgat taatcatgta tttattttag ttcagttaaa acaaacatac 60
attgtttcat tgaaaccggt gtagcattta ctctctgcca acaggccaca gaggcatgtt 120
ggcctctaac agtacagctg ggacatttac actatataca tacaaggagt cctctttcat 180
tgagatcaca cttgacatgt taccaaatag ccatgtggta gctactgcat tttgaaaatt 240
tagacctcat ttaaagcttt agattatctg aacttaagtt gatgtccaac cagtttattg 300
caaatacaat ttaaacaagt tattttcaat tttagtatta tacacaatgt caatttctta 360
agattgaatt ctctgtacaa gta 383

<210> 34
<211> 249
<212> DNA
<213> Mus musculus

<400> 34
tttttttttt tttttttgac cacacagaca tggagttacc aattgtagaa atcgaaaaaa 60
ttggcatcaa ctctcgaagt cagagtgaac tcttgcctgc gggttggctt gactacgccc 120
aggcactgag ctgcctcaac cagctaggga gctatgatga tgctgagtcc tgttttcatg 180
atgtcaccag atgtatgtag taggtaattt gtctcacaaa ggcctttgta ccaatggctg 240
tgaagcttg 249

<210> 35
<211> 469
<212> DNA
<213> Mus musculus

<400> 35
tttttttttt tttttttaat tacgcaaaaa tttatttgac caaaatgtag aaaaagtgat 60
actattacgt atgatacaat agcaagaaca taaagaacag agtagatttt atcaattgca 120
gtttgctcaa agttcatctc cctggtagtg tgatgcttaa taaataggag tgaggggcag 180
ggttcagata agctagaagc agggtgactt tcagtcagga ctgtaaactt gagttggccc 240
cgcactgctg gggaatgtgg aatgtttcag tgtgagatgt taactgagaa aggaaaacta 300
caaagccaaa aaatgtgcag cctacagagc actccacatc cagttcaatc aggaggtctt 360
ggtcttttat tagtgtcatt cccgaaggaa ttcaagtctt ggcaaagtca agcttccatt 420
tgttgttccc tgaacaatgg agatgaagtt aagccaattt ctttttcta 469

<210> 36
<211> 355

<212> DNA
<213> Mus musculus

<400> 36
tttttttttc ctactagact aattaaaaaa aaaagtcaac attgatatcg attacttgca 60
tgaagacaca ggaaaacagg cgacatacac tccttaaaga cacacaagat aagaagatga 120
aacttcaggt acataagaag tctgtacaaa aagctagatt tgatactctc aaaaagtgaa 180
gggtcctaca acagcataga gaaataattt aatgccttcc agaacagaac tccagctctg 240
tggaggtttt cctattctat gggggcagat ctcatgccaa cccacagagc aggcgcttcc 300
acctcctatc cgtttatgcg gtagttttca tggatttccg gccggcctcg tgccg 355

<210> 37
<211> 217
<212> DNA
<213> Mus musculus

<400> 37
atgttgaaaa atcttattta ttttcttaat taaaaagaag tatatttgtg ttaaatttag 60
aatatttagc tttccattga tcttcccaat acttgtcaga tagatactaa tagtgctcag 120
aactgtgaaa gttaatatcc aataccatat tttctaagaa gaccctcaag gcattgggga 180
ttttaattta aaaataaaag aattttaatt agcattg 217

<210> 38
<211> 362
<212> DNA
<213> Mus musculus

<400> 38
tttttttttt tttttttgaa aaagaaattt tttgccttta ttagaatggc attaggcctt 60
aaatatgcca attttggtaa tcacattatt gttttaataa gaaacgactc tacagaattg 120
caatactggt ccaacagtct tgtccttctt ttaaagcaag aaacagaatg taagtaacca 180
gaaagcaggg caggcatcgg ctaacccagg agactagctt cttagatcca agcatttgca 240
gagagaaccg ttgggctggg gaggggtgga gcagctcgag ataactggaa cccagagtgt 300
gcgccaagtc ccatgaggct gcttgttgaa ctcatctttt ccttggtcac attgtttccc 360
tc 362

<210> 39
<211> 280
<212> DNA
<213> Mus musculus

<400> 39
tttttttttt tttttttcaa gagaaaatag gatttattta tcacatttga gatgaacaca 60
caggactgtc ccagaaatct tttacatata tattattgat agattggatt tattcagcat 120
acagaaaaag caaagcctgc cataagacac ttccctcttg agaacagcaa gatgtcaggc 180
agtgtctgta agttttcacc acgggaattc acattgagca aaaagaccgg gctcacaggt 240
ccgttcccag gtcctgctgc ttctatttca aaggctttca 280